# EUROPEAN PATENT SPECIFICATION

(11) **EP 2 935 311 B1**
(45) Date of publication and mention of the grant of the patent: **31.03.2021**
(21) Application number: 13821554.6
(22) Date of filing: 17.12.2013
(51) Int. Cl.: C07K 7/08, C07K 7/60

(54) **APJ RECEPTOR AGONISTS AND USES THEREOF**
APJ-REZEPTORAGONISTEN UND VERWENDUNGEN DAVON
AGONISTES DU RÉCEPTEUR APJ ET LEURS UTILISATIONS

(30) Priority: 20.12.2012 US 201261740409 P; 13.03.2013 US 201361779985 P
(43) Date of publication of application: 28.10.2015
(73) Proprietor: Amgen Inc., Thousand Oaks, California 91320-1799 (US)
(72) Inventor: HOLDER, Jerry Ryan, Simi Valley, California 93065 (US); SWAMINATH, Gayathri, Fremont, California 94539 (US); MIRANDA, Leslie P., Thousand Oaks, California 91362 (US); ARAL, Jennifer, Newbury Park, California 91320 (US); DOHERTY, Elizabeth M., Thousand Oaks, California 91360 (US); NIXEY, Thomas, Newbury Park, California 91320 (US)
(74) Representative: Lee, Nicholas John
(86) International application number: PCT/US2013/075773
(87) International publication number: WO 2014/099984

(56) References cited:
- WO-A1-2012/125408
- WO-A2-2007/048026
- WO-A2-2013/111110
- MURZA ALEXANDRE ET AL: "Elucidation of the Structure-Activity Relationships of Apelin: Influence of Unnatural Amino Acids on Binding, Signaling, and Plasma Stability", CHEMMEDCHEM, vol. 7, no. 2, February 2012 (2012-02), pages 318-325, XP002720442,
- MURZA A ET AL: "Elucidation of the Structure-Activity Relationships of Apelin: Influence of Unnatural Amino Acids on Binding, Signaling and Plasma Stability", JOURNAL OF PEPTIDE SCIENCE, vol. 18, no. Suppl. 1, September 2012 (2012-09), page S104, XP009176212, & 32ND EUROPEAN PEPTIDE SYMPOSIUM; ATHENS, GREECE; SEPTEMBER 02 -07, 2012

## Description

### CROSS-REFERENCE TO RELATED APPLICATIONS

This application is an international PCT Application which claims the benefit of United States Provisional Application Number 61/740,409, filed on December 20, 2012 and United States Provisional Application Number 61/779,985, filed on March 13, 2013.

### SEQUENCE LISTING

The instant application contains a sequence listing which is being submitted herewith in ASCII format via EFS-Web. The Sequence Listing, created on December 17, 2013, is provided as a file entitled A-1716-WO-PCT_Seq_List.txt and is 1,045,666 bytes in size.

### FIELD OF THE INVENTION

The application relates to agonists of the APJ receptor that have increased stability, circulating half life and/or potency relative to native apelin.

### BACKGROUND

Apelin is the endogenous ligand for APJ (APLNR, angiotensin receptor like-1). The APJ receptor is a member of the rhodopsin-like G protein-coupled receptor (GPCR) family. The apelin/APJ system has been observed in many tissues such as heart, kidney, pancreas, lung and the central nervous system. This suggests diverse roles of the system in the physiology and pathology of mammals.

Apelin peptides are processed from a 77 residue pre-pro form into smaller bioactive fragments, mainly a 36 residue form (Apelin 42-77- also referred to as Apelin-36) and a smaller 13 residue polypeptide (Apelin 65-77-also referred to as Apelin-13) Hosoya et al., J. Biol. Chem. 275:21061-21067, 2000. Apelin peptides were previously determined to be endogenous ligands for the orphan APJ receptor, a member of the seven transmembrane G-protein-coupled receptor superfamily. Tatemoto et al., Biochem. Biophysi. Res. Commun. 251:471-476, 1998. One of the shorter more active isoforms identified, pyroglutamated apelin-13 ([PE65]Apelin-13 (65-77), has been reported to be the most potent and abundant form of apelin in cardiac tissue. Maguire et al., Hypertension 54:598-604, 2009. *In vitro* and preclinical models have suggested that the apelin/APJ system has a role in cardiovascular homeostasis as well as metabolism. Barnes et al., Heart 96:1011-1016, 2010. Circulating apelin levels are transient and Apelin-13 has a brief plasma half-life of <5 minutes leading to short-lived cardiovascular effects.

*In vitro,* exogenous apelin increases contractility at subnanomolar concentrations in atrial strips and whole rat hearts, and increases sarcomere shortening by up to 140% in isolated cardiomyocyctes. Barnes et al., Heart 96:1011-1016, 2010. Apelin also has a potent inotropic effect in an *ex-vivo* isolated heart assay. *In vivo,* acute apelin infusion restores ejection fraction, increases cardiac output and reduces left ventricular end-diastolic pressure in rats with chronic heart failure. Berry et al., Circulation 110:187-193, 2004. Exogenous apelin potently enhances myocardial contractility without inducing left ventricular hypertrophy concomitant with reduction in ventricular preload and afterload. Barnes et al., Heart 96:1011-1016, 2010.

There have been very limited structure activity relationship (SAR) studies reported for apelin peptides. The limited SAR studies have focused on *in vitro* affinity and/or potency enhancement. There are no reports with SAR focused on increasing proteolytic stability and prolonging circulating half-lives while improving or maintaining potency of APJ agonists.
Studies from Kawamata et al and Hosoya et al have shown that that shorter peptide apelin-13 had approximately a 3.5-fold higher *in vitro* affinity to the APJ receptor than apelin-36. Kawamata et al., BBA 1538: 162-171, 2001, Hosoya et al., JBC 275: 21061-21067. Apelin-13 analogues were reported havinga single substitution with either canonical or non-canonical amino acids. The authors also reported double and triple substitutions in apelin 66-77 and apelin 63-77, but not in apelin-13. The emphasis was on peptides reported to have higher *in vitro* affinity and potency than aplein-13. Nishizawa et al., in: T. Shioiri (ed.), Peptide Science 2000: Proceedings of the 37th Japanese Peptide Symposium, pp. 151-154. Several if not all of these modified peptides are reported in later studies. US 7,635,751.

In a 2003 study (Medhurst et al., J. Neurochemistry 84:1162-1172, 2003) *in vitro* activity of apelin-36, apelin-17 and apelin-13 was compared. It was concluded that all three peptides were approximately equipotent. C-terminal amidation resulted in about a 14-fold decrease in affinity. A more recent study (Hamada et al., J. Mol. Med. 22:547-552, 2008) reported cyclic analogues of apelin-13. When tested for *in vitro* activity all three analogues maintained function activity, although with reduced potency relative to apelin-13.

A 12 amino acid-apelin peptide having ligand activity on APJ was reported in a 2009 patent (US 7,635,751). The peptide could have a substitution of one non-canonical amino acid.

Another study reported synthesizing analogues of apelin-13 with amino acid substitutions with non-canonical amino acids at the C-terminal end of the molecule emphasized, but no pegylation at the N- or C-terminus or another site specific location. The use of internal PEG spacers (short PEG (n=4 or 6), however, was also reported in lower activity peptide analogs with deletions in the middle of the sequence that contained fewer amino acid residues than apelin-13. Murza et al. ChemMedChem 7:318-325, 2012.
Studies have also reported attempts to modify the apelin receptor in order to affect physiological function. WO 2010/053545. The invention relates generally to compounds which are allosteric modulators the APJ receptor. The allosteric modulators are derived from the intracellular loops and domains of the the APJ receptor. WO 2012/125408 provides compositions and methods for treating a disease or disorder assocated with Apelin. WO 2007/048026 provides a composition of matter that involves a CGRP peptide antagonist.

This application provides novel apelin therapeutic agents and uses of those therapeutics.

### SUMMARY OF THE INVENTION

The invention provides an APJ peptide agonist having increased *in vitro* stability relative to wild type Apelin-13 (SEQ ID NO: 2), wherein the agonist is modified with a half-life prolonging group at the N-terminus or at a side chain of the peptide agonist, wherein the peptide agonist comprises the amino acid sequence selected from any one of:
SEQ ID NOs 29, 30, 31, 32, 33, 34, 35, 36, 37, 43 and 48,
wherein the peptide agonist binds to and activates the APJ receptor,
wherein the half-life prolonging group is PEG.

The increased stability of the APJ peptide agonist may be increased *in vitro* half-life.

The PEG group of the APJ peptide agonist may be thiopropionyl(20K-mPEG) or Atz(20K-mPEG).

The APJ agonist may be used in a method of improving cardiac contractility, in a method of improving systolic or diastolic function and/or in a method of treating heart failure in a patient in need thereof.

Therapeutic agents that have APJ agonist activity are provided. The agonists can have advantageous pharmaceutical characteristics (e.g., half-life or potency). Such compounds can comprise an APJ receptor binding domain or sequences derived thereof by rational design, peptide screening, yeast-based screening, phage display, RNA-peptide screening, or the other screening techniques. The domain can have very little or no antagonist activity. The compound can also comprise a vehicle, such as a polymer (e.g. polyethylene glycol), protein (e.g. an Ab or Fc domain), or another peptide sequence (e.g. a targeting domain) where the vehicle is covalently attached to the APJ agonist domain. The vehicle and the APJ agonist domain may be linked through the N- or C-terminus, or any other site of the polypeptide. The vehicle can be a polymer, e.g. polyethylene glycol (PEG).

APJ agonist domains can be generated by rational design, yeast secretion screening, rational design, protein structural analysis, phage display, RNA-peptide screening and other techniques known in the art.

Various embodiments are directed to an APJ agonist having increased stability relative to wild type Apelin-13 (SEQ ID NO: 2), wherein the agonist is modified with at least one non-canonical amino acid. The APJ agonist may also include a half-life prolonging group at the N-terminus, C-terminus, or other residue of the agonist. The APJ agonist can have a half-life prolonging group at the N-terminus, C-terminus, or other residue of the agonist consisting of PEG, an Fc domain, IgG, HSA, PE, an Ab, a peptide, a lipid. cholesterol, a nanostructure or other half-life prolonging moiety known in the art. The agonist can be pyroglutamated at the N-terminus and/or can be PEGylated or conjugated at the N-terminus or elsewhere in the agonist. In various embodiments the agonist can bind to the APJ receptor. In other embodiments the APJ agonist is greater than 7, 8, 9, 10, 11, 12 amino acids long, e.g. 13 or more amino acids long and the half-life prolonging group can be anywhere in the molecule. In yet other embodiments, the APJ agonist can have increased potency relative to wild-type apelin-13 .

In various embodiments the APJ agonist can have at least 2 amino acid residues replaced with a non-canonical amino acid. Alternatively, the at least 2 non-canonical amino acid residues can be 3, 4 or 5 amino acid residues.

In various embodiments the APJ agonist with increased stability has an increased *in vitro* half-life. The moiety that provides increased stability can be any group that accomplishes this objective, e.g. amino acid substitutions or chemical modifications. In other embodiments the APJ agonist with increased stability has an increased *in vivo* half-life. The increased *in vivo* half-life may be a result of increased proteolytic or metabolic stability and/or inclusion of a half-life prolonging moiety. The moiety that provides increased *in* vivo half-life can be any group that accomplishes this objective, e.g. the APJ agonist can be PEGylated with thiopropionyl(20K-mPEG), Atz(PEG10), NPeg11 or Atz(20K-mPEG) or alternatively conjugated to a protein.

### BRIEF DESCRIPTION OF THE DRAWINGS

Figure 1. Examples of non-canonical amino acids

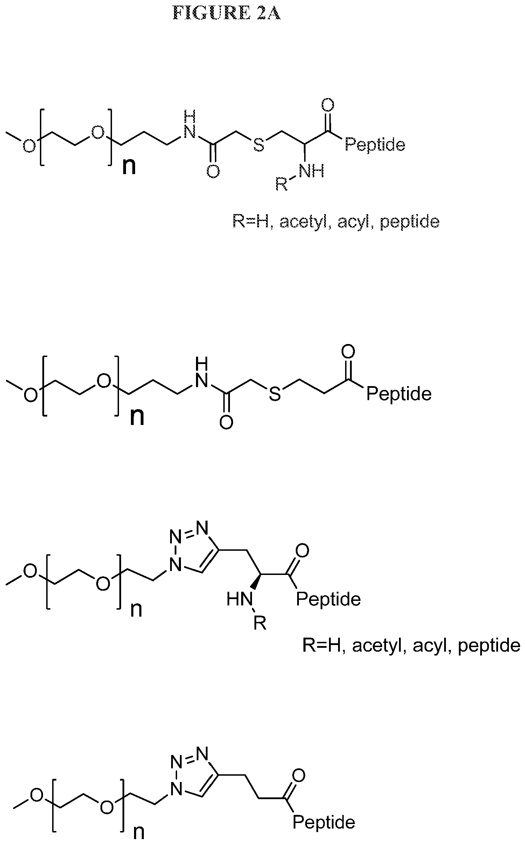

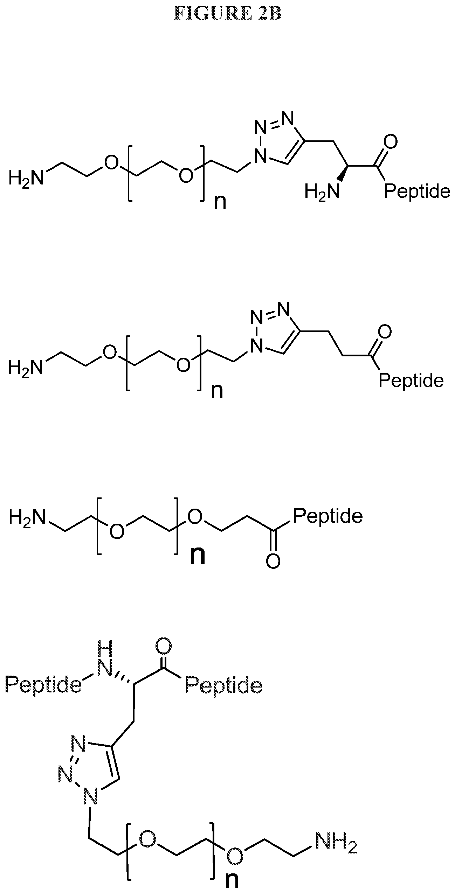

Figures 2A-B. Examples of PEG and linkers.
Figures 3A-C. Examples of APJ agonists.
Figures 4A-B. *In vitro* stability of APJ agonists in plasma.

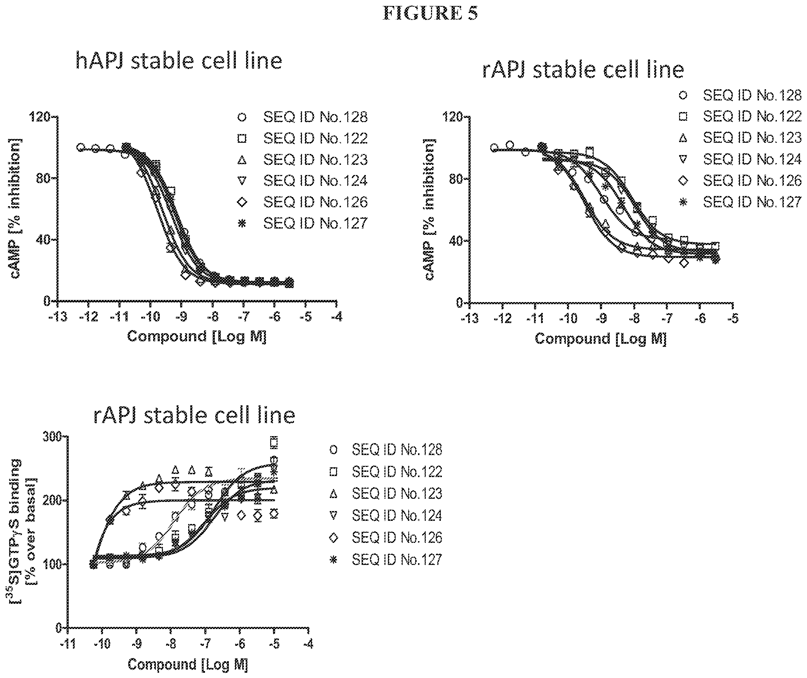

Figure 5. Agonist activity of 20 kDa PEGylated peptides. The peptides {Thiopropionyl(20K-mPEG)}LRP[hArg][Cha]SHKG[Oic][Nle]P[4-Cl-F]{FreeAcid} (SEQ ID NO: 122), {Thiopropionyl(20K-mPEG)}KFRRQRP[hArg][Cha]SHKG[Oic][Nle]P[4-Cl-F]{FreeAcid} (SEQ ID NO: 123), {Thiopropionyl(20K-mPEG)}LLRP[hArg][Cha] SHKG[Oic][Nle]P[4-Cl-F]{FreeAcid} (SEQ ID NO: 124), {Hydrogen}[Atz(20K-mPEG)]KFRRQRP[hArg][Cha]SHKG[Oic][Nle]P[4-Cl-F]{FreeAcid} (SEQ ID NO: 126) and {Hydrogen}[Atz(20K-mPEG)]LRP[hArg][Cha]SHKG[Oic][Nle]P[4-Cl-F]{FreeAcid} (SEQ ID NO: 127) are potent and act as a full agonist relative to [PE]RPRLSHKGPMPF (pyr apelin-13) (SEQ ID NO: 128).
Figure 6. Comparison of a 525 dalton PEGylated peptide (SEQ ID NO: 23) and pyr apelin 13 (SEQ ID NO: 128).
Figure 7. Demonstrates intrinsic load independent inotropic effect of an agonist (SEQ ID NO: 24) in an isolated Langendorff rat heart.
Figure 8. Demonstrates that an agonist (SEQ ID NO: 24) increases systolic and diastolic function in an isolated Langendorff heart.
Figure 9. Effects of a peptide (SEQ ID NO: 23) on cardiac contractility in normal and heart failure rats. MI = heart failure rats.
Figure 10. Effects of PEG size on *in vitro* activity.
Figure 11. Effects of peptide valency on *in vitro* activity.
Figure 12. Structures of Bis- and tetrakis- 20kDa PEG peptides.
Figure 13. Effects of peptide valency on *in vitro* activity.
Figure 14. Structures of Bis- 1.7 and 2.2 kDa PEG peptides.
Figure 15. Effects of Fc conjugates for apelin-13 and apelin-17 in rat and human GTPγ assays.
Figure 16. Effects of Fc conjugates in human GTPγ assay.

### DETAILED DESCRIPTION

The foregoing summary is not intended to define every aspect or embodiment of the invention, and additional aspects may be described in other sections. The entire document is intended to be related as a unified disclosure, and it should be understood that all combinations of features described herein may be contemplated, even if the combination of features is not found together in the same sentence, or paragraph, or section of this document.

In addition to the foregoing, as an additional aspect, all embodiments narrower in scope in any way than the variations defined by specific paragraphs herein can be included in this disclosure. For example, certain aspects are described as a genus, and it should be understood that every member of a genus can be, individually, an embodiment. Also, aspects described as a genus or selecting a member of a genus should be understood to embrace combinations of two or more members of the genus. It should also be understood that while various embodiments in the specification are presented using "comprising" language, under various circumstances, a related embodiment may also be described using "consisting of" or "consisting essentially of" language.

It will be understood that the descriptions herein are exemplary and explanatory only and are not restrictive of the invention as claimed. In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise. Also, the use of the term "portion" can include part of a moiety or the entire moiety.

Unless otherwise defined herein, scientific and technical terms used in connection with the application shall have the meanings that are commonly understood by those of ordinary skill in the art. Further, unless otherwise required by context, singular terms shall include pluralities and plural terms shall include the singular. Thus, as used in this specification and the appended claims, the singular forms "a", "an" and "the" include plural referents unless the context clearly indicates otherwise. For example, reference to "a protein" includes a plurality of proteins; reference to "a cell" includes populations of a plurality of cells.

It should also be understood that when describing a range of values, the characteristic being described could be an individual value found within the range. For example, "a pH from about pH 4 to about pH 6," could be, but is not limited to, pH 4, 4.2, 4.6, 5.1, 5.5, etc. and any value in between such values. Additionally, "a pH from about pH 4 to about pH 6," should not be construed to mean that the pH in question varies 2 pH units from pH 4 to pH 6, but rather a value may be picked from within a two pH range for the pH of the solution.

In some embodiments, when the term "about" is used, it means the recited number plus or minus 5%, 10%, 15% or more of that recited number. The actual variation intended is determinable from the context.

The section headings used herein are for organizational purposes only and are not to be construed as limiting the subject matter described. All documents, or portions of documents, cited in this application, including but not limited to patents, patent applications, articles, books, and treatises, are included. As utilized in accordance with the disclosure, the following terms, unless otherwise indicated, shall be understood to have the following meanings:

The term "acidic residue" or "acidic amino acid" should be understood to mean amino acid residues in D- or L-form having sidechains comprising acidic groups. Exemplary acidic residues can include D and E.

The term "amino acid" or "residue" should be understood to mean a compound containing an amino group (NH₂), a carboxylic acid group (COOH), and any of various side groups, that have the basic formula NH₂CHRCOOH, and that link together by peptide bonds to form proteins. Amino acids may, for example, be acidic, basic, aromatic, polar or derivatized. Non-standard amino acids may be referred to as "non-canonical" amino acids.

A one-letter abbreviation system is frequently applied to designate the identities of the twenty "canonical" amino acid residues generally incorporated into naturally occurring peptides and proteins (Table 1). Such one-letter abbreviations are entirely interchangeable in meaning with three-letter abbreviations, or non-abbreviated amino acid names. Within the one-letter abbreviation system used herein, an upper case letter indicates a L-amino acid, and a lower case letter indicates a D-amino acid. For example, the abbreviation "R" designates L-arginine and the abbreviation "r" designates D-arginine.

**TABLE 1. ONE-LETTER ABBREVIATIONS FOR THE CANONICAL AMINO ACIDS. (Three letter abbreviations are in parentheses.)**

| | |
|---|---|
| Alanine (Ala) | A |
| Glutamine (Gln) | Q |
| Leucine (Leu) | L |
| Serine (Ser) | S |
| Arginine (Arg) | R |
| Glutamic Acid (Glu) | E |
| Lysine (Lys) | K |
| Threonine (Thr) | T |
| Asparagine (Asn) | N |
| Glycine (Gly) | G |
| Methionine (Met) | M |
| Tryptophan (Trp) | W |
| Aspartic Acid (Asp) | D |
| Histidine (His) | H |
| Phenylalanine (Phe) | F |
| Tyrosine (Tyr) | Y |
| Cysteine (Cys) | C |
| Isoleucine (Ile) | I |
| Proline (Pro) | P |
| Valine (Val) | V |

An amino acid substitution in an amino acid sequence can be designated herein with a one-letter abbreviation for the amino acid residue in a particular position, followed by the numerical amino acid position relative to a native sequence of interest, which is then followed by the one-letter symbol for the amino acid residue substituted in. For example, "T30D" symbolizes a substitution of a threonine residue by an aspartate residue at amino acid position 30, relative to the native sequence of interest.

Amino acid residues are commonly categorized according to different chemical and/or physical characteristics. The term "acidic amino acid residue" refers to amino acid residues in D- or L-form having side chains comprising acidic groups. Exemplary acidic residues include aspartic acid and glutamic acid residues. The term "alkyl amino acid residue" refers to amino acid residues in D- or L-form having C₁₋₆alkyl side chains which may be linear, branched, or cyclized, including to the amino acid amine as in proline, wherein the C₁₋₆alkyl is substituted by 0, 1, 2 or 3 substituents selected from C₁₋₄haloalkyl, halo, cyano, nitro, -C(=O)R^{b}, -C(=O)OR^{a}, -C(=O)NR^{a}R^{a}, -C(=NR^{a})NR^{a}R^{a}, -NR^{a}C(=NR^{a})NR^{a}R^{a}, -OR^{a}, -OC (=O)R^{b}, -OC(=O)NR^{a}R^{a}, -OC₂₋₆alkylNR^{a}R^{a}, -OC₂₋₆alkylOR^{a}, -SR^{a}, -S(=O)R^{b}, -S(=O)₂R^{b}, -S(= O)₂NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})N R^{a}R^{a}, -N(R^{a})S(=O)₂R^{b}, -N(R^{a})S(=O)₂NR^{a}R^{a}, -NR^{a}C₂₋₆alkylNR^{a}R^{a} and -NR^{a}C₂₋₆alkylOR^{a}; wherein R^{a} is independently, at each instance, H or R^{b}; and R^{b} is independently, at each instance C₁₋₆alkyl substituted by 0, 1, 2 or 3 substituents selected from halo, C₁₋₄alk, C₁₋₃haloalk, -OC₁₋₄alk, -NH₂, -NHC₁₋₄alk, and -N(C₁₋₄alk)C₁₋₄alk; or any protonated form thereof, including alanine, valine, leucine, isoleucine, proline, serine, threonine, lysine, arginine, histidine, aspartate, glutamate, asparagine, glutamine, cysteine, methionine, hydroxyproline, cyclohexylalanine, norleucine, norvaline, 2-aminobutyric acid, but which residues do not contain an aryl or aromatic group. The term "aromatic amino acid residue" refers to amino acid residues in D- or L-form having side chains comprising aromatic groups. Exemplary aromatic residues include tryptophan, tyrosine, 3-(1-naphthyl)alanine, histidine, or phenylalanine residues. The term "basic amino acid residue" refers to amino acid residues in D- or L-form having side chains comprising basic groups. Exemplary basic amino acid residues include histidine, lysine, homolysine, ornithine, arginine, N-methyl-arginine, ω-aminoarginine, ω-methyl-arginine, 1-methyl-histidine, 3-methyl-histidine, and homoarginine (hR) residues. The term "hydrophilic amino acid residue" refers to amino acid residues in D- or L-form having side chains comprising polar groups. Exemplary hydrophilic residues include cysteine, serine, threonine, histidine, lysine, asparagine, aspartate, glutamate, glutamine, and citrulline (Cit) residues. The terms "lipophilic amino acid residue" refers to amino acid residues in D- or L-form having sidechains comprising uncharged, aliphatic or aromatic groups. Exemplary lipophilic sidechains include phenylalanine, isoleucine, leucine, methionine, valine, tryptophan, and tyrosine. Alanine (A) is amphiphilic-it is capable of acting as a hydrophilic, or lipophilic (i.e., hydrophobic), residue. Alanine, therefore, is included within the definition of both "lipophilic" (i.e., "hydrophobic") residue and "hydrophilic" residue. The term "nonfunctional" or "neutral" amino acid residue refers to amino acid residues in D- or L-form having side chains that lack acidic, basic, or aromatic groups. Exemplary neutral amino acid residues include methionine, glycine, alanine, valine, isoleucine, leucine, and norleucine

Polypeptides may have amino acid substitutions. Desired amino acid substitutions (whether conservative or non-conservative) can be determined by those skilled in the art. Amino acid substitutions can be used to identify important residues of the polypeptide sequence, or to increase or decrease the affinity of the peptide or vehicle-peptide molecules (see preceding formulae) described herein.

In certain embodiments, conservative amino acid substitutions can encompass non-naturally occurring amino acid residues which are typically incorporated by chemical peptide synthesis rather than by synthesis in biological systems.

Conservative modifications can produce half-life extending moiety-conjugated peptides having functional, physical, and chemical characteristics similar to those of the conjugated (e.g., PEG-conjugated) peptide from which such modifications are made. In contrast, substantial modifications in the functional and/or chemical characteristics of peptides may be accomplished by selecting substitutions in the amino acid sequence that differ significantly in their effect on maintaining (a) the structure of the molecular backbone in the region of the substitution, for example, as an α-helical conformation, (b) the charge or hydrophobicity of the molecule at the target site, or (c) the size of the molecule.

For example, a "conservative amino acid substitution" may involve a substitution of a native amino acid residue with a nonnative residue such that there is little or no effect on the polarity or charge of the amino acid residue at that position. Furthermore, any native residue in the polypeptide may also be substituted with alanine, as has been previously described for "alanine scanning mutagenesis" (see, for example, MacLennan et al., Acta Physiol. Scand. Suppl., 643:55-67 (1998); Sasaki et al., 1998, Adv. Biophys. 35:1-24 (1998), which discuss alanine scanning mutagenesis).

Desired amino acid substitutions (whether conservative or non-conservative) can be determined by those skilled in the art at the time such substitutions are desired. For example, amino acid substitutions can be used to identify important residues of the peptide sequence, or to increase or decrease the affinity of the peptide or vehicle-conjugated peptide molecules described herein.

Naturally occurring residues may be divided into classes based on common side chain properties:
1) hydrophobic: norleucine (Nor or Nle), Met, Ala, Val, Leu, Ile;
2) neutral hydrophilic: Cys, Ser, Thr, Asn, Gln;
3) acidic: Asp, Glu;
4) basic: His, Lys, Arg;
5) residues that influence chain orientation: Gly, Pro; and
6) aromatic: Trp, Tyr, Phe.

Conservative amino acid substitutions may involve exchange of a member of one of these classes with another member of the same class. Conservative amino acid substitutions may encompass non-naturally occurring amino acid residues, which are typically incorporated by chemical peptide synthesis rather than by synthesis in biological systems. These include peptidomimetics and other reversed or inverted forms of amino acid moieties. Non-conservative substitutions may involve the exchange of a member of one of these classes for a member from another class.

In making such changes, according to certain embodiments, the hydropathic index of amino acids may be considered. Each amino acid has been assigned a hydropathic index on the basis of its hydrophobicity and charge characteristics. They are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art (see, *for example,* Kyte et al., 1982, J. Mol. Biol. 157:105-131). It is known that certain amino acids may be substituted for other amino acids having a similar hydropathic index or score and still retain a similar biological activity. In making changes based upon the hydropathic index, in certain embodiments, the substitution of amino acids whose hydropathic indices are within ±2 is included. In certain embodiments, those that are within ±1 are included, and in certain embodiments, those within ±0.5 are included

Substitution of like amino acids can be made effectively on the basis of hydrophilicity. In certain embodiments, the greatest local average hydrophilicity of a protein, as governed by the hydrophilicity of its adjacent amino acids, correlates with its immunogenicity and antigenicity, *i.e.,* with a biological property of the protein.

The following hydrophilicity values have been assigned to these amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). In making changes based upon similar hydrophilicity values, in certain embodiments, the substitution of amino acids whose hydrophilicity values are within ±2 is included, in certain embodiments, those that are within ±1 are included, and in certain embodiments, those within ±0.5 are included.

Examples of conservative substitutions include the substitution of one non-polar (hydrophobic) amino acid residue such as isoleucine, valine, leucine norleucine, alanine, or methionine for another, the substitution of one polar (hydrophilic) amino acid residue for another such as between arginine and lysine, between glutamine and asparagine, between glycine and serine, the substitution of one basic amino acid residue such as lysine, arginine or histidine for another, or the substitution of one acidic residue, such as aspartic acid or glutamic acid for another. The phrase "conservative amino acid substitution" also includes the use of a chemically derivatized residue in place of a non-derivatized residue, provided that such polypeptide displays the requisite bioactivity. Other exemplary amino acid substitutions that can be useful are set forth in Table 2 below.

**TABLE 2. Some Useful Amino Acid Substitutions.**

| ORIGINAL RESIDUES | EXEMPLARY SUBSTITUTIONS |
|---|---|
| Ala | Val, Leu, Ile, Gly |
| Arg | Lys, Gln, Asn, His |
| Asn | Gln |
| Asp | Glu |
| Cys | Ser, Ala |
| Gln | Asn |
| Glu | Asp |
| Gly | Pro, Ala |
| His | Asn, Gln, Lys, Arg |
| Ile | Leu, Val, Met, Ala, Phe, Norleucine |
| Leu | Norleucine, Ile, Val, Met, Ala, Phe |
| Lys | Arg, 1,4-Diamino-butyric Acid, Gln, Asn, His |
| Met | Leu, Phe, Ile |
| Phe | Leu, Val, Ile, Ala, Tyr |
| Pro | Ala |
| Ser | Thr, Ala, Cys |
| Thr | Ser |
| Trp | Tyr, Phe |
| Tyr | Trp, Phe, Thr, Ser |
| Val | Ile, Met, Leu, Phe, Ala, Norleucine |

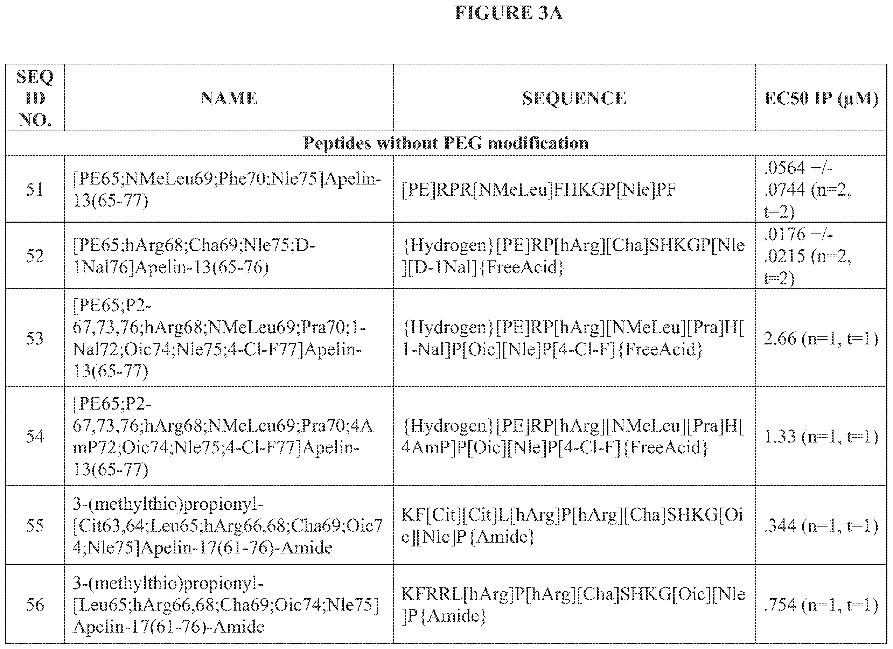

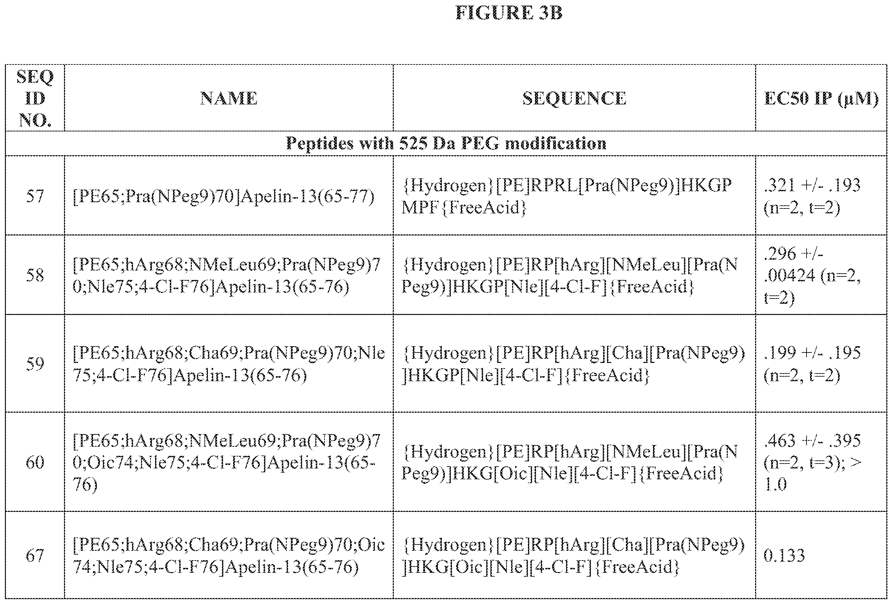

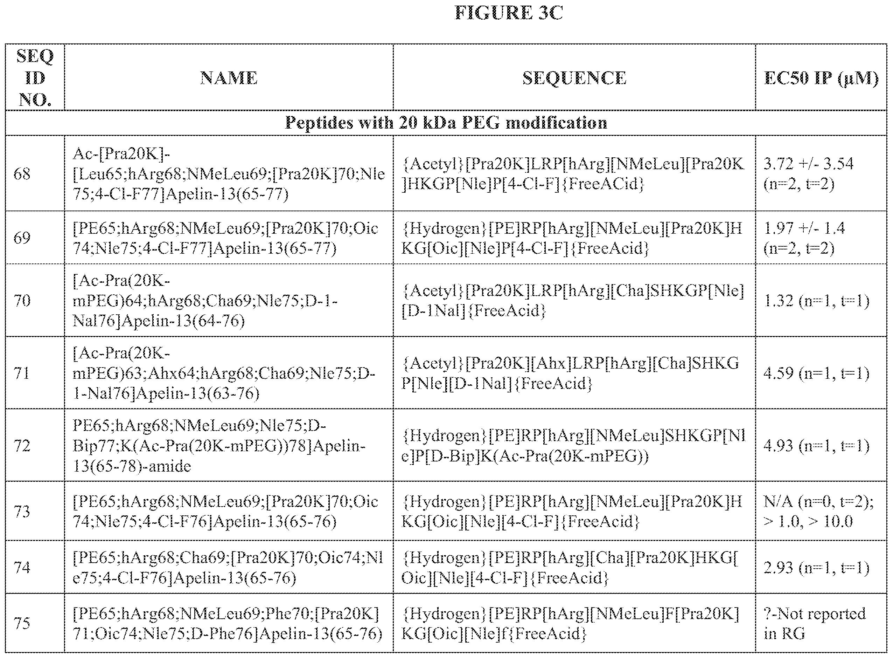

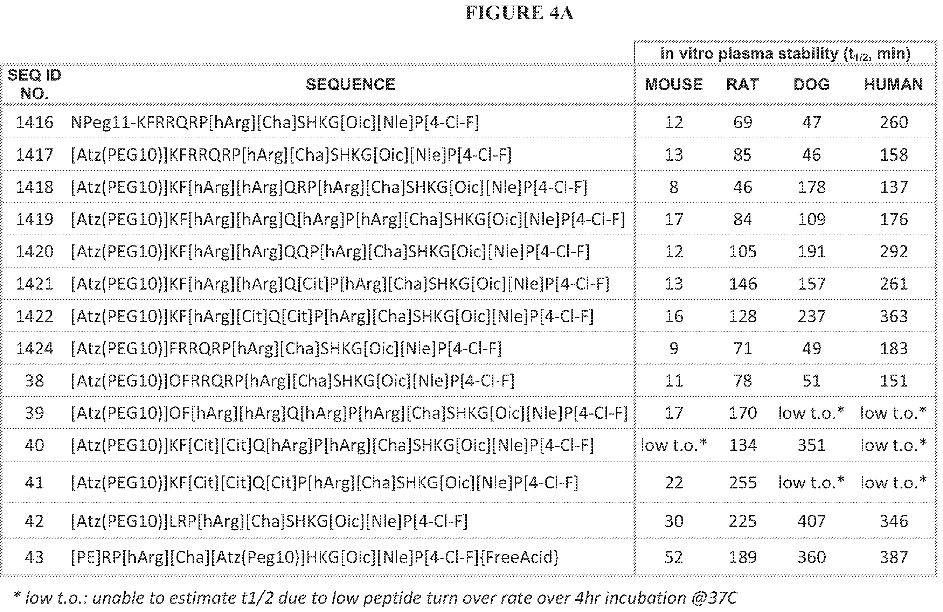

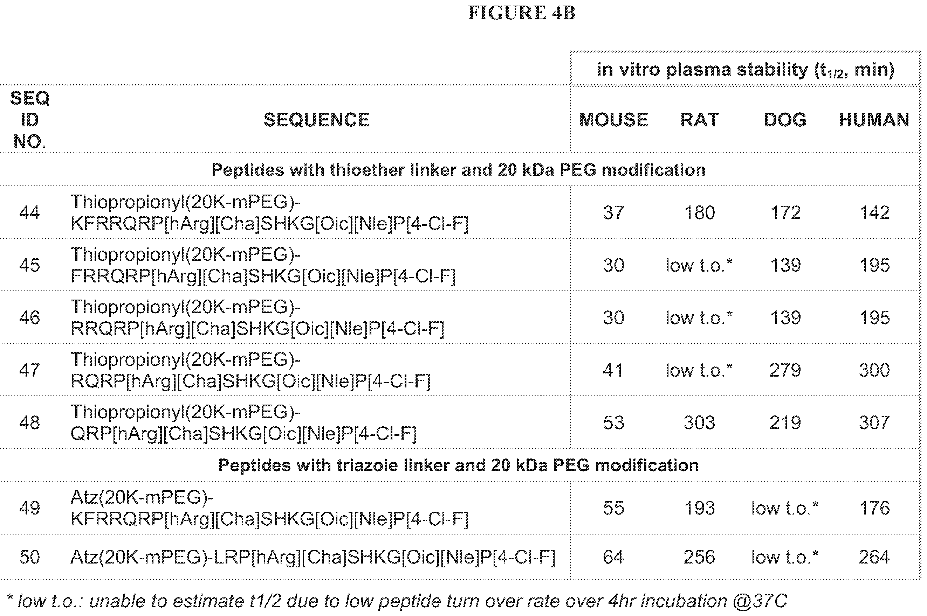

The term "agonist" should be understood to refer to something that can positively affect a process, e.g. activate or stimulate the process, the process can include, but is not limited to chemical, biochemical, cellular or physiological processes. An "APJ agonist" should be understood to refer to a molecule that is able to activate the APJ receptor. The APJ agonist may bind to the APJ receptor. Examples of agonists are provided in several Tables below and in several figures e.g. Figures 3 and 4. It can refer to a polypeptide having biological activity at least comparable to a naturally occurring apelin. The term further includes peptides that potentiate the effects of the naturally occurring molecule.

The APJ agonist sequence may have the following formula:
Nx¹x²x³x⁴x⁵x⁶x⁷x⁸x⁹x¹⁰x¹¹x¹²x¹³x¹⁴x¹⁵x¹⁶x¹⁷ (SEQ ID NO: 61)
wherein:
N is an extension or conjugation linker
x¹ is absent, is an amino acid residue (e.g. basic or polar), or is a conjugation linker;
x² is absent, is an amino acid residue (e.g. nonfunctional or hydrophobic), or is a conjugation linker;
x³ is absent, is an amino acid residue (e.g. basic or polar), or is a conjugation linker;
x⁴ is absent, is an amino acid residue (e.g. basic or polar),, or is a conjugation linker;
x⁵ is absent or is a nonfunctional, hydrophobic, or polar residue (e.g. pE, Q, Cit, L, V, G, H or P);, or a conjugation linker;
x⁶ is absent or is a polar or basic residue (e.g. K or Cit, R, NMeArg, or hArg);
x⁷ is a nonfunctional or hydrophobic residue (e.g. P, Oic, or G);
x⁸ is a basic or polar residue (e.g. Q or Cit, R, NMeArg, or hArg);
x⁹ is a nonfunctional or hydrophobic residue (e.g. V, I, or L preferred, NMeLeu or Cha);
x¹⁰ is a nonfunctional, polar, or hydrophobic residue, or a conjugation linker (e.g. S, F, and 4F-Phe);
x¹¹ is a nonfunctional, polar, basic, or hydrophobic residue, or a conjugation linker;
x¹² is a nonfunctional, hydrophobic, polar, or basic residue;
x¹³ is a nonfunctional or aromatic residue (e.g. G);
x¹⁴ is a nonfunctional or hydrophilic residue (e.g. P and Oic);
x¹⁵ is a nonfunctional, polar, or hydrophobic residue (e.g. aliphatic, aromatic, hydrophobic residues);
x¹⁶ is a nonfunctional or a hydrophobic residue (e.g. F, 4I-Phe, 4C1-Phe, Bip , P, Oic);
x¹⁷ is absent or is a hydrophobic residue (e.g. aromatic residues

As mentioned above, an agonist can affect a physiological process. Thus, a modified peptide can have agonistic activity on the heart by increasing, for example, various aspects of contractility, such as dp/dt or heart rate.

The term "-antagonist peptide," "peptide antagonist," and "inhibitor peptide" should be understood to mean a peptide or polypeptide that blocks or in some way interferes with the biological activity of a receptor of interest, or has biological activity comparable to a known antagonist or inhibitor of a receptor of interest. For an apelin antagonistic peptide, the receptor of interest is the APJ receptor.

The term "aromatic residue" or "aromatic amino acid" should be understood to mean amino acid residues in D- or L-form having side chains comprising aromatic groups. Exemplary aromatic residues can include F, Y, and W.

The term "basic residue" refers to amino acid residues in D- or L-form having sidechains comprising basic groups. Exemplary basic residues can include H, K, and R.

The terms "derivatizing" and "derivative" or "derivatized" should be understood to mean processes and resulting compounds respectively in which a portion of the parent molecule or vehicle has been modified chemically. Examples can include: (1) a compound having a cyclic portion; for example, cross-linking between residues within the compound; (2) a compound cross-linked or having a cross-linking site; for example, the compound has a cysteinyl residue and thus forms cross-linked dimers; (3) one or more peptidyl linkages are replaced by a non-peptidyl linkage; (4) an N-terminus is modified with agents capable of reacting with the amino group; (5) the C-terminus can be replaced with an amide or ester; and (6) compounds in which individual amino acid moieties are modified through treatment with agents capable of reacting with selected side chains or terminal residues.

In various embodiments, the polypeptide and/or vehicle portion of the compounds may be derivatized. Such derivatives may improve the solubility, absorption, stability, biological half life, and the like of the compounds. The moieties may alternatively eliminate or attenuate any undesirable effects of the compounds.

The term "fusion protein" should be understood to mean that the protein includes polypeptide components derived from more than one parental protein or polypeptide. Typically, a fusion protein is expressed from a fusion gene in which a nucleotide sequence encoding a polypeptide sequence from one protein is appended in frame with, and optionally separated by a linker from, a nucleotide sequence encoding a polypeptide sequence from a different protein. The fusion gene can then be expressed by a recombinant host cell as a single protein.

The term "endogenous apelin peptides" should be understood to mean full-length Apelin preproprotein having sequence MNLRLCVQAL LLLWLSLTAV CGGSLMPLPD GNGLEDGNVR HLVQPRGSRN GPGPWQGGRR KFRRQRPRLS HKGPMPF (SEQ ID NO: 138). Apelin (42-77) also referred to as Apelin-36 having sequence LVQPRGSRNGPGPWQG GRRKFRRQRPRLSHKGPMPF (SEQ ID NO: 1), Apelin (65-77) also referred to as Apelin-13 having sequence QRPRLSHKGPMPF (SEQ ID NO: 2) or fragments of full-length Apelin. The clipped apelin peptides can also be referred to as isoforms of the molecule.

The term "isolated polypeptide" should be understood to mean a polypeptide molecule that is purified or separated from at least one contaminant polypeptide molecule with which it is ordinarily associated in the natural source of the polypeptide. An isolated polypeptide molecule is other than in the form or setting in which it is found in nature.

The term "APJ ligand" should be understood to mean a molecule that binds to APJ or forms a complex with APJ. The ligand may be, but is not necessarily a signal triggering molecule. In some instances the term "ligand" may be used interchangeably with agonist

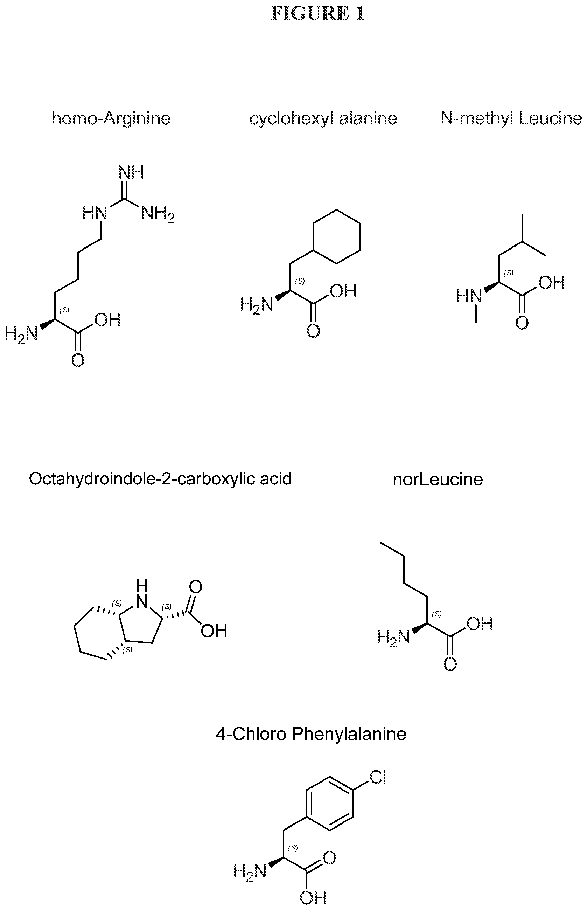

The term "non-canonical" or "unnatural" amino acids should be understood to mean amino acid residues in D- or L-form that are not among the 20 canonical amino acids generally incorporated into naturally occurring proteins. Examples of non-canonical amino acids can be found in Figure 1 and Table 3. Non-canonical amino acid residues can be incorporated into a peptide by employing known techniques of protein engineering that use recombinantly expressing cells. (See, e.g., Link et al., Non-canonical amino acids in protein engineering, Current Opinion in Biotechnology, 14(6):603-609 (2003). Additional examples of non-canonical amino acids include, for example, β-amino acids, homoamino acids, cyclic amino acids and amino acids with derivatized side chains. Additional examples can include (in the L-form or D-form) β-alanine, β-aminopropionic acid, piperidinic acid, aminocaprioic acid, aminoheptanoic acid, aminopimelic acid, desmosine, diaminopimelic acid, N*^{α}*-ethylglycine, N*^{α}*-ethylaspargine, hydroxylysine, allo-hydroxylysine, isodesmosine, allo-isoleucine, ω-methylarginine, N*^{α}*-methylglycine, N*^{α}*-methylisoleucine, N*^{α}*-methylvaline, γ-carboxyglutamate, ε-N,N,N-trimethyllysine, ε-N-acetyllysine, O-phosphoserine, N*^{α}*-acetylserine, N*^{α}*-formylmethionine, 3-methylhistidine, 5-hydroxylysine, and other similar amino acids, and those listed in Table 3 below, and derivatized forms of any of these as described herein. Table 3 and Figure 1 contain some exemplary non-canonical amino acid residues that may be useful and associated abbreviations as typically used herein, although the skilled practitioner will understand that different abbreviations and nomenclatures may be applicable to the same substance and appear interchangeably herein. Some amino acid sequences, as recited herein may include "{H}-" at the N-terminal, which represents an N-terminus amino group, and/or may include "-{Free Acid}" at the C-terminal, which represents a C-terminus carboxy group.

In the event an abbreviation listed in Table 3 differs from another abbreviation for the same substance disclosed elsewhere herein, both abbreviations are understood to be applicable. The amino acids listed in Table 3 can be in the L-form or D-form, unless otherwise noted.

**TABLE 3. Examples of non-canonical amino acids for substitution into peptide sequences.**

| AMINO ACID | ABBREVIATION(S) |
|---|---|
| Acetamidomethyl | Acm |
| Acetylarginine | acetylarg |
| α-aminoadipic acid | Aad |
| aminobutyric acid | Abu |
| 2-aminobutyric acid | 2-Abu |
| 6-aminohexanoic acid | Ahx; εAhx |
| 3-amino-6-hydroxy-2-piperidone | Ahp |
| 2-aminoindane-2-carboxylic acid | Aic |
| α-amino-isobutyric acid | Aib |
| 3-amino-2-naphthoic acid | Anc |
| 2-aminotetraline-2-carboxylic acid | Atc |
| Aminophenylalanine | Aminophe; Amino-Phe |
| 4-amino-phenylalanine (also known as paraaminophenylalanine) | 4AmP; 4-AminoF; 4-Amino-Phe |
| 4-amidino-phenylalanine | 4AmPhe |
| 2-amino-2-(1-carbamimidoylpiperidin-4-yl)acetic acid | 4AmPig |
| ω-N -methylarginine | R(Me) |
| Arg ψ(CH₂NH) -reduced amide bond | rArg |
| 3-(1,2,3-triazol-4-yl)Alanine | Atz |
| (S)-2-amino-3-(1-(1-bromo-2-oxo-6,9,12-trioxa-3-azatetradecan-14-yl)-1 H-1, 2,3-triazol-4-yl)propanoic acid | Atz(PEG3 -bromoacetamide) |
| 3-(1-(O-(aminoethyl)-O'-(ethylene)-decaethyleneglycol)-1,2,3-triazol-4-yl)Alanine | Atz(amino-PEG10) |
| 3-(1-(O-(aminoethyl)-O'-(ethylene)-ethyleneglycol450avg)-1,2,3-triazol-4-yl)Alanine | Atz(20kDa PEG) |
| (S)-2-amino-3-(1-(2-oxo-6,9,12,15,18,21,24,27,30,33,36-undecaoxa-3-azaoctatriacontan-38-yl)-1H-1,2,3-triazol-4-yl)propanoic acid | Atz(PEG11-(acetamidomethyl) |
| (S)-2-amino-3-(1-(1-hydroxy-5-oxo-9,12,15,18,21,24,27,30,33,36,39-undecaoxa-3-thia-6-azahentetracontan-4l-yl)-1H-1,2,3-triazol-4-yl)propanoic acid | Atz(PEG11-((2-hydroxyethyl)thio)acetamide) |
| (S)-2-amino-3-(1-(1-bromo-2-oxo-6,9,12,15,18,21,24,27,30,33,36-undecaoxa-3-azaoctatriacontan-38-yl)-1H-1,2,3-triazol-4-yl)propanoic acid | Atz(PEG11-bromoacetamide) |
| (S)-2-amino-6-(3-(1-(1-bromo-2-oxo-6,9,12,15,18,21,24,27,30,33,36-undecaoxa-3-azaoctatriacontan-38-yl)-1H-1,2,3-triazol-4-yl)propanamido)hexanoic acid | K(ethyl-triazole-PEG11-bromoacetamide) |
| β-alanine | bA |
| β-homoarginine | bhArg |
| β-homolysine | bhomoK |
| β-homo Tic | BhTic |
| β-homophenylalanine | BhPhe |
| β-homoproline | BhPro |
| β-homotryptophan | BhTrp |
| 4,4'-biphenylalanine; 4-phenyl-phenylalanine ; or biphenylalanine | Bip; 4Bip |
| β, β-diphenyl-alanine | BiPhA |
| β-phenylalanine | BPhe |
| *p*-carboxyl-phenylalanine | Cpa |
| Citrulline | Cit |
| Cyclohexylalanine | Cha |
| Cyclohexylglycine | Chg |
| Cyclopentylglycine | Cpg |
| 4-tert-butyl- L-phenylalanine | 4tBu-F |
| 4-benzoyl-L-phenylalanine | 4-Bz-F |
| 2-chloro-L-phenylalanine | 2-Cl-F |
| 4-trifluoromethyl-L-phenylalanine | 4CF3-F |
| 4-fluoro-L-phenylalanine | 4-F-F |
| 4-methyl-L-phenylalanine | 4-Me-F |
| 2-amino-3-guanidinopropanoic acid | 3G-Dpr |
| α, γ-diaminobutyric acid | Dab |
| 2,4-diaminobutyric acid | Dbu |
| diaminopropionic acid | Dap |
| 3,4-dichloro-L-phenylalanine | DiCl-F |
| 3,4-dimethoxy-L-phenylalanine | DiMeO-F |
| α, β-diaminopropionoic acid (or 2,3-diaminopropionic acid | Dpr |
| 3,3-diphenylalanine | Dip |
| 4-guanidino phenylalanine | Guf |
| 4-guanidino proline | 4GuaPr |
| Homoarginine | hArg; hR |
| Homocitrulline | hCit |
| Homoglutamine | hQ |
| Homoleucine | hLeu; hL |
| Homolysine | hLys; hK; homoLys |
| Homophenylalanine | hPhe; homoPhe |
| 4-hydroxyproline (or hydroxyproline) | Hyp |
| 2-indanylglycine (or indanylglycine) | IgI |
| indoline-2-carboxylic acid | Idc |
| Iodotyrosine | I-Tyr |
| Lys ψ(CH₂NH)-reduced amide bond | rLys |
| (S)-6-((S)-2-acetamidopent-4-ynamido)-2-aminohexanoic acid | K(Ac-Pra) |
| N-ε-biotinyl-L-lysine | K(Biotin) |
| (S)-2,2',2"-(10-(2-((5-amino-5-carboxypentyl)amino)-2-oxoethyl)-1,4,7,10-tetraazacyclododecane-1,4,7-triyl)triacetic acid | K(DOTA) |
| (S)-2-amino-6-(pent-4-ynamido)hexanoic acid | K(4-Pen) |
| methionine oxide | Met[O] |
| methionine sulfone | Met[O]₂ |
| *N^{α}*-methylarginine | NMeR |
| Nα-[(CH₂)₃NHCH(NH)NH₂] substituted glycine | N-Arg |
| *N^{α}*-methylcitrulline | NMeCit |
| *N^{α}*-methylglutamine | NMeQ |
| *N^{α}*-methylhomocitrulline | N *^{α}*-MeHoCit |
| *N^{α}*-methylhomolysine | NMeHoK |
| *N^{α}*-methylleucine | N*^{α}*-MeL; NMeL; NMeLeu; NMe-Leu |
| *N^{α}*-methyllysine | NMe-Lys |
| *Nε*-methyl-lysine | N-eMe-K |
| *Nε*-ethyl-lysine | N-eEt-K |
| *Nε*-isopropyl-lysine | N-eIPr-K |
| *N^{α}*-methylnorleucine | NMeNle; NMe-Nle |
| *N^{α}*-methylornithine | N *^{α}*-MeOrn; NMeOrn |
| *N^{α}*-methylphenylalanine | NMe-Phe |
| 1'N -methyltryptophan | 1'NMeW |
| 4-methyl-phenylalanine | MePhe |
| α-methylphenyalanine | AMeF |
| *N^{α}*-methylthreonine | NMe-Thr; NMeThr |
| *N^{α}*-methylvaline | NMeVal; NMe-Val |
| *Nε*-(O-(aminoethyl)-O'-(2-propanoyl)-undecaethyleneglycol)-Lysine | K(NPeg 11) |
| *Nε*-(O-(aminoethyl)-O'-(2-propanoyl)-(ethyleneglycol)27-Lysine | K(NPeg27) |
| 3-(1-naphthyl)alanine | 1-Nal; INal |
| 3-(2-naphthyl)alanine | 2-Nal; 2Nal |
| nipecotic acid | Nip |
| Nitrophenylalanine | nitrophe |
| norleucine | Nle |
| norvaline | Nva or Nvl |
| O-methyltyrosine | Ome-Tyr |
| (S)-octylglycine | OctylG |
| octahydroindole-2-carboxylic acid | Oic |
| Ornithine | Orn |
| Orn ψ(CH2NH)-reduced amide bond | rOrn |
| pyroglutamic acid | pGlu; PE; pE |
| L-phosphoserine | pS |
| 4-piperidinylalanine | 4PipA |
| 4-pyridinylalanine | 4Pal |
| 3-pyridinylalanine | 3Pal |
| 2-pyridinylalanine | 2Pal |
| para-iodophenylalanine (or 4-iodophenylalanine) | pI-Phe |
| Phenylglycine | Phg |
| Propargylglycine | Pra |
| pipecolic acid | Pip |
| 4-amino-1-piperidine-4-carboxylic acid | 4Pip |
| Sarcosine | Sar |
| 1,2,3,4-tetrahydroisoquinoline | Tic |
| 1,2,3,4-tetrahydroisoquinoline-1-carboxylic acid | Tiq |
| 1,2,3,4-tetrahydroisoquinoline-7-hydroxy-3-carboxylic acid | Hydroxyl-Tic |
| 1,2,3,4-tetrahydronorharman-3-carboxylic acid | Tpi |
| thiazolidine-4-carboxylic acid | Thz |
| 3-thienylalanine | Thi |
| (S)-tert-butylglycine | Tle |
| symmetrical N'-ω-dimethyl arginine | SDMA |
| N-ε-dimethyl lysine | K(Me2) |
| 4-carboxyphenylalanine | 4CO2-F |

The term "nonfunctional residue" or "nonfunctional amino acid" should be understood to mean amino acid residues in D- or L-form having sidechains that lack acidic, basic, or aromatic groups. Exemplary nonfunctional amino acid residues can include M, G, A, V, I, L and norleucine (Nle).

The term "polymeric nanostructure" may be understood to mean nanoparticle e.g. microsphere formulation containing the 50:50 poly(D,L-lactide-co-glycolide) polymer from Amylin (Diabetes Technol. Ther. 13:1145-1154, 2011; Endocrine J. 56:951-962, 2009 or Medincel (WO2012090070A2).

The term "physiologically acceptable salts" should be understood to mean any salts that are known or later discovered to be pharmaceutically acceptable to a formulation of the APJ agonists. Some specific examples are: acetate; trifluoroacetate; hydrohalides, such as hydrochloride and hydrobromide; sulfate; citrate; tartrate; glycolate; and oxalate. In various embodiments, physiologically acceptable salts of the polypeptides and compositions of matter are contemplated.

The term "serum protein binding moiety" should be understood to refer to binding to endogenous (native) proteins commonly found in the blood in high abundance. Human serum albumin is an example of such a protein. Binding to such molecules can allow a drug to adopt serum binding protein pharmacokinetics which can be an advantageous property.

The term "membrane protein binding moiety" should be understood to refer to binding to a protein embedded or spanning the cellular lipid bilayer.

The term "polar residue" or "polar amino acid" should be understood to refer to amino acid residues in D- or L-form having side chains comprising polar groups. Exemplary polar residues can include C, S, T, N, and Q.

The term "polynucleotide" or "nucleic acid" should be understood to include both single-stranded and double-stranded nucleotide polymers containing two or more nucleotide residues. The nucleotide residues comprising the polynucleotide can be ribonucleotides or deoxyribonucleotides or a modified form of either type of nucleotide. Said modifications include base modifications such as bromouridine and inosine derivatives, ribose modifications such as 2',3'-dideoxyribose, and internucleotide linkage modifications such as phosphorothioate, phosphorodithioate, phosphoroselenoate, phosphorodiselenoate, phosphoroanilothioate, phosphoraniladate and phosphoroamidate.

The term "polypeptide," "peptide" and "protein" are used interchangeably herein and include two or more amino acids linked covalently through peptide bonds. The terms do not refer to a specific length of the product. Thus, "peptides," and "oligopeptides," are included within the definition of polypeptide. The terms include post-translational modifications of the polypeptide, for example, glycosylations, acetylations, biotinylations, 4-pentynoylations, PEGylations, phosphorylations and the like. In addition, protein fragments, analogs, mutated or variant proteins, fusion proteins and the like are included within the meaning of polypeptide. The terms also include molecules in which one or more amino acid analogs or non-canonical or unnatural amino acids are included as can be expressed recombinantly using known protein engineering techniques. In addition, fusion proteins can be derivatized as described herein by well-known organic chemistry techniques.

A composition that includes a peptide or polypeptide covalently linked, attached, or bound, either directly or indirectly through a linker moiety, to another peptide or polypeptide or to a half-life extending moiety is a "conjugate" or "conjugated" molecule, whether conjugated by chemical means (e.g., post-translationally or post-synthetically) or by recombinant fusion.

The term "polypeptide analog" or "peptide analog" should be understood to mean a polypeptide having a sequence that differs from a polypeptide sequence existing in nature by at least one amino acid residue substitution, internal addition, or internal deletion of at least one amino acid, and/or amino- or carboxy- terminal end truncations or additions, and/or carboxy-terminal amidation. An "internal deletion" refers to absence of an amino acid from a sequence existing in nature at a position other than the N- or C-terminus. Likewise, an "internal addition" refers to presence of an amino acid in a sequence existing in nature at a position other than the N- or C-terminus.

The term "residue" can be used interchangeably with "amino acid"

The term "recombinant" should be understood to mean that the material (e.g., a nucleic acid or a polypeptide) has been artificially or synthetically (i.e., non-naturally) altered by human intervention. The alteration can be performed on the material within, or removed from, its natural environment or state. For example, a "recombinant nucleic acid" is one that is made by recombining nucleic acids, e.g., during cloning, DNA shuffling or other well known molecular biological procedures. Examples of such molecular biological procedures are found in Maniatis et al., Molecular Cloning. A Laboratory Manual. Cold Spring Harbor Laboratory, Cold Spring Harbor, N.Y. (1982). A "recombinant DNA molecule," is comprised of segments of DNA joined together by means of such molecular biological techniques. The term "recombinant protein" or "recombinant polypeptide" as used herein refers to a protein molecule which is expressed using a recombinant DNA molecule. A "recombinant host cell" is a cell that contains and/or expresses a recombinant nucleic acid.

The term "vehicle" should be understood to mean, *inter alia,* a molecule that prevents degradation and/or increases half-life, reduces toxicity, reduces immunogenicity, or increases biological activity of a therapeutic molecule. Exemplary vehicles can include polymers (e.g., polyethylene glycol (PEG); lipids; cholesterol group (such as a steroid); a carbohydrate or oligosaccharide (e.g., dextran); any natural or synthetic protein (e.g., an Ab or Fc domain); or any polypeptide or peptide that binds to a salvage receptor.

In various embodiments, a composition of matter is provided in which an APJ agonist polypeptide may be attached to a vehicle through the polypeptide's N-terminus, C-terminus, backbone, or side-chain via chemical modification. Thus, the vehicle-peptide molecules may be described by the following formula I: 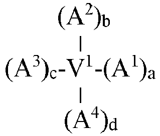 wherein:
V¹ is a vehicle (e.g. a PEG);
A¹, A², A³, and A⁴ are each independently selected from -(L¹)ₑ-P¹, -(L¹)ₑ-P¹⁻(L²)_{f} -P², - (L¹)ₑ-P¹-(L²)_{f}-P²-(L³)_{g}-P³, -(L¹)ₑ-P¹-(L²)_{f}-P²-(L³)_{g}-P³-(L⁴)ₕ-P⁴, and higher multimers thereof.
P¹, P², P³, and P⁴ are each independently sequences of APJ agonist domains;
L¹, L², L³, and L⁴ are each independently linkers; and
a, b, c, d, e, f, g, and h are each independently 0 or 1, provided that at least one of a, b, and c is 1.

Thus, compound II can comprise the formulae:

V¹-A¹

and multimers thereof wherein V¹ is a PEG and is attached, with or without linker, at the N-terminus of A¹;

Compound III can comprise the formulae : 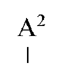 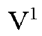 and multimers thereof wherein V¹ is a PEG and is attached, with or without linker, at the backbone or side-chain of A²;

Compound IV can comprise the formulae :

A³-V¹

and multimers thereof wherein V¹ is a PEG and is attached, with or without linker, at the C-terminus of A³;

Compound V can comprise the formulae :

A²-V¹-A¹

and multimers thereof wherein V¹ is a PEG and is attached, with or without linker, at any location of A1 and A2;

Compound VI can comprise the formulae 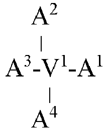 and multimers thereof wherein V¹ is a PEG and is attached, with or without linker, at any location of A1, A2, A3, or A4;

Compound VII can comprise the formulae: 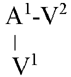 and multimers thereof wherein V¹ and V² are a PEG and are attached, with or without linker, at any location of A¹.

Recombinant DNA- and/or RNA-mediated protein expression and protein engineering techniques, or any other methods of preparing peptides, are applicable to the making of the polypeptides disclosed herein. For example, the peptides can be made in transformed host cells. Briefly, a recombinant DNA molecule, or construct, coding for the peptide is prepared. Methods of preparing such DNA molecules are well known in the art. For instance, sequences encoding the peptides can be excised from DNA using suitable restriction enzymes. Any of a large number of available and well-known host cells may be used in the practice of various embodiments. The selection of a particular host is dependent upon a number of factors recognized by the art. These include, for example, compatibility with the chosen expression vector, toxicity of the peptides encoded by the DNA molecule, rate of transformation, ease of recovery of the peptides, expression characteristics, bio-safety and costs. A balance of these factors should be struck with the understanding that not all hosts may be equally effective for the expression of a particular DNA sequence. Within these general guidelines, useful microbial host cells in culture include bacteria (such as *Escherichia coli* sp.), yeast (such as *Saccharomyces* sp.) and other fungal cells, insect cells, plant cells, mammalian (including human) cells, e.g., CHO cells and HEK293 cells. Modifications can be made at the DNA level, as well. The peptide-encoding DNA sequence may be changed to codons more compatible with the chosen host cell. *For E. coli,* optimized codons are known in the art. Codons can be substituted to eliminate restriction sites or to include silent restriction sites, which may aid in processing of the DNA in the selected host cell. Next, the transformed host is cultured and purified. Host cells may be cultured under conventional fermentation conditions so that the desired compounds are expressed. Such fermentation conditions are well known in the art. In addition, the DNA optionally further encodes, 5' to the coding region of a fusion protein, a signal peptide sequence (e.g., a secretory signal peptide) operably linked to the expressed peptide analog. For further examples of appropriate recombinant methods and exemplary DNA constructs useful for recombinant expression of the compositions by mammalian cells, including dimeric Fc fusion proteins ("peptibodies") or chimeric immunoglobulin(light chain + heavy chain)-Fc heterotrimers ("hemibodies"), see, e.g., Sullivan et al., Toxin Peptide Therapeutic Agents, US2007/0071764 and Sullivan et al., Toxin Peptide Therapeutic Agents, PCT/US2007/022831, published as WO 2008/088422.

Peptide compositions can also be made by synthetic methods. Solid phase synthesis can be used as a technique of making individual peptides since it is the most cost-effective method of making small peptides. For example, well known solid phase synthesis techniques include the use of protecting groups, linkers, and solid phase supports, as well as specific protection and deprotection reaction conditions, linker cleavage conditions, use of scavengers, and other aspects of solid phase peptide synthesis. Suitable techniques are well known in the art. (E.g., Merrifield (1973), Chem. Polypeptides, pp. 335-61 (Katsoyannis and Panayotis eds.); Merrifield (1963), J. Am. Chem. Soc. 85: 2149; Davis et al. (1985), Biochem. Intl. 10: 394-414; Stewart and Young (1969), Solid Phase Peptide Synthesis; U.S. Pat. No. 3,941,763; Finn et al. (1976), The Proteins (3rd ed.) 2: 105-253; and Erickson et al. (1976), The Proteins (3rd ed.) 2: 257-527; "Protecting Groups in Organic Synthesis," 3rd Edition, T. W. Greene and P. G. M. Wuts, Eds., John Wiley & Sons, Inc., 1999; NovaBiochem Catalog, 2000; "Synthetic Peptides, A User's Guide," G. A. Grant, Ed., W.H. Freeman & Company, New York, N.Y., 1992; "Advanced Chemtech Handbook of Combinatorial & Solid Phase Organic Chemistry," W. D. Bennet, J. W. Christensen, L. K. Hamaker, M. L. Peterson, M. R. Rhodes, and H. H. Saneii, Eds., Advanced Chemtech, 1998; "Principles of Peptide Synthesis, 2nd ed.," M. Bodanszky, Ed., Springer-Verlag, 1993; "The Practice of Peptide Synthesis, 2nd ed.," M. Bodanszky and A. Bodanszky, Eds., Springer-Verlag, 1994; "Protecting Groups," P. J. Kocienski, Ed., Georg Thieme Verlag, Stuttgart, Germany, 1994; "Fmoc Solid Phase Peptide Synthesis, A Practical Approach," W. C. Chan and P. D. White, Eds., Oxford Press, 2000, G. B. Fields et al., Synthetic Peptides: A User's Guide, 1990, 77-183). For further examples of synthetic and purification methods known in the art, which are applicable to making the compositions of matter, see, e.g., Sullivan et al, US2007/0071764 and Sullivan et al., PCT/US2007/022831, published as WO 2008/088422 A2.

One or more useful modifications to peptide domains can include amino acid additions or insertions, amino acid deletions, peptide truncations, amino acid substitutions, and/or chemical derivatization of amino acid residues, accomplished by known chemical techniques. For example, the thusly modified amino acid sequence includes at least one amino acid residue inserted or substituted therein, relative to the amino acid sequence of the native sequence of interest, in which the inserted or substituted amino acid residue has a side chain comprising a nucleophilic or electrophilic reactive functional group by which the peptide is covalently conjugated to a linker and/or half-life extending moiety. Useful examples of such a nucleophilic or electrophilic reactive functional group include, but are not limited to, a thiol, a primary amine, a seleno, a hydrazide, an aldehyde, a carboxylic acid, a ketone, an aminooxy, a masked (protected) aldehyde, or a masked (protected) keto functional group. Examples of amino acid residues having a side chain comprising a nucleophilic reactive functional group include, but are not limited to, a lysine residue, a homolysine, an α,β-diaminopropionic acid residue, an α,γ-diaminobutyric acid residue, an ornithine residue, a cysteine, a homocysteine, a glutamic acid residue, an aspartic acid residue, or a selenocysteine ("SeCys") residue.

The peptide portions of the compositions of matter can also be chemically derivatized at one or more amino acid residues by known organic chemistry techniques. "Chemical derivative" or "chemically derivatized" refers to a subject peptide having one or more residues chemically derivatized by reaction of a functional side group. Such derivatized molecules include, for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those peptides which contain one or more naturally occurring amino acid derivatives of the twenty canonical amino acids, whether in L- or D- form. For example, 4-hydroxyproline may be substituted for proline; 5-hydroxylysine maybe substituted for lysine; 3-methylhistidine may be substituted for histidine; homoserine may be substituted for serine; and ornithine may be substituted for lysine.

Useful derivatizations include, in some embodiments, those in which the amino terminal of the peptide is chemically blocked so that conjugation with the vehicle will be prevented from taking place at an N-terminal free amino group. There may also be other beneficial effects of such a modification, for example a reduction in the peptide analog's susceptibility to enzymatic proteolysis. The N-terminus can be acylated or modified to a substituted amine, or derivatized with another functional group, such as an aromatic moiety (e.g., an indole acid, benzyl (Bzl or Bn), dibenzyl (DiBzl or Bn₂), or benzyloxycarbonyl (Cbz or Z)), *N*,*N*-dimethylglycine or creatine. For example, in some embodiments, an acyl moiety, such as, but not limited to, a formyl, acetyl (Ac), propanoyl, butanyl, heptanyl, hexanoyl, octanoyl, or nonanoyl, can be covalently linked to the N-terminal end of the peptide, which can prevent undesired side reactions during conjugation of the vehicle to the peptide. Other exemplary N-terminal derivative groups include -NRR¹ (other than -NH₂), -NRC(O)R¹, - NRC(O)OR¹, -NRS(O)₂R¹, -NHC(O)NHR¹, succinimide, or benzyloxycarbonyl-NH- (Cbz-NH-), wherein R and R¹ are each independently hydrogen or lower alkyl and wherein the phenyl ring may be substituted with 1 to 3 substituents selected from C₁-C₄ alkyl, C₁-C₄ alkoxy, chloro, and bromo.

In some embodiments, one or more peptidyl [-C(O)NR-] linkages (bonds) between amino acid residues can be replaced by a non-peptidyl linkage. Exemplary non-peptidyl linkages are -CH₂-carbamate [-CH₂-OC(O)NR-], phosphonate , -CH₂-sulfonamide [-CH₂-S(O)₂NR-], urea [-NHC(O)NH-], -CH₂-secondary amine, and alkylated peptide [-C(O)NR⁶- wherein R⁶ is lower alkyl].

In some embodiments, one or more individual amino acid residues can be derivatized. Various derivatizing agents are known to react specifically with selected sidechains or terminal residues, as described in detail below by way of example.

Lysinyl residues and amino terminal residues may be reacted with succinic or other carboxylic acid anhydrides, which reverse the charge of the lysinyl residues. Other suitable reagents for derivatizing alpha-amino-containing residues include imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate.

Arginyl residues may be modified by reaction with any one or combination of several conventional reagents, including phenylglyoxal, 2,3-butanedione, 1,2-cyclohexanedione, and ninhydrin. Derivatization of arginyl residues requires that the reaction be performed in alkaline conditions because of the high pKa of the guanidine functional group. Furthermore, these reagents may react with the groups of lysine as well as the arginine epsilon-amino group.

Specific modification of tyrosyl residues has been studied extensively, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively.

Carboxyl sidechain groups (aspartyl or glutamyl) may be selectively modified by reaction with carbodiimides (R'-N=C=N-R') such as 1-cyclohexyl-3-(2-morpholinyl-(4-ethyl) carbodiimide or 1-ethyl-3-(4-azonia-4,4-dimethylpentyl) carbodiimide. Furthermore, aspartyl and glutamyl residues may be converted to asparaginyl and glutaminyl residues by reaction with ammonium ions.

Glutaminyl and asparaginyl residues may be deamidated to the corresponding glutamyl and aspartyl residues. Alternatively, these residues are deamidated under mildly acidic conditions. Either form of these residues falls within the scope of the various embodiments.

Cysteinyl residues can be replaced by amino acid residues or other moieties either to eliminate disulfide bonding or, conversely, to stabilize cross-linking. (See, e.g., Bhatnagar et al., J. Med. Chem., 39:3814-3819 (1996)).

Derivatization with bifunctional agents is useful for cross-linking the peptides or their functional derivatives to a water-insoluble support matrix, if desired, or to other macromolecular vehicles. Commonly used cross-linking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophenyl)dithio] propioimidate yield photoactivatable intermediates that are capable of forming crosslinks in the presence of light. Alternatively, reactive water-insoluble matrices such as cyanogen bromide-activated carbohydrates and the reactive substrates, e.g., as described in U.S. Pat. Nos. 3,969,287; 3,691,016; 4,195,128; 4,247,642; 4,229,537; and 4,330,440, are employed for protein immobilization.

Other possible modifications include hydroxylation of proline and lysine, phosphorylation of hydroxyl groups of seryl or threonyl residues, oxidation of the sulfur atom in Cys, methylation of the alpha-amino groups of lysine, arginine, and histidine side chains. Creighton, Proteins: Structure and Molecule Properties (W. H. Freeman & Co., San Francisco), 79-86 (1983).

The above examples of derivatizations are not intended to be an exhaustive treatment, but merely illustrative.

In various embodiments one may wish to modulate the pharmacokinetic profile of an APJ agonist polypeptide. For modulation of the pharmacokinetic profile to fit a therapeutic need, various embodiments can include one or more half-life extending moieties of various masses and configurations, which half-life extending moiety, or moieties, can be covalently fused, attached, linked or conjugated to the polypeptide. A "half-life extending moiety" refers to a molecule that prevents or mitigates *in vivo* degradation by proteolysis or other activity-diminishing chemical modification, increases *in vivo* or *in vitro* half-life or other pharmacokinetic properties such as, but not limited to decreasing the rate of renal or hepatic clearance rate, increasing the rate of absorption, reduces toxicity, reduces immunogenicity, improves solubility, increases biological activity and/or target selectivity of the peptide analog with respect to a target of interest, and/or increases manufacturability, compared to an unconjugated form of the polypeptide analog. The half-life extending moiety can be one that is pharmaceutically acceptable.

The half-life extending moiety can be selected such that a polypeptide or composition described herein can achieve a sufficient hydrodynamic size to prevent clearance by renal filtration *in vivo.* For example, a half-life extending moiety can be selected that is a polymeric macromolecule, which is substantially straight chain, branched-chain (br), or dendritic in form. Alternatively, a half-life extending moiety can be selected such that, *in vivo,* the polypeptide or composition of matter will bind to a serum protein to form a complex, such that the complex thus formed avoids substantial renal clearance. The half-life extending moiety can be, for example, a lipid; a cholesterol group (such as a steroid); a carbohydrate or oligosaccharide; a polymer; or any natural or synthetic protein, polypeptide or peptide that binds to a salvage receptor.

Exemplary half-life extending moieties that can be used can include an immunoglobulin Fc domain, or a portion thereof, or a biologically suitable polymer or copolymer, for example, a polyalkylene glycol compound, such as a polyethylene glycol (PEG) or a polypropylene glycol. Other appropriate polyalkylene glycol compounds include, but are not limited to, charged or neutral polymers of the following types: dextran, polylysine, colominic acids or other carbohydrate based polymers, polymers of amino acids, and biotin derivatives. In some monomeric fusion or conjugate proteins an immunoglobulin (including light and heavy chains) or a portion thereof, can be used as a half-life-extending moiety, preferably an immunoglobulin of human origin, and including any of the immunoglobulins, such as, but not limited to, IgG1, IgG2, IgG3 or IgG4.

Other examples of the half-life extending moiety, include a copolymer of ethylene glycol, a copolymer of propylene glycol, a carboxymethyl cellulose, a polyvinyl pyrrolidone, a poly-1,3-dioxolane, a poly-1,3,6-trioxane, an ethylene maleic anhydride copolymer, a polyaminoacid (e.g., polylysine or polyornithine), a dextran n-vinyl pyrrolidone, a poly n-vinyl pyrrolidone, a propylene glycol homopolymer, a propylene oxide polymer, an ethylene oxide polymer, a polyoxyethylated polyol, a polyvinyl alcohol, a linear or branched glycosylated chain, a polyacetal, a long chain fatty acid, a long chain hydrophobic aliphatic group, or a polysialic acid (e.g., PolyXen™ technology; Gregoriadis et al., Improving the therapeutic efficacy of peptides and proteins: a role for polysialic acids, Intl. J. Pharmaceutics, 300:125-30 (2005)).

The half-life extending moiety can be an anionically charged chemical entity, covalently linked to the N-terminus of the peptide analog, which anionically charged chemical entities include, but are not limited to, phosphotyrosine, phosphoserine, p-phosphono(difluoromethyl)-phenylalanine (Pfp), p-phosphono-methyl-phenylalanine (Pmp), p-phosphatidyl-phenylalanine (Ppa), or p-phosphono-methylketo-phenylalanine (Pkp), which can be covalently linked to the N-terminal of the peptide analog, optionally indirectly, via an AEEA linker or other linker as described herein. (See, Chandy et al.)

The half-life extending moiety can include peptide ligands or small (organic) molecule ligands that have binding affinity for a long half-life serum protein under physiological conditions of temperature, pH, and ionic strength. Examples include an albumin-binding peptide or small molecule ligand, a transthyretin-binding peptide or small molecule ligand, a thyroxine-binding globulin-binding peptide or small molecule ligand, an antibody-binding peptide or small molecule ligand, or another peptide or small molecule that has an affinity for a long half-life serum protein. (See, e.g., Blaney et al., Method and compositions for increasing the serum half-life of pharmacologically active agents by binding to transthyretin-selective ligands, US Patent. No. 5,714,142; Sato et al., Serum albumin binding moieties, US 2003/0069395 A1; Jones et al., Pharmaceutical active conjugates, US Patent No. 6,342,225).

A "long half-life serum protein" is one of the hundreds of different proteins dissolved in mammalian blood plasma, including so-called "carrier proteins" (such as albumin, transferrin and haptoglobin), fibrinogen and other blood coagulation factors, complement components, immunoglobulins, enzyme inhibitors, precursors of substances such as angiotensin and bradykinin and many other types of proteins. Various embodiments can encompass the use of any single species of pharmaceutically acceptable half-life extending moiety, such as, but not limited to, those described herein, or the use of a combination of two or more different half-life extending moieties, such as PEG and immunoglobulin Fc domain or a portion thereof (see, e.g., Feige et al., Modified peptides as therapeutic agents, US Patent No. 6,660,843), such as a CH2 domain of Fc, albumin (e.g., human serum albumin (HSA); see, e.g., Rosen et al., Albumin fusion proteins, US Patent No. 6,926,898 and US 2005/0054051; Bridon et al., Protection of endogenous therapeutic peptides from peptidase activity through conjugation to blood components, US 6,887,470), a transthyretin (TTR; see, e.g., Walker et al., Use of transthyretin peptide/protein fusions to increase the serum half-life of pharmacologically active peptides/proteins, US 2003/0195154 A1; 2003/0191056 A1), or a thyroxine-binding globulin (TBG), or a combination such as immunoglobulin(light chain+heavy chain) and Fc domain (the heterotrimeric combination a so-called "hemibody"), for example as described in Sullivan et al., Toxin Peptide Therapeutic Agents, PCT/US2007/022831, published as WO 2008/088422.

Conjugation of the peptide analogs(s) to the half-life extending moiety, or moieties, can be via the N-terminus and/or C-terminus of the peptide, or can be intercalary as to its primary amino acid sequence, F1 being linked closer to the peptide analog's N-terminus.

Particularly useful half-life extending moieties include immunoglobulins (e.g., human immunoglobulin, including IgG1, IgG2, IgG3 or IgG4). The term "immunoglobulin" encompasses full antibodies comprising two dimerized heavy chains (HC), each covalently linked to a light chain (LC); a single undimerized immunoglobulin heavy chain and covalently linked light chain (HC + LC); or a chimeric immunoglobulin (light chain + heavy chain)-Fc heterotrimer (a so-called "hemibody").

Extending the half-life of the non-degraded APJ agonist can also be referred to as increased "stability." In various circumstances, it can be imagined that modification of the peptide, e.g. addition of a PEG molecule could extend the half-life of a degraded fragment of the endogenous apelin molecule which may not necessarily lead to increased stability of the bioactive apelin. Increased stability can also be understood to mean reduced clearance of the agonist or overall increased exposure of the agonist or an agonist with a modification or combination of modifications that reduces the rate of metabolic degradation relative to unmodified agonist. Increased stability can prolong half-life of the agonist in biological matrices such as plasma and tissue homogenates *in vitro* and prolong plasma residence times *in vivo. In* vivo plasma residence times should be understood to mean circulating life-time of the intact peptide drug entity administered into an animal. *In* vitro plasma residence times should be understood to mean life-time of the intact peptide drug entity after addition in biological medium.

Modifications of the APJ agonist can also lead to increased "potency" of the molecule, e.g. improving the pharmokinetic or pharmacodynamic properties of the molecule. Increased potency can also be understood to mean an agonist with a modification or combination of modifications that reduces the rate of metabolic degradation relative to unmodified agonist. Increased stability can prolong half-life or the agonist in biological matrices such as plasma and tissue homogenates *in vitro* and prolong plasma residence times *in vivo.* Increased potency may further be understood to mean increased affinity and/or efficacyof the receptor.

An "Fc region", or used interchangeably herein, "Fc domain" or "immunoglobulin Fc domain", contains two heavy chain fragments, which in a full antibody comprise the _{CH}1 and _{CH}2 domains of the antibody. The two heavy chain fragments are held together by two or more disulfide bonds and by hydrophobic interactions of the _{CH}3 domains.

As noted above, polymer half-life extending moieties can also be used. Various means for attaching chemical moieties useful as half-life extending moieties are currently available, see, e.g., Patent Cooperation Treaty ("PCT") International Publication No. WO 96/11953, entitled "N-Terminally Chemically Modified Protein Compositions and Methods,". This PCT publication discloses, among other things, the selective attachment of water-soluble polymers to the APJ ligand.

In some embodiments, the polymer half-life extending moiety is polyethylene glycol (PEG), covalently linked at the N-terminus, C-terminus or at one or more intercalary side chains of the APJ agonist. In some embodiments the composition of matter can further include one or more PEG moieties conjugated to a non-PEG half-life extending moiety or to the APJ agonist, or to any combination of any of these. For example, an Fc domain or portion thereof can be made mono-PEGylated, di-PEGylated, or otherwise multi-PEGylated, by the process of covalent conjugation.

Covalent conjugation of proteins and peptides with poly(ethylene glycol) (PEG) can significantly extend the *in vivo* circulating half-lives. PEGylation is believed to achieve this effect predominately by retarding renal clearance, since the PEG moiety adds considerable hydrodynamic radius to the molecule. (Zalipsky, S., et al., Use of functionalized poly(ethylene glycol)s for modification of polypeptides., in poly(ethylene glycol) chemistry: Biotechnical and biomedical applications., J.M. Harris, Ed., Plenum Press: New York., 347-370 (1992)). Additional benefits often conferred by PEGylation of proteins and peptides include increased solubility, resistance to proteolytic degradation, and reduced immunogenicity of the therapeutic polypeptide. The merits of protein PEGylation are evidenced by the commercialization of several PEGylated proteins including PEG-Adenosine deaminase (Adagen™/Enzon Corp.), PEG-L-asparaginase (Oncaspar™/Enzon Corp.), PEG-Interferon α-2b (PEG-Intron™/Schering/Enzon), PEG-Interferon α-2a (PEGASYS™/Roche) and PEG-G-CSF (Neulasta™/Amgen) as well as many others in clinical trials.

By "PEGylated peptide", "PEGylated polypeptide" or "PEGylated protein" is meant a peptide having a polyethylene glycol (PEG) moiety covalently bound to an amino acid residue of the peptide itself or to a peptidyl or non-peptidyl linker that is covalently bound to a residue of the peptide, either directly or indirectly through another linker moiety. A nonlimiting example is N-terminal conjugation of the peptide with 3-(1-(1-bromo-2-oxo-6,9,12,15,18,21,24,27,30,33,36-undecaoxa-3-azaoctatriacontan-38-yl)-1H-1,2,3-triazol-4-yl)propanoyl (designated herein by the abbreviation "{bromoacetamide-PEG11-triazole}-").

By "polyethylene glycol" or "PEG" is meant a polyalkylene glycol compound or a derivative thereof, with or without coupling agents or derivatization with coupling or activating moieties (e.g., with aldehyde, hydroxysuccinimidyl, hydrazide, thiol, triflate, tresylate, azirdine, oxirane, orthopyridyl disulphide, vinylsulfone, iodoacetamide or a maleimide moiety). In accordance with various embodiments, useful PEG includes substantially linear, straight chain PEG, branched PEG (brPEG), or dendritic PEG. (See, e.g., Merrill, US Patent No. 5,171,264; Harris et al., Multiarmed, monofunctional, polymer for coupling to molecules and surfaces, US Patent No. 5,932,462; Shen, N-maleimidyl polymer derivatives, US Patent No. 6,602,498).

Briefly, the PEG groups can generally be attached to the peptide portion of the composition via acylation or alkylation (or reductive amination) through a reactive group on the PEG moiety (e.g., an aldehyde, amino, thiol, or ester group) to a reactive group on the compound (e.g., an aldehyde, amino, or ester group). A useful strategy for the PEGylation of synthetic peptides consists of combining, through forming a conjugate linkage in solution, a peptide and a PEG moiety, each bearing a special functionality that is mutually reactive toward the other. The peptides can be easily prepared with conventional solid phase synthesis . The peptides can be "preactivated" with an appropriate functional group at a specific site. The precursors are purified and fully characterized prior to reacting with the PEG moiety. Ligation of the peptide with PEG usually takes place in aqueous phase and can be easily monitored by reverse phase analytical HPLC. The PEGylated peptides can be easily purified by preparative HPLC and characterized by analytical HPLC, amino acid analysis and laser desorption mass spectrometry.

PEG is a well-known, water soluble polymer that is commercially available or can be prepared by ring-opening polymerization of ethylene glycol according to methods well known in the art (Sandler and Karo, Polymer Synthesis, Academic Press, New York, Vol. 3, pages 138-161). In the application, the term "PEG" is used broadly to encompass any polyethylene glycol molecule, in mono-, bi-, or poly- functional form, without regard to size or to modification at an end of the PEG, and can be represented by the formula:

X-O(CH₂CH₂O)ₙ-R, (I)

where n is 3 to 2300 and X is H or a terminal modification, e.g., a methyl or C₁₋₄ alkyl, and R is the reactive moiety used for covalent attachment.

In some embodiments, a PEG can be used that terminates on one end with hydroxy or methoxy, i.e., X is H or CH₃ ("methoxy PEG"). It is noted that the other end of the PEG, which is shown in formula (I) terminating in R, covalently attaches to an activating moiety via an ether oxygen bond, an amine linkage, or amide linkage. When used in a chemical structure, the term "PEG" includes the formula (I) above without the hydrogen of the hydroxyl group shown, leaving the oxygen available to react with a free carbon atom of a linker to form an ether bond. More specifically, in order to conjugate PEG to a peptide, the peptide must be reacted with PEG in an "activated" form. Activated PEG can be represented by the formula:

(PEG)-(A) (II)

where PEG (defined *supra*) covalently attaches to a carbon atom of the activation moiety (A) to form an ether bond, an amine linkage, or amide linkage, and (A) contains a reactive group which can react with an amino, azido, alkyne, imino, maleimido, N-succinimidyl, carboxyl, aminooxy, seleno, or thiol group on an amino acid residue of a peptide or a linker moiety covalently attached to the peptide, e.g., the APJ agonist.

Examples of various PEG's are below.
Aminoalkyl PEG 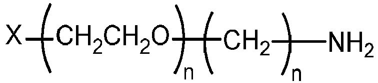
Thiol PEG 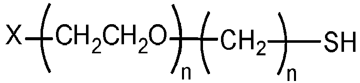
Carboxylic acid PEG 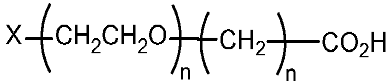
Hydrazide PEG 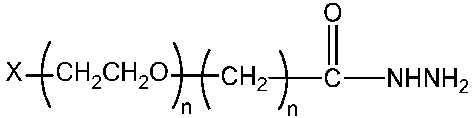
Azido PEG 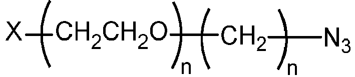
Mesylate and Tosylate PEGs 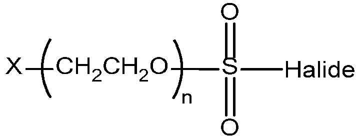 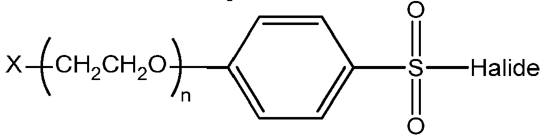 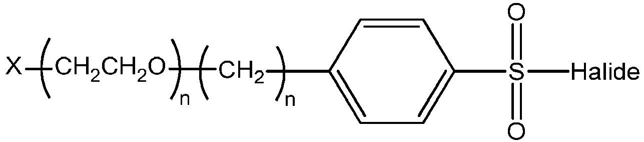
Maleimide PEGs 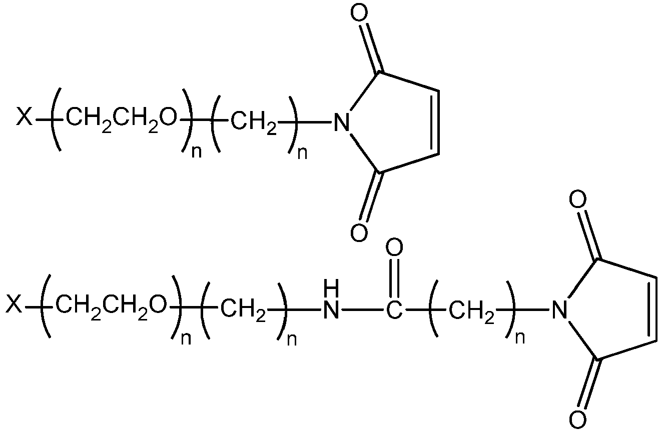
p-Nitrophenyl Carbonate PEG 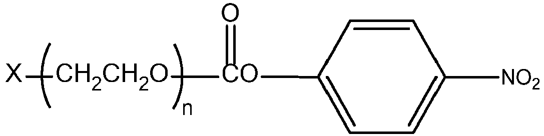
NHS Carbonate PEG 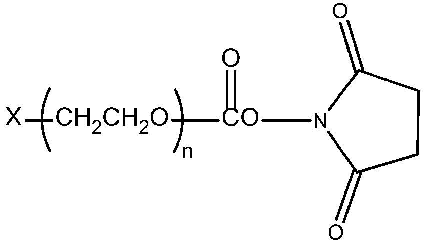
NHS active esters 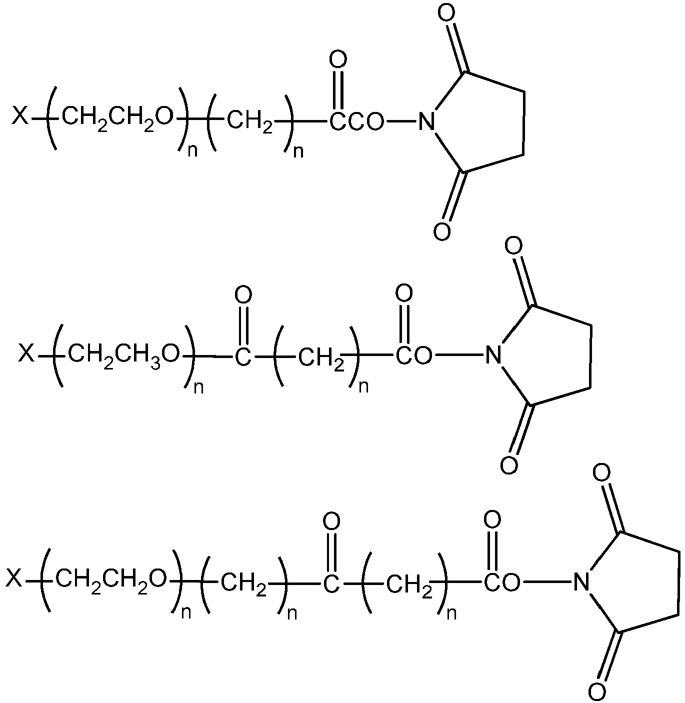
Aldehyde PEG 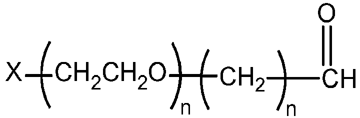
Aminoxy PEGs 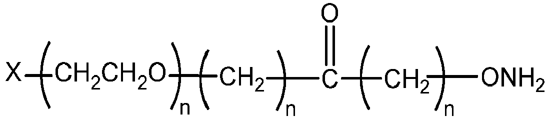 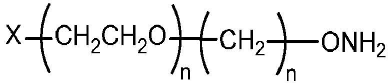
Iodoacetamide PEG 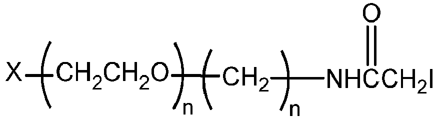
Ortho-pyridyldisulfide PEG 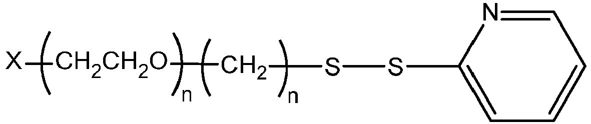
Alkyne PEG

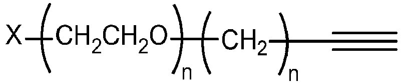

Techniques for the preparation of activated PEG and its conjugation to biologically active peptides are well known in the art. (E.g., see U.S. Pat. Nos. 5,643,575, 5,919,455, 5,932,462, and 5,990,237; Kinstler et al., N-terminally chemically modified protein compositions and methods, US Patent Nos. 5,985,265, and 5,824,784; Thompson et al., PEGylation of polypeptides, EP 0575545 B1; Petit, Site specific protein modification, US Patent Nos. 6,451,986, and 6,548,644; S. Herman et al., Poly(ethylene glycol) with reactive endgroups: I. Modification of proteins, J. Bioactive Compatible Polymers, 10:145-187 (1995); Y. Lu et al., PEGylated peptides III: Solid-phase synthesis with PEGylating reagents of varying molecular weight: synthesis of multiply PEGylated peptides, Reactive Polymers, 22:221-229 (1994); A.M. Felix et al., PEGylated Peptides IV: Enhanced biological activity of site-directed PEGylated GRF analogs, Int. J. Peptide Protein Res., 46:253-264 (1995); A.M. Felix, Site-specific poly(ethylene glycol)ylation of peptides, ACS Symposium Series 680(poly(ethylene glycol)): 218-238 (1997); Y. Ikeda et al., Polyethylene glycol derivatives, their modified peptides, methods for producing them and use of the modified peptides, EP 0473084 B1; G.E. Means et al., Selected techniques for the modification of protein side chains, in: Chemical modification of proteins, Holden Day, Inc., 219 (1971)).

Activated PEG, such as PEG-aldehydes or PEG-aldehyde hydrates, can be chemically synthesized by known means or obtained from commercial sources, e.g., Shearwater Polymers, (Huntsville, Al) or Enzon, Inc. (Piscataway, N.J.).

An example of a useful activated PEG can be a PEG-aldehyde compound (e.g., a methoxy PEG-aldehyde), such as PEG-propionaldehyde, which is commercially available from Shearwater Polymers (Huntsville, Al). PEG-propionaldehyde is represented by the formula PEG-CH₂CH₂CHO. (See, e.g., U.S. Pat. No. 5,252,714). Also included within the meaning of "PEG aldehyde compound" are PEG aldehyde hydrates, e.g., PEG acetaldehyde hydrate and PEG bis aldehyde hydrate, which latter yields a bifunctionally activated structure. (See., e.g., Bentley et al., Poly(ethylene glycol) aldehyde hydrates and related polymers and applications in modifying amines, US Patent No. 5,990,237) (See., e.g., Bentley et al., Poly(ethylene glycol) aldehyde hydrates and related polymers and applications in modifying amines, US Patent No. 5,990,237). An activated multi-branched PEG-aldehyde compound can be used (PEG derivatives comprising multiple arms to give divalent, trivalent, tetravalent, octavalent constructs). Using a 4-arm PEG derivative four (4) APJ agonists can be attached to each PEG molecule. For example, the APJ agonist can be conjugated to a polyethylene glycol (PEG) at 1, 2, 3 or 4 amino functionalized sites of the PEG.

In being conjugated, the polyethylene glycol (PEG), as described herein, is covalently bound by alkylation of a thiol present in the peptide or is covalently bound by cycloaddition reaction between azido and alkyne moieties present in the PEG and peptide. Alternatively, the PEG can be covalently bound by reductive amination directly to at least one solvent-exposed free amine moiety of an amino acid residue of the APJ agonist itself. In some embodiments, the APJ agonist can be conjugated to a PEG at one or more primary or secondary amines on the peptide analog, or to two PEG groups at a single primary amine site on the peptide analog (e.g., this can occur when the reductive amination reaction involves the presence of excess PEG-aldehyde compound). It has been observed that when PEGylation by reductive amination is at a primary amine on the peptide, it is not uncommon to have amounts (1 to 100% range) of reaction product that have two or more PEGs present per molecule, and if the desired PEGylation product is one with only one PEG per molecule, then this "over-PEGylation" may be undesirable. When PEGylated product with a single PEG per PEGylation product molecule is desired, an embodiment can be employed that involves PEGylation using secondary amines of the pharmacologically active peptide, because only one PEG group per molecule will be transferred in the reductive amination reaction.

Amino acid residues that can provide a primary amine moiety include residues of lysine, homolysine, ornithine, a, β-diaminopropionic acid (Dap), a, β-diaminopropionoic acid (Dpr), and a, γ-diaminobutyric acid (Dab), aminobutyric acid (Abu), and α-amino-isobutyric acid (Aib). The polypeptide N-terminus also provides a useful α-amino group for PEGylation. Amino acid residues that can provide a secondary amine moiety include ε-N-alkyl lysine, α-N-alkyl lysine, δ-N-alkyl ornithine, α-N-alkyl ornithine, or an N-terminal proline, where the alkyl is C₁ to C₆.

Another useful activated PEG for generating the PEGylated analogs is a PEG-maleimide compound, such as, but not limited to, a methoxy PEG-maleimide, such as maleimido monomethoxy PEG, are particularly useful for generating PEG-conjugated peptides. (E.g., Shen, *N*-maleimidyl polymer derivatives, US Patent No. 6,602,498; C. Delgado et al., The uses and properties of PEG-linked proteins., Crit. Rev. Therap. Drug Carrier Systems, 9:249-304 (1992); S. Zalipsky et al., Use of functionalized poly(ethylene glycol)s for modification of polypeptides, in: Poly(ethylene glycol) chemistry: Biotechnical and biomedical applications (J.M. Harris, Editor, Plenum Press: New York, 347-370 (1992); S. Herman et al., Poly(ethylene glycol) with reactive endgroups: I. Modification of proteins, J. Bioactive Compatible Polymers, 10:145-187 (1995); P.J. Shadle et al., Conjugation of polymer to colony stimulating factor-1, U.S. Patent No. 4,847,325; G. Shaw et al., Cysteine added variants IL-3 and chemical modifications thereof, U.S. Patent No. 5,166,322 and EP 0469074 B1; G. Shaw et al., Cysteine added variants of EPO and chemical modifications thereof, EP 0668353 A1; G. Shaw et al., Cysteine added variants G-CSF and chemical modifications thereof, EP 0668354 A1; N.V. Katre et al., Interleukin-2 muteins and polymer conjugation thereof, U.S. Patent No. 5,206,344; R.J. Goodson and N.V. Katre, Site-directed pegylation of recombinant interleukin-2 at its glycosylation site, Biotechnology, 8:343-346 (1990)).

A poly(ethylene glycol) vinyl sulfone is another useful activated PEG for generating the PEG-conjugated the APJ agonists by conjugation at thiolated amino acid residues, e.g., at C residues. (E.g., M. Morpurgo et al., Preparation and characterization of poly(ethylene glycol) vinyl sulfone, Bioconj. Chem., 7:363-368 (1996); see also Harris, Functionalization of polyethylene glycol for formation of active sulfone-terminated PEG derivatives for binding to proteins and biologically compatible materials, U.S. Patent Nos. 5,446,090; 5,739,208; 5,900,461; 6,610,281 and 6,894,025; and Harris, Water soluble active sulfones of poly(ethylene glycol), WO 95/13312 A1). Another activated form of PEG that is useful in accordance with various embodiments, is a PEG-N-hydroxysuccinimide ester compound, for example, methoxy PEG-N-hydroxysuccinimidyl (NHS) ester.

Heterobifunctionally activated forms of PEG are also useful. (See, e.g., Thompson et al., PEGylation reagents and biologically active compounds formed therewith, U.S. Patent No. 6,552,170).

In still other embodiments of producing a composition of matter, the APJ agonist is reacted by known chemical techniques with an activated PEG compound, such as but not limited to, a thiol-activated PEG compound, a diol-activated PEG compound, a PEG-hydrazide compound, a PEG-oxyamine compound, or a PEG-bromoacetyl compound. (See, e.g., S. Herman, Poly(ethylene glycol) with Reactive Endgroups: I. Modification of Proteins, J. Bioactive and Compatible Polymers, 10:145-187 (1995); S. Zalipsky, Chemistry of Polyethylene Glycol Conjugates with Biologically Active Molecules, Advanced Drug Delivery Reviews, 16:157-182 (1995); R. Greenwald et al., Poly(ethylene glycol) conjugated drugs and prodrugs: a comprehensive review, Critical Reviews in Therapeutic Drug Carrier Systems, 17:101-161 (2000)).

In another embodiment the activated PEG for generating a PEG-conjugated APJ agonist can be a multivalent PEG having more than one activated residues. Multivalent PEG moieties can include, but are not limited to, those shown below in Table 4:

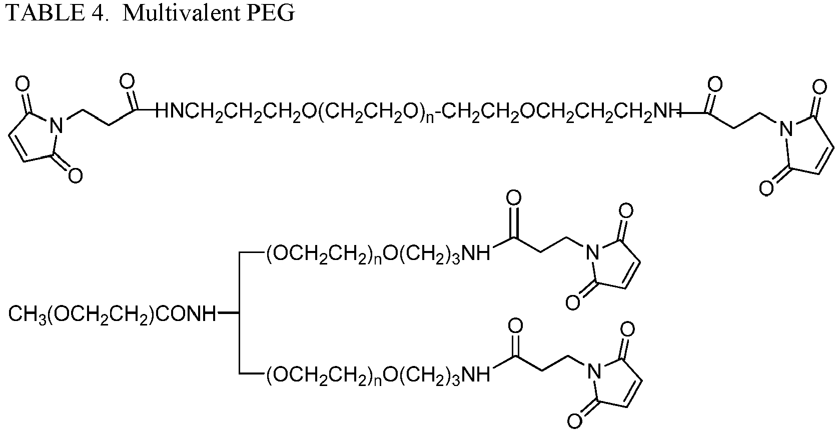

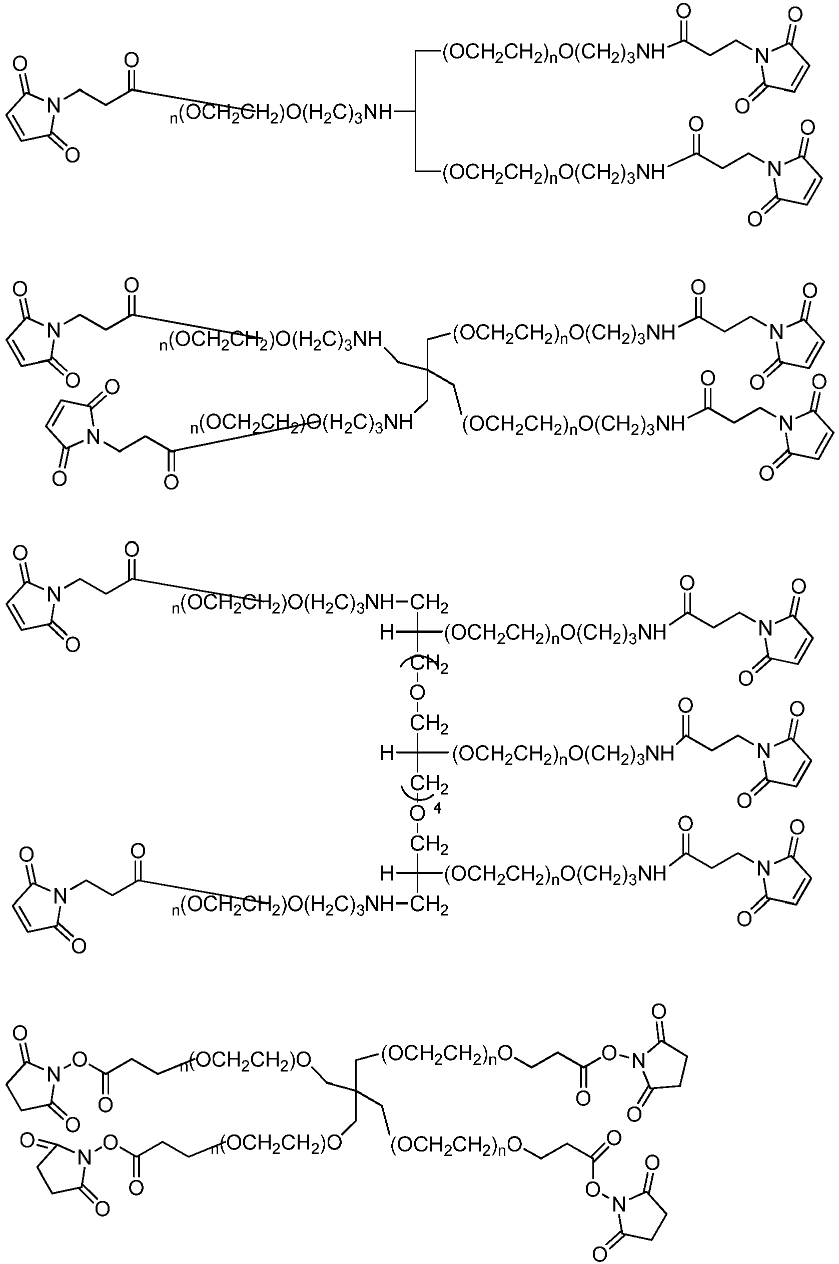

In still other embodiments the APJ agonist can be reacted by known chemical techniques with an activated multi-branched PEG compound (PEG derivatives comprising multiple arms to give divalent, trivalent, tetravalent, octavalent constructs), such as but not limited to, pentaerythritol tetra-polyethyleneglycol ether. Functionalization and activated derivatives , such as, but not limited to, N-succinimidyloxycarbonyl)propyl, p-nitrophenyloxycarbonyl, (-CO₂-p-C₆H₄NO₂), 3-(N-maleimido)propanamido, 2-sulfanylethyl, and 3-aminopropyl. Using a 4-arm PEG derivative, four peptide analogs can be attached to each PEG molecule. For example, the APJ agonist can be conjugated to a polyethylene glycol (PEG) at:
(a) 1, 2, 3 or 4 amino functionalized sites of the PEG;
(b) 1, 2, 3 or 4 thiol functionalized sites of the PEG;
(c) 1, 2, 3 or 4 maleimido functionalized sites of the PEG;
(d) 1, 2, 3 or 4 N-succinimidyl functionalized sites of the PEG;
(e) 1, 2, 3 or 4 carboxyl functionalized sites of the PEG;
(f) 1, 2, 3 or 4 *p*-nitrophenyloxycarbonyl functionalized sites of the PEG;
(g) 1, 2, 3 or 4 azido functionalized sites of the PEG;
(h) 1, 2, 3 or 4 alkene or alkyne functionalized sites of the PEG; or
(i) 1, 2, 3 or 4 halide or halide-acetylamide functionalized sites of the PEG.

The smallest practical size of PEG is about 500 Daltons (Da), below which PEG becomes toxic. Above about 500 Da, any molecular mass for a PEG can be used as practically desired, e.g., from about 500 Daltons (Da) to 100,000 Da (n is 10 to 2300). The number of PEG monomers (n) is approximated from the average molecular mass using a MW = 44 Da for each monomer. In some embodiments, the combined or total average molecular mass of PEG used in a PEG-conjugated APJ agonist can be from about 500 Da to 10,000 Da (total n is from 10 to 230), or from about 10,000 to 40,000 Da (total n is from 230 to 910), or from about 40,000 to 100,000 Da (total n is from 910 to 2300).

In various other embodiments, the combined molecular mass of the PEG molecule should not exceed about 100,000 Da. In some embodiments, the combined or total average molecular mass of PEG used in a PEG-conjugated APJ agonist can be from about 3,000 Da to 60,000 Da (total n is from 70 to 1,400), from about 10,000 Da to 40,000 Da (total n is about 230 to about 910). In other embodiments the combined mass for PEG is from about 20,000 Da to 30,000 Da (total n is about 450 to about 680).

It will be appreciated that "multimers" of the composition of matter can be made, since the half-life extending moiety employed for conjugation of the APJ agonist (with or without an intervening linker moiety) can be multivalent (e.g., bivalent, trivalent, tetravalent or a higher order valency) as to the number of amino acid residues at which the half-life extending moiety can be conjugated. In some embodiments the peptide can be multivalent (e.g., bivalent, trivalent, tetravalent or a higher order valency), and, thus, some "multimers" may have more than one half life extending moiety. Consequently, it is possible to produce a variety of conjugated half-life extending moiety peptide structures. By way of example, a univalent half-life extending moiety and a univalent peptide can produce a 1:1 conjugate; a bivalent peptide and a univalent half-life extending moiety may form conjugates wherein the peptide conjugates bear two half-life extending moiety moieties, whereas a bivalent half-life extending moiety and a univalent peptide may produce species where two peptide entities are linked to a single half-life extending moiety; use of higher-valence half-life extending moiety can lead to the formation of clusters of peptide entities bound to a single half-life extending moiety, whereas higher-valence peptides may become encrusted with a plurality of half-life extending moiety moieties. By way of further example, if the site of conjugation of a multivalent half-life extending moiety to the APJ agonist is a cysteine or other aminothiol the methods disclosed by D'Amico et al. may be employed (D'Amico et al., Method of conjugating aminothiol containing molecules to vehicles, published as US 2006/0199812).

The peptide moieties may have more than one reactive group which will react with the activated half-life extending moiety and the possibility of forming complex structures must always be considered; when it is desired to form simple structures such as 1:1 adducts of half-life extending moiety and peptide, or to use bivalent half-life extending moiety to form peptide:half-life extending moiety:peptide adducts, it will be beneficial to use predetermined ratios of activated half-life extending moiety and peptide material, predetermined concentrations thereof and to conduct the reaction under predetermined conditions (such as duration, temperature, pH, etc.) so as to form a proportion of the described product and then to separate the described product from the other reaction products. The reaction conditions, proportions and concentrations of the reagents can be obtained by relatively simple trial-and- error experiments which are within the ability of an ordinarily skilled artisan with appropriate scaling-up as necessary. Purification and separation of the products is similarly achieved by conventional techniques well known to those skilled in the art.

Additionally, physiologically acceptable salts of the half-life extending moiety-fused or conjugated to the APJ agonist are also encompassed by various embodiments described within.

The above-described half-life extending moieties and other half-life extending moieties described herein are useful, either individually or in combination, and as further described in the art, for example, in Sullivan et al., Toxin Peptide Therapeutic Agents, US2007/0071764 and Sullivan et al., Toxin Peptide Therapeutic Agents, PCT/US2007/022831, published as WO 2008/088422. Various embodiments encompass the use of any single species of pharmaceutically acceptable half-life extending moiety, such as, but not limited to, those described herein, in conjugation with the peptide analog, or the use of a combination of two or more like or different half-life extending moieties.

A "linker" or "linker moiety" is optional in the polypeptides and/or compositions of matter described herein. When present, its chemical structure is not critical, since it serves primarily as a means of covalent attachment of the APJ agonist to a vehicle. The linker, e.g., may consist of a thioether, amine, imine, amide, triazole, disulfide, or a carbon-carbon bond. Thus, in various embodiments, e.g. a thioether can tether the vehicle to APJ agonist.

The "linker" or "linker moiety" can be a biologically acceptable peptidyl or non-peptidyl organic group that is covalently bound to an amino acid residue of an APJ agonist or other polypeptide chain (e.g., an immunoglobulin HC or LC or immunoglobulin Fc domain) contained in a composition, which linker moiety covalently joins or conjugates the peptide analog or other polypeptide chain to another peptide or polypeptide chain in the composition, or to a half-life extending moiety. In some embodiments, a half-life extending moiety, as described herein, is conjugated, i.e., covalently bound directly to an amino acid residue of the APJ agonist itself, or optionally, to a peptidyl or non-peptidyl linker moiety (including but not limited to aromatic or aryl linkers) that is covalently bound to an amino acid residue of the peptide analog. The presence of any linker moiety is optional. When present, its chemical structure is not critical, since it serves primarily as a spacer to position, join, connect, or optimize presentation or position of one functional moiety in relation to one or more other functional moieties of a molecule in various embodiments.

The presence of a linker moiety can be useful in optimizing pharamcologial activity of some embodiments. The linker may be made up of amino acids linked together by peptide bonds. The linker moiety, if present, can be independently the same or different from any other linker, or linkers, that may be present in the composition. As stated above, the linker moiety, if present (whether within the primary amino acid sequence of the peptide analog, or as a linker for attaching a half-life extending moiety to the peptide analog), can be "peptidyl" in nature (i.e., made up of amino acids linked together by peptide bonds) and made up in length, preferably, of from 1 up to about 40 amino acid residues, of from 1 up to about 20 amino acid residues, or from 1 to about 10 amino acid residues. The amino acid residues in the linker are from among the twenty canonical amino acids, e.g., cysteine, glycine, alanine, proline, asparagine, glutamine, and /or serine. In various embodiments, a peptidyl linker can be made up of a majority of amino acids that are sterically unhindered, such as glycine, serine, and alanine linked by a peptide bond. It may also be desirable that, if present, a peptidyl linker be selected that avoids rapid proteolytic turnover in circulation *in vivo.* Some of these amino acids may be glycosylated, as is well understood by those in the art. For example, a useful linker sequence constituting a sialylation site is X₁X₂NX₄X₅G (SEQ ID NO: 76), wherein X₁, X₂, X₄ and X₅ are each independently any amino acid residue. It may also be desirable that, if present, a peptidyl linker can consist of any non-canonical amino acids or a combination of non-canonical and canonical amino acids selected to avoid or reduce rapid proteolytic turnover in vitro and/or *in vivo.*

In other embodiments, the 1 to 40 amino acids of the peptidyl linker moiety can be selected from glycine, alanine, proline, asparagine, glutamine, and lysine. A linker can be made up of a majority of amino acids that are sterically unhindered, such as glycine and alanine. Thus, linkers can include polyglycines, polyserines, and polyalanines, or combinations of any of these. Some exemplary peptidyl linkers are poly(Gly)₁₋₈ (SEQ ID NO: 77), particularly (Gly)₃ (SEQ ID NO: 78), (Gly)₄ (SEQ ID NO: 79), (Gly)₅ (SEQ ID NO: 80) and (Gly)₇ (SEQ ID NO: 81), as well as, GlySer and poly(Gly)₄Ser (SEQ ID NO: 82), such as "L15" (GGGGSGGGGSGGGGS; SEQ ID NO: 83), poly(Gly-Ala)₂₋₄ (SEQ ID NO: 62) and poly(Ala)₁₋₈ (SEQ ID NO: 84). Other specific examples of peptidyl linkers include (Gly)₅Lys (SEQ ID NO: 85), and (Gly)₅LysArg (SEQ ID NO: 86). Other examples of useful peptidyl linkers are: Other examples of useful peptidyl linkers are:
(Gly)₃Lys(Gly)₄ (SEQ ID NO: 87);
(Gly)₃AsnGlySer(Gly)₂ (SEQ ID NO: 88);
(Gly)₃Cys(Gly)₄ (SEQ ID NO: 89); and
GlyProAsnGlyGly (SEQ ID NO: 90).

To explain the above nomenclature, for example, (Gly)₃Lys(Gly)₄ (SEQ ID NO: 91) means Gly-Gly-Gly-Lys-Gly-Gly-Gly-Gly (SEQ ID NO: 91). Other combinations of Gly and Ala are also useful.

Other linkers are those identified herein as "L5" (GGGGS; or "G₄S"; SEQ ID NO: 92), "L10" (GGGGSGGGGS; SEQ ID NO: 93); "L20" (GGGGSGGGGSGGGGSGGGGS; SEQ ID NO: 94); "L25" (GGGGSGGGGSGGGGSGGGGSGGGGS; SEQ ID NO: 95) and any linkers used in the working examples hereinafter.

In some embodiments which comprise a peptide linker moiety, acidic residues, for example, glutamate or aspartate residues are placed in the amino acid sequence of the linker moiety. Examples include the following peptide linker sequences:
GGEGGG (SEQ ID NO: 96);
GGEEEGGG (SEQ ID NO: 97);
GEEEG (SEQ ID NO: 98);
GEEE (SEQ ID NO: 99);
GGDGGG (SEQ ID NO: 100);
GGDDDGG (SEQ ID NO: 101);
GDDDG (SEQ ID NO: 102);
GDDD (SEQ ID NO: 103);
GGGGSDDSDEGSDGEDGGGGS (SEQ ID NO: 1412);
WEWEW (SEQ ID NO: 104);
FEFEF (SEQ ID NO: 105);
EEEWWW (SEQ ID NO: 106);
EEEFFF (SEQ ID NO: 107);
WWEEEWW (SEQ ID NO: 108); or
FFEEEFF (SEQ ID NO: 109).

In other embodiments, the linker constitutes a phosphorylation site, e.g., X₁X₂YX₄X₅G (SEQ ID NO: 110), wherein X₁, X₂, X₄, and X₅ are each independently any amino acid residue; X₁X₂SX₄X₅G (SEQ ID NO: 111), wherein X₁, X₂, X₄ and X₅ are each independently any amino acid residue; or X₁X₂TX₄X₅G (SEQ ID NO: 112), wherein X₁, X₂, X₄ and X₅ are each independently any amino acid residue.

The linkers shown here are exemplary; peptidyl linkers may be much longer and may include other residues. A peptidyl linker can contain, e.g., a cysteine, another thiol, or nucleophile for conjugation with a half-life extending moiety. In another embodiment, the linker can contain a cysteine or homocysteine residue, or other 2-amino-ethanethiol or 3-amino-propanethiol moiety for conjugation to maleimide, iodoacetaamide or thioester, functionalized half-life extending moiety.

Another useful peptidyl linker is a large, flexible linker comprising a random Gly/Ser/Thr sequence, for example: GSGSATGGSGSTASSGSGSATH (SEQ ID NO: 113) or HGSGSATGGSGSTASSGSGSAT (SEQ ID NO: 114), that is estimated to be about the size of a 1 kDa PEG molecule. Alternatively, a useful peptidyl linker may be comprised of amino acid sequences known in the art to form rigid helical structures (e.g., Rigid linker: - AEAAAKEAAAKEAAAKAGG (SEQ ID NO: 115). Additionally, a peptidyl linker can also comprise a non-peptidyl segment such as a 6 carbon aliphatic molecule of the formula -CH₂-CH₂-CH₂-CH₂-CH₂-CH₂-. The peptidyl linkers can be altered to form derivatives as described herein.

Optionally, a non-peptidyl linker moiety is also useful for conjugating the half-life extending moiety to the peptide portion of the half-life extending moiety-conjugated peptide. For example, alkyl linkers such as -NH-(CH₂)ₛ-C(O)-, wherein s = 2-20 can be used. These alkyl linkers may further be substituted by any non-sterically hindering group such as lower alkyl (e.g., C₁-C₆) lower acyl, halogen (e.g., Cl, Br), CN, NH₂, phenyl, *etc.* Exemplary non-peptidyl linkers are PEG linkers (e.g., shown below): 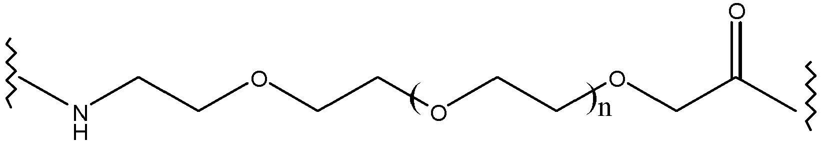 wherein n is such that the linker has a molecular weight of about 100 to about 5000 Daltons (Da).

The linker could also be a unnatural amino acid, such as aminohenxanoic acid, or an organic group, such as a dicarbohylic acid, such as succinic acid (butanedioic acid). 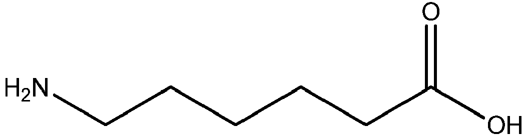 6-aminohexanoic acid 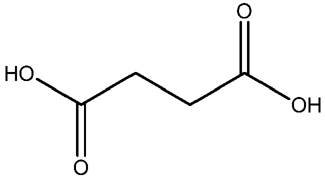 butanedioic acid

In one embodiment, the non-peptidyl linker is aryl. The linkers may be altered to form derivatives in the same manner as described herein. In addition, PEG moieties may be attached to the N-terminal amine or selected side chain amines by either reductive alkylation using PEG aldehydes or acylation using hydroxysuccinimido or carbonate esters of PEG, or by thiol conjugation.

"Aryl" is phenyl or phenyl vicinally-fused with a saturated, partially-saturated, or unsaturated 3-, 4-, or 5 membered carbon bridge, the phenyl or bridge being substituted by 0, 1, 2 or 3 substituents selected from C₁₋₈ alkyl, C₁₋₄ haloalkyl or halo. "Heteroaryl" is an unsaturated 5, 6 or 7 membered monocyclic or partially-saturated or unsaturated 6-, 7-, 8-, 9-, 10- or 11 membered bicyclic ring, wherein at least one ring is unsaturated, the monocyclic and the bicyclic rings containing 1, 2, 3 or 4 atoms selected from N, O and S, wherein the ring is substituted by 0, 1, 2 or 3 substituents selected from C₁₋₈ alkyl, C₁₋₄ haloalkyl and halo. Non-peptide portions, such as non-peptidyl linkers or non-peptide half-life extending moieties can be synthesized by conventional organic chemistry reactions.

The above is merely illustrative and not an exhaustive treatment of the kinds of linkers that can optionally be employed in accordance with various embodiments.

Various embodiments also relate to the described compositions of matter for use in a method of treating or preventing a disease, disorder, or other medical condition, for example, cardiac disorders. The cardiac disorders can comprise, for example, any disorder affecting contractility of the heart or other heart functions. Additionally, various embodiments relate to any form of heart disease, e.g. hypertrophy, hypertension, heart failure, congestive heart failure or cardiomyopathy. The heart failure can be congestive heart failure. Other embodiments concern the use of APJ agonists described herein to treat disorders of the heart resulting from hypertension including pulmonary arterial hypertension cancer, diabetes, obesity, metastatic diseases and HIV. The APJ agonists may be for use in a method of treating or preventing various other disorders or diseases, such as but not limited to disorders associated with lipid and glucose metabolism, disorders associated with regulation and function of the GI tract, protection against HIV cell entry, and protection from cell apoptosis.

Pharmaceutical compositions can be configured for administration to a patient by a wide variety of delivery routes, e.g., an intravascular delivery route such as by injection or infusion, subcutaneous ("s.c."), intravenous ("i.v."), intramuscular, intraperitoneal ("i.p."), epidural, or intrathecal delivery routes, or for oral, enteral, pulmonary (e.g., inhalant), intranasal, transmucosal (e.g., sublingual administration), transdermal or other delivery routes and/or forms of administration known in the art. Delivery of a drug or pharmaceutical composition containing and APJ agonist, may take place via standard injectable modalities, whether self-administered using prefilled syringes or in hospital setting, or also via a delivery pump such as an autoinjector, a patch pump or large volume injector to achieve the most accurate dosing and the most stable plasma exposure levels. The pharmaceutical compositions may be prepared in liquid form, or may be in dried powder form, such as lyophilized form, or crystalline form. For oral or enteral use, the pharmaceutical compositions can be configured, for example, as tablets, troches, lozenges, aqueous or oily suspensions, dispersible powders or granules, emulsions, hard or soft capsules, syrups, elixirs or enteral formulas.

In general, various embodiments provide pharmaceutical compositions comprising an APJ agonist and a pharmaceutically acceptable carrier. Such pharmaceutical compositions can be configured for administration to a patient by a wide variety of delivery routes, e.g., an intravascular delivery route such as by injection or infusion, subcutaneous, intramuscular, intraperitoneal, epidural, or intrathecal delivery routes, or for oral, enteral, pulmonary (e.g., inhalant), intranasal, transmucosal (e.g., sublingual administration), transdermal or other delivery routes and/or forms of administration known in the art. The pharmaceutical compositions may be prepared in liquid form, or may be in dried powder form, such as lyophilized form. For oral or enteral use, the pharmaceutical compositions can be configured, for example, as tablets, troches, lozenges, aqueous or oily suspensions, dispersible powders or granules, emulsions, hard or soft capsules, syrups, elixirs or enteral formulas.

Enhanced pharmaceutical properties can refer to molecules with improved pharmacokinetics, pharmacodynamics and/or improved dosing frequency for drug efficacy, e.g. not every few hours.

In the practice, the "pharmaceutically acceptable carrier" can be any physiologically tolerated substance known to those of ordinary skill in the art useful in formulating pharmaceutical compositions, including, any pharmaceutically acceptable diluents, excipients, dispersants, binders, fillers, glidants, anti-frictional agents, compression aids, tablet-disintegrating agents (disintegrants), suspending agents, lubricants, flavorants, odorants, sweeteners, permeation or penetration enhancers, preservatives, surfactants, solubilizers, emulsifiers, thickeners, adjuvants, dyes, coatings, encapsulating material(s), and/or other additives singly or in combination. Such pharmaceutical compositions can include diluents of various buffer content (*e.g.*, Tris-HCl, acetate, phosphate), pH and ionic strength; additives such as detergents and solubilizing agents (*e.g*., Tween® 80, Polysorbate 80), anti-oxidants (*e.g.,* ascorbic acid, sodium metabisulfite), preservatives (*e.g.,* Thimersol®, benzyl alcohol) and bulking substances (*e.g*., lactose, mannitol); incorporation of the material into particulate preparations of polymeric compounds such as polylactic acid, polyglycolic acid, etc. or into liposomes. Hyaluronic acid can also be used, and this can have the effect of promoting sustained duration in the circulation. Such compositions can influence the physical state, stability, rate of in vivo release, and rate of in vivo clearance of the present proteins and derivatives. See, e.g., Remington's Pharmaceutical Sciences, 18th Ed. (1990, Mack Publishing Co., Easton, PA 18042) pages 1435-1712. The compositions can be prepared in liquid form, or can be in dried powder, such as lyophilized form. Implantable sustained release formulations are also useful, as are transdermal or transmucosal formulations. Additionally (or alternatively), various embodiments provide compositions for use in any of the various slow or sustained release formulations or microparticle formulations known to the skilled artisan, for example, sustained release microparticle formulations, which can be administered via pulmonary, intranasal, or subcutaneous delivery routes. (See, e.g., Murthy et al., Injectable compositions for the controlled delivery of pharmacologically active compound, U.S. Patent No.6,887,487; Manning et al., Solubilization of pharmaceutical substances in an organic solvent and preparation of pharmaceutical powders using the same, U.S. Patent Nos. 5,770,559 and 5,981,474; Lieberman et al., Lipophilic complexes of pharmacologically active inorganic mineral acid esters of organic compounds, U.S. Patent No. 5,002,936; Gen, Formative agent of protein complex, US 2002/0119946 A1; Goldenberg et al., Sustained release formulations, WO 2005/105057 A1).

One can dilute the compositions or increase the volume of the pharmaceutical compositions with an inert material. Such diluents can include carbohydrates, especially, mannitol, α-lactose, anhydrous lactose, cellulose, sucrose, modified dextrans and starch. Certain inorganic salts may also be used as fillers, including calcium triphosphate, magnesium carbonate and sodium chloride. Some commercially available diluents are Fast-Flo, Emdex, STA-Rx 1500, Emcompress and Avicell.

A variety of conventional thickeners are useful in creams, ointments, suppository and gel configurations of the pharmaceutical composition, such as, but not limited to, alginate, xanthan gum, or petrolatum, may also be employed in various configurations of the pharmaceutical composition.

In various embodiments, liquid pharmaceutical compositions that are sterile solutions or suspensions can be administered to a patient by injection, for example, intramuscularly, intrathecally, epidurally, intravascularly (e.g., intravenously or intraarterially), intraperitoneally or subcutaneously. (See, e.g., Goldenberg et al., Suspensions for the sustained release of proteins, U.S. Patent No. 6,245,740 and WO 00/38652 A1). Sterile solutions can also be administered by intravenous infusion. The composition can be included in a sterile solid pharmaceutical composition, such as a lyophilized powder, which can be dissolved or suspended at a convenient time before administration to a patient using sterile water, saline, buffered saline or other appropriate sterile injectable medium.

Implantable sustained release formulations are also useful embodiments of the pharmaceutical compositions. For example, the pharmaceutically acceptable carrier, being a biodegradable matrix implanted within the body or under the skin of a human or non-human vertebrate, can be a hydrogel similar to those described above. Alternatively, it may be formed from a poly-alpha-amino acid component. (Sidman, Biodegradable, implantable drug delivery device, and process for preparing and using same, U.S. Patent No. 4,351,337). Other techniques for making implants for delivery of drugs are also known and useful.

In powder forms, the pharmaceutically acceptable carrier is a finely divided solid, which is in admixture with finely divided active ingredient(s), including the composition. For example, in some embodiments, a powder form is useful when the pharmaceutical composition is configured as an inhalant. (See, e.g., Zeng et al., Method of preparing dry powder inhalation compositions, WO 2004/017918; Trunk et al., Salts of the CGRP antagonist BIBN4096 and inhalable powdered medicaments containing them, U.S. Patent No. 6,900,317).

One can dilute or increase the volume of the compound with an inert material. These diluents could include carbohydrates, especially mannitol, α-lactose, anhydrous lactose, cellulose, sucrose, modified dextrans and starch. Certain inorganic salts can also be used as fillers including calcium triphosphate, magnesium carbonate and sodium chloride. Some commercially available diluents are Fast-Flo™, Emdex™, STA-Rx™ 1500, Emcompress™ and Avicell™.

Binders can be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic. An antifrictional agent can be included in the formulation of the therapeutic to prevent sticking during the formulation process. Lubricants can be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants can also be used such as sodium lauryl sulfate, magnesium lauryl sulfate, polyethylene glycol of various molecular weights, Carbowax 4000 and 6000.

Also useful in various embodiments are oral dosage forms. If necessary, the composition can be chemically modified so that oral delivery is efficacious. Generally, the chemical modification contemplated is the attachment of at least one moiety to the molecule itself, where said moiety permits (a) inhibition of proteolysis; and (b) uptake into the blood stream from the stomach or intestine. Also desired is the increase in overall stability of the compound and increase in circulation time in the body. Moieties useful as covalently attached half-life extending moieties can also be used for this purpose. Examples of such moieties include: PEG, copolymers of ethylene glycol and propylene glycol, carboxymethyl cellulose, dextran, polyvinyl alcohol, polyvinyl pyrrolidone and polyproline. See, for example, Abuchowski and Davis (1981), Soluble Polymer-Enzyme Adducts, Enzymes as Drugs (Hocenberg and Roberts, eds.), Wiley-Interscience, New York, NY, pp 367-83; Newmark, et al. (1982), J. Appl. Biochem. 4:185-9. Other polymers that could be used are poly-1,3-dioxolane and poly-1,3,6-tioxocane. Preferred for pharmaceutical usage, as indicated above, are PEG moieties.

For oral delivery dosage forms, it is also possible to use a salt of a modified aliphatic amino acid, such as sodium N-(8-[2-hydroxybenzoyl] amino) caprylate (SNAC), as a carrier to enhance absorption of the therapeutic compounds. The clinical efficacy of a heparin formulation using SNAC has been demonstrated in a Phase II trial conducted by Emisphere Technologies. See US Patent No. 5,792,451, "Oral drug delivery composition and methods."

In one embodiment, the pharmaceutically acceptable carrier can be a liquid and the pharmaceutical composition is prepared in the form of a solution, suspension, emulsion, syrup, elixir or pressurized composition. The active ingredient(s) (e.g., the composition of matter) can be dissolved, diluted or suspended in a pharmaceutically acceptable liquid carrier such as water, an organic solvent, a mixture of both, or pharmaceutically acceptable oils or fats. The liquid carrier can contain other suitable pharmaceutical additives such as detergents and/or solubilizers (e.g., Tween 80, Polysorbate 80), emulsifiers, buffers at appropriate pH (e.g., Tris-HCl, acetate, phosphate), adjuvants, anti-oxidants (e.g., ascorbic acid, sodium metabisulfite), preservatives (e.g., Thimersol, benzyl alcohol), sweeteners, flavoring agents, suspending agents, thickening agents, bulking substances (e.g., lactose, mannitol), colors, viscosity regulators, stabilizers, electrolytes, osmolutes or osmo-regulators. Additives can also be included in the formulation to enhance uptake of the composition. Additives potentially having this property are for instance the fatty acids oleic acid, linoleic acid and linolenic acid.

Also useful are oral solid dosage forms, which are described generally in Remington's Pharmaceutical Sciences (1990), supra, in Chapter 89. Solid dosage forms include tablets, capsules, pills, troches or lozenges, cachets or pellets. Also, liposomal or proteinoid encapsulation can be used to formulate the present compositions (as, for example, proteinoid microspheres reported in U.S. Patent No. 4,925,673). Liposomal encapsulation can be used and the liposomes can be derivatized with various polymers (e.g., U.S. Patent No. 5,013,556). A description of possible solid dosage forms for the therapeutic is given in Marshall, K., Modern Pharmaceutics (1979), edited by G. S. Banker and C. T. Rhodes, in Chapter 10. In general, the formulation will include the compound, and inert ingredients that allow for protection against the stomach environment, and release of the biologically active material in the intestine.

In tablet form, the active ingredient(s) are mixed with a pharmaceutically acceptable carrier having the necessary compression properties in suitable proportions and compacted in the shape and size desired.

The powders and tablets can contain up to 99% of the active ingredient(s). Suitable solid carriers include, for example, calcium phosphate, magnesium stearate, talc, sugars, lactose, dextrin, starch, gelatin, cellulose, polyvinylpyrrolidine, low melting waxes and ion exchange resins.

Controlled release formulation can be desirable. In various embodiments the composition can be incorporated into an inert matrix that permits release by either diffusion or leaching mechanisms e.g., gums. Slowly degenerating matrices can also be incorporated into the formulation, e.g., alginates, polysaccharides. Another form of a controlled release of the compositions is by a method based on the Oros™ therapeutic system (Alza Corp.), i.e., the drug is enclosed in a semipermeable membrane which allows water to enter and push drug out through a single small opening due to osmotic effects. Some enteric coatings also have a delayed release effect.

Pulmonary delivery of the compositions may also be useful. The protein (or derivative) is delivered to the lungs of a mammal while inhaling and traverses across the lung epithelial lining to the blood stream. (Other reports of this include Adjei et al., Pharma. Res. (1990) 7: 565-9; Adjei et al. (1990), Internatl. J. Pharmaceutics 63: 135-44 (leuprolide acetate); Braquet et al. (1989), J. Cardiovasc. Pharmacol. 13 (suppl.5): s. 143-146 (endothelin-1); Hubbard et al. (1989), Annals Int. Med. 3: 206-12 (α1-antitrypsin); Smith et al. (1989), J. Clin. Invest. 84: 1145-6 (α1-proteinase); Oswein et al. (March 1990), "Aerosolization of Proteins," Proc. Symp. Resp. Drug Delivery II, Keystone, Colorado (recombinant human growth hormone); Debs et al. (1988), J. Immunol. 140: 3482-8 (interferon-y and tumor necrosis factor α) and Platz et al., U.S. Patent No. 5,284,656 (granulocyte colony stimulating factor).

The dosage regimen involved in a method for treating a condition will be determined by the attending physician, considering various factors which modify the action of drugs, e.g. the age, condition, body weight, sex and diet of the patient, the severity of any infection, time of administration and other clinical factors. Generally, the daily regimen should be in the range of 0.1-1000 micrograms of the compound per kilogram of body weight, preferably 0.1-150 micrograms per kilogram.

Table 5 contains SEQ ID NOs: 139-161. In the table, a free acid can be a carboxylic acid and an amide can be an amidated C-terminus.

**TABLE 5.**

| AMINO ACID SEQUENCE | DESIGNATION | SEQ ID NO. |
|---|---|---|
| {Acetyl}[Atz(PEG10)]GGLRPRLSHKGPMPF{Free Acid} | Ac-Atz(PEG10)-Gly-Gly-[Leu65]Apelin-13(65-77) | 139 |
| {Acetyl}[Atz(PEG10)]GLRPRLSHKGPMPF{Free Acid} | Ac-Atz(PEG 10)-Gly-[Leu65]Apelin-13(65-77) | 140 |
| {Acetyl}[Atz(PEG10)]LRPRLSHKGPMPF{Free Acid} | Ac-Atz(PEG 10)-[Leu65]Apelin-13(65-77) | 141 |
| {Acetyl}[Atz(PEG10)][Atz(PEG10)]PRLSHKGPMPF {FreeAcid} | Ac-[Atz(PEG10)65,66]Apelin-13(65-77) | 142 |
| {Hydrogen}[PE][Atz(PEG10)]PRLSHKGPMPF{Free Acid} | [PE65;Atz(PEG10)66]Apel in-13(65-77) | 143 |
| {Hydrogen}[PE]R[Atz(PEG10)]RLSHKGPMPF{Free Acid} | [PE65;Atz(PEG10)67]Apel in-13(65-77) | 144 |
| {Hydrogen}[PE]RP[Atz(PEG10)]LSHKGPMPF{Free Acid} | [PE65;Atz(PEG10)68]Apel in-13(65-77) | 145 |
| {Hydrogen} [PE]RPR[Atz(PEG10)]SHKGPMPF {Free Acid} | [PE65;Atz(PEG10)69]Apel in-13(65-77) | 146 |
| {Hydrogen} [PE]RPRL[Atz(PEG10)]HKGPMPF {Free Acid} | [PE65;Atz(PEG10)70]Apel in-13(65-77) | 147 |
| {Hydrogen}[PE]RPRLS[Atz(PEG10)]KGPMPF{Free Acid} | [PE65;Atz(PEG10)71]Apel in-13(65-77) | 148 |
| {Hydrogen}[PE]RPRLSH[Atz(PEG10)]GPMPF{Free Acid} | [PE65;Atz(PEG10)72]Apel in-13(65-77) | 149 |
| {Hydrogen}[PE]RPRLSHK[Atz(PEG10)]PMPF{Free Acid} | [PE65;Atz(PEG10)73]Apel in-13(65-77) | 150 |
| {Hydrogen}[PE]RPRLSHKG[Atz(PEG10)]MPF{Free Acid} | [PE65;Atz(PEG10)74]Apel in-13(65-77) | 151 |
| {Hydrogen}[PE]RPRLSHKGP[Atz(PEG10)]PF{Free Acid} | [PE65;Atz(PEG10)75]Apel in-13(65-77) | 152 |
| {Hydrogen} [PE]RPRLSHKGPM[Atz(PEG10)]F {Free Acid} | [PE65;Atz(PEG10)76]Apel in-13(65-77) | 153 |
| {Hydrogen}[PE]RPRLSHKGPMP[Atz(PEG10)]{Ami de} | [PE65;Atz(PEG10)77]Apel in-13(65-77)-amide | 154 |
| {Hydrogen}[PE]RPRLSHKGPMPF[Atz(PEG10)]{Am ide} | [PE65]Apelin-13(65-77)-Atz(PEG10)-amide | 155 |
| {Hydrogen} [PE]RPRLSHKGPMPFG[Atz(PEG10)] {A mide} | [PE65]Apelin-13(65-77)-Gly-Atz(PEG10)-amide | 156 |
| {Hydrogen}[PE]RPRLSHKGPMPFGG[Atz(PEG10)]{ Amide} | [PE65]Apelin-13(65-77)-Gly-Gly-Atz(PEG10)-amide | 157 |
| {Hydrogen} [Atz(PEG10)]LVQPRGSRNGPGPWQGG RRKFRRQRPRLSHKGP[Nle]PF{FreeAcid} | [Atz(PEG10)41;Nle75]Ape lin-36(41-77) | 158 |
| {Hydrogen} [Atz(PEG10)]PGPWQGGRRKFRRQRPR LSHKGP[Nle]PF{FreeAcid} | [Atz(PEG10)51;N1e75]Ape lin-26(51-77) | 159 |
| {Hydrogen} [Atz(PEG10)]KFRRQRPRLSHKGP[Nle] PF{FreeAcid} | [Atz(PEG10)60;Nle75]Ape lin-17(60-77) | 160 |
| {Hydrogen} [Atz(PEG10)]QRPRLSHKGP [Nle]PF {FreeAcid} | Atz(PEG10)-[Nle75]Apelin-13(65-77) | 161 |

Table 6 is shown below. In the table a free acid can be a carboxylic acid and an amide can be an amidated C-terminus

**TABLE 6.**

| AMINO ACID SEQUENCE | DESIGNATION | SEQ ID NO. |
|---|---|---|
| {Hydrogen}[PE]RP[hArg][NMeLeu]S HKGP[pI-Phe]P[4-F-F]{FreeAcid} | [PE65;hArg68;NMeLeu69;pI-Phe75;4-F-F77]Apelin-13(65-77) | 162 |
| {Hydrogen} [PE]RP[hArg][Cha]SHKG P[pI-Phe]P[4-F-F]{FreeAcid} | [PE65;hArg68;Cha69;pI-Phe75;4-F-F77]Apelin-13(65-77) | 163 |
| {Hydrogen}[PE]RP[hArg][NMeLeu]S HKGP[pI-Phe]P[4-Me-F]{FreeAcid} | [PE65;hArg68;NMeLeu69;pI-Phe75;4-Me-F77]Apelin-13(65-77) | 164 |
| {Hydrogen} [PE]RP[hArg][Cha]SHKG P[pI-Phe]P[4-Me-F]{FreeAcid} | [PE65;hArg68;Cha69;pI-Phe75;4-Me-F77]Apelin-13(65-77) | 165 |
| {Hydrogen} [PE]RP[hArg][Cha]SHKG P[Nle]P[4-Me-F]{FreeAcid} | [PE65;hArg68;Cha69;Nle75;4-Me-F77]Apelin-13(65-77) | 166 |
| {Hydrogen}[PE]RP[hArg][Cha]SHKG P[Nle]P[D-Bip]{FreeAcid} | [PE65;hArg68;Cha69;Nle75;D-Bip77]Apelin-13(65-77) | 167 |
| {Hydrogen}[PE]RP[hArg][NMeLeu]S HKGP[Nle]P[D-1Nal]{FreeAcid} | [PE65;hArg68;NMeLeu69;Nle75;D-1Nal77]Apelin-13(65-77) | 168 |
| {Hydrogen}[PE]RP[hArg][NMeLeu]S HKGP[pI-Phe]P[D-1Nal]{FreeAcid} | [PE65;hArg68;NMeLeu69;pI-Phe75;D-1Nal77]Apelin-13(65-77) | 169 |
| {Hydrogen} [PE]RP[hArg][Cha]SHKG P[pI-Phe]P[D-1Nal]{FreeAcid} | [PE65;hArg68;Cha69;pI-Phe75;D-1Nal77]Apelin-13(65-77) | 170 |
| {Hydrogen} [PE]RP[hArg][Cha]SHKG P[Nle]P[D-1Nal]{FreeAcid} | [PE65;hArg68;Cha69;Nle75;D-1Nal77]Apelin-13(65-77) | 171 |
| {Hydrogen}[PE]RP[hArg][Cha]SHKG P[pI-Phe]P[pI-Phe]{FreeAcid} | [PE65;hArg68;Cha69;pI-Phe75,77]Apelin-13(65-77) | 172 |
| {Hydrogen}[PE]RP[hArg][Cha]SHKG P[pI-Phe]PY{FreeAcid} | [PE65;hArg68;Cha69;pI-Phe75;Tyr77]Apelin-13(65-77) | 173 |
| {Hydrogen}[PE]RP[hArg][Cha]SHKG P[Nle]P[pI-Phe]{FreeAcid} | [PE65;hArg68;Cha69;Nle75;pI-Phe77]Apelin-13(65-77) | 174 |
| {Hydrogen}[PE]RP[hArg][Cha]SHKG P[pI-Phe]Pf{FreeAcid} | [PE65;hArg68;Cha69;pI-Phe75;D-Phe77]Apelin-13(65-77) | 175 |
| {Hydrogen} [PE]RP[hArg][Cha]SHKG P[Nle]Pf{FreeAcid} | [PE65;hArg68;Cha69;Nle75;D-Phe77]Apelin-13(65-77) | 176 |
| {Hydrogen}[PE]RP[hArg][NMeLeu]S HKGP[Nle]P[NMePhe]{Amide} | [PE65;hArg68;NMeLeu69;Nle75;NMe Phe77]Apelin-13(65-77)-amide | 177 |
| {Hydrogen}[PE]RP[hArg][NMeLeu]S HKGP[Nle]Pw{FreeAcid} | [PE65;hArg68;NMeLeu69;Nle75;D-Trp77]Apelin-1 3(65-77) | 178 |
| {Hydrogen}[PE]RP[hArg][NMeLeu]S HKGP[pI-Phe]Pw{FreeAcid} | [PE65;hArg68;NMeLeu69;pI-Phe75;D-Trp77]Apelin-13(65-77) | 179 |
| {Hydrogen}[PE]RP[hArg][Cha]SHKG P[pI-Phe]Pw{FreeAcid} | [PE65;hArg68;Cha69;pI-Phe75;D-Trp77]Apelin-13(65-77) | 180 |
| {Hydrogen} [PE]RP[hArg][Cha]SHKG P[Nle]Pw{FreeAcid} | [PE65;hArg68;Cha69;Nle75;D-Trp77]Apelin-13(65-77) | 181 |
| {Hydrogen} [PE]RP[hArg][Cha]SHKG P[Nle]PY{FreeAcid} | [PE65;hArg68;Cha69;Nle75;Tyr77]Ape lin-13(65-77) | 182 |
| {Hydrogen}[PE]RP[hArg][NMeLeu]S HKGP[Nle]Py{FreeAcid} | [PE65;hArg68;NMeLeu69;Nle75;D-Tyr77]Apelin-13 (65-77) | 183 |
| {Hydrogen}[PE]RP[hArg][NMeLeu]S HKGP[pI-Phe]Py{FreeAcid} | [PE65;hArg68;NMeLeu69;pI-Phe75;D-Tyr77]Apelin-13(65-77) | 184 |
| {Hydrogen}[PE]RP[hArg][Cha]SHKG P[pI-Phe]Py{FreeAcid} | [PE65;hArg68;Cha69;pI-Phe75;D-Tyr77]Apelin-13 (65-77) | 185 |
| {Hydrogen} [PE]RP[hArg][Cha]SHKG P[Nle]Py{FreeAcid} | [PE65;hArg68;Cha69;Nle75;D-Tyr77]Apelin-13 (65-77) | 186 |
| {Hydrogen}[PE]RP[hArg][NMeLeu]F HKGP[pI-Phe]P[2-Nal]{FreeAcid} | [PE65;hArg68;NMeLeu69;Phe70;pI-Phe75;2-Nal77]Apelin-13(65-77) | 187 |
| {Hydrogen}[PE]RP[hArg][NMeLeu]F HKGP[Nle]P[D-Bip]{Amide} | [PE65;hArg68;NMeLeu69;Phe70;Nle75 ;D-Bip77]Apelin-13(65-77)-amide | 188 |
| {Hydrogen}[PE]RP[hArg][NMeLeu]F HKGP[Nle]P[D-Bip]{FreeAcid} | [PE65;hArg68;NMeLeu69;Phe70;Nle75 ;D-Bip77]Apelin-13(65-77) | 189 |
| {Hydrogen}[PE]RP[hArg][NMeLeu]F HKGP[pI-Phe]P[D-Bip]{FreeAcid} | [PE65;hArg68;NMeLeu69;Phe70;pI-Phe75;D-Bip77]Apelin-13(65-77) | 190 |
| {Palmitoyl}LRP[hArg][NMeLeu]FHK GP[Nle]P[D-Bip]{FreeAcid} | Palmitoyl-[Leu65;hArg68;NMeLeu69;Phe70;Nle7 5;D-Bip77]Apelin-13(65-77) | 191 |
| {Hydrogen}[PE]RP[hArg][Cha]SHKG P[pI-Phe]P[D-Bip]{FreeAcid} | [PE65;hArg68;Cha69;pI-Phe75;D-Bip77]Apelin-13(65-77) | 192 |
| {Hydrogen}[PE]RP[hArg][NMeLeu]S HKGP[Nle]P[4-Me-F]{FreeAcid} | [PE65;hArg68;NMeLeu69;Nle75;4-Me-F77]Apelin-13(65-77) | 193 |
| {Hydrogen}[PE]RP[hArg][NMeLeu]F HKGP[pI-Phe]P[D-Bip]{Amide} | [PE65;hArg68;NMeLeu69;Phe70;pI-Phe75;D-Bip77]Apelin-13(65-77)-amide | 194 |
| {Hydrogen}[PE]RP[hArg][Cha]SHKG P[Nle]Pf{Amide} | [PE65;hArg68;Cha69;Nle75;D-Phe77]Apelin-13(65-77)-amide | 195 |
| {Hydrogen}[PE]RP[hArg][NMeLeu]S HKGP[Nle]P[4-Cl-F]{Amide} | [PE65;hArg68;NMeLeu69;Nle75;4-Cl-F77]Apelin-13(65-77)-amide | 200 |
| {Hydrogen}[PE]RP[hArg][NMeLeu]S HKGP[Nle]P[4-Me-F]{Amide} | [PE65;hArg68;NMeLeu69;Nle75;4-Me-F77]Apelin-13(65-77)-amide | 201 |
| {Hydrogen}[PE]RP[hArg][NMeLeu]S HKGP[Nle]P[Bip]{Amide} | [PE65;hArg68;NMeLeu69;Nle75;Bip77 ]Apelin-13(65-77)-amide | 202 |
| {Hydrogen}[PE]RP[hArg][NMeLeu]S HKGP[Nle]P[D-Bip]{Amide} | [PE65;hArg68;NMeLeu69;Nle75;D-Bip77]Apelin-13(65-77)-amide | 203 |
| {Hydrogen}[PE]RP[hArg][NMeLeu]S HKGP[Nle]P[4-NO2-F]{Amide} | [PE65;hArg68;NMeLeu69;Nle75;4-NO2-F77]Apelin-13(65-77)-amide | 204 |
| {Hydrogen}[PE]RP[hArg][NMeLeu]S HKG[1-Nal][Nle]P[4-Cl-F]{Amide} | [PE65;hArg68;NMeLeu69;1-Nal74;Nle75;4-Cl-F77]Apelin-13(65-77)-amide | 205 |
| {Hydrogen}[PE]RP[hArg][NMeLeu]F HKG[1-Nal][Nle]P[4-Cl-F]{Amide} | [PE65;hArg68;NMeLeu69;Phe70;1-Nal74;Nle75;4-Cl-F77]Apelin-13(65-77)-amide | 206 |
| {Hydrogen}[PE]RP[hArg][NMeLeu]F HKGP[Nle]P[4-Cl-F]{Amide} | [PE65;hArg68;NMeLeu69;Phe70;Nle75 ;4-Cl-F77]Apelin-13(65-77)-amide | 207 |
| {Hydrogen}[PE]RP[hArg][NMeLeu]S AKG[1-Nal][Nle]P[4-Cl-F]{Amide} | [PE65;hArg68;NMeLeu69;Ala71;1-Nal74;Nle75;4-Cl-F77]Apelin-13(65-77)-amide | 208 |
| {Hydrogen}[PE]RP[hArg][NMeLeu]F AKG[1-Nal][Nle]P[4-Cl-F]{Amide} | [PE65;hArg68;NMeLeu69;Phe70;Ala71 ;1-Nal74;Nle75;4-Cl-F77]Apelin-13(65-77)-amide | 209 |
| {Hydrogen}[PE]RP[hArg][NMeLeu]F AKGP[Nle]P[4-Cl-F]{Amide} | [PE65;hArg68;NMeLeu69;Phe70;Ala71 ;Nle75;4-Cl-F77]Apelin-13(65-77)-amide | 210 |
| {Hydrogen}[PE]RP[hArg][NMeLeu]S[ 1-Nal]KG[1-Nal][Nle]P[4-Cl-F]{Amide} | [PE65;hArg68;NMeLeu69;1-Nal71,74;Nle75;4-Cl-F77]Apelin-13(65-77)-amide | 211 |
| {Hydrogen}[PE]RP[hArg][NMeLeu]F[ 1-Nal]KG[1-Nal][Nle]P[4-Cl-F]{Amide} | [PE65;hArg68;NMeLeu69;Phe70;1-Nal71,74;Nle75;4-Cl-F77]Apelin-13(65-77)-amide | 212 |
| {Hydrogen}[PE]RP[hArg][NMeLeu]F[ 1-Nal]KGP[Nle]P[4-Cl-F] {Amide} | [PE65;hArg68;NMeLeu69;Phe70;1-Nal71;Nle75;4-Cl-F77]Apelin-13(65-77)-amide | 213 |
| {Hydrogen}[PE]RP[hArg][NMeLeu]F[ 1-Nal]KGP[Nle]P[4-Cl-F] {Amide} | [PE65;hArg68;NMeLeu69;Phe70;1-Nal71;Nle75;4-Cl-F77]Apelin-13(65-77)-amide | 214 |
| {Hydrogen}[PE]RP[hArg][NMeLeu][N MePhe][1-Nal]KGP[Nle]P[4-Cl-F]{Amide} | [PE65;hArg68;NMeLeu69;NMePhe70; 1-Nal71;Nle75;4-Cl-F77]Apelin-13(65-77)-amide | 215 |
| {Hydrogen}[PE]RP[hArg][NMeLeu][N MePhe]AKG[ 1-Nal][Nle]P[4-Cl-F]{Amide} | [PE65;hArg68;NMeLeu69;NMePhe70; Ala71;1-Nal74;Nle75;4-Cl-F77]Apelin-13(65-77)-amide | 216 |
| {Hydrogen}[PE]RP[hArg][NMeLeu][N MePhe]AKG[ 1-Nal][Nle]P[4-Cl-F]{Amide} | [PE65;hArg68;NMeLeu69;NMePhe70; Ala71;1-Nal74;Nle75;4-Cl-F77]Apelin-13(65-77)-amide | 217 |
| {Hydrogen}[PE]RP[hArg][NMeLeu]F[ 2-Nal]KG[1-Nal][Nle]P[4-Cl-F]{Amide} | [PE65;hArg68;NMeLeu69;Phe70;2-Nal71;1-Nal74;Nle75;4-Cl-F77]Apelin-13(65-77)-amide | 218 |
| {Hydrogen}[PE]RP[hArg][NMeLeu]F[ 2-Nal]KGP[Nle]P[4-Cl-F]{Amide} | [PE65;hArg68;NMeLeu69;Phe70;2-Nal71;Nle75;4-Cl-F77]Apelin-13(65-77)-amide | 219 |
| {Hydrogen}[PE]RP[hArg][NMeLeu][N MePhe][2-Nal]KGP[Nle]P[4-Cl-F]{Amide} | [PE65;hArg68;NMeLeu69;NMePhe70; 2-Nal71;Nle75;4-Cl-F77]Apelin-13(65-77)-amide | 220 |
| [PE]RP[hArg][NMeLeu]SHKGP[Nle]P [D-Bip] | [PE65;hArg68;NMeLeu69;Nle75;D-Bip77]Apelin-13(65-77) | 221 |
| [PE]RP[hArg][NMeLeu]SHKGP[pI-Phe]P[D-Bip] | [PE65;hArg68;NMeLeu69;pI-Phe75;D-Bip77]Apelin-13(65-77) | 222 |
| [PE]RP[hArg][NMeLeu]SHKGP[pI-Phe]P[2-Nal] | [PE65;hArg68;NMeLeu69;pI-Phe75;2-Nal77]Apelin-13(65-77) | 223 |
| {Palmitoyl}LRP[hArg][NMeLeu]SHK GP[pI-Phe]P[D-Bip] | Palmitoyl-Leu65;hArg68;NMeLeu69;pI-Phe75;D-Bip77]Apelin-13(65-77) | 224 |
| [PE]RP[hArg][NMeLeu]SHKGP[Nle]P [D-Bip]K{Amide} | PE65;hArg68;NMeLeu69;Nle75;D-Bip77;K(Palmitoyl)78]Apelin-13(65-78)-amide | 225 |
| [PE]RP[hArg][NMeLeu]SHKGP[Nle]P [2-Nal] | [PE65;hArg68;NMeLeu69;Nle75;2-Nal77]Apelin-13(65-77) | 226 |
| [PE]RP[hArg][NMeLeu]SHKGP[pI-Phe]P[4-Cl-F] | [PE65;hArg68;NMeLeu69;pI-Phe75;4-Cl-F77]Apelin-13(65-77) | 227 |
| [PE]RP[hArg][NMeLeu]SHKGP[Nle]P [4-Cl-F] | [PE65;hArg68;NMeLeu69;Nle75;4-Cl-F77]Apelin-13(65-77) | 228 |
| [PE]RP[hArg][NMeLeu]SHKGP[Nle]P [4-F-F] | [PE65;hArg68;NMeLeu69;Nle75;4-F-F77]Apelin-13(65-77) | 229 |
| [PE]RP[hArg][Cha]SHKGP[Nle]P[4-F-F] | [PE65;hArg68;Cha69;Nle75;4-F-F77]Apelin-13(65-77) | 230 |
| [PE]RP[hArg][NMeLeu]SHKGP[Nle]P [pI-Phe] | [PE65;hArg68;NMeLeu69;Nle75;pI-Phe77]Apelin-13(65-77) | 231 |
| [PE]RP[hArg][NMeLeu]SHKGP[Nle]P f | [PE65;hArg68;NMeLeu69;Nle75;D-Phe77]Apelin-13(65-77) | 232 |
| {Palmitoyl}LRP[hArg][NMeLeu]SHK GP[pI-Phe]P[D-Bip] | Palmitoyl-Leu65;hArg68;NMeLeu69;pI-Phe75;D-Bip77]Apelin-13(65-77) | 233 |
| [PE]RP[hArg][NMeLeu]SHKGP[Nle]P [D-Bip]K{Amide} | PE65;hArg68;NMeLeu69;Nle75;D-Bip77;K(Palmitoyl)78]Apelin-13(65-78)-amide | 234 |
| [PE]RP[hArg][Cha]SHKGP[pI-Phe]P[2-Nal] | [PE65;hArg68;Cha69;pI-Phe75;2-Nal77]Apelin-13(65-77) | 235 |
| {Acetyl}[Pra]LRP[hArg][NMeLeu][Pra ]HKGP[Nle]P[4-Cl-F]{FreeAcid} | Ac-Pra-[Leu65;hArg68;NMeLeu69;Pra70;Nle7 5;4-Cl-F77]Apelin-13(65-77) | 236 |
| {Acetyl}[Pra]LRP[hArg][NMeLeu][Pra ]HKGP[Nle]P[4-Cl-F]{FreeAcid} | Ac-Pra-[Leu65;hArg68;NMeLeu69;Pra70;Nle7 5;4-Cl-F77]Apelin-13(65-77) | 237 |
| {Acetyl}[Pra[NMeArg][NMeLeu] [Pra]HKGP[Nle]P[4-Cl-F]{FreeAcid} | Ac-Pra-[Leu65;NMeArg68;NMeLeu69;Pra70;N le75;4-Cl-F77]Apelin-13(65-77) | 238 |
| {Acetyl}[Pra]L[NMeArg]P[hArg][NM eLeu][Pra]HKGP[Nle]P[4-Cl-F]{FreeAcid} | Ac-Pra-[Leu65;NMeArg66;hArg68;NMeLeu69; Pra70;Nle75 ;4-Cl-F77]Apelin-13(65-77) | 239 |
| {Acetyl}[Pra]L[NMeArg]P[NMeArg][ NMeLeu][Pra]HKGP[Nle]P[4-Cl-F]{FreeAcid} | Ac-Pra-[Leu65;NMeArg66,68;NMeLeu69;Pra7 0;Nle75;4-Cl-F77]Apelin-13(65-77) | 240 |
| [PE]RP[hArg][NMeLeu][Pra]HKG[Oic ][Nle]P[4-Cl-F]{FreeAcid} | [PE65;hArg68;NMeLeu69;Pra70;Oic74 ;Nle75;4-Cl-F77]Apelin-13(65-77) | 241 |
| [PE]RP[hArg][NMeLeu][Pra]HKG[Oic ][Nle]P[4-Cl-F]{FreeAcid} | [PE65;hArg68;NMeLeu69;Pra70;Oic74 ;Nle75;4-Cl-F77]Apelin-13(65-77) | 242 |
| [PE]RP[NMeArg][NMeLeu][Pra]HKG[ Oic][Nle]P[4-Cl-F]{FreeAcid} | [PE65;NMeArg68;NMeLeu69;Pra70;Oi c74;Nle75;4-Cl-F77]Apelin-13(65-77) | 243 |
| [PE][NMeArg]P[hArg][NMeLeu][Pra] HKG[Oic][Nle]P[4-Cl-F]{FreeAcid} | [PE65;NMeArg66;hArg68;NMeLeu69; Pra70;Oic74;Nle75;4-Cl-F77]Apelin-13(65-77) | 244 |
| [PE][NMeArg]P[NMeArg][NMeLeu][P ra]HKG[Oic][Nle]P[4-Cl-F]{FreeAcid} | [PE65;NMeArg66,68;NMeLeu69;Pra70 ;Oic74;Nle75;4-Cl-F77]Apelin-13(65-77) | 245 |
| KFRRQRP[hArg] [Cha]SHKG[Oic][Nl e]P[4-Cl-F] | [hArg68;Cha69;Oic74;Nle75;4-Cl-F77]Apelin-17(61-77) | 246 |
| {MeS-Propionyl}KFRRQRP[hArg][Cha]SHK G[Oic][Nle]P[4-Cl-F] | 3-(methylthio)propionyl-[hArg68;Cha69;Oic74;Nle75;4-Cl-F77]Apelin-17(61-77) | 247 |
| [Pra]KFRRQRP[hArg][Cha]SHKG[Oic ][Nle]P[4-Cl-F] | [Pra60; hArg68;Cha69;Oic74;Nle75;4-Cl-F77]Apelin-17(60-77) | 248 |
| [Pra]PGPWQGGRRKFRRQRP[hArg][ Cha]SHKG[Oic][Nle]P[4-Cl-F] | [Pra51;hArg68;Cha69;Oic74;Nle75;4-Cl-F77]Apelin-26(51-77) | 249 |
| [Pra]LVQPRGSRNGPGPWQGGRRK FRRQRP[hArg] [Cha]SHKG[Oic][Nle] P[4-C1-F] | [Pra41;hArg68;Cha69;Oic74;Nle75;4-Cl-F77]Apelin-36(41-77) | 250 |
| [Pra]KF[hArg][hArg]QRP[hArg][Cha] SHKG[Oic][Nle]P[4-Cl-F] | Pra-[hArg63,64,68;Cha69;Oic74;Nle75;4-Cl-F77]Apelin-17(61-77) | 251 |
| [Pra]KF[hArg][hArg]Q[hArg]P[hArg][ Cha]SHKG[Oic][Nle]P[4-Cl-F] | Pra-[hArg63,64,66,68;Cha69;Oic74;Nle75;4 -Cl-F77]Apelin-17(61-77) | 252 |
| [Pra]KF[hArg][hArg]QQP[hArg][Cha] SHKG[Oic][Nle]P[4-Cl-F] | Pra-[hArg63,64,68;Gln66;Cha69;Oic74;Nle 75;4-Cl-F77]Apelin-17(61-77) | 253 |
| [Pra]KF[hArg][hArg]Q[Cit]P[hArg][Ch a]SHKG[Oic][Nle]P[4-Cl-F] | Pra-[hArg63,64,68;Cit66;Cha69;Oic74;Nle7 5;4-Cl-F77]Apelin-17(61-77) | 254 |
| [Pra]KF[hArg][Cit]Q[Cit]P[hArg][Cha] SHKG[Oic][Nle]P[4-Cl-F] | Pra-[hArg63,68;Cit64,66;Cha69;Oic74;Nle7 5;4-Cl-F77]Apelin-17(61-77) | 255 |
| [Pra]KF[Cit][Cit]Q[hArg]P[hArg][Cha] SHKG[Oic][Nle]P[4-Cl-F] | Pra-[Cit63,64;hArg66,68;Cha69;Oic74;Nle7 5;4-Cl-F77]Apelin-17(61-77) | 256 |
| [Pra]KF[Cit][Cit]Q[Cit]P[hArg][Cha]S HKG[Oic][Nle]P[4-Cl-F] | Pra-[Cit63,64,66;hArg68;Cha69;Oic74;Nle7 5;4-Cl-F77]Apelin-17(61-77) | 257 |
| [Pra]OFRRQRP[hArg][Cha]SHKG[Oic ][Nle]P[4-Cl-F] | Pra-[Orn61;hArg68;Cha69;Oic74;Nle75;4-Cl-F77]Apelin-17(61-77) | 258 |
| [Pra]OF[hArg][hArg]Q[hArg]P[hArg][ Cha]SHKG[Oic][Nle]P[4-Cl-F] | Pra-[Orn61;hArg63,64,66,68;Cha69;Oic74; Nle75;4-Cl-F77]Apelin-17(61-77) | 259 |
| [Pra]FRRQRP[hArg][Cha]SHKG[Oic][ Nle]P[4-Cl-F] | [Pra61;hArg68;Cha69;Oic74;Nle75;4-Cl-F77]Apelin-17(61-77) | 260 |
| {Bromoacetyl} [NPeg11]LRP[hArg][C ha]SHKG [Oic][Nle]P[4-Cl-F]{FreeAcid} | Bromoacetyl-NPeg11-[Leu65;hArg68;Cha69;Oic74;Nle75;4-Cl-F77]Apelin-13(65-77) | 261 |
| {Bromoacetyl}[NPeg11]KFRRQRP[h Arg][Cha]SHKG[Oic][Nle]P[4-Cl-F]{FreeAcid} | Bromoacetyl-NPegl1-[hArg68;Cha69;Oic74;Nle75;4-Cl-F77]Apelin-17(61-77) | 262 |
| {Mercaptopropionyl}KFRRQRP[hArg] [Cha]SHKG[Oic][Nle]P[4-Cl-F]{FreeAcid} | Mercaptopropionyl-[hArg68;Cha69;Oic74;Nle75;4-Cl-F77]Apelin-17(61-77) | 263 |
| {Mercaptopropionyl}FRRQRP[hArg][ Cha]SHKG[Oic][Nle]P[4-Cl-F]{FreeAcid} | Mercaptopropionyl-[hArg68;Cha69;Oic74;Nle75;4-Cl-F77]Apelin-17(62-77) | 264 |
| {Mercaptopropionyl }RRQRP[hArg][C ha]SHKG[Oic][Nle]P[4-Cl-F]{FreeAcid} | Mercaptopropionyl-[hArg68;Cha69;Oic74;Nle75;4-Cl-F77]Apelin-17(63-77) | 265 |
| {Mercaptopropionyl}RQRP[hArg][Cha ]SHKG[Oic][Nle]P[4-Cl-F]{FreeAcid} | Mercaptopropionyl-[hArg68;Cha69;Oic74;Nle75;4-Cl-F77]Apelin-17(64-77) | 266 |
| {Mercaptopropionyl}QRP[hArg][Cha] SHKG[Oic][Nle]P[4-Cl-F]{FreeAcid} | Mercaptopropionyl-[hArg68;Cha69;Oic74;Nle75;4-Cl-F77]Apelin-17(65-77) | 267 |
| {Mercaptopropionyl }LRP[hArg][Cha]S HKG[Oic][Nle]P[4-Cl-F]{FreeAcid} | Mercaptopropionyl-[Leu65;hArg68;Cha69;Oic74;Nle75;4-Cl-F77]Apelin-17(65-77) | 268 |
| {MercaptoproplOnyl}LLRP[hArg][Cha] SHKG[Oic][Nle]P[4-Cl-F]{FreeAcid} | Mercaptopropionyl-[Leu65;hArg68;Cha69;Oic74;Nle75;4-Cl-F77]Apelin-17(65-77) | 269 |
| {Pra}LRP[hArg][Cha]SHKG[Oic][Nle] P[4-Cl-F]{FreeAcid} | Pra-[Leu65;hArg68;Cha69;Oic74;Nle75;4-Cl-F77]Apelin-17(65-77) | 270 |
| [PE]RP[hArg][Cha][Pra]HKG[Oic][Nle ]P[4-Cl-F] {FreeAcid} | [PE65;hArg68;Cha69;Pra70;Oic74;Nle7 5;4-Cl-F77]Apelin-13(65-77) | 271 |
| [PE]RP[hArg][Cha]S[Pra]KG[Oic][Nle ]P[4-Cl-F] {FreeAcid} | [PE65;hArg68;Cha69;Pra71;Oic74;Nle7 5;4-Cl-F77]Apelin-13(65-77) | 272 |
| {MeS-Propionyl}KFRRLRP[hArg][Cha]SHK G[Oic][Nle]P[4-Cl-F] | 3-(methylthio)propionyl-[Leu65;hArg68;Cha69;Oic74;Nle75;4-Cl-F77]Apelin-17(61-77) | 273 |
| {MeS-Propionyl }KFRRARP[hArg][Cha]SHK G[Oic][Nle]P[4-Cl-F] | 3-(methylthio)propionyl-[Ala65;hArg68;Cha69;Oic74;Nle75;4-Cl-F77]Apelin-17(61-77) | 274 |
| {MeS-Propionyl }KFRRVRP[hArg][Cha]SHK G[Oic][Nle]P[4-Cl-F] | 3-(methylthio)propionyl-[Val65;hArg68;Cha69;Oic74;Nle75;4-Cl-F77]Apelin-17(61-77) | 275 |
| {MeS-Propionyl }KFRRIRP[hArg][Cha]SHK G[Oic][Nle]P[4-Cl-F] | 3-(methylthio)propionyl-[Ile65;hArg68;Cha69;Oic74;Nle75;4-Cl-F77]Apelin-17(61-77) | 276 |
| {MeS-Propionyl }KFRRFRP[hArg][Cha]SHK G[Oic][Nle]P[4-Cl-F] | 3-(methylthio)propionyl-[Phe65;hArg68;Cha69;Oic74;Nle75;4-Cl-F77]Apelin-17(61-77) | 277 |
| {MeS-Propionyl} KFRRYRP [hArg][Cha]SHK G[Oic][Nle]P[4-Cl-F] | 3-(methylthio)propionyl-[Tyr65;hArg68;Cha69;Oic74;Nle75;4-Cl-F77]Apelin-17(61-77) | 278 |
| {MeS-Propionyl }KFRRL[hArg]P[hArg][Cha] SHKG[Oic][Nle]P[4-Cl-F] | 3-(methylthio)propionyl-[Leu65;hArg66,68;Cha69;Oic74;Nle75; 4-Cl-F77]Apelin-17(61-77) | 279 |
| {MeS-Propionyl }KFRKLRP[hArg][Cha]SHK G[Oic][Nle]P[4-Cl-F] | 3-(methylthio)propionyl-[Lys64;Leu65;hArg68;Cha69;Oic74;Nl e75;4-Cl-F77]Apelin-17(61-77) | 280 |
| {MeS-Propionyl }KFR[Orn]LRP[hArg][Cha]S HKG[Oic][Nle]P[4-Cl-F] | 3-(methylthio)propionyl-[Orn64;Leu65;hArg68;Cha69;Oic74;Nl e75;4-Cl-F77]Apelin-17(61-77) | 281 |
| {MeS-Propionyl }KFRHLRP[hArg][Cha]SHK G[Oic][Nle]P[4-Cl-F] | 3-(methylthio)propionyl-[His64;Leu65;hArg68;Cha69;Oic74;Nle 75;4-Cl-F77]Apelin-17(61-77) | 282 |
| {MeS-Propionyl }KFRWLRP[hArg][Cha]SH KG[Oic][Nle]P[4-Cl-F] | 3-(methylthio)propionyl-[Trp64;Leu65;hArg68;Cha69;Oic74;Nle 75;4-Cl-F77]Apelin-17(61-77) | 283 |
| {MeS-Propionyl}KFRFLRP[hArg][Cha]SHK G[Oic][Nle]P[4-Cl-F] | 3-(methylthio)propionyl-[Phe64;Leu65;hArg68;Cha69;Oic74;Nl e75;4-Cl-F77]Apelin-17(61-77) | 284 |
| {MeS-Propionyl}KFRYLRP[hArg][Cha]SHK G[Oic][Nle]P[4-Cl-F] | 3-(methylthio)propionyl-[Tyr64;Leu65;hArg68;Cha69;Oic74;Nle 75;4-Cl-F77]Apelin-17(61-77) | 285 |
| {MeS-Propionyl }KFR[3Pal]LRP[hArg][Cha] SHKG[Oic][Nle]P[4-Cl-F] | 3-(methylthio)propionyl-[3Pal64;Leu65;hArg68;Cha69;Oic74;Nl e75;4-Cl-F77]Apelin-17(61-77) | 286 |
| {MeS-Propionyl }KFR[2Pal]LRP[hArg][Cha] SHKG[Oic][Nle]P[4-Cl-F] | 3-(methylthio)propionyl-[2Pal64;Leu65;hArg68;Cha69;Oic74;Nl e75;4-Cl-F77]Apelin-17(61-77) | 287 |
| {MeS-Propionyl}KFHRLRP[hArg][Cha]SHK G[Oic][Nle]P[4-Cl-F] | 3-(methylthio)propionyl-[His63;Leu65;hArg68;Cha69;Oic74;Nle 75;4-Cl-F77]Apelin-17(61-77) | 288 |
| {MeS-Propionyl}KFWRLRP[hArg][Cha]SH KG[Oic][Nle]P[4-Cl-F] | 3-(methylthio)propionyl-[Trp63;Leu65;hArg68;Cha69;Oic74;Nle 75;4-Cl-F77]Apelin-17(61-77) | 289 |
| {MeS-Propionyl }KFFRLRP[hArg][Cha]SHK G[Oic][Nle]P[4-Cl-F] | 3-(methylthio)propionyl-[Phe63;Leu65;hArg68;Cha69;Oic74;Nl e75;4-Cl-F77]Apelin-17(61-77) | 290 |
| {MeS-Propionyl }KFYRLRP[hArg][Cha]SHK G[Oic][Nle]P[4-Cl-F] | 3-(methylthio)propionyl-[Tyr63;Leu65;hArg68;Cha69;Oic74;Nle 75;4-Cl-F77]Apelin-17(61-77) | 291 |
| {MeS-Propionyl}KF[3Pal]RLRP[hArg][Cha] SHKG[Oic][Nle]P[4-Cl-F] | 3-(methylthio)propionyl-[3Pal63;Leu65;hArg68;Cha69;Oic74;Nl e75;4-Cl-F77]Apelin-17(61-77) | 292 |
| {MeS-Propionyl }KF[2Pal]RLRP[hArg][Cha] SHKG[Oic][Nle]P[4-Cl-F] | 3-(methylthio)propionyl-[2Pal63;Leu65;hArg68;Cha69;Oic74;Nl e75;4-Cl-F77]Apelin-17(61-77) | 293 |
| {MeS-Propionyl}FRKLRP[hArg][Cha]SHKG [Oic][Nle]P[4-Cl-F] | 3-(methylthio)propionyl-[Lys64;Leu65;hArg68;Cha69;Oic74;Nl e75;4-Cl-F77]Apelin-17(62-77) | 294 |
| {MeS-Propionyl }FR[Orn]LRP[hArg][Cha]SH KG[Oic][Nle]P[4-Cl-F] | 3-(methylthio)propionyl-[Orn64;Leu65;hArg68;Cha69;Oic74;Nl e75;4-Cl-F77]Apelin-17(62-77) | 295 |
| {MeS-Propionyl }FRHLRP[hArg][Cha]SHKG [Oic][Nle]P[4-Cl-F] | 3-(methylthio)propionyl-[His64;Leu65;hArg68;Cha69;Oic74;Nle 75;4-Cl-F77]Apelin-17(62-77) | 296 |
| {MeS-Propionyl }FRWLRP[hArg][Cha]SHK G[Oic][Nle]P[4-Cl-F] | 3-(methylthio)propionyl-[Trp64;Leu65;hArg68;Cha69;Oic74;Nle 75;4-Cl-F77]Apelin-17(62-77) | 297 |
| {MeS-Propionyl }FRFLRP[hArg][Cha]SHKG[ Oic][Nle]P[4-Cl-F] | 3-(methylthio)propionyl-[Phe64;Leu65;hArg68;Cha69;Oic74;Nl e75;4-Cl-F77]Apelin-17(62-77) | 298 |
| {MeS-Propionyl}FRYLRP[hArg][Cha]SHKG [Oic][Nle]P[4-Cl-F] | 3-(methylthio)propionyl-[Tyr64;Leu65;hArg68;Cha69;Oic74;Nle 75;4-Cl-F77]Apelin-17(62-77) | 299 |
| {MeS-Propionyl}FKRLRP[hArg][Cha]SHKG [Oic][Nle]P[4-Cl-F] | 3-(methylthio)propionyl-[Lys63;Leu65;hArg68;Cha69;Oic74;Nl e75;4-Cl-F77]Apelin-17(62-77) | 300 |
| {MeS-Propionyl }F[Orn]RLRP[hArg][Cha]SH KG[Oic] [Nle]P[4-Cl-F] | 3-(methylthio)propionyl-[Orn63;Leu65;hArg68;Cha69;Oic74;Nl e75;4-Cl-F77]Apelin-17(62-77) | 301 |
| {MeS-Propionyl }FHRLRP[hArg][Cha]SHKG [Oic][Nle]P[4-Cl-F] | 3-(methylthio)propionyl-[His63;Leu65;hArg68;Cha69;Oic74;Nle 75;4-Cl-F77]Apelin-17(62-77) | 302 |
| {MeS-Propionyl }FWRLRP[hArg][Cha]SHK G[Oic][Nle]P[4-Cl-F] | 3-(methylthio)propionyl-[Trp63;Leu65;hArg68;Cha69;Oic74;Nle 75;4-Cl-F77]Apelin-17(62-77) | 303 |
| {MeS-Propionyl }FFRLRP[hArg][Cha]SHKG[ Oic][Nle]P[4-Cl-F] | 3-(methylthio)propionyl-[Phe63;Leu65;hArg68;Cha69;Oic74;Nl e75;4-Cl-F77]Apelin-17(62-77) | 304 |
| {MeS-Propionyl }FYRLRP[hArg][Cha]SHKG [Oic][Nle]P[4-Cl-F] | 3-(methylthio)propionyl-[Tyr63;Leu65;hArg68;Cha69;Oic74;Nle 75;4-Cl-F77]Apelin-17(62-77) | 305 |
| {MeS-Propionyl }F[3Pal]RLRP[hArg][Cha]S HKG[Oic][Nle]P[4-Cl-F] | 3-(methylthio)propionyl-[3Pal63;Leu65;hArg68;Cha69;Oic74;Nl e75;4-Cl-F77]Apelin-17(62-77) | 306 |
| {MeS-Propionyl}F[2Pal]RLRP[hArg][Cha]S HKG[Oic][Nle]P[4-Cl-F] | 3-(methylthio)propionyl-[2Pal63;Leu65;hArg68;Cha69;Oic74;Nl e75;4-Cl-F77]Apelin-17(62-77) | 307 |
| {MeS-Propionyl }KFRRL[hArg]P[hArg][Cha] SHKG[Oic][Nle]P[4-Cl-F] | 3-(methylthio)propionyl-[Leu65;hArg66,68;Cha69;Oic74;Nle75; 4-Cl-F77]Apelin-17(61-77) | 308 |
| {MeS-Propionyl }FQQL[hArg]P[hArg][Cha]S HKG[Oic][Nle]P[4-Cl-F] | 3-(methylthio)propionyl-[Gln63,64;Leu65;hArg66,68;Cha69;Oic 74;Nle75;4-Cl-F77]Apelin-17(62-77) | 309 |
| {MeS-Propionyl}KF[Cit][Cit]L[hArg]P[hArg] [Cha]SHKG[Oic][Nle]P[4-Cl-F] | 3-(methylthio)propionyl-[Cit63,64;Leu65;hArg66,68;Cha69;Oic 74;Nle75;4-Cl-F77]Apelin-17(61-77) | 310 |
| {MeS-Propionyl}F[Cit][Cit]L[hArg]P[hArg][ Cha]SHKG[Oic][Nle]P[4-Cl-F] | 3-(methylthio)propionyl-[Cit63,64;Leu65;hArg66,68;Cha69;Oic 74;Nle75;4-Cl-F77]Apelin-17(62-77) | 311 |
| {MeS-Propionyl}KFARL[hArg]P[hArg][Cha] SHKG[Oic][Nle]P[4-Cl-F] | 3-(methylthio)propionyl-[Ala63;Leu65;hArg66,68;Cha69;Oic74; Nle75;4-Cl-F77]Apelin-17(61-77) | 312 |
| {MeS-Propionyl }KFERL[hArg]P[hArg][Cha] SHKG[Oic][Nle]P[4-Cl-F] | 3-(methylthio)propionyl-[Glu63;Leu65;hArg66,68;Cha69;Oic74; Nle75;4-Cl-F77]Apelin-17(61-77) | 313 |
| {MeS-Propionyl }KFTRL[hArg]P[hArg][Cha] SHKG[Oic][Nle]P[4-Cl-F] | 3-(methylthio)propionyl-[Thr63;Leu65;hArg66,68;Cha69;Oic74; Nle75;4-Cl-F77]Apelin-17(61-77) | 314 |
| {MeS-Propionyl }KFYRL[hArg]P[hArg][Cha] SHKG[Oic][Nle]P[4-Cl-F] | 3-(methylthio)propionyl-[Tyr63;Leu65;hArg66,68;Cha69;Oic74; Nle75;4-Cl-F77]Apelin-17(61-77) | 315 |
| {MeS-Propionyl }KFIRL[hArg]P[hArg][Cha] SHKG[Oic][Nle]P[4-Cl-F] | 3-(methylthio)propionyl-[Ile63;Leu65;hArg66,68;Cha69;Oic74; Nle75;4-Cl-F77]Apelin-17(61-77) | 316 |
| {MeS-Propionyl }KFPRL[hArg]P[hArg][Cha] SHKG[Oic][Nle]P[4-Cl-F] | 3-(methylthio)propionyl-[Pro63;Leu65;hArg66,68;Cha69;Oic74; Nle75;4-Cl-F77]Apelin-17(61-77) | 317 |
| {MeS-Propionyl}KFSRL[hArg]P[hArg][Cha] SHKG[Oic][Nle]P[4-Cl-F] | 3-(methylthio)propionyl-[Ser63;Leu65;hArg66,68;Cha69;Oic74; Nle75;4-Cl-F77]Apelin-17(61-77) | 318 |
| {MeS-Propionyl }KFFRL[hArg]P[hArg][Cha] SHKG[Oic][Nle]P[4-Cl-F] | 3-(methylthio)propionyl-[Phe63;Leu65;hArg66,68;Cha69;Oic74; Nle75;4-Cl-F77]Apelin-17(61-77) | 319 |
| {MeS-Propionyl }KFLRL[hArg]P[hArg][Cha] SHKG[Oic][Nle]P[4-Cl-F] | 3-(methylthio)propionyl-[Leu63,65;hArg66,68;Cha69;Oic74;Nle 75;4-Cl-F77]Apelin-17(61-77) | 320 |
| {MeS-Propionyl } KFVRL[hArg]P [hArg][Cha] SHKG[Oic][Nle]P[4-Cl-F] | 3-(methylthio)propionyl-[Val63;Leu65;hArg66,68;Cha69;Oic74; Nle75;4-Cl-F77]Apelin-17(61-77) | 321 |
| {MeS-Propionyl }KFRQL[hArg]P[hArg][Cha] SHKG[Oic][Nle]P[4-Cl-F] | 3-(methylthio)propionyl-[Gln64;Leu65;hArg66,68;Cha69;Oic74; Nle75;4-Cl-F77]Apelin-17(61-77) | 322 |
| {MeS-Propionyl }KFRAL[hArg]P[hArg][Cha] SHKG[Oic][Nle]P[4-Cl-F] | 3-(methylthio)propionyl-[Ala64;Leu65;hArg66,68;Cha69;Oic74; Nle75;4-Cl-F77]Apelin-17(61-77) | 323 |
| {MeS-Propionyl }KFREL[hArg]P[hArg][Cha] SHKG[Oic][Nle]P[4-Cl-F] | 3-(methylthio)propionyl-[Glu64;Leu65;hArg66,68;Cha69;Oic74; Nle75;4-Cl-F77]Apelin-17(61-77) | 324 |
| {MeS-Propionyl }KFRTL[hArg]P[hArg][Cha] SHKG[Oic][Nle]P[4-Cl-F] | 3-(methylthio)propionyl-[Thr64;Leu65;hArg66,68;Cha69;Oic74; Nle75;4-Cl-F77]Apelin-17(61-77) | 325 |
| {MeS-Propionyl }KFRYL[hArg]P[hArg][Cha] SHKG[Oic][Nle]P[4-Cl-F] | 3-(methylthio)propionyl-[Tyr64;Leu65;hArg66,68;Cha69;Oic74; Nle75;4-Cl-F77]Apelin-17(61-77) | 326 |
| {MeS-Propionyl }KFRIL[hArg]P[hArg][Cha] SHKG[Oic][Nle]P[4-Cl-F] | 3-(methylthio)propionyl-[Ile64;Leu65;hArg66,68;Cha69;Oic74; Nle75;4-Cl-F77]Apelin-17(61-77) | 327 |
| {MeS-Propionyl }KFRPL[hArg]P[hArg][Cha] SHKG[Oic][Nle]P[4-Cl-F] | 3-(methylthio)propionyl-[Pro64;Leu65;hArg66,68;Cha69;Oic74; Nle75;4-Cl-F77]Apelin-17(61-77) | 328 |
| {MeS-Propionyl }KFRSL[hArg]P[hArg][Cha] SHKG[Oic][Nle]P[4-Cl-F] | 3-(methylthio)propionyl-[Ser64;Leu65;hArg66,68;Cha69;Oic74; Nle75;4-Cl-F77]Apelin-17(61-77) | 329 |
| {MeS-Propionyl }KFRFL[hArg]P[hArg][Cha] SHKG[Oic][Nle]P[4-Cl-F] | 3-(methylthio)propionyl-[Phe64;Leu65;hArg66,68;Cha69;Oic74; Nle75;4-Cl-F77]Apelin-17(61-77) | 330 |
| {MeS-Propionyl }KFRLL[hArg]P[hArg][Cha] SHKG[Oic][Nle]P[4-Cl-F] | 3-(methylthio)propionyl-[Leu64,65;hArg66,68;Cha69;Oic74;Nle 75;4-Cl-F77]Apelin-17(61-77) | 331 |
| {MeS-Propionyl }KFRVL[hArg]P[hArg][Cha] SHKG[Oic][Nle]P[4-Cl-F] | 3-(methylthio)propionyl-[Val64;Leu65;hArg66,68;Cha69;Oic74; Nle75;4-Cl-F77]Apelin-17(61-77) | 332 |
| {MeS-Propionyl }FARL[hArg]P[hArg][Cha]S HKG[Oic][Nle]P[4-Cl-F] | 3-(methylthio)propionyl-[Ala63;Leu65;hArg66,68;Cha69;Oic74; Nle75;4-Cl-F77]Apelin-17(62-77) | 333 |
| {MeS-Propionyl }FERL[hArg]P[hArg][Cha]S HKG[Oic][Nle]P[4-Cl-F] | 3-(methylthio)propionyl-[Glu63;Leu65;hArg66,68;Cha69;Oic74; Nle75;4-Cl-F77]Apelin-17(62-77) | 334 |
| {MeS-Propionyl}FTRL[hArg]P[hArg][Cha]S HKG[Oic][Nle]P[4-Cl-F] | 3-(methylthio)propionyl-[Thr63;Leu65;hArg66,68;Cha69;Oic74; Nle75;4-Cl-F77]Apelin-17(62-77) | 335 |
| {MeS-Propionyl}FYRL[hArg]P[hArg][Cha]S HKG[Oic][Nle]P[4-Cl-F] | 3-(methylthio)propionyl-[Tyr63;Leu65;hArg66,68;Cha69;Oic74; Nle75;4-Cl-F77]Apelin-17(62-77) | 336 |
| {MeS-Propionyl}FIRL[hArg]P[hArg][Cha]S HKG[Oic][Nle]P[4-Cl-F] | 3-(methylthio)propionyl-[Ile63;Leu65;hArg66,68;Cha69;Oic74; Nle75;4-Cl-F77]Apelin-17(62-77) | 337 |
| {MeS-Propionyl }FPRL[hArg]P[hArg][Cha]S HKG[Oic][Nle]P[4-Cl-F] | 3-(methylthio)propionyl-[Pro63;Leu65;hArg66,68;Cha69;Oic74; Nle75;4-Cl-F77]Apelin-17(62-77) | 338 |
| {MeS-Propionyl }FSRL[hArg]P[hArg][Cha]S HKG[Oic][Nle]P[4-Cl-F] | 3-(methylthio)propionyl-[Ser63;Leu65;hArg66,68;Cha69;Oic74; Nle75;4-Cl-F77]Apelin-17(62-77) | 339 |
| {MeS-Propionyl }FFRL[hArg]P[hArg][Cha]S HKG[Oic][Nle]P[4-Cl-F] | 3-(methylthio)propionyl-[Phe63;Leu65;hArg66,68;Cha69;Oic74; Nle75;4-Cl-F77]Apelin-17(62-77) | 340 |
| {MeS-Propionyl }FLRL[hArg]P[hArg][Cha]S HKG[Oic][Nle]P[4-Cl-F] | 3-(methylthio)propionyl-[Leu63;Leu65;hArg66,68;Cha69;Oic74; Nle75;4-Cl-F77]Apelin-17(62-77) | 341 |
| {MeS-Propionyl}FVRL[hArg]P[hArg][Cha]S HKG[Oic][Nle]P[4-Cl-F] | 3-(methylthio)propionyl-[Val63;Leu65;hArg66,68;Cha69;Oic74; Nle75;4-Cl-F77]Apelin-17(62-77) | 342 |
| {MeS-Propionyl }FRQL[hArg]P[hArg][Cha]S HKG[Oic][Nle]P[4-Cl-F] | 3-(methylthio)propionyl-[Gln64;Leu65;hArg66,68;Cha69;Oic74; Nle75;4-Cl-F77]Apelin-17(62-77) | 343 |
| {MeS-Propionyl}FRAL[hArg]P[hArg][Cha]S HKG[Oic][Nle]P[4-Cl-F] | 3-(methylthio)propionyl-[Ala64;Leu65;hArg66,68;Cha69;Oic74; Nle75;4-Cl-F77]Apelin-17(62-77) | 344 |
| {MeS-Propionyl}FREL[hArg]P[hArg][Cha]S HKG[Oic][Nle]P[4-Cl-F] | 3-(methylthio)propionyl-[Glu64;Leu65;hArg66,68;Cha69;Oic74; Nle75;4-Cl-F77]Apelin-17(62-77) | 345 |
| {MeS-Propionyl }FRTL[hArg]P[hArg][Cha]S HKG[Oic][Nle]P[4-Cl-F] | 3-(methylthio)propionyl-[Thr64;Leu65;hArg66,68;Cha69;Oic74; Nle75;4-Cl-F77]Apelin-17(62-77) | 346 |
| {MeS-Propionyl }FRYL[hArg]P[hArg][Cha]S HKG[Oic][Nle]P[4-Cl-F] | 3-(methylthio)propionyl-[Tyr64;Leu65;hArg66,68;Cha69;Oic74; Nle75;4-Cl-F77]Apelin-17(62-77) | 347 |
| {MeS-Propionyl }FRIL[hArg]P[hArg][Cha]S HKG[Oic][Nle]P[4-Cl-F] | 3-(methylthio)propionyl-[Ile64;Leu65;hArg66,68;Cha69;Oic74; Nle75;4-Cl-F77]Apelin-17(62-77) | 348 |
| {MeS-Propionyl }FRPL[hArg]P[hArg][Cha]S HKG[Oic][Nle]P[4-Cl-F] | 3-(methylthio)propionyl-[Pro64;Leu65;hArg66,68;Cha69;Oic74; Nle75;4-Cl-F77]Apelin-17(62-77) | 349 |
| {MeS-Propionyl}FRSL[hArg]P[hArg][Cha]S HKG[Oic][Nle]P[4-Cl-F] | 3-(methylthio)propionyl-[Ser64;Leu65;hArg66,68;Cha69;Oic74; Nle75;4-Cl-F77]Apelin-17(62-77) | 350 |
| {MeS-Propionyl }FRFL[hArg]P[hArg][Cha]S HKG[Oic][Nle]P[4-Cl-F] | 3-(methylthio)propionyl-[Phe64;Leu65;hArg66,68;Cha69;Oic74; Nle75;4-Cl-F77]Apelin-17(62-77) | 351 |
| {MeS-Propionyl }FRLL[hArg]P[hArg][Cha]S HKG[Oic][Nle]P[4-Cl-F] | 3-(methylthio)propionyl-[Leu64,65;hArg66,68;Cha69;Oic74;Nle 75;4-Cl-F77]Apelin-17(62-77) | 352 |
| {MeS-Propionyl}FRVL[hArg]P[hArg][Cha]S HKG[Oic][Nle]P[4-Cl-F] | 3-(methylthio)propionyl-[Val64;Leu65;hArg66,68;Cha69;Oic74; Nle75;4-Cl-F77]Apelin-17(62-77) | 353 |
| {MeS-Propionyl}KFRRQRP[hArg][Cha]SHK G[Oic][Nle]P{Amide} | 3-(methylthio)propionyl-[hArg68;Cha69;Oic74;Nle75]Apelin-17(61-76)-Amide | 354 |
| {MeS-Propionyl}KF[Cit][Cit]L[hArg]P[hArg] [Cha]SHKG[Oic][Nle]P{Amide} | 3-(methylthio)propionyl-[Cit63,64;Leu65;hArg66,68;Cha69;Oic 74;Nle75]Apelin-17(61-76)-Amide | 355 |
| {MeS-Propionyl}F[Cit][Cit]L[hArg]P[hArg][ Cha]SHKG[Oic][Nle]P{Amide} | 3-(methylthio)propionyl-[Cit63,64;Leu65;hArg66,68;Cha69;Oic 74;Nle75]Apelin-17(62-76)-Amide | 356 |
| {MeS-Propionyl }KFRRL[hArg]P[hArg][Cha] SHKG[Oic][Nle]P{Amide} | 3-(methylthio)propionyl-[Leu65;hArg66,68;Cha69;Oic74;Nle75] Apelin-17(61-76)-Amide | 357 |
| {MeS-Propionyl}FQQL[hArg]P[hArg][Cha]S HKG[Oic][Nle]P{Amide} | 3-(methylthio)propionyl-[Gln63,64;Leu65;hArg66,68;Cha69;Oic 74;Nle75]Apelin-17(62-76)-Amide | 358 |
| {MeS-Propionyl }FRRQRP[hArg][Cha]SHKG [Oic][Nle]P[4-Cl-F]{COOH} | 3-(methylthio)propionyl-[hArg68;Cha69;Oic74;Nle75;4-Cl-F77]Apelin-17(62-77) | 359 |
| {MeS-Propionyl}F[hArg][hArg]Q[hArg]P[hA rg] [Cha]SHKG[Oic][Nle]P[4-Cl-F]{COOH} | 3-(methylthio)propionyl-[hArg63,64,66,68;Cha69;Oic74;Nle75;4 -Cl-F77]Apelin-17(62-77) | 360 |
| {MeS-Propionyl}A[hArg][hArg]Q[hArg]P[h Arg] [Cha]SHKG[Oic][Nle]P[4-Cl-F] {COOH} | 3-(methylthio)propionyl-[Ala62;hArg63,64,66,68;Cha69;Oic74; Nle75;4-Cl-F77]Apelin-17(62-77) | 361 |
| {MeS-Propionyl}FAQ[hArg]P[hArg][Cha]SH KG[Oic][Nle]P[4-Cl-F]{COOH} | 3-(methylthio)propionyl-[Phe63;Ala64;hArg66,68;Cha69;Oic74; Nle75;4-Cl-F77]Apelin-17(63-77) | 362 |
| {MeS-Propionyl}F[hArg]AQ[hArg]P[hArg][ Cha]SHKG[Oic][Nle]P[4-Cl-F]{COOH} | 3-(methylthio)propionyl-[hArg63,66,68;Ala64;Cha69;Oic74;Nle 75;4-Cl-F77]Apelin-17(62-77) | 363 |
| {MeS-Propionyl}F[hArg][hArg]A[hArg]P[hA rg][Cha]SHKG[Oic][Nle]P[4-Cl-F]{COOH} | 3-(methylthio)propionyl-[hArg63,64,66,68;Ala65;Cha69;Oic74; Nle75;4-Cl-F77]Apelin-17(62-77) | 364 |
| {MeS-Propionyl}F[hArg][hArg]QAP[hArg][ Cha]SHKG[Oic][Nle]P[4-Cl-F]{COOH} | 3-(methylthio)propionyl-[hArg63,64,68;Ala66;Cha69;Oic74;Nle 75;4-Cl-F77]Apelin-17(62-77) | 365 |
| {MeS-Propionyl}F[hArg][hArg]Q[hArg]P[hA rg][Cha][K(NPeg11)]HKG[Oic][Nle]P[ 4-Cl-F]{COOH} | 3-(methylthio)propionyl-[hArg63,64,66,68;Cha69;K(NPeg11)70; Oic74;Nle75;4-Cl-F77]Apelin-17(62-77) | 366 |
| {MeS-Propionyl}F[hArg][hArg]Q[hArg]P[hA rg][Cha]S[K(NPeg11)]KG[Oic][Nle]P[ 4-Cl-F]{COOH} | 3-(methylthio)propionyl-[hArg63,64,66,68;Cha69;K(NPeg11)71; Oic74;Nle75;4-Cl-F77]Apelin-17(62-77) | 367 |
| {MeS-Propionyl}F[hArg][hArg]Q[hArg]P[hA rg] [Cha] [Atz(PEG10)]HKG[Oic][Nle]P 4-Cl-F]{COOH} | 3-(methylthio)propionyl-[hArg63,64,66,68;Cha69;Atz(PEG10)7 0;Oic74;Nle75;4-Cl-F77]Apelin-17(62-77) | 368 |
| {MeS-Propionyl}F[hArg][hArg]Q[hArg]P[hA rg] [Cha]S[Atz(PEG10)]KG[Oic][Nle]P [4-Cl-F]{COOH} | 3-(methylthio)propionyl-[hArg63,64,66,68;Cha69;Atz(PEG10)7 1;Oic74;Nle75;4-Cl-F77]Apelin-17(62-77) | 369 |
| | | |
| {MeS-Propionyl}F[hArg][hArg]Q[hArg]P[hA rg][Cha]THKG[Oic][Nle]P[4-Cl-F]{COOH} | 3-(methylthio)propionyl-[hArg63,64,66,68;Cha69;Thr70;Oic74; Nle75;4-Cl-F77]Apelin-17(62-77) | 370 |
| {MeS-Propionyl}F[hArg][hArg]Q[hArg]P[hA rg][Cha]AHKG[Oic][Nle]P[4-Cl-F]{COOH} | 3-(methylthio)propionyl-[hArg63,64,66,68;Cha69;Ala70;Oic74; Nle75;4-Cl-F77]Apelin-17(62-77) | 371 |
| {MeS-Propionyl}F[hArg][hArg]Q[hArg]P[hA rg][Cha][Abu]HKG[Oic][Nle]P[4-Cl-F]{COOH} | 3-(methylthio)propionyl-[hArg63,64,66,68;Cha69;Abu70;Oic74; Nle75;4-Cl-F77]Apelin-17(62-77) | 372 |
| {MeS-Propionyl}F[hArg][hArg]Q[hArg]P[hA rg] [Cha][Nle]HKG[Oic][Nle]P[4-Cl-F] {COOH} | 3-(methylthio)propionyl-[hArg63,64,66,68;Cha69;Nle70,75;Oic7 4;4-Cl-F77]Apelin-17(62-77) | 373 |
| {MeS-Propionyl}F[hArg][hArg]Q[hArg]P[hA rg][Cha][Nva]HKG[Oic][Nle]P[4-Cl-F]{COOH} | 3-(methylthio)propionyl-[hArg63,64,66,68;Cha69;Nva70;Oic74; Nle75;4-Cl-F77]Apelin-17(62-77) | 374 |
| {MeS-Propionyl}F[hArg][hArg]Q[hArg]P[hA rg][Cha]VHKG[Oic][Nle]P[4-Cl-F]{COOH} | 3-(methylthio)propionyl-[hArg63,64,66,68;Cha69; Val70; Oic74; Nle75;4-Cl-F77]Apelin-17(62-77) | 375 |
| {MeS-Propionyl}F[hArg][hArg]Q[hArg]P[hA rg][Cha]GHKG[Oic][Nle]P[4-Cl-F]{COOH} | 3-(methylthio)propionyl-[hArg63,64,66,68;Cha69;Gly70;Oic74; Nle75;4-Cl-F77]Apelin-17(62-77) | 376 |
| {MeS-Propionyl}F[hArg][hArg]Q[hArg]P[hA rg][Cha]LHKG[Oic][Nle]P[4-Cl-F]{COOH} | 3-(methylthio)propionyl-[hArg63,64,66,68;Cha69;Leu70;Oic74; Nle75;4-Cl-F77]Apelin-17(62-77) | 378 |
| {MeS-Propionyl}F[hArg][hArg]Q[hArg]P[hA rg][Cha]IHKG[Oic][Nle]P[4-Cl-F]{COOH} | 3-(methylthio)propionyl-[hArg63,64,66,68;Cha69;Ile70;Oic74;N le75;4-Cl-F77]Apelin-17(62-77) | 379 |
| {MeS-rg][Cha][CPG]HKG[Oic][Nle]P[4-Cl-F]{COOH} | 3-(methylthio)propionyl-[hArg63,64,66,68;Cha69;CPG70;Oic74; Nle75;4-Cl-F77]Apelin-17(62-77) | 380 |
| {MeS-Propiony1}F[hArg][hArg]Q[hArg]P[hA rg] [Cha][Chg]HKG[Oic][Nle]P[4-Cl-F]{COOH} | 3-(methylthio)propionyl-[hArg63,64,66,68;Cha69;Chg7C);Oic74; Nle75;4-Cl-F77]Apelin-17(62-77) | 381 |
| {MeS-Propionyl}F[hArg][hArg]Q[hArg]P[hA rg] [Cha][Cha]HKG[Oic][Nle]P[4-Cl-F]{COOH} | 3-(methylthio)propionyl-[hArg63,64,66,68;Cha69,70;Oic74;Nle7 5;4-Cl-F77]Apelin-17(62-77) | 382 |
| {MeS-Propionyl}F[hArg][hArg]Q[hArg]P[hA rg][Cha]HHKG[Oic][Nle]P[4-Cl-F]{COOH} | 3-(methylthio)propionyl-[hArg63,64,66,68;Cha69;His70;Oic74; Nle75;4-Cl-F77]Apelin-17(62-77) | 383 |
| {MeS-Propionyl}F[hArg][hArg]Q[hArg]P[hA rg][Cha]YHKG[Oic][Nle]P[4-Cl-F]{COOH} | 3-(methylthio)propionyl-[hArg63,64,66,68;Cha69;Tyr70;Oic74; Nle75;4-Cl-F77]Apelin-17(62-77) | 384 |
| {MeS-Propionyl}F[hArg][hArg]Q[hArg]P[hA rg][Cha]WHKG[Oic][Nle]P[4-Cl-F]{COOH} | 3-(methylthio)propionyl-[hArg63,64,66,68;Cha69;Trp70;Oic74; Nle75;4-Cl-F77]Apelin-17(62-77) | 385 |
| {MeS-Propionyl}F[hArg][hArg]Q[hArg]P[hA rg] [Cha]FHKG[Oic][Nle]P[4-Cl-F] {COOH} | 3-(methylthio)propionyl-[hArg63,64,66,68;Cha69;Phe70;Oic74; Nle75;4-Cl-F77]Apelin-17(62-77) | 386 |
| {MeS-Propionyl}F[hArg][hArg]Q[hArg]P[hA rg] [Cha][NMePhe]HKG[Oic][Nle]P[4-Cl-F]{COOH} | 3-(methylthio)propionyl-[hArg63,64,66,68;Cha69;NMePhe70;Oi c74;Nle75;4-Cl-F77]Apelin-17(62-77) | 387 |
| {MeS-Propionyl}F[hArg][hArg]Q[hArg]P[hA rg] [Cha][1-Nal]HKG[Oic][Nle]P[4-Cl-F] {COOH} | 3-(methylthio)propionyl-[hArg63,64,66,68;Cha69;1-Nal70;Oic74;Nle75;4-Cl-F77]Apelin-17(62-77) | 388 |
| {MeS-Propionyl}F[hArg][hArg]Q[hArg]P[hA rg][Cha][2-Nal]HKG[Oic][Nle]P[4-Cl-F]{COOH} | 3-(methylthio)propionyl-[hArg63,64,66,68;Cha69;2-Nal70;Oic74;Nle75;4-Cl-F77]Apelin-17(62-77) | 389 |
| {MeS-Propionyl}F[hArg][hArg]Q[hArg]P[hA rg] [Cha][Bip]HKG[Oic][Nle]P[4-Cl-F] {COOH} | 3-(methylthio)propionyl-[hArg63,64,66,68;Cha69;Bip70;Oic74; Nle75;4-Cl-F77]Apelin-17(62-77) | 390 |
| {MeS-Propionyl}F[hArg][hArg]Q[hArg]P[hA rg] [Cha][Igl]HKG[Oic][Nle]P[4-Cl-F] {COOH} | 3-(methylthio)propionyl-[hArg63,64,66,68;Cha69;Igl70;Oic74;N le75;4-Cl-F77]Apelin-17(62-77) | 391 |
| {MeS-Propionyl}F[hArg][hArg]Q[hArg]P[hA rg] [Cha][4-Cl-F]HKG[Oic][Nle]P[4-Cl-F]{COOH} | 3-(methylthio)propionyl-[hArg63,64,66,68;Cha69;4-Cl-F70,77;Oic74;Nle75]Apelin-17(62-77) | 392 |
| {MeS-Propionyl}F[hArg][hArg]Q[hArg]P[hA rg][Cha][4-F-F]HKG[Oic][Nle]P[4-Cl-F]{COOH} | 3-(methylthio)propionyl-[hArg63,64,66,68;Cha69;4-F-F70;Oic74;Nle75;4-Cl-F77]Apelin-17(62-77) | 393 |
| {MeS-Propionyl}F[hArg][hArg]Q[hArg]P[hA rg] [Cha][pI-Phe]HKG[Oic][Nle]P[4-Cl-F]{COOH} | 3-(methylthio)propionyl-[hArg63,64,66,68;Cha69;pI-Phe70;Oic74;Nle75;4-Cl-F77]Apelin-17(62-77) | 394 |
| {MeS-Propionyl}F[hArg][hArg]Q[hArg]P[hA rg] [Cha][2Pal]HKG[Oic][Nle]P[4-Cl-F] {COOH} | 3-(methylthio)propionyl-[hArg63,64,66,68;Cha69;2Pal70;Oic74; Nle75;4-Cl-F77]Apelin-17(62-77) | 395 |
| {MeS-Propionyl}F[hArg][hArg]Q[hArg]P[hA rg] [Cha][3Pal]HKG[Oic][Nle]P[4-Cl-F] {COOH} | 3-(methylthio)propionyl-[hArg63,64,66,68;Cha69;3Pal70;Oic74; Nle75;4-Cl-F77]Apelin-17(62-77) | 396 |
| {MeS-Propionyl}F[hArg][hArg]Q[hArg]P[hA rg] [Cha][Tic]HKG[Oic][Nle]P[4-Cl-F] {COOH} | 3-(methylthio)propionyl-[hArg63,64,66,68;Cha69;Tic70;Oic74; Nle75;4-Cl-F77]Apelin-17(62-77) | 397 |
| {MeS-Propionyl}F[hArg][hArg]Q[hArg]P[hA rg][Cha][Orn]HKG[Oic][Nle]P[4-Cl-F]{COOH} | 3-(methylthio)propionyl-[hArg63,64,66,68;Cha69;Orn70;Oic74; Nle75;4-Cl-F77]Apelin-17(62-77) | 398 |
| {MeS-Propionyl}F[hArg][hArg]Q[hArg]P[hA rg] [Cha][Dab]HKG[Oic][Nle]P[4-Cl-F] {COOH} | 3-(methylthio)propionyl-[hArg63,64,66,68;Cha69;Dab70;Oic74; Nle75;4-Cl-F77]Apelin-17(62-77) | 399 |
| {MeS-Propionyl}F[hArg][hArg]Q[hArg]P[hA rg][Cha][Dap]HKG[Oic][Nle]P[4-Cl-F]{COOH} | 3-(methylthio)propionyl-[hArg63,64,66,68;Cha69;Dap70;Oic74; Nle75;4-Cl-F77]Apelin-17(62-77) | 400 |
| | | |
| {MeS-Propionyl}F[hArg][hArg]Q[hArg]P[hA rg] [Cha]STKG[Oic][Nle]P[4-Cl-F]{COOH} | 3-(methylthio)propionyl-[hArg63,64,66,68;Cha69;Thr71;Oic74; Nle75;4-Cl-F77]Apelin-17(62-77) | 401 |
| {MeS-Propionyl}F[hArg][hArg]Q[hArg]P[hA rg] [Cha]SAKG[Oic][Nle]P[4-Cl-F]{COOH} | 3-(methylthio)propionyl-[hArg63,64,66,68;Cha69;Ala71;Oic74; Nle75;4-Cl-F77]Apelin-17(62-77) | 402 |
| {MeS-Propionyl}F[hArg][hArg]Q[hArg]P[hA rg] [Cha]S[Abu]KG[Oic][Nle]P[4-Cl-F]{COOH} | 3-(methylthio)propionyl-[hArg63,64,66,68;Cha69;Abu71;Oic74; Nle75;4-Cl-F77]Apelin-17(62-77) | 403 |
| {MeS-Propionyl}F[hArg][hArg]Q[hArg]P[hA rg][Cha]S[Nle]KG[Oic][Nle]P[4-Cl-F]{COOH} | 3-(methylthio)propionyl-[hArg63,64,66,68;Cha69;Nle71,75;Oic7 4;4-Cl-F77]Apelin-17(62-77) | 404 |
| {MeS-Propionyl}F[hArg][hArg]Q[hArg]P[hA rg][Cha]S[Nva]KG[Oic][Nle]P[4-Cl-F]{COOH} | 3-(methylthio)propionyl-[hArg63,64,66,68;Cha69;Nva71;Oic74; Nle75;4-Cl-F77]Apelin-17(62-77) | 405 |
| {MeS-Propionyl}F[hArg][hArg]Q[hArg]P[hA rg] [Cha]SVKG[Oic][Nle]P[4-Cl-F] {COOH} | 3-(methylthio)propionyl-[hArg63,64,66,68;Cha69;Val71;Oic74; Nle75;4-Cl-F77]Apelin-17(62-77) | 406 |
| {MeS-Propionyl}F[hArg][hArg]Q[hArg]P[hA rg] [Cha]SGKG[Oic][Nle]P[4-Cl-F] {COOH} | 3-(methylthio)propionyl-[hArg63,64,66,68;Cha69;Gly71;Oic74; Nle75;4-Cl-F77]Apelin-17(62-77) | 407 |
| {MeS-Propionyl}F[hArg][hArg]Q[hArg]P[hA rg][Cha]SLKG[Oic][Nle]P[4-Cl-F]{COOH} | 3-(methylthio)propionyl-[hArg63,64,66,68;Cha69;Leu71;Oic74; Nle75;4-Cl-F77]Apelin-17(62-77) | 408 |
| {MeS-Propionyl}F[hArg][hArg]Q[hArg]P[hA rg] [Cha]SIKG[Oic][Nle]P[4-Cl-F] {COOH} | 3-(methylthio)propionyl-[hArg63,64,66,68;Cha69;Ile71;Oic74;N le75;4-Cl-F77]Apelin-17(62-77) | 409 |
| {MeS-Propionyl}F[hArg][hArg]Q[hArg]P[hA rg] [Cha]S[CPG]KG[Oic][Nle]P[4-Cl-F] {COOH} | 3-(methylthio)propionyl-[hArg63,64,66,68;Cha69;CPG71;Oic74; Nle75;4-Cl-F77]Apelin-17(62-77) | 410 |
| {MeS-Propionyl}F[hArg][hArg]Q[hArg]P[hA rg][Cha]S[Chg]KG[Oic][Nle]P[4-Cl-F] {COOH} | 3-(methylthio)propionyl-[hArg63,64,66,68;Cha69;Chg71;Oic74; Nle75;4-Cl-F77]Apelin-17(62-77) | 411 |
| {MeS-Propionyl}F[hArg][hArg]Q[hArg]P[hA rg][Cha]S[Cha]KG[Oic][Nle]P[4-Cl-F]{COOH} | 3-(methylthio)propionyl-[hArg63,64,66,68;Cha69,71;Oic74;Nle7 5;4-Cl-F77]Apelin-17(62-77) | 412 |
| {MeS-Propionyl}F[hArg][hArg]Q[hArg]P[hA rg] [Cha]SHKG[Oic][Nle]P[4-Cl-F]{COOH} | 3-(methylthio)propionyl-[hArg63,64,66,68;Cha69;Oic74,Nle75;4 -Cl-F77]Apelin-17(62-77) | 413 |
| {MeS-Propionyl}F[hArg][hArg]Q[hArg]P[hA rg] [Cha]SYKG[Oic][Nle]P[4-Cl-F]{COOH} | 3-(methylthio)propionyl-[hArg63,64,66,68;Cha69;Tyr71;Oic74; Nle75;4-Cl-F77]Apelin-17(62-77) | 414 |
| {MeS-Propionyl}F[hArg][hArg]Q[hArg]P[hA rg] [Cha]SWKG[Oic][Nle]P[4-Cl-F] {COOH} | 3-(methylthio)propionyl-[hArg63,64,66,68;Cha69;Trp71;Oic74; Nle75;4-Cl-F77]Apelin-17(62-77) | 415 |
| {MeS-Propionyl}F[hArg][hArg]Q[hArg]P[hA rg][Cha]SFKG[Oic][Nle]P[4-Cl-F]{COOH} | 3-(methylthio)propionyl-[hArg63,64,66,68;Cha69;Phe71;Oic74; Nle75;4-Cl-F77]Apelin-17(62-77) | 416 |
| {MeS-Propionyl}F[hArg][hArg]Q[hArg]P[hA rg][Cha]S[NMePhe]KG[Oic][Nle]P[4-Cl-F]{COOH} | 3-(methylthio)propionyl-[hArg63,64,66,68;Cha69;NMePhe71;Oi c74;Nle75;4-Cl-F77]Apelin-17(62-77) | 417 |
| {MeS-Propionyl}F[hArg][hArg]Q[hArg]P[hA rg] [Cha]S[1-Nal]KG[Oic][Nle]P[4-Cl-F] {COOH} | 3-(methylthio)propionyl-[hArg63,64,66,68;Cha69;1-Nal71;Oic74;Nle75;4-Cl-F77]Apelin-17(62-77) | 418 |
| {MeS-Propionyl}F[hArg][hArg]Q[hArg]P[hA rg][Cha]S[2-Nal]KG[Oic][Nle]P[4-Cl-F]{COOH} | 3-(methylthio)propionyl-[hArg63,64,66,68;Cha69;2-Nal71;Oic74;Nle75;4-Cl-F77]Apelin-17(62-77) | 419 |
| {MeS-Propionyl}F[hArg][hArg]Q[hArg]P[hA rg] [Cha]S[Bip]KG[Oic][Nle]P[4-Cl-F] {COOH} | 3-(methylthio)propionyl-[hArg63,64,66,68;Cha69;Bip71;Oic74; Nle75;4-Cl-F77]Apelin-17(62-77) | 420 |
| {MeS-Propionyl}F[hArg][hArg]Q[hArg]P[hA rg][Cha]S[Igl]KG[Oic][Nle]P[4-Cl-F]{COOH} | 3-(methylthio)propionyl-[hArg63,64,66,68;Cha69;Igl71;Oic74;N le75;4-Cl-F77]Apelin-17(62-77) | 421 |
| {MeS-Propionyl}F[hArg][hArg]Q[hArg]P[hA rg] [Cha]S[4-Cl-F]KG[Oic][Nle]P[4-Cl-F] {COOH} | 3-(methylthio)propionyl-[hArg63,64,66,68;Cha69;4-Cl-F71,77;Oic74;Nle75]Apelin-17(62-77) | 422 |
| {MeS-Propionyl}F[hArg][hArg]Q[hArg]P[hA rg][Cha]S[4-F-F]KG[Oic][Nle]P[4-Cl-F]{COOH} | 3-(methylthio)propionyl-[hArg63,64,66,68;Cha69;4-F-F71;Oic74;Nle75;4-Cl-F77]Apelin-17(62-77) | 423 |
| {MeS-Propionyl}F[hArg][hArg]Q[hArg]P[hA rg] [Cha]S[4-I-F]KG[Oic][Nle]P[4-Cl-F]{COOH} | 3-(methylthio)propionyl-[hArg63,64,66,68;Cha69;4-IF71;Oic74;Nle75;4-Cl-F77]Apelin-17(62-77) | 424 |
| {MeS-Propionyl}F[hArg][hArg]Q[hArg]P[hA rg][Cha]S[2Pal]KG[Oic][Nle]P[4-Cl-F]{COOH} | 3-(methylthio)propionyl-[hArg63,64,66,68;Cha69;2Pal71;Oic74; Nle75;4-Cl-F77]Apelin-17(62-77) | 425 |
| {MeS-Propionyl}F[hArg][hArg]Q[hArg]P[hA rg][Cha]S[3Pal]KG[Ole][Nle]P[4-Cl-F]{COOH} | 3-(methylthio)propionyl-[hArg63,64,66,68;Cha69;3Pal71;Oic74; Nle75;4-Cl-F77]Apelin-17(62-77) | 426 |
| {MeS-Propionyl}F[hArg][hArg]Q[hArg]P[hA rg] [Cha]S[Tic]KG[Oic][Nle]P[4-Cl-F] {COOH} | 3-(methylthio)propionyl-[hArg63,64,66,68;Cha69;Tic71;Oic74; Nle75;4-Cl-F77]Apelin-17(62-77) | 427 |
| {MeS-Propionyl}F[hArg][hArg]Q[hArg]P[hA rg][Cha]S[Orn]KG[Oic][Nle]P[4-Cl-F]{COOH} | 3-(methylthio)propionyl-[hArg63,64,66,68;Cha69;Orn71;Oic74; Nle75;4-Cl-F77]Apelin-17(62-77) | 428 |
| {MeS-Propionyl}F[hArg][hArg]Q[hArg]P[hA rg][Cha]S[Dab]KG[Oic][Nle]P[4-Cl-F]{COOH} | 3-(methylthio)propionyl-[hAr63,64,66,68;Cha69;Dab71;Oic74; Nle75;4-Cl-F77]Apelin-17(62-77) | 429 |
| {MeS-Propionyl}F[hArg][hArg]Q[hArg]P[hA rg][Cha]S[Dap]KG[Oic][Nle]P[4-Cl-F]{COOH} | 3-(methylthio)propionyl-[hArg63,64,66,68;Cha69;Dap71;Oic74; Nle75;4-Cl-F77]Apelin-17(62-77) | 430 |

The following table shows examples of peptides. In the table, the free acid can be a carboxylic acid and the amide can be at the C-terminus and the agonist can include a PEG derivative or serum half-life prolonging group.

**TABLE 7.**

| AMINO ACID SEQUENCE | DESIGNATION | SEQ IDNO. |
|---|---|---|
| {Acetyl}[Atz(PEG10)]GGLRP[hArg][NMeLeu ]SHKGP[Nle]P[4-Cl-F]{FreeAcid} | Ac-Atz(PEG10)-Gly-Gly-[Leu65;hArg68;NMeLeu69;Nle75;4 -Cl-F77]Apelin-13(65-77) | 431 |
| {Acetyl}[Atz(PEG10)]GLRP[hArg][NMeLeu] SHKGP[Nle]P[4-Cl-F]{FreeAcid} | Ac-Atz(PEG10)-Gly-[Leu65;hArg68;NMeLeu69;Nle75;4 -Cl-F77]Apelin-13(65-77) | 432 |
| {Acetyl}[Atz(PEG10)]LRP[hArg][NMeLeu]S HKGP[Nle]P[4-Cl-F]{FreeAcid} | Ac-Atz(PEG10)-[Leu65;hArg68;NMeLeu69;Nle75;4 -Cl-F77]Apelin-13(65-77) | 433 |
| {Hydrogen}[PE][Atz(PEG10)]P[hArg][NMeLe u]SHKGP[Nle]P[4-Cl-F]{FreeAcid} | [PE65;Atz(PEG10)66;hArg68;NMe Leu69;Nle75;4-Cl-F77]Apelin-13(65-77) | 434 |
| {Hydrogen} [PE]RP[Atz(PEG10)][NMeLeu]SH KGP[Nle]P[4-Cl-F]{FreeAcid} | [PE65;Atz(PEG10)68;NMeLeu69; Nle75;4-Cl-F77]Apelin-13(65-77) | 435 |
| {Hydrogen}[PE]RP[hArg][Atz(PEG10)]SHKG P[Nle]P[4-Cl-F]{FreeAcid} | [PE65;hArg68;Atz(PEG10)69;Nle7 5;4-Cl-F77]Apelin-13(65-77) | 436 |
| {Hydrogen} [PE]RP[hArg][NMeLeu][Atz(PEG 10)]HKGP[Nle]P[4-Cl-F]{FreeAcid} | [PE65;hArg68;NMeLeu69;Atz(PE G10)70;Nle75;4-Cl-F77]Apelin-13(65-77) | 437 |
| {Hydrogen} [PE]RP[hArg][NMeLeu]S[Atz(PE G10)]KGP[Nle]P[4-Cl-F]{FreeAcid} | [PE65;hArg68;NMeLeu69;Atz(PE G10)71;Nle75;4-Cl-F77]Apelin-13(65-77) | 438 |
| {Hydrogen}[PE]RP[hArg][NMeLeu]SHKGP[ Atz(PEG10)]P[4-Cl-F]{FreeAcid} | [PE65;hArg68;NMeLeu69;Atz(PE G10)75;4-Cl-F77]Apelin-13(65-77) | 439 |
| {Hydrogen}[PE]RP[hArg][NMeLeu]SHKGP[ Nle]P[Atz(PEG10)]Amide} | [PE65;hArg68;NMeLeu69;Nle75;A tz(PEG10)77]Apelin-13(65-77)-amide | 440 |
| {Hydrogen}[PE]RP[hArg][NMeLeu]SHKGP[ Nle]P[4-Cl-F][Atz(PEG10)]{Amide} | [PE65;hArg68;NMeLeu69;Nle75;4-Cl-F77]Apelin-13(65-77)-Atz(PEG10)-amide | 441 |
| {Hydrogen}[PE]RP[hArg][NMeLeu]SHKGP[ Nle]P[4-Cl-F]G[Atz(PEG10)]{Amide} | [PE65;hArg68;NMeLeu69;Nle75;4-Cl-F77]Apelin-13(65-77)-Gly-Atz(PEG10)-amide | 442 |
| {Hydrogen}[PE]RP[hArg][NMeLeu]SHKGP[ Nle]P[4-Cl-F]GG[Atz(PEG10)]{Amide} | [PE65;hArg68;NMeLeu69;Nle75;4-Cl-F77]Apelin-13(65-77)-Gly-Gly-Atz(PEG10)-amide | 443 |
| {Acetyl}[Atz(PEG10)]LRP[hArg][NMeLeu][A tz(PEG10)]HKGP[Nle]P[4-Cl-F]{FreeAcid} | Ac-Atz(PEG 10)-[Leu65;hArg68;NMeLeu69;Atz(PE G10)70;Nle75;4-Cl-F77]Apelin-13(65-77) | 444 |
| {Hydrogen}[PE]RP[hArg][Cha][Atz(PEG10)] HKGP[Nle]P[4-Cl-F]{FreeAcid} | [PE65;hArg68;Cha69;Atz(PEG10)7 0;Nle75;4-Cl-F77]Apelin-13(65-77) | 445 |
| {Hydrogen}[PE]RP[hArg][Cha][Atz(PEG10)] HKG[Oic][Nle]P[4-Cl-F]{FreeAcid} | [PE65;hArg68;Cha69;Atz(PEG10)7 0;Oic74;Nle75;4-Cl-F77]Apelin-13(65-77) | 446 |
| {Hydrogen}[PE]RP[hArg][Cha][Atz(PEG10)] HKG[Oic][Nle]P[4-Cl-F]{Amide} | [PE65;hArg68;Cha69;Atz(PEG10)7 0;Oic74;Nle75;4-Cl-F77]Apelin-13(65-77)-amide | 447 |
| {Hydrogen} [PE]RP[hArg][NMeLeu][Atz(PEG 10)]HKGP[Nle]P[4-Cl-F]{FreeAcid} | [PE65;hArg68;NMeLeu69;Atz(PE G10)70;Nle75;4-Cl-F77]Apelin-13(65-77) | 448 |
| {Hydrogen} [PE]RP[hArg][NMeLeu][Atz(PEG 10)]HKG[Oic][Nle]P[4-Cl-F]{FreeAcid} | [PE65;hArg68;NMeLeu69;Atz(PE G10)70;Oic74;Nle75;4-Cl-F77]Apelin-13(65-77) | 449 |
| {Hydrogen} [PE]RP[hArg][NMeLeu][Atz(PEG 10)]HKG[Oic][Nle]P[4-Cl-F]{Amide} | [PE65;hArg68;NMeLeu69;Atz(PE G10)70;Oic74;Nle75;4-Cl-F77]Apelin-13 (65-77)-amide | 450 |
| {Hydrogen}[PE]RP[hArg][Cha][Atz(1K-mPEG)]HKG[Oic][Nle]P[4-Cl-F]{FreeAcid} | [PE65;hArg68;Cha69;Atz(1K-mPEG)70;Oic74;Nle75;4-Cl-F77]Apelin-13(65-77) | 451 |
| Hydrogen}[PE]RP[hArg][Cha][Atz(2K-mPEG)]HKG[Oic][Nle]P[4-Cl-F]{FreeAcid} | [PE65;hArg68;Cha69;Atz(2K-mPEG)70;Oic74;Nle75;4-Cl-F77]Apelin-13(65-77) | 452 |
| {Hydrogen} [PE]RP[hArg] [Cha] [Atz(5K-mPEG)]HKG[Oic][Nle]P[4-Cl-F]{FreeAcid} | [PE65;hArg68;Cha69;Atz(5K-mPEG)70;Oic74;Nle75;4-Cl-F77]Apelin-13(65-77) | 453 |
| {Hydrogen}[PE]RP[hArg][Cha][Atz(10K-mPEG)]HKG[Oic][Nle]P[4-Cl-F]{FreeAcid} | [PE65;hArg68;Cha69;Atz(10K-mPEG);Oic74;Nle75;4-Cl-F77]Apelin-13(65-77) | 454 |
| {Hydrogen} [PE]RP[hArg] [Cha] [Atz(20K-mPEG)]HKG[Oic][Nle]P[4-Cl-F]{FreeAcid} | [PE65;hArg68;Cha69;Atz(20K-mPEG)70;Oic74;Nle75;4-Cl-F77]Apelin-13(65-77) | 456 |
| {Hydrogen} [PE]RP[hArg][NMeLeu][Atz(20K-mPEG)]HKG[Oic][Nle]P[4-Cl-F]{FreeAcid} | [PE65;hArg68;NMeLeu69;[Atz(20 K-mPEG)]70;Oic74;N1e75;4-Cl-F77]Apelin-13(65-77) | 457 |
| {Hydrogen} [PE]RP[hArg] [Cha] S[Atz(1K-mPEG)]KG[Oic][Nle]P[4-Cl-F]{FreeAcid} | [PE65;hArg68;Cha69;Atz(1K-mPEG)71;Oic74;Nle75;4-Cl-F77]Apelin-13(65-77) | 458 |
| {Hydrogen} [PE]RP[hArg] [Cha] S[Atz(2K-mPEG)]KG[Oic][Nle]P[4-Cl-F]{FreeAcid} | [PE65;hArg68;Cha69;Atz(2K-mPEG)K71;Oic74;Nle75;4-Cl-F77]Apelin-13(65-77) | 459 |
| {Hydrogen} [PE]RP[hArg] [Cha] S[Atz(5K-mPEG)]KG[Oic][Nle]P[4-Cl-F]{FreeAcid} | [PE65;hArg68;Cha69;Atz(5K-mPEG)71;Oic74;Nle75;4-Cl-F77]Apelin-13(65-77) | 460 |
| {Hydrogen}[PE]RP[hArg][Cha]S[Atz(10K-mPEG)]KG[Oic][Nle]P[4-Cl-F]{FreeAcid} | [PE65;hArg68;Cha69;Atz(10K-mPEG);Oic74;Nle75;4-Cl-F77]Apelin-13(65-77) | 461 |
| {Hydrogen} [PE]RP[hArg] [Cha] S[Atz(20K-mPEG)]KG[Oic][Nle]P[4-Cl-F]{FreeAcid} | [PE65;hArg68;Cha69;Atz(20K-mPEG)71;Oic74;Nle75;4-Cl-F77]Apelin-13(65-77) | 462 |
| {Acetyl}[Atz(20K-mPEG)]LRP[hArg][NMeLeu][Atz(20K-mPEG)]HKGP[Nle]P[4-Cl-F]{FreeACid} | Ac-[Atz(20K-mPEG)]-[Leu65;hArg68;NMeLeu69;[Atz(20 K-mPEG)]70;Nle75;4-Cl-F77]Apelin-13(65-77) | 463 |
| {Hydrogen}[PE]RP[hArg][NMeLeu]SHKGP[ Nle]P[D-Bip]K(Ac-Atz(20K-mPEG)) | PE65;hArg68;NMeLeu69;Nle75;D-Bip77;K(Ac-Atz(20K-mPEG))78]Apelin-13 (65-78)-amide | 464 |
| {Acetyl}[Atz(1K-mPEG)]LRP[hArg][Cha]SHKG[Oic][Nle]P[4-Cl-F] {FreeAcid} | Ac-Atz(1K-mPEG)-[Leu65;hArg68;Cha69;Oic74;Nle75 ;4-Cl-F77]Apelin-13(65-77) | 465 |
| {Acetyl}[Atz(2K-mPEG)]LRP[hArg][Cha]SHKG[Oic][Nle]P[4-Cl-F] {FreeAcid} | Ac-Atz(2K-mPEG)-[Leu65;hArg68;Cha69;Oic74;Nle75 ;4-Cl-F77]Apelin-13(65-77) | 466 |
| {Acetyl}[Atz(5K-mPEG)]LRP[hArg][Cha]SHKG[Oic][Nle]P[4-Cl-F] {FreeAcid} | Ac-Atz(5K-mPEG)-[Leu65;hArg68;Cha69;Oic74;Nle75 ;4-Cl-F77]Apelin-13(65-77) | 467 |
| {Acetyl}[Atz(10K-mPEG)]LRP[hArg][Cha]SHKG[Oic][Nle]P[4-Cl-F]{FreeAcid} | Ac-Atz(10K-mPEG)-[Leu65;hArg68;Cha69;Oic74;Nle75 ;4-Cl-F77]Apelin-13(65-77) | 468 |
| {Acetyl}[Atz(20K-mPEG)]LRP[hArg][Cha]SHKG[Oic][Nle]P[4-Cl-F] {FreeAcid} | Ac-Atz(20K-mPEG)-[Leu65;hArg68;Cha69;Oic74;Nle75 ;4-Cl-F77]Apelin-13(65-77) | 469 |
| {Hydrogen} [Atz(20K-mPEG)]KFRRQRP[hArg] [Cha]SHKG[Oic][N1 e]P[4-Cl-F] {FreeAcid} | Atz(20K-mPEG)-[hArg68;Cha69;Oic74;Nle75;4-Cl-F77]Apelin-17(61-77) | 470 |
| {Hydrogen}[Atz(20K-mPEG)]LRP[hArg][Cha]SHKG[Oic][Nle]P[4-Cl-F]{FreeAcid} | Atz(20K-mPEG)-[Leu65;hArg68;Cha69;Oic74;Nle75 ;4-Cl-F77]Apelin-13(65-77) | 471 |
| {Thiopropionyl(20K-mPEG)}LRP[hArg][Cha]SHKG[Oic][Nle]P[4-Cl-F]{FreeAcid} | Thiopropionyl(20K-mPEG)-[Leu65;hArg68;Cha69;Oic74;Nle75 ;4-Cl-F77]Apelin-13(65-77) | 472 |
| {Thiopropionyl(20K-mPEG)}KFRRQRP[hArg] [Cha]SHKG[Oic][N1 e]P[4-Cl-F] {FreeAcid} | Thiopropionyl(20K-mPEG)-[hArg68;Cha69;Oic74;Nle75;4-Cl-F77]Apelin-17(61-77) | 473 |
| {Thiopropionyl(40K-mPEG)}KFRRQRP[hArg] [Cha]SHKG[Oic][N1 e]P[4-Cl-F] {FreeAcid} | Thiopropionyl(40K-mPEG)-[hArg68;Cha69;Oic74;Nle75;4-Cl-F77]Apelin-17(61-77) | 474 |
| Thiopropionyl(20K-mPEG)}LLRP[hArg][Cha]SHKG[Oic][Nle]P[ 4-Cl-F]{FreeAcid} | Thiopropionyl(20K-mPEG)-Leu-[Leu65;hArg68;Cha69;Oic74;Nle75 ;4-Cl-F77]Apelin-13(65-77) | 475 |
| {Thiopropionyl(20K-mPEG)}FRRQRP[hArg][Cha]SHKG[Oic][Nle ]P[4-Cl-F]{COOH} | Thiopropionyl(20K-mPEG)-[hArg68;Cha69;Oic74;Nle75;4-Cl-F77]Apelin-17(62-77) | 476 |
| {Thiopropionyl(20K-mPEG)}RRQRP[hArg] [Cha]SHKG[Oic][Nle] P[4-Cl-F]{COOH} | Thiopropionyl(20K-mPEG)-[hArg68;Cha69;Oic74;Nle75;4-Cl-F77]Apelin-17(63-77) | 477 |
| {Thiopropionyl(20K-mPEG)}RQRP[hArg][Cha]SHKG[Oic][Nle]P[ 4-Cl-F]{COOH} | Thiopropionyl(20K-mPEG)-[hArg68;Cha69;Oic74;Nle75;4-Cl-F77]Apelin-17(64-77) | 478 |
| {Thiopropionyl(20K-mPEG)}QRP[hArg][Cha]SHKG[Oic][Nle]P[4-Cl-F]{COOH} | Thiopropionyl(20K-mPEG)-[hArg68;Cha69;Oic74;Nle75;4-Cl-F77]Apelin-17(65-77) | 479 |
| {Thiopropionyl(1K-mPEG)}QRP[hArg][Cha]SHKG[Oic][Nle]P[4-Cl-F]{FreeAcid} | Thiopropionyl(1K-mPEG)-[hArg68;Cha69;Oic74;Nle75;4-Cl-F77]Apelin-17(65-77) | 480 |
| {Thiopropionyl(2K-mPEG)}QRP[hArg][Cha]SHKG[Oic][Nle]P[4-Cl-F] {FreeAcid} | Thiopropionyl(2K-mPEG)-[hArg68;Cha69;Oic74;Nle75;4-Cl-F77]Apelin-17(65-77) | 483 |
| {Thiopropionyl(5K-mPEG)}QRP[hArg][Cha]SHKG[Oic][Nle]P[4-Cl-F]{FreeAcid} | Thiopropionyl(5K-mPEG)-[hArg68;Cha69;Oic74;Nle75;4-Cl-F77]Apelin-17(65-77) | 481 |
| {Thiopropionyl(10K-mPEG)}QRP[hArg][Cha]SHKG[Oic][Nle]P[4-Cl-F]{FreeAcid} | Thiopropionyl(10K-mPEG)-[hArg68;Cha69;Oic74;Nle75;4-Cl-F77]Apelin-17(65-77) | 482 |
| {Thiopropionyl(40K-mPEG)}QRP[hArg][Cha]SHKG[Oic][Nle]P[4-Cl-F]{FreeAcid} | Thiopropionyl(40K-mPEG)-[hArg68;Cha69;Oic74;Nle75;4-Cl-F77]Apelin-17(65-77) | 484 |
| {Thiopropionyl(1K-mPEG)}KFRRQRP[hArg] [Cha]SHKG[Oic][N1 e]P[4-Cl-F] {FreeAcid} | Thiopropionyl(1K-mPEG)-[hArg68;Cha69;Oic74;Nle75;4-Cl-F77]Apelin-17(61-77) | 485 |
| {Thiopropionyl(2K-mPEG)}KFRRQRP[hArg] [Cha]SHKG[Oic][N1 e]P[4-Cl-F] {FreeAcid} | Thiopropionyl(2K-mPEG)-[hArg68;Cha69;Oic74;Nle75;4-Cl-F77]Apelin-17(61-77) | 486 |
| {Thiopropionyl(5K-mPEG)}KFRRQRP[hArg] [Cha]SHKG[Oic][N1 e]P[4-Cl-F] {FreeAcid} | Thiopropionyl(5K-mPEG)-[hArg68;Cha69;Oic74;Nle75;4-Cl-F77]Apelin-17(61-77) | 487 |
| {Thiopropionyl(10K-mPEG)}KFRRQRP[hArg][Cha]SHKG[Oic][Nl e]P[4-Cl-F]{FreeAcid} | Thiopropionyl(10K-mPEG)-[hArg68;Cha69;Oic74;Nle75;4-Cl-F77]Apelin-17(61-77) | 488 |
| {Thiopropionyl(40K-mPEG)}KFRRQRP[hArg] [Cha]SHKG[Oic][N1 e]P[4-Cl-F] {FreeAcid} | Thiopropionyl(40K-mPEG)-[hArg68;Cha69;Oic74;Nle75;4-Cl-F77]Apelin-17(61-77) | 489 |
| {Thiopropionyl(20K-mPEG) }F[hArg][hArg]Q[hArg]P[hArg][Cha]S HKG[Oic][Nle]P[4-Cl-F] | Thiopropionyl(20K-mPEG)-[hArg63,64,66,68;Cha69;O ic74;Nle75;4-Cl-F77]Apelin-17(62-77) | 490 |
| {Thiopropionyl(20K-mPEG) }OF[hArg][hArg]Q[hArg]P[hArg][Cha] SHKG[Oic][Nle]P[4-Cl-F] | Thiopropionyl(20K-mPEG)-74;Nle75;4-Cl-F77]Apelin-17(61-[Orn61;hArg63,64,66,68;Cha69;Oic 77) | 491 |
| {Thiopropionyl(20K-mPEG)}OF[hArg][hArg]Q[Cit]P[hArg][Cha]S HKG[Oic][Nle]P[4-Cl-F] | Thiopropionyl(20K-mPEG)-[Orn61;hArg63,64,68;Cit66;Cha69; Oic74;Nle75;4-Cl-F77]Apelin-17(61-77) | 492 |
| {Thiopropionyl(20K-mPEG)}F[hArg][hArg]Q[Cit]P[hArg][Cha]SH KG[Oic] [Nle]P[4-Cl-F] | Thiopropionyl(20K-mPEG)-[hArg63,64,68;Cit66;Cha6 9;Oic74;Nle75;4-Cl-F77]Apelin-17(62-77) | 493 |
| {Thiopropionyl(20K-mPEG)}F[Cit][Cit]Q[hArg]P[hArg][Cha]SHK G[Oic][Nle]P[4-Cl-F] | Thiopropionyl(20K-mPEG)-[Cit63,64;hArg66,68;Cha6 9;Oic74;Nle75;4-Cl-F77]Apelin-17(62-77) | 494 |
| [NPeg11]KFRRQRP[hArg][Cha]SHKG[Oic][ Nle]P[4-Cl-F] | NPeg11-[hArg68;Cha69;Oic74;Nle75;4-Cl-F77]Apelin-17(61-77) | 495 |
| [Atz(PEG10)]KFRRQRP[hArg][Cha]SHKG[O ic][Nle]P[4-Cl-F] | Atz(Peg10)-[hArg68;Cha69;Oic74;Nle75;4-Cl-F77]Apelin-17(61-77) | 496 |
| [Atz(PEG10)]KF[hArg][hArg]QRP[hArg][Cha ]SHKG[Oic][Nle]P[4-Cl-F] | Atz(Peg10)-[hArg63,64,68;Cha69;Oic74;Nle75; 4-Cl-F77]Apelin-17(61-77) | 497 |
| [Atz(PEG10KF[hAr[hArg]Q[hArg]P[hArg] [Cha]SHKG[Oic][Nle]P[4-Cl-F] | Atz(Peg10)-[hArg63,64,66,68;Cha69;Oic74;Nle 75;4-Cl-F77]Apelin-17(61-77) | 498 |
| [Atz(PEG10)]KF[hArg][hArg]QQP[hArg][Cha ]SHKG[Oic][Nle]P[4-Cl-F] | Atz(Peg10)-[hArg63,64,68;Gln66;Cha69;Oic74; Nle75;4-Cl-F77]Apelin-17(61-77) | 499 |
| [Atz(PEG10)]KFhArg]Q[Cit]P[hArg][ Cha]SHKG[Oic][Nle]P[4-Cl-F] | Atz(Peg10)-[hArg63,64,68;Cit66;Cha69;Oic74; Nle75;4-Cl-F77]Apelin-17(61-77) | 500 |
| [Atz(PEG10)]KF[hArg][Cit]Q[Cit]P[hArg][Ch a]SHKG[Oic][Nle]P[4-Cl-F] | Atz(Peg10)-[hArg63,68;Cit64,66;Cha69;Oic74; Nle75;4-Cl-F77]Apelin-17(61-77) | 501 |
| [Atz(PEG10)]KF[Cit][Cit]Q[hArg]P[hArg][Ch a]SHKG[Oic][Nle]P[4-Cl-F] | Atz(Peg10)-[Cit63,64;hArg66,68;Cha69;Oic74; Nle75;4-Cl-F77]Apelin-17(61-77) | 502 |
| [Atz(PEG10)]KF[Cit][Cit]Q[Cit]P[hArg][Cha] SHKG[Oic][Nle]P[4-Cl-F] | Atz(Peg10)-[Cit63,64,66;hArg68;Cha69;Oic74; Nle75;4-Cl-F77]Apelin-17(61-77) | 503 |
| [Atz(PEG10)]OFRRQRP[hArg][Cha]SHKG[O ic][Nle]P[4-Cl-F] | Atz(Peg10)-[Orn61;hArg68;Cha69;Oic74;Nle75 ;4-Cl-F77]Apelin-17(61-77) | 504 |
| [Atz(PEG10)]OF[hArg][hArg]Q[hArg]P[hArg] [Cha]SHKG[Oic][Nle]P[4-Cl-F] | Atz(Peg10)-[Orn61;hArg63,64,66,68;Cha69;Oic 74;Nle75;4-Cl-F77]Apelin-17(61-77) | 505 |
| [Atz(PEG10)]FRRQRP[hArg][Cha]SHKG[Oic ][Nle]P[4-Cl-F] | Atz(Peg10)-[hArg68;Cha69;Oic74;Nle75;4-Cl-F77]Apelin-17(62-77) | 506 |
| [Atz(PEG10)]LRP[hArg][Cha]SHKG[Oic][Nle ]P[4-Cl-F] | Atz(Peg10)-[Leu65;hArg68;Cha69;Oic74;Nle75 ;4-Cl-F77]Apelin-17(65-77) | 507 |

The following table shows examples of peptides . In the table, the free acid can be a carboxylic acid and the amide can be at the C-terminus.

**TABLE 8.**

| AMINO ACID SEQUENCE | DESIGNATION | SEQ ID NO. |
|---|---|---|
| {Hydrogen}[PE]RP[hArg][NMeLeu]FH KPPFPF{FreeAcid} | [PE65;hArg68;NMeLeu69;Phe70,75;Pro73] Apelin-13(65-77) | 508 |
| {Hydrogen}[PE]RP[hArg][NMeLeu]SH KGP[pI-Phe]P[4-F-F]{FreeAcid} | [PE65;hArg68;NMeLeu69;pI-Phe75;4-F-F77]Apelin-13(65-77) | 509 |
| {Hydrogen}[PE]RP[hArg][Cha]SHKGP[ pI-Phe]P[4-F-F]{FreeAcid} | [PE65;hArg68;Cha69;pI-Phe75;4-F-F77]Apelin-13(65-77) | 510 |
| {Hydrogen}[PE]RP[hArg][NMeLeu]SH KGP[pI-Phe]P[4-Me-F]{FreeAcid} | [PE65;hArg68;NMeLeu69;pI-Phe75;4-Me-F77]Apelin-13(65-77) | 511 |
| {Hydrogen} [PE]RP[hArg][Cha]SHKGP[ pI-Phe]P[4-Me-F]{FreeAcid} | [PE65;hArg68;Cha69;pI-Phe75;4-Me-F77]Apelin-13(65-77) | 512 |
| {Hydrogen} [PE]RP[hArg][Cha]SHKGP[ Nle]P[4-Me-F]{FreeAcid} | [PE65;hArg68;Cha69;Nle75;4-Me-F77]Apelin-13(65-77) | 513 |
| {Hydrogen} [PE]RP[hArg][Cha]SHKGP[ Nle]P[D-Bip]{FreeAcid} | [PE65;hArg68;Cha69;Nle75;D-Bip77]Apelin-13(65-77) | 514 |
| {Hydrogen}[PE]RP[hArg][NMeLeu]SH KGP[Nle]P[D-1Nal]{FreeAcid} | [PE65;hArg68;NMeLeu69;Nle75;D-1Nal77]Apelin-13(65-77) | 515 |
| {Hydrogen}[PE]RP[hArg][NMeLeu]SH KGP[pI-Phe]P[D-1Nal]{FreeAcid} | [PE65;hArg68;NMeLeu69;pI-Phe75;D-1Nal77]Apelin-13(65-77) | 516 |
| {Hydrogen} [PE]RP[hArg][Cha]SHKGP[ pI-Phe]P[D-1Nal]{FreeAcid} | [PE65;hArg68;Cha69;pI-Phe75;D-1Nal77]Apelin-13(65-77) | 517 |
| {Hydrogen}[PE]RP[hArg][Cha]SHKGP[ Nle]P[D-1Nal]{FreeAcid} | [PE65;hArg68;Cha69;Nle75;D-1Nal77]Apelin-13(65-77) | 518 |
| {Hydrogen} [PE]RP[hArg][Cha]SHKGP[ pI-Phe]P[pI-Phe]{FreeAcid} | [PE65;hArg68;Cha69;pI-Phe75,77]Apelin-13(65-77) | 519 |
| {Hydrogen}[PE]RP[hArg][Cha]SHKGP[ pI-Phe]PY{FreeAcid} | [PE65;hArg68;Cha69;pI-Phe75;Tyr77]Apelin-13(65-77) | 520 |
| {Hydrogen} [PE]RP[hArg][Cha]SHKGP[ Nle]P[pI-Phe]{FreeAcid} | [PE65;hArg68;Cha69;Nle75;pI-Phe77]Apelin-13(65-77) | 521 |
| {Hydrogen}[PE]RP[hArg][Cha]SHKGP[ pI-Phe]Pf{FreeAcid} | [PE65;hArg68;Cha69;pI-Phe75;D-Phe77]Apelin-13(65-77) | 522 |
| {Hydrogen} [PE]RP[hArg][Cha]SHKGP[ Nle]Pf{FreeAcid} | [PE65;hArg68;Cha69;Nle75;D-Phe77]Apelin-13(65-77) | 523 |
| {Hydrogen}[PE]RP[hArg][NMeLeu]SH KGP[Nle]P[NMePhe]{Amide} | [PE65;hArg68;NMeLeu69;Nle75;NMePhe7 7]Apelin-13(65-77)-amide | 524 |
| {Hydrogen}[PE]RP[hArg][NMeLeu]SH KGP[Nle]Pw{FreeAcid} | [PE65;hArg68;NMeLeu69;Nle75;D-Trp77]Apelin-13(65-77) | 525 |
| {Hydrogen}[PE]RP[hArg][NMeLeu]SH KGP[pI-Phe]Pw{FreeAcid} | [PE65;hArg68;NMeLeu69;pI-Phe75;D-Trp77]Apelin-13(65-77) | 526 |
| {Hydrogen}[PE]RP[hArg][Cha]SHKGP[ pI-Phe]Pw{FreeAcid} | [PE65;hArg68;Cha69;pI-Phe75;D-Trp77]Apelin-13(65-77) | 527 |
| {Hydrogen} [PE]RP[hArg][Cha]SHKGP[ Nle]Pw{FreeAcid} | [PE65;hArg68;Cha69;Nle75;D-Trp77]Apelin-13(65-77) | 528 |
| {Hydrogen}[PE]RP[hArg][Cha]SHKGP[ Nle]PY{FreeAcid} | [PE65;hArg68;Cha69;Nle75;Tyr77]Apelin-13(65-77) | 529 |
| {Hydrogen}[PE]RP[hArg][NMeLeu]SH KGP[Nle]Py{FreeAcid} | [PE65;hArg68;NMeLeu69;Nle75;D-Tyr77]Apelin-13 (65-77) | 530 |
| {Hydrogen}[PE]RP[hArg][NMeLeu]SH KGP[pI-Phe]Py{FreeAeid} | [PE65;hArg68;NMeLeu69;pI-Phe75;D-Tyr77]Apelin-13(65-77) | 531 |
| {Hydrogen} [PE]RP[hArg][Cha]SHKGP[ pI-Phe]Py{FreeAcid} | [PE65;hArg68;Cha69;pI-Phe75;D-Tyr77]Apelin-13 (65-77) | 532 |
| {Hydrogen}[PE]RP[hArg][Cha]SHKGP[ Nle]Py{FreeAcid} | [PE65;hArg68;Cha69;Nle75;D-Tyr77]Apelin-13(65-77) | 533 |
| {Hydrogen}[PE]RP[hArg][NMeLeu]FH KGP[pI-Phe]P[2-Nal]{FreeAcid} | [PE65;hArg68;NMeLeu69;Phe70;pI-Phe75;2-Nal77]Apelin-13 (65-77) | 534 |
| {Hydrogen}[PE]RP[hArg][NMeLeu]FH KGP[Nle]P[D-Bip]{Amide} | [PE65;hArg68;NMeLeu69;Phe70;Nle75;D-Bip77]Apelin-13(65-77)-amide | 535 |
| {Hydrogen}[PE]RP[hArg][NMeLeu]FH KGP[Nle]P[D-Bip]{FreeAcid} | [PE65;hArg68;NMeLeu69;Phe70;Nle75;D-Bip77]Apelin-13(65-77) | 536 |
| {Hydrogen}[PE]RP[hArg][NMeLeu]FH KGP[pI-Phe]P[D-Bip]{FreeAcid} | [PE65;hArg68;NMeLeu69;Phe70;pI-Phe75;D-Bip77]Apelin-1 3(65-77) | 537 |
| {Palmitoyl}LRP[hArg][NMeLeu]FHKGP [Nle]P[D-Bip]{FreeAcid} | Palmitoyl-[Leu65;hArg68;NMeLeu69;Phe70;Nle75;D-Bip77]Apelin-13 (65-77) | 538 |
| {Hydrogen} [PE]RP[hArg][Cha]SHKGP[ pI-Phe]P[D-Bip]{FreeAcid} | [PE65;hArg68;Cha69;pI-Phe75;D-Bip77]Apelin-13(65-77) | 539 |
| {Hydrogen}[PE]RP[hArg][NMeLeu]SH KGP[Nle]P[4-Me-F]{FreeAcid} | [PE65;hArg68;NMeLeu69;Nle75;4-Me-F77]Apelin-13(65-77) | 540 |
| {Hydrogen}[PE]RP[hArg][NMeLeu]FH KGP[pI-Phe]P[D-Bip]{Amide} | [PE65;hArg68;NMeLeu69;Phe70;pI-Phe75;D-Bip77]Apelin-13(65-77)-amide | 541 |
| {Hydrogen}[PE]RP[hArg][Cha]SHKGP[ Nle]Pf{Amide} | [PE65;hArg68;Cha69;Nle75;D-Phe77]Apelin-13(65-77)-amide | 542 |
| {Hydrogen}[PE]RP[hArg][NMeLeu]SH KGP[Nle]P[4-Cl-F]{Amide} | [PE65;hArg68;NMeLeu69;Nle75;4-Cl-F77]Apelin-13(65-77)-amide | 543 |
| {Hydrogen}[PE]RP[hArg][NMeLeu]SH KGP[Nle]P[4-Me-F]{Amide} | [PE65;hArg68;NMeLeu69;Nle75;4-Me-F77]Apelin-13(65-77)-amide | 544 |
| {Hydrogen}[PE]RP[hArg][NMeLeu]SH KGP[Nle]P[Bip]{Amide} | [PE65;hArg68;NMeLeu69;Nle75;Bip77]Ap elin-13(65-77)-amide | 545 |
| {Hydrogen}[PE]RP[hArg][NMeLeu]SH KGP[Nle]P[D-Bip]{Amide} | [PE65;hArg68;NMeLeu69;Nle75;D-Bip77]Apelin-13(65-77)-amide | 546 |
| {Hydrogen}[PE]RP[hArg][NMeLeu]SH KGP[Nle]P[4-NO2-F]{Amide} | [PE65;hArg68;NMeLeu69;Nle75;4-NO2-F77]Apelin-13(65-77)-amide | 547 |
| {Hydrogen}[PE]RP[hArg][NMeLeu]SH KG[1-Nal][Nle]P[4-Cl-F]{Amide} | [PE65;hArg68;NMeLeu69;1-Nal74;Nle75;4-Cl-F77]Apelin-13(65-77)-amide | 548 |
| {Hydrogen}[PE]RP[hArg][NMeLeu]FH KG[1-Nal][Nle]P[4-Cl-F]{Amide} | [PE65;hArg68;NMeLeu69;Phe70;1-Nal74;Nle75;4-Cl-F77]Apelin-13(65-77)-amide | 549 |
| {Hydrogen}[PE]RP[hArg][NMeLeu]FH KGP[Nle]P[4-Cl-F]{Amide} | [PE65;hArg68;NMeLeu69;Phe70;Nle75;4-Cl-F77]Apelin-13(65-77)-amide | 550 |
| {Hydrogen} [PE]RP[hArg][NMeLeu]SA KG[1-Nal][Nle]P[4-Cl-F]{Amide} | [PE65;hArg68;NMeLeu69;Ala71;1-Nal74;Nle75;4-Cl-F77]Apelin-13(65-77)-amide | 551 |
| {Hydrogen}[PE]RP[hArg][NMeLeu]FA KG[1-Nal][Nle]P[4-Cl-F]{Amide} | [PE65;hArg68;NMeLeu69;Phe70;Ala71;1-Nal74;Nle75;4-Cl-F77]Apelin-13(65-77)-amide | 552 |
| {Hydrogen}[PE]RP[hArg][NMeLeu]FA KGP[Nle]P[4-Cl-F]{Amide} | [PE65;hArg68;NMeLeu69;Phe70;Ala71;Nle 75;4-Cl-F77]Apelin-13(65-77)-amide | 553 |
| {Hydrogen}[PE]RP[hArg][NMeLeu]S[1-Nal]KG[1-Nal][Nle]P[4-Cl-F]{Amide} | [PE65;hArg68;NMeLeu69;1-Nal71,74;Nle75;4-Cl-F77]Apelin-13(65-77)-amide | 554 |
| {Hydrogen}[PE]RP[hArg][NMeLeu]F[1-Nal]KG[1-Nal][Nle]P[4-Cl-F]{Amide} | [PE65;hArg68;NMeLeu69;Phe70;1-Nal71,74;Nle75;4-Cl-F77]Apelin-13(65-77)-amide | 555 |
| {Hydrogen}[PE]RP[hArg][NMeLeu]F[1-Nal]KGP[Nle]P[4-Cl-F]{Amide} | [PE65;hArg68;NMeLeu69;Phe70;1-Nal71;Nle75;4-Cl-F77]Apelin-13(65-77)-amide | 556 |
| {Hydrogen}[PE]RP[hArg][NMeLeu]F[1- Nal]KGP[Nle]P[4-Cl-F]{Amide} | [PE65;hArg68;NMeLeu69;Phe70;1-Nal71;Nle75;4-Cl-F77]Apelin-13(65-77)-amide | 557 |
| {Hydrogen}[PE]RP[hArg][NMeLeu][NM ePhe][1-Nal]KGP[Nle]P[4-Cl-F] {Amide} | [PE65;hArg68;NMeLeu69;NMePhe70;1-Na171;Nle75;4-Cl-F77]Apelin-13 (65-77)-amide | 558 |
| {Hydrogen}[PE]RP[hArg][NMeLeu][NM ePhe]AKG[1-Nal][Nle]P[4-Cl-F]{Amide} | [PE65;hArg68;NMeLeu69;NMePhe70;Ala7 1;1-Nal74;Nle75;4-Cl-F77]Apelin-13(65-77)-amide | 559 |
| {Hydrogen}[PE]RP[hArg][NMeLeu][NM ePhe]AKG[1-Nal][Nle]P[4-Cl-F]{Amide} | [PE65;hArg68;NMeLeu69;NMePhe70;Ala7 1;1-Nal74;Nle75;4-Cl-F77]Apelin-13(65-77)-amide | 560 |
| {Hydrogen}[PE]RP[hArg][NMeLeu]F[2-Nal]KG[1-Nal][Nle]P[4-Cl-F]{Amide} | [PE65;hArg68;NMeLeu69;Phe70;2-Nal71;1-Nal74;Nle75;4-Cl-F77]Apelin-13(65-77)-amide | 561 |
| {Hydrogen}[PE]RP[hArg][NMeLeu]F[2-Nal]KGP[Nle]P[4-Cl-F]{Amide} | [PE65;hArg68;NMeLeu69;Phe70;2-Nal71;Nle75;4-Cl-F77]Apelin-13 (65-77)-amide | 562 |
| {Hydrogen}[PE]RP[hArg][NMeLeu][NM ePhe][2-Nal]KGP[Nle]P[4-Cl-F] {Amide} | [PE65;hArg68;NMeLeu69;NMePhe70;2-Nal71;Nle75;4-Cl-F77]Apelin-13 (65-77)-amide | 563 |
| [PE]RP[hArg][NMeLeu]SHKGP[Nle]P[ D-Bip] | [PE65;hArg68;NMeLeu69;Nle75;D-Bip77]Apelin-13(65-77) | 564 |
| [PE]RP[hArg][NMeLeu]SHKGP[pI-Phe]P[D-Bip] | [PE65;hArg68;NMeLeu69;pI-Phe75;D-Bip77]Apelin-13(65-77) | 565 |
| [PE]RP[hArg][NMeLeu]SHKGP[pI-Phe]P[2-Nal] | [PE65;hArg68;NMeLeu69;pI-Phe75;2-Nal77]Apelin-13(65-77) | 566 |
| {Palmitoyl}LRP[hArg][NMeLeu]SHKGP [pI-Phe]P[D-Bip]{Amide} | Palmitoyl-Leu65;hArg68;NMeLeu69;pI-Phe75;D-Bip77]Apelin-13(65-77) | 567 |
| [PE]RP[hArg][NMeLeu]SHKGP[Nle]P[ D-Bip]K(Palmitoyl){Amide} | PE65;hArg68;NMeLeu69;Nle75;D-Bip77;K(Palmitoyl)78]Apelin-13 (65-78)-amide | 568 |
| [PE]RP[hArg][NMeLeu]SHKGP[Nle]P[2 -Nal] | [PE65;hArg68;NMeLeu69;Nle75;2-Nal77]Apelin-13(65-77) | 569 |
| [PE]RP[hArg][NMeLeu]SHKGP[pI-Phe]P[4-Cl-F] | [PE65;hArg68;NMeLeu69;pI-Phe75;4-Cl-F77]Apelin-13(65-77) | 570 |
| [PE]RP[hArg][NMeLeu]SHKGP[Nle]P[4 -Cl-F] | [PE65;hArg68;NMeLeu69;Nle75;4-Cl-F77]Apelin-13(65-77) | 571 |
| [PE]RP[hArg][NMeLeu]SHKGP[Nle]P[4 -F-F] | [PE65;hArg68;NMeLeu69;Nle75;4-F-F77]Apelin-13(65-77) | 572 |
| [PE]RP[hArg][Cha]SHKGP[Nle]P[4-F-F] | [PE65;hArg68;Cha69;Nle75;4-F-F77]Apelin-13(65-77) | 573 |
| [PE]RP[hArg][NMeLeu]SHKGP[Nle]P[p I-Phe] | [PE65;hArg68;NMeLeu69;Nle75;pI-Phe77]Apelin-13(65-77) | 574 |
| [PE]RP[hArg][NMeLeu]SHKGP[Nle]Pf | [PE65;hArg68;NMeLeu69;Nle75;D-Phe77]Apelin-13(65-77) | 575 |
| {Palmitoyl}LRP[hArg][NMeLeu]SHKGP [pI-Phe]P[D-Bip]{Amide} | Palmitoyl-Leu65;hArg68;NMeLeu69;pI-Phe75;D-Bip77]Apelin-13(65-77) | 576 |
| [PE]RP[hArg][NMeLeu]SHKGP[Nle]P[ D-Bip]K(Palmitoyl){Amide} | PE65;hArg68;NMeLeu69;Nle75;D-Bip77;K(Palmitoyl)78]Apelin-13 (65-78)-amide | 577 |
| [PE]RP[hArg][Cha]SHKGP[pI-Phe]P[2-Nal] | [PE65;hArg68;Cha69;pI-Phe75;2-Nal77]Apelin-13(65-77) | 578 |
| {Acetyl}[Pra]LRP[hArg][NMeLeu][Pra] HKGP[Nle]P[4-Cl-F]{FreeAcid} | Ac-Pra-[Leu65,hArg68,NMeLeu69,Pra70,Nle75,4-Cl-F77]Apelin-13(65-77) | 579 |
| {Acetyl}[Pra]LRP[hArg][NMeLeu][Pra] HKGP[Nle]P[4-Cl-F]{FreeAcid} | Ac-Pra-[Leu65;hArg68;NMeLeu69;Pra70;Nle75;4-Cl-F77]Apelin-13(65-77) | 580 |
| {Acetyl}[Pra]LRP[NMeArg][NMeLeu][P ra]HKGP[Nle]P[4-Cl-F]{FreeAcid} | Ac-Pra-[Leu65;NMeArg68;NMeLeu69;Pra70;Nle7 5;4-Cl-F77]Apelin-13(65-77) | 581 |
| {Acetyl }[Pra]L[NMeArg]P[hArg][NMeL eu][Pra]HKGP[Nle]P[4-Cl-F]{FreeAcid} | Ac-Pra-[Leu65;NMeArg66;hArg68;NMeLeu69;Pra 70;Nle75;4-Cl-F77]Apelin-13(65-77) | 582 |
| {Acetyl }[Pra]L[NMeArg]P[NMeArg][N MeLeu][Pra]HKGP[Nle]P[4-Cl-F]{FreeAcid} | Ac-Pra-[Leu65;NMeArg66,68;NMeLeu69;Pra70;Nl e75;4-Cl-F77]Apelin-13(65-77) | 583 |
| [PE]RP[hArg][NMeLeu][Pra]HKG[Oic][ Nle]P[4-Cl-F]{FreeAcid} | [PE65;hArg68;NMeLeu69;Pra70;Oic74;Nle 75;4-Cl-F77]Apelin-13(65-77) | 584 |
| [PE]RP[hArg][NMeLeu][Pra]HKG[Oic][ Nle]P[4-Cl-F]{FreeAcid} | [PE65;hArg68;NMeLeu69;Pra70;Oic74;Nle 75;4-Cl-F77]Apelin-13(65-77) | 585 |
| [PE]RP[NMeArg][NMeLeu][Pra]HKG[O ic][Nle]P[4-Cl-F]{FreeAcid} | [PE65;NMeArg68;NMeLeu69;Pra70;Oic74; Nle75;4-Cl-F77]Apelin-13(65-77) | 586 |
| [PE][NMeArg]P[hArg][NMeLeu][Pra]H KG[Oic][Nle]P[4-Cl-F]{FreeAcid} | [PE65;NMeArg66;hArg68;NMeLeu69;Pra7 0;Oic74;Nle75;4-Cl-F77]Apelin-13(65-77) | 587 |
| [PE][NMeArg]P[NMeArg][NMeLeu][Pra ]HKG[Oic][Nle]P[4-Cl-F] {FreeAcid} | [PE65;NMeArg66,68;NMeLeu69;Pra70;Oic 74;Nle75;4-Cl-F77]Apelin-13(65-77) | 588 |
| {Hydrogen} [PE]RP[hArg]LFHKGPF[1-Nal]F{FreeAcid} | [PE65;hArg68;Phe70,75;1-Nal76]Apelin-13(65-77) | 589 |
| {Hydrogen}[PE]RPR[Chg]FHKGPF[1-Nal]F{FreeAcid} | [PE65;Chg69;Phe70,75;1-Nal76]Apelin-13(65-77) | 132 |
| {Hydrogen} [PE]RPR[NMeLeu]FHKGPF [1-Nal]F{FreeAcid} | [PE65;NMeLeu69;Phe70,75;1-Nal76]Apelin-13(65-77) | 137 |
| {Hydrogen}[PE]RPR[Cha]FHKGP[Nle][ 1-Nal]F{FreeAcid} | [PE65;Cha69;Phe70;Nle75;1-Nal76]Apelin-13(65-77) | 196 |
| {Hydrogen}[PE]RPRLFHKGP[Nle][1-Nal]F{FreeAcid} | [PE65;Phe70;Nle75;1-Nal76]Apelin-13(65-77) | 197 |
| {Hydrogen}[PE]RP[hArg][Cha]FHKGP[ Nle][1-Nal]F{FreeAcid} | [PE65;hArg68;Cha69;Phe70;Nle75;1-Nal76]Apelin-13(65-77) | 198 |
| {Hydrogen} [PE]RP[hArg]LFHKGP[Nle] [1-Nal]F{FreeAcid} | [PE65;hArg68;Phe70;Nle75;1-Nal76]Apelin-13(65-77) | 199 |
| {Hydrogen}[PE]RPR[Chg]FHKGP[Nle][ 1-Nal]F{FreeAcid} | [PE65;Chg69;Phe70;Nle75;1-Nal76]Apelin-13(65-77) | 377 |
| {Hydrogen}[PE]RPRLFHKGPF[1-Nal]F{FreeAcid} | [PE65;Phe70,75;1-Nal76]Apelin-13(65-77) | 455 |
| {Hydrogen} [PE]RPR[Cha]FHKGPM[1-Nal]F{FreeAcid} | [PE65;Cha69;Phe70;1-Nal76]Apelin-13(65-77) | 595 |
| {Hydrogen}[PE]RP[hArg][Cha]FHKGP M[1-Nal]F{FreeAcid} | [PE65;hArg68;Cha69;Phe70;1-Nal76]Apelin-13(65-77) | 1014 |
| {Hydrogen}[PE]RPRLFHKGPM[1-Nal]F{FreeAcid} | [PE65;Phe70;1-Nal76]Apelin-13(65-77) | 1075 |
| {Hydrogen} [PE]RP[hArg]LFHKGPM[1-Nal]F{FreeAcid} | [PE65;hArg68;Phe70;1-Nal76]Apelin-13(65-77) | 1154 |
| {Hydrogen}[PE]RPR[Chg]FHKG,PM[1-Nal]F{FreeAcid} | [PE65;Chg69;Phe70;1-Nal76]Apelin-13(65-77) | 1172 |
| {Hydrogen}[PE]RPR[NMeLeu]FHKGP M[1-Nal]F{FreeAcid} | [PE65;NMeLeu69;Phe70;1-Nal76]Apelin-13(65-77) | 1173 |
| {Hydrogen}[PE]RPR[Cha]FHKGPF[1-Nal]F{FreeAcid} | [PE65;Cha69;Phe70,75;1-Nal76]Apelin-13(65-77) | 1174 |
| {Hydrogen}[PE]RP[hArg][Cha]FHKGPF [1-Nal]F{FreeAcid} | [PE65;hArg68;Cha69;Phe70,75;1-Nal76]Apelin-13(65-77) | 1175 |
| {Hydrogen}[PE]RPR[NMeLeu]FHKGP[ Nle][1-Nal]F{FreeAcid} | [PE65;NMeLeu69;Phe70;Nle75;1-Nal76]Apelin-13(65-77) | 1176 |
| {Hydrogen}[PE]RP[hArg][Cha]FHKGP[ pI-Phe][1-Nal]F{FreeAcid} | [PE65;hArg68;Cha69;Phe70;pI-Phe75;1-Nal76]Apelin-13(65-77) | 1177 |
| {Hydrogen}[PE]RP[hArg]LFHKGP[pI-Phe][1-Nal]F{FreeAcid} | [PE65;hArg68;Phe70;pI-Phe75;1-Nal76]Apelin-13(65-77) | 1425 |
| {Hydrogen}[PE]RPR[NMeLeu]FHKGP[ pI-Phe][1-Nal]F{FreeAcid} | [PE65;NMeLeu69;Phe70;pI-Phe75;1-Nal76]Apelin-13(65-77) | 1440 |
| [PE]RP[hArg][Cha]SHKGP[Nle][4-Cl-F]K(palmitoyl){Amide} | PE65;hArg68;Cha69;Nle75;4-Cl-F77;K(Palmitoyl)78]Apelin-13(65-78)-amide | 1441 |
| [PE]RP[hArg][Cha]SHKGP[Nle][D-1Nal]K(palmitoyl){Amide} | PE65;hArg68;Cha69;Nle75;D-1Nal77;K(Palmitoyl)78]Apelin-13(65-78)-amide | 1442 |
| {Acetyl}[Pra]LRP[hArg][NMeLeu][Pra] HKGP[Nle][Oic][4-Cl-F]{FreeAcid} | Ac-Pra-[Leu65;hArg68;NMeLeu69;Pra70;Nle75;Oi c76;4-Cl-F77]Apelin-13(65-77) | 1443 |
| {Acetyl}[Pra]LRP[hArg][NMeLeu][Pra] HKG[Oic][Nle][Oic][4-Cl-F]{FreeAcid} | Ac-Pra-[Leu65;hArg68;NMeLeu69;Pra70;Oic74,76; Nle75;4-Cl-F77]Apelin-13(65-77) | 1444 |
| {Acetyl}[Pra]LRP[hArg][NMeLeu][Pra] HKG[Oic][Nle][Tic][4-Cl-F]{FreeAcid} | Ac-Pra-[Leu65;hArg68;NMeLeu69;Pra70;Oic74;Nl e75;Tic76;4-Cl-F77]Apelin-13(65-77) | 1445 |
| [PE]RP[hArg][NMeLeu][Pra]HKG[Oic][ Nle][Oic][4-Cl-F]{FreeAcid} | [PE65;hArg68;NMeLeu69;Pra70;Oic74,76; Nle75;4-Cl-F77]Apelin-13(65-77) | 1446 |
| {Hydrogen} [PE]RP[hArg]LFHKGPF[1-Nal]F{FreeAcid} | [PE65;hArg68;Phe70,75;1-Nal76]Apelin-13(65-77) | 1447 |
| {Hydrogen} [PE]RP[hArg]LFKKPPFPF { FreeAcid} | [PE65;hArg68;Phe70,75;Lys71;Pro73]Apeli n-13(65-77) | 1448 |
| {Hydrogen} [PE]RPR[Chg]FHKPPFPF {F reeAcid} | [PE65;Chg69;Phe70,75;Pro73]Apelin-13(65-77) | 1449 |
| {Hydrogen}[PE]RPR[Chg]SHKPPF[1-Nal]F{FreeAcid} | [PE65;Chg69;Pro73;Phe75;1-Nal76]Apelin-13(65-77) | 1560 |
| {Hydrogen}[PE]RPR[Chg]FHKGPF[1-Nal]F{FreeAcid} | [PE65;Chg69;Phe70,75;1-Nal76]Apelin-13(65-77) | 590 |
| {Hydrogen}[PE]RPR[Chg]FKKPPF[1-Nal]F{FreeAcid} | [PE65;Chg69;Phe70,75;Lys71;Pro73;1-Nal76]Apelin-13(65-77) | 591 |
| {Hydrogen} [PE]RPR[Chg]FKKPPFPF {F reeAcid} | [PE65;Chg69;Phe70,75;Lys71;Pro73]Apelin -13(65-77) | 592 |
| {Hydrogen}[PE]RPR[NMeLeu]FHKPPF PF{FreeAcid} | [PE65;NMeLeu69;Phe70,75;Pro73]Apelin-13(65-77) | 593 |
| {Hydrogen}[PE]RPR[NMeLeu]SHKPPF[ 1-Nal]F{FreeAcid} | [PE65;NMeLeu69;Pro73;Phe75;1-Nal76]Apelin-13(65-77) | 594 |
| {Hydrogen}[PE]RPR[NMeLeu]FHKPPF[ 1-Nal]F {FreeAcid} | [PE65;NMeLeu69;Phe70,75;Pro73;1-Nal76]Apelin-13(65-77) | 596 |
| {Hydrogen} [PE]RPR[NMeLeu]FHKGPF [1-Nal]F{FreeAcid} | [PE65;NMeLeu69;Phe70,75;1-Nal76]Apelin-13(65-77) | 597 |
| {Hydrogen} [PE]RPR[NMeLeu]FKKPPF PF{FreeAcid} | [PE65;NMeLeu69;Phe70,75;Lys71;Pro73]A pelin-13(65-77) | 598 |
| {Hydrogen}[PE]RPR[Cha]FHKPP[Nle]P F{FreeAcid} | [PE65;Cha69;Phe70;Pro73;Nle75]Apelin-13(65-77) | 599 |
| {Hydrogen}[PE]RPR[Cha]SHKPP[Nle][ 1-Nal]F{FreeAcid} | [PE65;Cha69;Pro73;Nle75;1-Nal76]Apelin-13(65-77) | 600 |
| {Hydrogen}[PE]RPR[Cha]FHKPP[Nle][ 1-Nal]F{FreeAcid} | [PE65;Cha69;Phe70;Pro73;Nle75;1-Nal76]Apelin-13(65-77) | 601 |
| {Hydrogen}[PE]RPR[Cha]FHKGP[Nle][ 1-Nal]F{FreeAcid} | [PE65;Cha69;Phe70;Nle75;1-Nal76]Apelin-13(65-77) | 602 |
| {Hydrogen}[PE]RPR[Cha]FKKPP[Nle][ 1-Nal]F{FreeAcid} | [PE65;Cha69;Phe70;Lys71;Pro73;Nle75;1-Nal76]Apelin-13(65-77) | 603 |
| {Hydrogen}[PE]RPR[Cha]FKKPP[Nle]P F{FreeAcid} | [PE65;Cha69;Phe70;Lys71;Pro73;Nle75]Ap elin-13(65-77) | 604 |
| {Hydrogen}[PE]RP[hArg][Cha]SHKPP[ Nle]PF{FreeAcid} | [PE65;hArg68;Cha69;Pro73;Nle75]Apelin-13(65-77) | 605 |
| {Hydrogen}[PE]RP[hArg][Cha]FHKPP[ Nle]PF{FreeAcid} | [PE65;hArg68;Cha69;Phe70;Pro73;Nle75]A pelin-13(65-77) | 606 |
| {Hydrogen}[PE]RP[hArg][Cha]SHKPP[ Nle][1-Nal]F{FreeAcid} | [PE65;hArg68;Cha69;Pro73;Nle75;1-Nal76]Apelin-13(65-77) | 607 |
| {Hydrogen}[PE]RP[hArg][Cha]FHKPP[ Nle][1-Nal]F{FreeAcid} | [PE65;hArg68;Cha69;Phe70;Pro73;Nle75;1 -Nal76]Apelin-13(65-77) | 608 |
| {Hydrogen}[PE]RP[hArg][Cha]FHKGP[ Nle][1-Nal]F{FreeAcid} | [PE65;hArg68;Cha69;Phe70;Nle75;1-Nal76]Apelin-13(65-77) | 609 |
| {Hydrogen}[PE]RP[hArg][Cha]FKKPP[ Nle][1-Nal]F{FreeAcid} | [PE65;hArg68;Cha69;Phe70;Lys71;Pro73;N le75;1-Nal76]Apelin-13(65-77) | 610 |
| {Hydrogen}[PE]RP[hArg][Cha]FKKPP[ Nle]PF{FreeAcid} | [PE65;hArg68;Cha69;Phe70;Lys71;Pro73;N le75]Apelin-13(65-77) | 611 |
| {Hydrogen}[PE]RP[hArg]LFHKPP[Nle] PF{FreeAcid} | [PE65;hArg68;Phe70;Pro73;Nle75]Apelin-13(65-77) | 612 |
| {Hydrogen}[PE]RP[hArg]LSHKPP[Nle][ 1-Nal]F{FreeAcid} | [PE65;hArg68;Pro73;Nle75;1-Nal76]Apelin-13(65-77) | 613 |
| {Hydrogen}[PE]RP[hArg]LFHKPP[Nle][ 1-Nal]F{FreeAcid} | [PE65;hArg68;Phe70;Pro73;Nle75;1-Nal76]Apelin-13(65-77) | 614 |
| {Hydrogen} [PE]RP[hArg]LFHKGP[Nle] [1-Nal]F{FreeAcid} | [PE65;hArg68;Phe70;Nle75;1-Nal76]Apelin-13(65-77) | 615 |
| {Hydrogen}[PE]RP[hArg]LFKKPP[Nle][ 1-Nal]F{FreeAcid} | [PE65;hArg68;Phe70;Lys71;Pro73;Nle75;1-Nal76]Apelin-13(65-77) | 616 |
| {Hydrogen}[PE]RPR[Chg]SHKPP[Nle][ 1-Nal]F{FreeAcid} | [PE65;Chg69;Pro73;Nle75;1-Nal76]Apelin-13(65-77) | 617 |
| {Hydrogen}[PE]RPR[Chg]FHKPP[Nle][ 1-Nal]F{FreeAcid} | [PE65;Chg69;Phe70;Pro73;Nle75;1-Nal76]Apelin-13(65-77) | 618 |
| {Hydrogen}[PE]RPR[Chg]FHKGP[Nle][ 1-Nal]F{FreeAcid} | [PE65;Chg69;Phe70;Nle75;1-Nal76]Apelin-13(65-77) | 619 |
| {Hydrogen}[PE]RPR[Chg]FKKPP[Nle][ 1-Nal]F{FreeAcid} | [PE65;Chg69;Phe70;Lys71;Pro73;Nle75;1-Nal76]Apelin-13(65-77) | 620 |
| {Hydrogen}[PE]RPR[Chg]FKKPP[Nle]P F{FreeAcid} | [PE65;Chg69;Phe70;Lys71;Pro73;Nle75]Ap elin-13(65-77) | 621 |
| {Hydrogen} [PE]RPR[Cha]FHKPPM[1-Nal]F{FreeAcid} | [PE65;Cha69;Phe70;Pro73;1-Nal76]Apelin-13(65-77) | 622 |
| {Hydrogen} [PE]RPR[Cha]FHKGPM[1-Nal]F{FreeAcid} | [PE65;Cha69;Phe70;1-Nal76]Apelin-13(65-77) | 623 |
| {Hydrogen} [PE]RPR[Cha]FKKPPM[1-Nal]F{FreeAcid} | [PE65;Cha69;Phe70;Lys71;Pro73;1-Nal76]Apelin-13(65-77) | 624 |
| {Hydrogen} [PE]RPR[Cha]FKKPPMPF { FreeAcid} | [PE65;Cha69;Phe70;Lys71;Pro73]Apelin-13(65-77) | 625 |
| {Hydrogen}[PE]RP[hArg][Cha]FHKPP MPF{FreeAcid} | [PE65;hArg68;Cha69;Phe70;Pro73]Apelin-13(65-77) | 626 |
| {Hydrogen} [PE]RP[hArg][Cha]SHKPP M[1-Nal]F{FreeAcid} | [PE65;hArg68;Cha69;Pro73;1-Nal76]Apelin-13(65-77) | 627 |
| {Hydrogen}[PE]RP[hArg][Cha]FHKPP M[1-Nal]F{FreeAcid} | [PE65;hArg68;Cha69;Phe70;Pro73;1-Nal76]Apelin-13(65-77) | 628 |
| {Hydrogen}[PE]RP[hArg][Cha]FHKGP M[1-Nal]F{FreeAcid} | [PE65;hArg68;Cha69;Phe70;1-Nal76]Apelin-13(65-77) | 629 |
| {Hydrogen}[PE]RP[hArg][Cha]FKKPP M[1-Nal]F{FreeAcid} | [PE65;hArg68;Cha69;Phe70;Lys71;Pro73;1 -Nal76]Apelin-13(65-77) | 630 |
| {Hydrogen}[PE]RP[hArg][Cha]FKKPP MPF{FreeAcid} | [PE65;hArg68;Cha69;Phe70;Lys71;Pro73] Apelin-13(65-77) | 631 |
| {Hydrogen}[PE]RP[hArg]LFKKPPM[1-Nal]F{FreeAcid} | [PE65;hArg68;Phe70;Lys71;Pro73;1-Nal76]Apelin-13(65-77) | 632 |
| {Hydrogen}[PE]RP[hArg]LFKKPPMPF{ FreeAcid} | [PE65;hArg68;Phe70;Lys71;Pro73]Apelin-13(65-77) | 633 |
| {Hydrogen} [PE]RPRLFKKPPM[1-Nal]F{FreeAcid} | [PE65;Phe70;Lys71;Pro73;1-Nal76]Apelin-13(65-77) | 634 |
| {Hydrogen}[PE]RPR[Chg]FHKPPM[1-Nal]F{FreeAcid} | [PE65;Chg69;Phe70;Pro73;1-Nal76]Apelin-13(65-77) | 635 |
| {Hydrogen}[PE]RPR[Chg]FHKG,PM[1-Nal]F{FreeAcid} | [PE65;Chg69;Phe70;1-Nal76]Apelin-13(65-77) | 636 |
| {Hydrogen}[PE]RPR[Chg]FKKPPM[1-Nal]F{FreeAcid} | [PE65;Chg69;Phe70;Lys71;Pro73;1-Nal76]Apelin-13(65-77) | 637 |
| {Hydrogen} [PE]RPR[Chg]FKKPPMPF { FreeAcid} | [PE65;Chg69;Phe70;Lys71;Pro73]Apelin-13(65-77) | 638 |
| {Hydrogen} [PE]RPRLFK[1-Nal]PPM[1-Nal]F{FreeAcid} | [PE65;Phe70;Lys71;1-Nal72,76;Pro73]Apelin-13(65-77) | 639 |
| {Hydrogen}[PE]RPR[NMeLeu]FHKPPM [1-Nal]F{FreeAcid} | [PE65;NMeLeu69;Phe70;Pro73;1-Nal76]Apelin-13(65-77) | 640 |
| {Hydrogen}[PE]RPR[NMeLeu]FHKGP M[1-Nal]F{FreeAcid} | [PE65;NMeLeu69;Phe70;1-Nal76]Apelin-13(65-77) | 641 |
| {Hydrogen}[PE]RPR[NMeLeu]FKKPPM [1-Nal]F{FreeAcid} | [PE65;NMeLeu69;Phe70;Lys71;Pro73;1-Nal76]Apelin-13(65-77) | 642 |
| {Hydrogen}[PE]RPR[NMeLeu]FKKPPM PF{FreeAcid} | [PE65;NMeLeu69;Phe70;Lys71;Pro73]Apel in-13(65-77) | 643 |
| {Hydrogen}[PE]RPR[Cha]SHKPPF[1-Nal]F{FreeAcid} | [PE65;Cha69;Pro73;Phe75;1-Nal76]Apelin-13(65-77) | 644 |
| {Hydrogen}[PE]RPRLFKKPPMPF{Free Acid} | [PE65;Phe70;Lys71;Pro73]Apelin-13(65-77) | 645 |
| {Hydrogen}[PE]RPR[Cha]FHKPPF[1-Nal]F{FreeAcid} | [PE65;Cha69;Phe70,75;Pro73;1-Nal76]Apelin-13(65-77) | 646 |
| {Hydrogen}[PE]RPR[Cha]FHKGPF[1-Nal]F{FreeAcid} | [PE65;Cha69;Phe70,75;1-Nal76]Apelin-13(65-77) | 647 |
| {Hydrogen}[PE]RPR[Cha]FKKPPF[1-Nal]F{FreeAcid} | [PE65;Cha69;Phe70,75;Lys71;Pro73;1-Nal76]Apelin-13(65-77) | 648 |
| {Hydrogen} [PE]RPR[Cha]FKKPPFPF {F reeAcid} | [PE65;Cha69;Phe70,75;Lys71;Pro73]Apelin -13(65-77) | 649 |
| {Hydrogen}[PE]RP[hArg][Cha]SHKPPF PF{FreeAcid} | [PE65;hArg68;Cha69;Pro73;Phe75]Apelin-13(65-77) | 650 |
| {Hydrogen}[PE]RP[hArg][Cha]FHKPPF PF{FreeAcid} | [PE65;hArg68;Cha69;Phe70,75;Pro73]Apeli n-13(65-77) | 651 |
| {Hydrogen} [PE]RP[hArg][Cha]SHKPPF [1-Nal]F{FreeAcid} | [PE65;hArg68;Cha69;Pro73;Phe75;1-Nal76]Apelin-13(65-77) | 652 |
| {Hydrogen}[PE]RP[hArg][Cha]FHKPPF [1-Nal]F{FreeAcid} | [PE65;hArg68;Cha69;Phe70,75;Pro73;1-Nal76]Apelin-13(65-77) | 653 |
| {Hydrogen}[PE]RP[hArg][Cha]FHKGPF [1-Nal]F{FreeAcid} | [PE65;hArg68;Cha69;Phe70,75;1-Nal76]Apelin-13(65-77) | 654 |
| {Hydrogen}[PE]RP[hArg][Cha]FKKPPF [1-Nal]F{FreeAcid} | [PE65;hArg68;Cha69;Phe70,75;Lys71;Pro7 3;1-Nal76]Apelin-13(65-77) | 655 |
| {Hydrogen}[PE]RP[hArg][Cha]FKKPPF PF{FreeAcid} | [PE65;hArg68;Cha69;Phe70,75;Lys71;Pro7 3]Apelin-13(65-77) | 656 |
| {Hydrogen} [PE]RP[hArg]LFHKPPFPF { FreeAcid} | [PE65;hArg68;Phe70,75;Pro73]Apelin-13(65-77) | 657 |
| {Hydrogen} [PE]RPR[NMeLeu]FKKPP[ Nle]PF{FreeAcid} | [PE65;NMeLeu69;Phe70;Lys71;Pro73;Nle7 5]Apelin-13(65-77) | 658 |
| {Hydrogen} [PE]RPRLFHKPP[pI-Phe]PF{FreeAcid} | [PE65;Phe70;Pro73;pI-Phe75]Apelin-13(65-77) | 659 |
| {Hydrogen}[PE]RPRLFHKPP[pI-Phe][1-Nal]F{FreeAcid} | [PE65;Phe70;Pro73;pI-Phe75;1-Nal76]Apelin-13(65-77) | 660 |
| {Hydrogen} [PE]RP[hArg]LFHKPP[pI-Phe]PF{FreeAcid} | [PE65;hArg68;Phe70;Pro73;pI-Phe75]Apelin-13(65-77) | 661 |
| {Hydrogen}[PE]RP[hArg]LSHKPP[pI-Phe][1-Nal]F{FreeAcid} | [PE65;hArg68;Pro73;pI-Phe75;1-Nal76]Apelin-13(65-77) | 662 |
| {Hydrogen}[PE]RP[hArg]LFHKPP[pI-Phe][1-Nal]F{FreeAcid} | [PE65;hArg68;Phe70;Pro73;pI-Phe75;1-Nal76]Apelin-13(65-77) | 663 |
| {Hydrogen} [PE]RPR[NMeLeu]FHKPP[ Nle]PF{FreeAcid} | [PE65;NMeLeu69;Phe70;Pro73;Nle75]Apel in-13(65-77) | 664 |
| {Hydrogen}[PE]RPR[NMeLeu]SHKPP[ Nle][1-Nal]F{FreeAcid} | [PE65;NMeLeu69;Pro73;Nle75;1-Nal76]Apelin-13(65-77) | 665 |
| {Hydrogen}[PE]RPR[NMeLeu]SH[1-Nal]PP[Nle]MF{FreeAcid} | [PE65;NMeLeu69;1-Na172;Pro73;Nle75;Met76]Apelin-13(65-77) | 666 |
| {Hydrogen}[PE]RPR[NMeLeu]FHKPP[ Nle][1-Nal]F{FreeAcid} | [PE65;NMeLeu69;Phe70;Pro73;Nle75;1-Nal76]Apelin-13(65-77) | 667 |
| {Hydrogen}[PE]RPR[NMeLeu]FHKGP[ Nle][1-Nal]F{FreeAcid} | [PE65;NMeLeu69;Phe70;Nle75;1-Nal76]Apelin-13(65-77) | 668 |
| {Hydrogen} [PE]RPR[Cha]FKKPP[pI-Phe]PF{FreeAcid} | [PE65;Cha69;Phe70;Lys71;Pro73;pI-Phe75]Apelin-13(65-77) | 669 |
| {Hydrogen}[PE]RP[hArg][Cha]SHKPP[p I-Phe]PF{FreeAcid} | [PE65;hArg68;Cha69;Pro73;pI-Phe75]Apelin-13(65-77) | 670 |
| {Hydrogen}[PE]RP[hArg][Cha]FHKPP[p I-Phe]PF{FreeAcid} | [PE65;hArg68;Cha69;Phe70;Pro73;pI-Phe75]Apelin-13(65-77) | 671 |
| {Hydrogen}[PE]RP[hArg][NMeLeu]SH KGP[pI-Phe]P[4-F-F]{FreeAcid} | [PE65;hArg68;NMeLeu69;pI-Phe75;4-F-F77]Apelin-13(65-77) | 672 |
| {Hydrogen}[PE]RP[hArg][Cha]SHKGP[ pI-Phe]P[4-F-F]{FreeAcid} | [PE65;hArg68;Cha69;pI-Phe75;4-F-F77]Apelin-13(65-77) | 673 |
| {Hydrogen}[PE]RP[hArg][Cha]SHKGP[ Nle][4-F-F]{FreeAcid} | [PE65;hArg68;Cha69;Nle75;4-F-F76]Apelin-13(65-76) | 674 |
| {Hydrogen}[PE]RP[hArg][NMeLeu]SH KGP[pI-Phe]P[4-Me-F]{FreeAcid} | [PE65;hArg68;NMeLeu69;pI-Phe75;4-Me-F77]Apelin-13(65-77) | 675 |
| {Hydrogen} [PE]RP[hArg][Cha]SHKGP[ pI-Phe]P[4-Me-F]{FreeAcid} | [PE65;hArg68;Cha69;pI-Phe75;4-Me-F77]Apelin-13(65-77) | 676 |
| {Hydrogen} [PE]RP[hArg][Cha]SHKGP[ Nle]P[4-Me-F]{FreeAcid} | [PE65;hArg68;Cha69;Nle75;4-Me-F77]Apelin-13(65-77) | 677 |
| {Hydrogen} [PE]RP[hArg][Cha]SHKGP[ Nle][4-Me-F]{FreeAcid} | [PE65;hArg68;Cha69;Nle75;4-Me-F76]Apelin-13(65-76) | 678 |
| {Hydrogen} [PE]RP[hArg][Cha]SHKGP[ Nle]P[D-Bip]{FreeAcid} | [PE65;hArg68;Cha69;Nle75;D-Bip77]Apelin-13(65-77) | 679 |
| {Hydrogen} [PE]RP[hArg][Cha]SHKGP[ Nle][D-Bip]{FreeAcid} | [PE65;hArg68;Cha69;Nle75;D-Bip76]Apelin-13(65-76) | 680 |
| {Hydrogen}[PE]RP[hArg][NMeLeu]SH KGP[Nle]P[D-1Nal]{FreeAcid} | [PE65;hArg68;NMeLeu69;Nle75;D-1Nal77]Apelin-13(65-77) | 681 |
| {Hydrogen}[PE]RP[hArg][NMeLeu]SH KGP[pI-Phe]P[D-1Nal]{FreeAcid} | [PE65;hArg68;NMeLeu69;pI-Phe75;D-1Nal77]Apelin-13(65-77) | 682 |
| {Hydrogen} [PE]RP[hArg][Cha]SHKGP[ pI-Phe]P[D-1Nal]{FreeAcid} | [PE65;hArg68;Cha69;pI-Phe75;D-1Nal77]Apelin-13(65-77) | 683 |
| {Hydrogen}[PE]RP[hArg][Cha]SHKGP[ Nle]P[D-1Nal]{FreeAcid} | [PE65;hArg68;Cha69;Nle75;D-1Nal77]Apelin-13(65-77) | 684 |
| {Hydrogen}[PE]RP[hArg][Cha]FHKPP[p I-Phe][1-Nal]F{FreeAcid} | [PE65;hArg68;Cha69;Phe70;Pro73;pI-Phe75;1-Nal76]Apelin-13(65-77) | 685 |
| {Hydrogen} [PE]RP[hArg][Cha]SHKGP[ pI-Phe]P[pI-Phe]{FreeAcid} | [PE65;hArg68;Cha69;pI-Phe75,77]Apelin-13(65-77) | 686 |
| {Hydrogen}[PE]RP[hArg][Cha]FHKGP[ pI-Phe][1-Nal]F{FreeAcid} | [PE65;hArg68;Cha69;Phe70;pI-Phe75;1-Nal76]Apelin-13(65-77) | 687 |
| {Hydrogen}[PE]RPR[Cha]SHKGP[pI-Phe]PY{FreeAcid} | [PE65;Cha69;pI-Phe75;Tyr77]Apelin-13(65-77) | 688 |
| {Hydrogen}[PE]RP[hArg][Cha]SHKGP[ pI-Phe]PY{FreeAcid} | [PE65;hArg68;Cha69;pI-Phe75;Tyr77]Apelin-13(65-77) | 689 |
| {Hydrogen} [PE]RP[hArg][Cha]SHKGP[ Nle]y{FreeAcid} | [PE65;hArg68;Cha69;Nle75;D-Tyr76]Apelin-13(65-76) | 690 |
| {Hydrogen} [PE]RPRLSHKGPMPy {Free Acid} | [PE65;D-Tyr77]Apelin-13(65-77) | 691 |
| {Hydrogen}[PE]RP[hArg][Cha]FKKPP[p I-Phe][1-Nal]F{FreeAcid} | [PE65;hArg68;Cha69;Phe70;Lys71;Pro73;p I-Phe75;1-Nal76]Apelin-13(65-77) | 692 |
| {Hydrogen} [PE]RPR[Cha] SHKPP[pI-Phe]PF{FreeAcid} | [PE65;Cha69;Pro73;pI-Phe75]Apelin-13(65-77) | 693 |
| {Hydrogen}[PE]RP[hArg][Cha]FKKPP[p I-Phe]PF{FreeAcid} | [PE65;hArg68;Cha69;Phe70;Lys71;Pro73;p I-Phe75]Apelin-13(65-77) | 694 |
| {Hydrogen}[PE]RP[hArg]LFHKGP[pI-Phe][1-Nal]F{FreeAcid} | [PE65;hArg68;Phe70;pI-Phe75;1-Nal76]Apelin-13(65-77) | 695 |
| {Hydrogen}[PE]RP[hArg]LFKKPP[pI-Phe][1-Nal]F{FreeAcid} | [PE65;hArg68;Phe70;Lys71;Pro73;pI-Phe75;1-Nal76]Apelin-13(65-77) | 696 |
| {Hydrogen} [PE]RP[hArg]LFKKPP[pI-Phe]PF{FreeAcid} | [PE65;hArg68;Phe70;Lys71;Pro73;pI-Phe75]Apelin-13(65-77) | 697 |
| {Hydrogen} [PE]RPR[Chg]FHKPP[pI-Phe]PF{FreeAcid} | [PE65;Chg69;Phe70;Pro73;pI-Phe75]Apelin-13(65-77) | 698 |
| {Hydrogen} [PE]RPR[Cha]FHKPP[pI-Phe]PF{FreeAcid} | [PE65;Cha69;Phe70;Pro73;pI-Phe75]Apelin-13(65-77) | 699 |
| {Hydrogen} [PE]RPR[Chg]FHKPP[pI-Phe][1-Nal]F{FreeAcid} | [PE65;Chg69;Phe70;Pro73;pI-Phe75;1-Nal76]Apelin-13(65-77) | 700 |
| {Hydrogen}[PE]RPR[Chg]FKKPP[pI-Phe][1-Nal]F{FreeAcid} | [PE65;Chg69;Phe70;Lys71;Pro73;pI-Phe75;1-Nal76]Apelin-13(65-77) | 701 |
| {Hydrogen} [PE]RPR[NMeLeu]FHKPP[p I-Phe]PF{FreeAcid} | [PE65;NMeLeu69;Phe70;Pro73;pI-Phe75]Apelin-13(65-77) | 702 |
| {Hydrogen}[PE]RPR[NMeLeu]SHKPP[p I-Phe][1-Nal]F{FreeAcid} | [PE65;NMeLeu69;Pro73;pI-Phe75;1-Nal76]Apelin-13(65-77) | 703 |
| {Hydrogen} [PE]RPR[Cha]SHKPP[pI-Phe][1-Nal]F{FreeAcid} | [PE65;Cha69;Pro73;pI-Phe75;1-Nal76]Apelin-13(65-77) | 704 |
| {Hydrogen}[PE]RPR[NMeLeu]FHKPP[p I-Phe][1-Nal]F{FreeAcid} | [PE65;NMeLeu69;Phe70;Pro73;pI-Phe75;1-Nal76]Apelin-13(65-77) | 705 |
| {Hydrogen}[PE]RPR[NMeLeu]FHKGP[ pI-Phe][1-Nal]F{FreeAcid} | [PE65;NMeLeu69;Phe70;pI-Phe75;1-Nal76]Apelin-13(65-77) | 706 |
| {Hydrogen}[PE]RPR[NMeLeu]FKKPP[p I-Phe][1-Nal]F{FreeAcid} | [PE65;NMeLeu69;Phe70;Lys71;Pro73;pI-Phe75;1-Nal76]Apelin-13(65-77) | 707 |
| {Hydrogen} [PE]RPR[NMeLeu]FKKPP[p I-Phe]PF{FreeAcid} | [PE65;NMeLeu69;Phe70;Lys71;Pro73;pI-Phe75]Apelin-13(65-77) | 708 |
| {Hydrogen}[PE]RPR[Cha]FHKPP[pI-Phe][1-Nal]F{FreeAcid} | [PE65;Cha69;Phe70;Pro73;pI-Phe75;1-Nal76]Apelin-13(65-77) | 709 |
| {Hydrogen}[PE]RPR[Cha]FKKPP[pI-Phe][1-Nal]F{FreeAcid} | [PE65;Cha69;Phe70;Lys71;Pro73;pI-Phe75;1-Nal76]Apelin-13(65-77) | 710 |
| {Hydrogen} [PE]RP[hArg][Cha]SHKGP[ Nle]P[pI-Phe]{FreeAcid} | [PE65;hArg68;Cha69;Nle75;pI-Phe77]Apelin-13(65-77) | 711 |
| {Hydrogen}[PE]RP[hArg][Cha]SHKGP[ pI-Phe]Pf{FreeAcid} | [PE65;hArg68;Cha69;pI-Phe75;D-Phe77]Apelin-13(65-77) | 712 |
| {Hydrogen} [PE]RP[hArg][Cha]SHKGP[ Nle]Pf{FreeAcid} | [PE65;hArg68;Cha69;Nle75;D-Phe77]Apelin-13(65-77) | 713 |
| {Hydrogen}[PE]RP[hArg][NMeLeu]SH KGP[Nle]P[NMePhe]{Amide} | [PE65;hArg68;NMeLeu69;Nle75;NMePhe7 7]Apelin-13(65-77)-amide | 714 |
| {Hydrogen}[PE]RP[hArg][NMeLeu]SH KGP[Nle]Pw{FreeAcid} | [PE65;hArg68;NMeLeu69;Nle75;D-Trp77]Apelin-13(65-77) | 715 |
| {Hydrogen}[PE]RP[hArg][NMeLeu]SH KGP[pI-Phe]Pw{FreeAcid} | [PE65;hArg68;NMeLeu69;pI-Phe75;D-Trp77]Apelin-13(65-77) | 716 |
| {Hydrogen}[PE]RP[hArg][Cha]SHKGP[ pI-Phe]Pw{FreeAcid} | [PE65;hArg68;Cha69;pI-Phe75;D-Trp77]Apelin-13(65-77) | 717 |
| {Hydrogen} [PE]RP[hArg][Cha]SHKGP[ Nle]Pw{FreeAcid} | [PE65;hArg68;Cha69;Nle75;D-Trp77]Apelin-13(65-77) | 718 |
| {Hydrogen}[PE]RP[hArg][Cha]SHKGP[ Nle]PY{FreeAcid} | [PE65;hArg68;Cha69;Nle75;Tyr77]Apelin-13(65-77) | 719 |
| {Hydrogen}[PE]RP[hArg][NMeLeu]SH KGP[Nle]Py{FreeAcid} | [PE65;hArg68;NMeLeu69;Nle75;D-Tyr77]Apelin-13 (65-77) | 720 |
| {Hydrogen}[PE]RP[hArg][NMeLeu]SH KGP[pI-Phe]Py{FreeAcid} | [PE65;hArg68;NMeLeu69;pI-Phe75;D-Tyr77]Apelin-13(65-77) | 721 |
| {Hydrogen} [PE]RP[hArg][Cha]SHKGP[ pI-Phe]Py{FreeAcid} | [PE65;hArg68;Cha69;pI-Phe75;D-Tyr77]Apelin-13 (65-77) | 722 |
| {Hydrogen} [PE]RP[hArg][Cha]SHKGP[ Nle]Py{FreeAcid} | [PE65;hArg68;Cha69;Nle75;D-Tyr77]Apelin-13 (65-77) | 723 |
| {Hydrogen}[PE]RP[hArg][NMeLeu]SH KPP[Nle]P[D-Bip]{Amide} | [PE65;hArg68;NMeLeu69;Pro73;Nle75;D-Bip77]Apelin-13(65-77)-amide | 724 |
| {Hydrogen}[PE]RP[hArg][NMeLeu]FH KGP[pI-Phe]P[2-Nal]{FreeAcid} | [PE65;hArg68;NMeLeu69;Phe70;pI-Phe75;2-Nal77]Apelin-13 (65-77) | 725 |
| {Hydrogen}[PE]RP[hArg][NMeLeu]FH KGP[Nle]P[D-Bip]{Amide} | [PE65;hArg68;NMeLeu69;Phe70;Nle75;D-Bip77]Apelin-13(65-77)-amide | 726 |
| {Hydrogen}[PE]RP[hArg][NMeLeu]FH KGP[Nle]P[D-Bip]{FreeAcid} | [PE65;hArg68;NMeLeu69;Phe70;Nle75;D-Bip77]Apelin-13(65-77) | 727 |
| {Hydrogen}[PE]RP[hArg][NMeLeu]FH KPP[Nle]P[D-Bip]{FreeAcid} | [PE65;hArg68;NMeLeu69;Phe70;Pro73;Nle 75;D-Bip77]Apelin-13(65-77) | 728 |
| {Hydrogen}[PE]RP[hArg][NMeLeu]FH KGP[pI-Phe]P[D-Bip]{FreeAcid} | [PE65;hArg68;NMeLeu69;Phe70;pI-Phe75;D-Bip77]Apelin-1 3(65-77) | 729 |
| {Hydrogen}[PE]RP[hArg][NMeLeu]FH KPP[Nle]P[D-Bip]{Amide} | [PE65;hArg68;NMeLeu69;Phe70;Pro73;Nle 75;D-Bip77]Apelin-13(65-77)-amide | 730 |
| {Hydrogen}[PE]RP[hArg][NMeLeu]SH KPP[pI-Phe]P[D-Bip]{FreeAcid} | [PE65;hArg68;NMeLeu69;Pro73;pI-Phe75;D-Bip77]Apelin-1 3(65-77) | 731 |
| {Palmitoyl}LRP[hArg][NMeLeu]FHKGP [Nle]P[D-Bip]{FreeAcid} | Palmitoyl-[Leu65;hArg68;NMeLeu69;Phe70;Nle75;D-Bip77]Apelin-13 (65-77) | 732 |
| {Hydrogen} [PE]RP[hArg][Cha]SHKGP[ pI-Phe]P[D-Bip]{FreeAcid} | [PE65;hArg68;Cha69;pI-Phe75;D-Bip77]Apelin-13(65-77) | 733 |
| {Hydrogen}[PE]RP[hArg][NMeLeu]SH KGP[Nle]P[4-Me-F]{FreeAcid} | [PE65;hArg68;NMeLeu69;Nle75;4-Me-F77]Apelin-13(65-77) | 734 |
| {Hydrogen}[PE]RP[hArg][NMeLeu]FH KGP[pI-Phe]P[D-Bip]{Amide} | [PE65;hArg68;NMeLeu69;Phe70;pI-Phe75;D-Bip77]Apelin-13(65-77)-amide | 735 |
| {Hydrogen}[PE]RP[hArg][Cha]SHKGP[ Nle]Pf{Amide} | [PE65;hArg68;Cha69;Nle75;D-Phe77]Apelin-13(65-77)-amide | 736 |
| {Hydrogen}[PE]RP[hArg][NMeLeu]SH KGP[Nle]P[4-Cl-F]{Amide} | [PE65;hArg68;NMeLeu69;Nle75;4-Cl-F77]Apelin-13(65-77)-amide | 737 |
| {Hydrogen}[PE]RP[hArg][NMeLeu]SH KGP[Nle]P[4-Me-F]{Amide} | [PE65;hArg68;NMeLeu69;Nle75;4-Me-F77]Apelin-13(65-77)-amide | 738 |
| {Hydrogen}[PE]RP[hArg][NMeLeu]SH KGP[Nle]P[Bip]{Amide} | [PE65;hArg68;NMeLeu69;Nle75;Bip77]Ap elin-13(65-77)-amide | 739 |
| {Hydrogen}[PE]RP[hArg][NMeLeu]SH KGP[Nle]P[D-Bip]{Amide} | [PE65;hArg68;NMeLeu69;Nle75;D-Bip77]Apelin-13(65-77)-amide | 740 |
| {Hydrogen}[PE]RP[hArg][NMeLeu]SH KGP[Nle]P[4-NO2-F]{Amide} | [PE65;hArg68;NMeLeu69;Nle75;4-NO2-F77]Apelin-13(65-77)-amide | 741 |
| {Hydrogen}[PE]RP[hArg][NMeLeu]SH KG[1-Nal][Nle]P[4-Cl-F]{Amide} | [PE65;hArg68;NMeLeu69;1-Nal74;Nle75;4-Cl-F77]Apelin-13(65-77)-amide | 742 |
| {Hydrogen}[PE]RP[hArg][NMeLeu]FH KG[1-Nal][Nle]P[4-Cl-F]{Amide} | [PE65;hArg68;NMeLeu69;Phe70;1-Nal74;Nle75;4-Cl-F77]Apelin-13(65-77)-amide | 743 |
| {Hydrogen}[PE]RP[hArg][NMeLeu]FH KGP[Nle]P[4-Cl-F]{Amide} | [PE65;hArg68;NMeLeu69;Phe70;Nle75;4-Cl-F77]Apelin-13(65-77)-amide | 744 |
| {Hydrogen} [PE]RP[hArg][NMeLeu]SA KG[1-Nal][Nle]P[4-Cl-F]{Amide} | [PE65;hArg68;NMeLeu69;Ala71;1-Nal74;Nle75;4-Cl-F77]Apelin-13(65-77)-amide | 745 |
| {Hydrogen}[PE]RP[hArg][NMeLeu]FA KG[1-Nal][Nle]P[4-Cl-F]{Amide} | [PE65;hArg68;NMeLeu69;Phe70;Ala71;1-Nal74;Nle75;4-Cl-F77]Apelin-13(65-77)-amide | 746 |
| {Hydrogen}[PE]RP[hArg][NMeLeu]FA KGP[Nle]P[4-Cl-F]{Amide} | [PE65;hArg68;NMeLeu69;Phe70748;Ala71 ;Nle75;4-Cl-F77]Apelin-13(65-77)-amide | 747 |
| {Hydrogen}[PE]RP[hArg][NMeLeu]S[1-Nal]KG[1-Nal][Nle]P[4-Cl-F]{Amide} | [PE65;hArg68;NMeLeu69;1-Nal71,74;Nle75;4-Cl-F77]Apelin-13(65-77)-amide | 748 |
| {Hydrogen}[PE]R-P[hArg][NMeLeu]F[1-Nal]KG[1-Nal][Nle]P[4-Cl-F]{Amide} | [PE65;hArg68;NMeLeu69;Phe70;1-Nal71,74;Nle75;4-Cl-F77]Apelin-13(65-77)-amide | 749 |
| {Hydrogen}[PE]RP[hArg][NMeLeu]F[1-Nal] [1-Nal]G[1-Nal][Nle]P[4-Cl-F]{Amide} | [PE65;hArg68;NMeLeu69;Phe70;1-Nal71,72, 74;Nle75;4-Cl-F77]Apelin-13(65-77)-amide | 750 |
| {Hydrogen}[PE]RP[hArg][NMeLeu]F[1-Nal]KGP[Nle]P[4-Cl-F]{Amide} | [PE65;hArg68;NMeLeu69;Phe70;1-Nal71;Nle75;4-Cl-F77]Apelin-13(65-77)-amide | 751 |
| {Hydrogen}[PE]RP[hArg][NMeLeu]F[1-Nal]KGP[Nle]P[4-Cl-F]{Amide} | [PE65;hArg68;NMeLeu69;Phe70;1-Nal71;Nle75;4-Cl-F77]Apelin-13(65-77)-amide | 752 |
| {Hydrogen}[PE]RP[hArg][NMeLeu][NM ePhe][1-Nal][1-Nal]G[1-Nal][Nle]P[4-Cl-F]{Amide} | [PE65;hArg68;NMeLeu69;NMePhe70;1-Nal71,72,74;Nle75;4-Cl-F77]Apelin-13(65-77)-amide | 753 |
| {Hydrogen}[PE]RP[hArg][NMeLeu][NM ePhe][1-Nal]KGP[Nle]P[4-Cl-F] {Amide} | [PE65;hArg68;NMeLeu69;NMePhe70;1-Nal71;Nle75;4-Cl-F77]Apelin-13 (65-77)-amide | 754 |
| {Hydrogen}[PE]RP[hArg][NMeLeu][NM ePhe]AKG[1-Nal][Nle]P[4-Cl-F]{Amide} | [PE65;hArg68;NMeLeu69;NMePhe70;Ala7 1;1-Nal74;Nle75;4-Cl-F77]Apelin-13(65-77)-amide | 755 |
| {Hydrogen}[PE]RP[hArg][NMeLeu][NM ePhe]AKG[1-Nal][Nle]P[4-Cl-F]{Amide} | [PE65;hArg68;NMeLeu69;NMePhe70;Ala7 1;1-Nal74;Nle75;4-Cl-F77]Apelin-13(65-77)-amide | 756 |
| {Hydrogen}[PE]RP[hArg][NMeLeu]S[2-Nal]KG[1-Nal][Nle]P[4-Cl-F]{Amide} | [PE65;hArg68;NMeLeu69;2-Nal71:1-Nal74;Nle75;4-Cl-F77]Apelin-13(65-77)-amide | 757 |
| {Hydrogen}[PE]RP[hArg][NMeLeu]F[2-Nal]KG[1-Nal][Nle]P[4-Cl-F]{Amide} | [PE65;hArg68;NMeLeu69;Phe70;2-Nal71;1-Nal74;Nle75;4-Cl-F77]Apelin-13(65-77)-amide | 758 |
| {Hydrogen}[PE]RP[hArg][NMeLeu]F[2-Nal] [1-Nal]G[1-Nal][Nle]P[4-Cl-F]{Amide} | [PE65;hArg68;NMeLeu69;Phe70;2-Nal71;1-Nal72,74;Nle75;4-Cl-F77]Apelin-13(65-77)-amide | 759 |
| {Hydrogen}[PE]RP[hArg][NMeLeu]F[2-Nal]KGP[Nle]P[4-Cl-F]{Amide} | [PE65;hArg68;NMeLeu69;Phe70;2-Nal71;Nle75;4-Cl-F77]Apelin-13 (65-77)-amide | 760 |
| {Hydrogen}[PE]RP[hArg][NMeLeu][NM ePhe][2-Nal]KGP[Nle]P[4-Cl-F] {Amide} | [PE65;hArg68;NMeLeu69;NMePhe70;2-Nal71;Nle75;4-Cl-F77]Apelin-13 (65-77)-amide | 761 |
| {Hydrogen}[PE]RP[hArg][NMeLeu]FH KPP[Nle]PF | [PE65;hArg68;NMeLeu69;Phe70;Pro73;Nle 75]Apelin-13(65-77) | 762 |
| {Hydrogen}[PE]RP[hArg][NMeLeu]FH K[Thz]P[Nle]PF | [PE65;hArg68;NMeLeu69;Phe70;Thz73;Nl e75]Apelin-13(65-77) | 763 |
| Palmitoyl-RP[hArg] [NMeLeu]FHKPP[Nle]PF | Palmitoyl-[hArg68;NMeLeu69;Phe70;Pro73;Nle75]A pelin-13(65-77) | 764 |
| {Hydrogen}[PE]RP[hArg][NMeLeu]FH KPP[Nle]PFK(palmitoyl){Amide} | [PE65;hArg68;NMeLeu69;Phe70;Pro73;Nle 75;K(palmitoyl)78]Apelin-13(65-77) | 765 |
| Palmitoyl-RP[hArg][NMeLeu]FHKPP[Nle]PF{Ami de} | Palmitoyl-[hArg68;NMeLeu69;Phe70;Pro73;Nle75]A pelin-13(65-77)-amide | 766 |
| {Hydrogen}[PE]RP[hArg][NMeLeu]SH KGP[Nle]P[D-Bip] | [PE65;hArg68;NMeLeu69;Nle75;D-Bip77]Apelin-13(65-77) | 767 |
| {Hydrogen}[PE]RP[hArg][NMeLeu]SH KGP[pI-Phe]P[D-Bip] | [PE65;hArg68;NMeLeu69;pI-Phe75;D-Bip77]Apelin-13(65-77) | 768 |
| {Hydrogen}[PE]RP[hArg][NMeLeu]SH KGP[pI-Phe]P[2-Nal] | [PE65;hArg68;NMeLeu69;pI-Phe75;2-Nal77]Apelin-13(65-77) | 769 |
| Palmitoyl-LRP [hArg][NMeLeu]SHKGP[pI-Phe]P[D-Bip] | Palmitoyl-Leu65;hArg68;NMeLeu69;pI-Phe75;D-Bip77]Apelin-1 3(65-77) | 770 |
| {Hydrogen} [PE]RP[hArg][Cha]SHKGP[ Nle][4-Cl-F]K(palmitoyl){Amide} | PE65;hArg68;Cha69;Nle75;4-Cl-F77;K(Palmitoyl)78]Apelin-13(65-78)-amide | 771 |
| {Hydrogen}[PE]RP[hArg][NMeLeu]SH KGP[Nle]P[D-Bip]K(palmitoyl){Amide} | PE65;hArg68;NMeLeu69;Nle75;D-Bip77;K(Palmitoyl)78]Apelin-13 (65-78)-amide | 772 |
| {Hydrogen} [PE]RP[hArg][Cha]SHKGP[ Nle][D-1Nal]K(palmitoyl){Amide} | PE65;hArg68;Cha69;Nle75;D-1Nal77;K(Palmitoyl)78]Apelin-13(65-78)-amide | 773 |
| {Hydrogen}[PE]RP[hArg][NMeLeu]SH KGP[Nle]P[2-Nal]{Amide} | [PE65;hArg68;NMeLeu69;Nle75;2-Nal77]Apelin-13(65-77) | 774 |
| {Hydrogen}[PE]RP[hArg][NMeLeu]SH KGP[pI-Phe]P[4-Cl-F] | [PE65;hArg68;NMeLeu69;pI-Phe75;4-Cl-F77]Apelin-13(65-77) | 775 |
| {Hydrogen}[PE]RP[hArg][NMeLeu]SH KGP[Nle]P[4-Cl-F] | [PE65;hArg68;NMeLeu69;Nle75;4-Cl-F77]Apelin-13(65-77) | 776 |
| {Hydrogen}[PE]RP[hArg][NMeLeu]SH KGP[Nle]P[4-F-F] | [PE65;hArg68;NMeLeu69;Nle75;4-F-F77]Apelin-13(65-77) | 777 |
| {Hydrogen} [PE]RP[hArg][Cha]SHKGP[ Nle]P[4-F-F] | [PE65;hArg68;Cha69;Nle75;4-F-F77]Apelin-13(65-77) | 778 |
| {Hydrogen}[PE]RP[hArg][NMeLeu]SH KGP[Nle]P[pI-Phe] | [PE65;hArg68;NMeLeu69;Nle75;pI-Phe77]Apelin-13(65-77) | 779 |
| {Hydrogen}[PE]RPR[NMeLeu]FKKPPF[ 1-Nal]F | [PE65;NMeLeu69;Phe70,75;Lys71;Pro73;1-Nal76]Apelin-13(65-77) | 780 |
| {Hydrogen} [PE]RPR[NMeLeu]FHKGP[ Nle]PF | [PE65;NMeLeu69;Phe70;Nle75]Apelin-13(65-77) | 781 |
| {Hydrogen}[PE]RP[hArg][NMeLeu]SH KGP[Nle]Pf | [PE65;hArg68;NMeLeu69;Nle75;D-Phe77]Apelin-13(65-77) | 782 |
| {Hydrogen}[PE]RP[hArg][NMeLeu]FH KPP[Nle]PF | [PE65;hArg68;NMeLeu69;Phe70;Pro73;Nle 75]Apelin-13(65-77) | 783 |
| Palmitoyl-LRP [hArg][NMeLeu]SHKGP[pI-Phe]P[D-Bip] {Amide} | Palmitoyl-Leu65;hArg68;NMeLeu69;pI-Phe75;D-Bip77]Apelin-1 3(65-77) | 784 |
| {Hydrogen}[PE]RP[hArg][NMeLeu]SH KGP[Nle]P[D-Bip]K(palmitoyl){Amide} | PE65;hArg68;NMeLeu69;Nle75;D-Bip77;K(Palmitoyl)78]Apelin-13 (65-78)-amide | 785 |
| {Hydrogen}[PE]RP[hArg] [NMeLeu]FH KPP[Nle]PF | [PE65;hArg68;NMeLeu69;Phe70;Pro73;Nle 75]Apelin-13(65-77) | 786 |
| {Hydrogen} [PE]RPR[NMeLeu]FHKGP[ Nle]PF | [PE65;NMeLeu69;Phe70;Nle75]Apelin-13(65-77) | 787 |
| {Hydrogen} [PE]RPR[NMeLeu]FHKGP[ Nle]PF | [PE65;NMeLeu69;Phe70;Nle75]Apelin-13(65-77) | 788 |
| {Hydrogen} [PE]RP[hArg][Cha]SHKGP[ Nle][1-Nal] | [PE65;hArg68;Cha69;Nle75;1-Nal76]Apelin-13(65-76) | 789 |
| {Hydrogen} [PE]RP[hArg][Cha]SHKGP[ pI-Phe]P[2-Nal] | [PE65;hArg68;Cha69;pI-Phe75;2-Nal77]Apelin-13(65-77) | 790 |
| {Acetyl}[Pra]LRP[hArg][NMeLeu][Pra] H[Dpr]GP[Nle]P[4-Cl-F]{FreeAcid} | Ac-Pra-[Leu65;hArg68;NMeLeu69;Pra70;Dpr72;Nl e75;4-Cl-F77]Apelin-13(65-77) | 791 |
| {Acetyl}[Pra]LRP[hArg][NMeLeu][Pra] HHGP[Nle]P[4-Cl-F]{FreeAcid} | Ac-Pra-[Leu65;hArg68;NMeLeu69;Pra70;His72;Nl e75;4-Cl-F77]Apelin-13(65-77) | 792 |
| {Acetyl}[Pra]LRP[hArg][NMeLeu][Pra] H[2Pal]GP[Nle]P[4-Cl-F]{FreeAcid} | Ac-Pra-[Leu65;hArg68;NMeLeu69;Pra70;2Pal72;N le75;4-Cl-F77]Apelin-13(65-77) | 793 |
| {Acetyl}[Pra]LRP[hArg][NMeLeu][Pra] H[3Pal]GP[Nle]P[4-Cl-F]{FreeAcid} | Ac-Pra-[Leu65;hArg68;NMeLeu69;Pra70;3Pal72;N le75;4-Cl-F77]Apelin-13(65-77) | 794 |
| {Acetyl}[Pra]LRP[hArg][NMeLeu][Pra] HRGP[Nle]P[4-Cl-F]{FreeAcid} | Ac-Pra-[Leu65;hArg68;NMeLeu69;Pra70;Arg72;Nl e75;4-Cl-F77]Apelin-13(65-77) | 795 |
| {Acetyl}[Pra]LRP[hArg][NMeLeu][Pra] H[hArg]GP[Nle]P[4-Cl-F]{FreeAcid} | Ac-Pra-[Leu65;hArg68,72;NMeLeu69;Pra70;Nle75; 4-Cl-F77]Apelin-13 (65-77) | 796 |
| {Acetyl}[Pra]LRP[hArg][NMeLeu][Pra] HKPP[Nle]P[4-Cl-F]{FreeAcid} | Ac-Pra-[Leu65;hArg68;NMeLeu69;Pra70;Pro73;Nl e75;4-Cl-F77]Apelin-13(65-77) | 797 |
| {Acetyl}[Pra]LRP[hArg][NMeLeu][Pra] HK[Oic]P[Nle]P[4-Cl-F]{FreeAcid} | Ac-Pra-[Leu65;hArg68;NMeLeu69;Pra70;Oic73;Nl e75;4-Cl-F77]Apelin-13(65-77) | 798 |
| {Acetyl}[Pra]LRP[hArg][NMeLeu][Pra] HK[bAla]P[Nle]P[4-Cl-F]{FreeAcid} | Ac-Pra-[Leu65;hArg68;NMeLeu69;Pra70;bAla73;N le75;4-Cl-F77]Apelin-13(65-77) | 799 |
| {Acetyl}[Pra]LRP[hArg][NMeLeu][Pra] H[1-Nal]PP[Nle]P[4-Cl-F]{FreeAcid} | Ac-Pra-[Leu65;hArg68;NMeLeu69;Pra70;1-Nal72;Pro73;Nle75;4-Cl-F77]Apelin-13(65-77) | 800 |
| {Acetyl}[Pra]LRP[hArg][NMeLeu][Pra] HKGP[Nle]P[4-Cl-F]{FreeAcid} | Ac-Pra-[Leu65;hArg68;NMeLeu69;Pra70;Nle75;4-Cl-F77]Apelin-13(65-77) | 801 |
| {Acetyl}[Pra]LRP[hArg][NMeLeu][Pra] H[1-Nal][Oic]P[Nle]P[4-Cl-F]{FreeAcid} | Ac-Pra-[Leu65;hArg68;NMeLeu69;Pra70;1-Nal72;Oic73;Nle75;4-Cl-F77]Apelin-13(65-77) | 802 |
| {Acetyl}[Pra]LRP[hArg][NMeLeu][Pra] H[1-Nal][bAla]P[Nle]P[4-Cl-F]{FreeAcid} | Ac-Pra-[Leu65;hArg68;NMeLeu69;Pra70;1-Nal72;bAla73;Nle75;4-Cl-F77]Apelin-13(65-77) | 803 |
| {Acetyl}[Pra]LRP[hArg][NMeLeu][Pra] HKPP[Nle]P[4-Cl-F]{FreeAcid} | Ac-Pra-[Leu65;hArg68;NMeLeu69;Pra70;Pro73;Nl e75;4-Cl-F77]Apelin-13(65-77) | 804 |
| {Acetyl}[Pra]LRP[hArg][NMeLeu][Pra] H[2Nal]PP[Nle]P[4-Cl-F]{FreeAcid} | Ac-Pra-[Leu65;hArg68;NMeLeu69;Pra70;2-Nal72;Pro73;Nle75;4-Cl-F77]Apelin-13(65-77) | 805 |
| {Acetyl}[Pra]LRP[hArg] [NMeLeu] [Pra] H[Bip]PP[Nle]P[4-Cl-F] {FreeAcid} | Ac-Pra-[Leu65;hArg68;NMeLeu69;Pra70;Bip72;Pr o73;Nle75;4-Cl-F77]Apelin-13(65-77) | 806 |
| {Acetyl}[Pra]LRP[hArg] [NMeLeu] [Pra] HFPP[Nle]P[4-Cl-F] {FreeAcid} | Ac-Pra-[Leu65;hArg68;NMeLeu69;Pra70;Phe72;Pr o73;Nle75;4-Cl-F77]Apelin-13(65-77) | 807 |
| {Acetyl}[Pra]LRP[hArg] [NMeLeu] [Pra] H[4AmP]PP[Nle]P[4-Cl-F] {FreeAcid} | Ac-Pra-[Leu65;hArg68;NMeLeu69;Pra70;4AmP72; Pro73;Nle75;4-Cl-F77]Apelin-13(65-77) | 808 |
| {Acetyl}[Pra]LRP[hArg] [NMeLeu] [Pra] H[Orn]PP[Nle]P[4-Cl-F]{FreeAcid} | Ac-Pra-[Leu65;hArg68;NMeLeu69;Pra70;Orn72;Pr o73;Nle75;4-Cl-F77]Apelin-13(65-77) | 809 |
| {Acetyl}[Pra]LRP[hArg][NMeLeu][Pra] H[Dab]PP[Nle]P[4-Cl-F]{FreeAcid} | Ac-Pra-[Leu65;hArg68;NMeLeu69;Pra70;Dab72;Pr o73;Nle75;4-Cl-F77]Apelin-13(65-77) | 810 |
| {Acetyl}[Pra]LRP[hArg] [NMeLeu] [Pra] H[Dpr]PP[Nle]P[4-Cl-F]{FreeAcid} | Ac-Pra-[Leu65;hArg68;NMeLeu69;Pra70;Dpr72;Pr o73;Nle75;4-Cl-F77]Apelin-13(65-77) | 811 |
| {Acetyl}[Pra]LRP[hArg] [NMeLeu] [Pra] H[1-Nal]GP[Nle]P[4-Cl-F]{FreeAcid} | Ac-Pra-[Leu65;hArg68;NMeLeu69;Pra70;1-Nal72;Nle75;4-Cl-F77]Apelin-13(65-77) | 812 |
| {Acetyl}[Pra]LRP[hArg] [NMeLeu] [Pra] HHPP[Nle]P[4-Cl-F] {FreeAcid} | Ac-Pra-[Leu65;hArg68;NMeLeu69;Pra70;His72;Pr o73;Nle75;4-Cl-F77]Apelin-13(65-77) | 813 |
| {Acetyl}[Pra]LRP[hArg] [NMeLeu] [Pra] H[2Pal]PP[Nle]P[4-Cl-F]{FreeAcid} | Ac-Pra-[Leu65;hArg68;NMeLeu69;Pra70;2Pal72;Pr o73;Nle75;4-Cl-F77]Apelin-13(65-77) | 814 |
| {Acetyl}[Pra]LRP[hArg] [NMeLeu] [Pra] H[3Pal]PP[Nle]P[4-Cl-F]{FreeAcid} | Ac-Pra-[Leu65;hArg68;NMeLeu69;Pra70;3Pal72;Pr o73;Nle75;4-Cl-F77]Apelin-13(65-77) | 815 |
| {Acetyl}[Pra]LRP[hArg] [NMeLeu] [Pra] HRPP[Nle]P[4-Cl-F] {FreeAcid} | Ac-Pra-[Leu65;hArg68;NMeLeu69;Pra70;Arg72;Pr o73;Nle75;4-Cl-F77]Apelin-1 3(65-77) | 816 |
| {Acetyl}[Pra]LRP[hArg][NMeLeu][Pra] H[hArg]PP[Nle]P[4-Cl-F]{FreeAcid} | Ac-Pra-[Leu65;hArg68,72;NMeLeu69;Pra70;Pro73; Nle75;4-Cl-F77]Apelin-13(65-77) | 817 |
| {Acetyl}[Pra]LRP[hArg] [NMeLeu] [Pra] HKGP[Nle]P[4-Cl-F]{FreeAcid} | Ac-Pra-[Leu65;hArg68;NMeLeu69;Pra70;Nle75;4-Cl-F77]Apelin-13(65-77) | 818 |
| {Acetyl}[Pra]LRP[hArg] [NMeLeu] [Pra] HKG[Oic][Nle]P[4-Cl-F]{FreeAcid} | Ac-Pra-[Leu65;hArg68;NMeLeu69;Pra70;Oic74;N1 e75;4-Cl-F77]Apelin-13(65-77) | 819 |
| {Acetyl}[Pra]LRP[hArg][NMeLeu][Pra] HKGP[Nle][Oic][4-Cl-F]{FreeAcid} | Ac-Pra-[Leu65;hArg68;NMeLeu69;Pra70;Nle75;Oi c76;4-Cl-F77]Apelin-13(65-77) | 820 |
| {Acetyl}[Pra]LR[Oic] [hArg] [NMeLeu] [P ra]HKGP[Nle]P[4-Cl-F]{FreeAcid} | Ac-Pra-[Leu65;Oic67;hArg68;NMeLeu69;Pra70;Nl e75;4-Cl-F77]Apelin-13(65-77) | 821 |
| {Acetyl}[Pra]LR[Oic][hArg][NMeLeu] [P ra]HKG[Oic][Nle][Oic][4-Cl-F]{FreeAcid} | Ac-Pra-[Leu65;Oic67,74,76;hArg68;NMeLeu69;Pra 70;Nle75;4-Cl-F77]Apelin-13(65-77) | 822 |
| {Acetyl}[Pra]LRP[hArg][NMeLeu] [Pra] H[2Nal]GP[Nle]P[4-Cl-F]{FreeAcid} | Ac-Pra-[Leu65;hArg68;NMeLeu69;Pra70;2-Nal72;Nle75;4-Cl-F77]Apelin-13(65-77) | 823 |
| {Acetyl}[Pra]LR[Oic][hArg][NMeLeu][P ra]HKGP[Nle][Oic][4-Cl-F]{FreeAcid} | Ac-Pra-[Leu65;Oic67,76;hArg68;NMeLeu69;Pra70; Nle75;4-Cl-F77]Apelin-13(65-77) | 824 |
| {Acetyl}[Pra]LR[Oic][hArg][NMeLeu][P ra]HKG[Oic][Nle]P[4-Cl-F]{FreeAcid} | Ac-Pra-[Leu65;Oic67,74;hArg68;NMeLeu69;Pra70; Nle75;4-Cl-F77]Apelin-13(65-77) | 825 |
| {Acetyl}[Pra]LRP[hArg][NMeLeu][Pra] HKG[Oic][Nle][Oic][4-Cl-F]{FreeAcid} | Ac-Pra-[Leu65;hArg68;NMeLeu69;Pra70;Oic74,76; 13(65-77) | 826 |
| {Acetyl}[Pra]LRP[NMeArg][NMeLeu] [P ra]HKGP[Nle]P[4-Cl-F]{FreeAcid} | Ac-Pra-[Leu65;NMeArg68;NMeLeu69;Pra70;N1e7 5;4-Cl-F77]Apelin-13(65-77) | 827 |
| {Acetyl}[Pra]L[NMeArg]P[hArg][NMeL eu][Pra]HKGP[Nle]P[4-Cl-F]{FreeAcid} | Ac-Pra-[Leu65;NMeArg66;hArg68;NMeLeu69;Pra 70;Nle75;4-Cl-F77]Apelin-13(65-77) | 828 |
| {Acetyl }[Pra]L[NMeArg]P[NMeArg] [N MeLeu][Pra]HKGP[Nle]P[4-Cl-F]{FreeAcid} | Ac-Pra-[Leu65;NMeArg66,68;NMeLeu69;Pra70;Nl e75;4-Cl-F77]Apelin-13(65-77) | 829 |
| {Acetyl}[Pra]LRP[hArg][NMeLeu][Pra] HKG[Oic][Nle][Tic][4-Cl-F]{FreeAcid} | Ac-Pra-[Leu65;hArg68;NMeLeu69;Pra70;Oic74;Nl e75;Tic76;4-Cl-F77]Apelin-13(65-77) | 830 |
| {Acetyl}[Pra]LR[Tic][hArg][NMeLeu][P ra]HKG[Oic][Nle]P[4-Cl-F]{FreeAcid} | Ac-Pra-[Leu65;Tic67;hArg68;NMeLeu69;Pra70;Oi c74;Nle75;4-Cl-F77]Apelin-13(65-77) | 831 |
| [PE]RP[hArg][NMeLeu][Pra]HKG[Oic][ Nle]P[4-Cl-F] {FreeAcid} | [PE65;hArg68;NMeLeu69;Pra70;Oic74;Nle 75;4-Cl-F77]Apelin-13(65-77) | 832 |
| [PE]RP[hArg][NMeLeu][Pra]H[1-Nal]G[Oic][Nle]P[4-Cl-F] {FreeAcid} | [PE65;hArg68;NMeLeu69;Pra70;1-Nal72;Oic74;Nle75;4-Cl-F77]Apelin-13(65-77) | 833 |
| {Acetyl}[Pra]LRP[hArg][NMeLeu][Pra] H[Bip]GP[Nle]P[4-Cl-F]{FreeAcid} | Ac-Pra-[Leu65;hArg68;NMeLeu69;Pra70;Bip72;Nl e75;4-Cl-F77]Apelin-13(65-77) | 834 |
| [PE]RP[hArg][NMeLeu][Pra]H[2Nal]G[ Oic][Nle]P[4-Cl-F]{FreeAcid} | [PE65;hArg68;NMeLeu69;Pra70;2-Nal72;Oic74;Nle75;4-Cl-F77]Apelin-13(65-77) | 835 |
| [PE]RP[hArg][NMeLeu][Pra]H[Bip]G[Oi c][Nle]P[4-Cl-F] {FreeAcid} | [PE65;hArg68;NMeLeu69;Pra70;Bip72;Oic 74;Nle75;4-Cl-F77]Apelin-13(65-77) | 836 |
| [PE]RP[hArg][NMeLeu] [Pra]HFG[Oic][ Nle]P[4-Cl-F]{FreeAcid} | [PE65;hArg68;NMeLeu69;Pra70;Phe72;Oic 74;Nle75;4-Cl-F77]Apelin-13(65-77) | 837 |
| [PE]RP[hArg][NMeLeu][Pra]H[4AmP]G [Oic][Nle]P[4-Cl-F] {FreeAcid} | [PE65;hArg68;NMeLeu69;Pra70;4AmP72; Oic74;Nle75;4-Cl-F77]Apelin-13(65-77) | 838 |
| [PE]RP[hArg][NMeLeu][Pra]H[Orn]G[O ic][Nle]P[4-Cl-F]{FreeAcid} | [PE65;hArg68;NMeLeu69;Pra70;Orn72;Oic 74;Nle75;4-Cl-F77]Apelin-13(65-77) | 839 |
| [PE]RP[hArg][NMeLeu][Pra]H[Dab]G[O ic][Nle]P[4-Cl-F]{FreeAcid} | [PE65;hArg68;NMeLeu69;Pra70;Dab72;Oi c74;Nle75;4-Cl-F77]Apelin-13(65-77) | 840 |
| [PE]RP[hArg][NMeLeu][Pra]H[Dpr]G[O ic][Nle]P[4-Cl-F]{FreeAcid} | [PE65;hArg68;NMeLeu69;Pra70;Dpr72;Oic 74;Nle75;4-Cl-F77]Apelin-13(65-77) | 841 |
| [PE]RP[hArg][NMeLeu][Pra]HHG[Oic][ Nle]P[4-Cl-F]{FreeAcid} | [PE65;hArg68;NMeLeu69;Pra70;His72;Oic 74;Nle75;4-Cl-F77]Apelin-13(65-77) | 842 |
| [PE]RP[hArg][NMeLeu][Pra]H[2Pal]G[O ic][Nle]P[4-Cl-F]{FreeAcid} | [PE65;hArg68;NMeLeu69;Pra70;2Pa172;Oi c74;Nle75;4-Cl-F77]Apelin-13(65-77) | 843 |
| [PE]RP[hArg][NMeLeu][Pra]H[3Pal]G[O ic][Nle]P[4-Cl-F]{FreeAcid} | [PE65;hArg68;NMeLeu69;Pra70;3Pal72;Oi c74;Nle75;4-Cl-F77]Apelin-13(65-77) | 844 |
| {Acetyl}[Pra]LRP[hArg][NMeLeu][Pra] HFGP[Nle]P[4-Cl-F]{FreeAcid} | Ac-Pra-[Leu65;hArg68;NMeLeu69;Pra70;Phe72;Nl e75;4-Cl-F77]Apelin-13(65-77) | 845 |
| [PE]RP[hArg][NMeLeu][Pra]HRG[Oic][ Nle]P[4-Cl-F]{FreeAcid} | [PE65;hArg68;NMeLeu69;Pra70;Arg72;Oic 74;Nle75;4-Cl-F77]Apelin-13(65-77) | 846 |
| [PE]RP[hArg][NMeLeu][Pra]H[hArg]G[ Oic][Nle]P[4-Cl-F]{FreeAcid} | [PE65;hArg68,72;NMeLeu69;Pra70;Oic74; Nle75;4-Cl-F77]Apelin-13(65-77) | 847 |
| [PE]RP[hArg][NMeLeu][Pra]HKP[Oic][ Nle]P[4-Cl-F]{FreeAcid} | [PE65;hArg68;NMeLeu69;Pra70;Pro73;Oic 74;Nle75;4-Cl-F77]Apelin-13(65-77) | 848 |
| [PE]RP[hArg][NMeLeu][Pra]HK[Oic][Oi c][Nle]P[4-Cl-F]{FreeAcid} | [PE65;hArg68;NMeLeu69;Pra70;Oic73,74; Nle75;4-Cl-F77]Apelin-13(65-77) | 849 |
| [PE]RP[hArg][NMeLeu] [Pra]HK[bAla][ Oic][Nle]P[4-Cl-F] {FreeAcid} | [PE65;hArg68;NMeLeu69;Pra70;bAla73;Oi c74;Nle75;4-Cl-F77]Apelin-13(65-77) | 850 |
| [PE]RP[hArg][NMeLeu][Pra]H[1-Nal]P[Oic][Nle]P[4-Cl-F]{FreeAcid} | [PE65;hArg68;NMeLeu69;Pra70;1-Nal72;Pro73;Oic74;Nle75;4-Cl-F77]Apelin-13(65-77) | 851 |
| [PE]RP[hArg][NMeLeu][Pra]H[1-Nal][Oic][Oic][Nle]P[4-Cl-F]{FreeAcid} | [PE65;hArg68;NMeLeu69;Pra70;1-Nal72;Oic73,74;Nle75;4-Cl-F77]Apelin-13(65-77) | 852 |
| [PE]RP[hArg][NMeLeu] [Pra]H[1-Nal] [bAla] [Oic] [Nle]P[4-Cl-F] {FreeAcid} | [PE65;hArg68;NMeLeu69;Pra70;1-Nal72;bAla73;Oic74;Nle75;4-Cl-F77]Apelin-13(65-77) | 853 |
| [PE]RP[hArg] [NMeLeu] [Pra]HKP[Oic] [ Nle]P[4-Cl-F] {FreeAcid} | [PE65;hArg68;NMeLeu69;Pra70;Pro73;Oic 74;Nle75;4-Cl-F77]Apelin-13(65-77) | 854 |
| [PE]RP[hArg][NMeLeu][Pra]H[2Nal]P[O ie][Nle]P[4-Cl-F]{FreeAcid} | [PE65;hArg68;NMeLeu69;Pra70;2-Nal72;Pro73;Oic74;Nle75;4-Cl-F77]Apelin-13(65-77) | 855 |
| {Acetyl}[Pra]LRP[hArg] [NMeLeu] [Pra] H[4AmP]GP[Nle]P[4-Cl-F]{FreeAcid} | Ac-Pra-[Leu65;hArg68;NMeLeu69;Pra70;4AmP72; Nle75;4-Cl-F77]Apelin-13(65-77) | 856 |
| [PE]RP[hArg][NMeLeu][Pra]H[Bip]P[Oi c][Nle]P[4-Cl-F] {FreeAcid} | [PE65;hArg68;NMeLeu69;Pra70;Bip72;Pro 73;Oic74;Nle75;4-Cl-F77]Apelin-13(65-77) | 857 |
| [PE]RP[hArg][NMeLeu][Pra]HFP[Oic][ Nle]P[4-Cl-F]{FreeAcid} | [PE65;hArg68;NMeLeu69;Pra70;Phe72;Pro 73;Oic74;Nle75;4-Cl-F77]Apelin-13(65-77) | 858 |
| [PE]RP[hArg][NMeLeu][Pra]H[4AmP]P[ Oic][Nle]P[4-Cl-F]{FreeAcid} | [PE65;hArg68;NMeLeu69;Pra70;4AmP72; Pro73;Oic74;Nle75;4-Cl-F77]Apelin-13(65-77) | 859 |
| [PE]RP[hArg][NMeLeu][Pra]H[Orn]P[Oi c][Nle]P[4-Cl-F]{FreeAcid} | [PE65;hArg68;NMeLeu69;Pra70;Orn72;Pro 73;Oic74;Nle75;4-Cl-F77]Apelin-13(65-77) | 860 |
| [PE]RP[hArg][NMeLeu][Pra]H[Dab]P[Oi c][Nle]P[4-Cl-F]{FreeAcid} | [PE65;hArg68;NMeLeu69;Pra70;Dab72;Pro 73;Oic74;Nle75;4-Cl-F77]Apelin-13(65-77) | 861 |
| [PE]RP[hArg][NMeLeu][Pra]H[Dpr]P[Oi c][Nle]P[4-Cl-F]{FreeAcid} | [PE65;hArg68;NMeLeu69;Pra70;Dpr72;Pro 73;Oic74;Nle75;4-Cl-F77]Apelin-13(65-77) | 862 |
| [PE]RP[hArg][NMeLeu][Pra]HHP[Oic][ Nle]P[4-Cl-F]{FreeAcid} | [PE65;hArg68;NMeLeu69;Pra70;His72;Pro 73;Oic74;Nle75;4-Cl-F77]Apelin-13(65-77) | 863 |
| [PE]RP[hArg][NMeLeu][Pra]H[2Pal]P[O ic][Nle]P[4-Cl-F]{FreeAcid} | [PE65;hArg68;NMeLeu69;Pra70;2Pal72;Pr o73;Oic74;Nle75;4-Cl-F77]Apelin-13(65-77) | 864 |
| [PE]RP[hArg][NMeLeu][Pra]H[3Pal]P[O ic][Nle]P[4-Cl-F]{FreeAcid} | [PE65;hArg68;NMeLeu69;Pra70;3Pal72;Pr o73;Oic74;Nle75;4-Cl-F77]Apelin-13(65-77) | 865 |
| [PE]RP[hArg][NMeLeu][Pra]HRP[Oic][ Nle]P[4-Cl-F]{FreeAcid} | [PE65;hArg68;NMeLeu69;Pra70;Arg72;Pro 73;Oic74;Nle75;4-Cl-F77]Apelin-13(65-77) | 866 |
| {Acetyl}[Pra]LRP[hArg][NMeLeu][Pra] H[Orn]GP[Nle]P[4-Cl-F]{FreeAcid} | Ac-Pra-[Leu65;hArg68;NMeLeu69;Pra70;Orn72;Nl e75;4-Cl-F77]Apelin-13(65-77) | 867 |
| [PE]RP[hArg][NMeLeu][Pra]H[hArg]P[ Oic][Nle]P[4-Cl-F]{FreeAcid} | [PE65;hArg68,72;NMeLeu69;Pra70;Pro73; Oic74;Nle75;4-Cl-F77]Apelin-13(65-77) | 868 |
| [PE]RP[hArg][NMeLeu][Pra]HKG[Oic][ Nle]P[4-Cl-F]{FreeAcid} | [PE65;hArg68;NMeLeu69;Pra70;Oic74;Nle 75;4-Cl-F77]Apelin-13(65-77) | 869 |
| [PE]RP[hArg][NMeLeu][Pra]HKG[Oic][ Nle][Oic][4-Cl-F]{FreeAcid} | [PE65;hArg68;NMeLeu69;Pra70;Oic74,76; Nle75;4-Cl-F77]Apelin-13(65-77) | 870 |
| [PE]R[Oic][hArg][NMeLeu][Pra]HKG[O ic][Nle][Oic][4-Cl-F]{FreeAcid} | [PE65;Oic67,74,76;hArg68;NMeLeu69;Pra 70;Nle75;4-Cl-F77]Apelin-13(65-77) | 871 |
| [PE]R[Oic][hArg][NMeLeu][Pra]HKG[O ic][Nle]P[4-Cl-F]{FreeAcid} | [PE65;Oic67,74;hArg68;NMeLeu69;Pra70; Nle75;4-Cl-F77]Apelin-13(65-77) | 872 |
| [PE]RP[NMeArg][NMeLeu][Pra]HKG[O ic][Nle]P[4-Cl-F]{FreeAcid} | [PE65;NMeArg68;NMeLeu69;Pra70;Oic74; Nle75;4-Cl-F77]Apelin-13(65-77) | 873 |
| [PE][NMeArg]P[hArg][NMeLeu][Pra]H KG[Oic][Nle]P[4-Cl-F]{FreeAcid} | [PE65;NMeArg66;hArg68;NMeLeu69;Pra7 0;Oic74;Nle75;4-Cl-F77]Apelin-13(65-77) | 874 |
| [PE][NMeArg]P[NMeArg][NMeLeu][Pra ]HKG[Oic][Nle]P[4-Cl-F] {FreeAcid} | [PE65;NMeArg66,68;NMeLeu69;Pra70;Oic 74;Nle75;4-Cl-F77]Apelin-13(65-77) | 875 |
| [PE]RP[hArg][NMeLeu][Pra]H[1-Nal]G[Oic][Nle]P[4-Cl-F]{FreeAcid} | [PE65;hArg68;NMeLeu69;Pra70;1-Nal72;Oic74;Nle75;4-Cl-F77]Apelin-13(65-77) | 876 |
| [PE]RP[hArg][NMeLeu][Pra]H[1-Nal]G[Oic][Nle][Oic][4-Cl-F]{FreeAcid} | [PE65;hArg68;NMeLeu69;Pra70;1-Nal72;Oic74,76;Nle75;4-Cl-F77]Apelin-13(65-77) | 877 |
| {Acetyl}[Pra]LRP[hArg][NMeLeu][Pra] H[Dab]GP[Nle]P[4-Cl-F]{FreeAcid} | Ac-Pra-[Leu65;hArg68;NMeLeu69;Pra70;Dab72;Nl e75;4-Cl-F77]Apelin-13(65-77) | 878 |
| [PE]R[Oic][hArg][NMeLeu][Pra]H[1-Nal]G[Oic][Nle][Oic][4-Cl-F]{FreeAcid} | [PE65;Oic67,74,76;hArg68;NMeLeu69;Pra 70;1-Nal72;Nle75;4-Cl-F77]Apelin-13(65-77) | 879 |
| [PE]R[Oic][hArg][NMeLeu][Pra]H[1-Nal]G[Oic][Nle]P[4-Cl-F]{FreeAcid} | [PE65;Oic67,74;hArg68;NMeLeu69;Pra70; 1-Nal72;Nle75;4-Cl-F77]Apelin-13(65-77) | 880 |
| [PE]RP[NMeArg][NMeLeu][Pra]H[1-Nal]G[Oic][Nle]P[4-Cl-F]{FreeAcid} | [PE65;NMeArg68;NMeLeu69;Pra70;1-Nal72;Oic74;Nle75;4-Cl-F77]Apelin-13(65-77) | 881 |
| [PE][NMeArg]P[hArg][NMeLeu][Pra]H[ 1-Nal]G[Oic][Nle]P[4-Cl-F] {FreeAcid} | [PE65;NMeArg66;hArg68;NMeLeu69;Pra7 0;1-Nal72;Oic74;Nle75;4-Cl-F77]Apelin-13(65-77) | 882 |
| [PE][NMeArg]P[NMeArg][NMeLeu][Pra ]H[1-Nal]G[Oic][Nle]P[4-Cl-F]{FreeAcid} | [PE65;NMeArg66,68;NMeLeu69;Pra70;1-Nal72;Oic74;Nle75;4-Cl-F77]Apelin-13(65-77) | 883 |
| [PE]RP[hArg][NMeLeu][Pra]H[LYSIPR] G[Oic][Nle]P[4-Cl-F]{FreeAcid} | [PE65;hArg68;NMeLeu69;Pra70;LYSIPR7 2;Oic74;Nle75;4-Cl-F77]Apelin-13(65-77) | 884 |
| [PE]RP[hArg][NMeLeu][Pra]H[K(Me2)] G[Oic][Nle]P[4-Cl-F]{FreeAcid} | [PE65;hArg68;NMeLeu69;Pra70;K(Me2)72 ;Oic74;Nle75;4-Cl-F77]Apelin-13(65-77) | 885 |
| KFRRQRP[hArg] [Cha]SHKG[Oic][Nle] P[4-Cl-F] | [hArg68;Cha69;Oic74;Nle75;4-Cl-F77]Apelin-17(61-77) | 886 |
| {MeS-Propionyl }KFRRQRP[hArg][Cha]SHKG [Oic][Nle]P[4-Cl-F] | 3-(methylthio)propionyl-[hArg68;Cha69;Oic74;Nle75;4-Cl-F77]Apelin-17(61-77) | 887 |
| [Pra]KFRRQRP[hArg][Cha]SHKG[Oic][ Nle]P[4-Cl-F] | [Pra60; hArg68;Cha69;Oic74;Nle75;4-Cl-F77]Apelin-17(60-77) | 888 |
| [Pra]PGPWQGGRRKFRRQRP[hArg][C ha]SHKG[Oic][Nle]P[4-Cl-F] | [Pra51;hArg68;Cha69;Oic74;Nle75;4-Cl-F77]Apelin-26(51-77) | 889 |
| [Pra]LVQPRGSRNGPGPWQGGRRKFR RQRP[hArg][Cha]SHKG[Oic][Nle]P[4-Cl-F] | [Pra41; hArg68;Cha69;Oic74;Nle75;4-Cl-F77]Apelin-36(41-77) | 890 |
| [Pra]KF[hArg][hArg]QRP[hArg][Cha]SH KG[Oic] [Nle]P[4-Cl-F] | Pra-[hArg63,64,68;Cha69;Oic74;Nle75;4-Cl-F77]Apelin-17(61-77) | 891 |
| [Pra]KF[hArg][hArg]Q[hArg]P[hArg][Ch a]SHKG[Oic][Nle]P[4-Cl-F] | Pra-[hArg63,64,66,68;Cha69;Oic74;Nle75;4-Cl-F77]Apelin-17(61-77) | 892 |
| [Pra]KF[hArg][hArg]QQP[hArg][Cha]SH KG[Oic][Nle]P[4-Cl-F] | Pra-[hArg63,64,68;Gln66;Cha69;Oic74;Nle75;4 -Cl-F77]Apelin-17(61-77) | 893 |
| [Pra]KF[hArg][hArg]Q[Cit]P[hArg][Cha] SHKG[Oic][Nle]P[4-Cl-F] | Pra-[hArg63,64,68;Cit66;Cha69;Oic74;Nle75;4-Cl-F77]Apelin-17(61-77) | 894 |
| [Pra]KF[hArg][Cit]Q[Cit]P[hArg][Cha]S HKG[Oic][Nle]P[4-Cl-F] | Pra-[hArg63,68;Cit64,66;Cha69;Oic74;Nle75;4-Cl-F77]Apelin-17(61-77) | 895 |
| [Pra]KF[Cit][Cit]Q[hArg]P[hArg][Cha]S HKG[Oic][Nle]P[4-Cl-F] | Pra-[Cit63,64;hArg66,68;Cha69;Oic74;Nle75;4-Cl-F77]Apelin-17(61-77) | 896 |
| [Pra]KF[Cit][Cit]Q[Cit]P[hArg][Cha]SH KG[Oic] [Nle]P[4-Cl-F] | Pra-[Cit63,64,66;hArg68;Cha69;Oic74;Nle75;4-Cl-F77]Apelin-17(61-77) | 897 |
| [Pra]OFRRQRP[hArg][Cha]SHKG[Oic][ Nle]P[4-Cl-F] | Pra-[Orn61;hArg68;Cha69;Oic74;Nle75;4-Cl-F77]Apelin-17(61-77) | 898 |
| [Pra]OF[hArg][hArg]Q[hArg]P[hArg][Ch a]SHKG[Oic][Nle]P[4-Cl-F] | Pra-[Orn61;hArg63,64,66,68;Cha69;Oic74;Nle7 5;4-Cl-F77]Apelin-17(61-77) | 899 |
| [Pra]FRRQRP[hArg][Cha]SHKG[Oic][N1 e]P[4-Cl-F] | [Pra61;hArg68;Cha69;Oic74;Nle75;4-Cl-F77]Apelin-17(61-77) | 900 |
| {Bromoacetyl} [NPeg11]LRP[hArg] [Cha ]SHKG[Oic][Nle]P[4-Cl-F] {FreeAcid} | Bromoacetyl-NPegl1-[Leu65;hArg68;Cha69;Oic74;Nle75;4-Cl-F77]Apelin-13(65-77) | 901 |
| {Bromoacetyl} [NPeg1 1]KFRR QRP[hAr g][Cha]SHKG[Oic][Nle]P[4-Cl-F]{FreeAcid} | Bromoacetyl-NPegl1-[hArg68;Cha69;Oic74;Nle75;4-Cl-F77]Apelin-17(61-77) | 902 |
| {Mercaptopropionyl}KFRRQRP[hArg][C ha]SHKG[Oic][Nle]P[4-Cl-F]{FreeAcid} | Mercaptopropionyl-[hArg68;Cha69;Oic74;Nle75;4-Cl-F77]Apelin-17(61-77) | 903 |
| {Mercaptopropionyl}FRRQRP[hArg][Ch a]SHKG[Oic][Nle]P[4-Cl-F]{FreeAcid} | Mercaptopropionyl-[hArg68;Cha69;Oic74;Nle75;4-Cl-F77]Apelin-17(62-77) | 904 |
| {Mercaptopropionyl}RRQRP[hArg][Cha] SHKG[Oic][Nle]P[4-Cl-F]{FreeAcid} | Mercaptopropionyl-[hArg68;Cha69;Oic74;Nle75;4-Cl-F77]Apelin-17(63-77) | 905 |
| {Mercaptopropionyl}RQRP[hArg][Cha]S HKG[Oic][Nle]P[4-Cl-F]{FreeAcid} | Mercaptopropionyl-[hArg68;Cha69;Oic74;Nle75;4-Cl-F77]Apelin-17(64-77) | 906 |
| {Mercaptopropionyl}QRP[hArg][Cha]SH KG[Oic][Nle]P[4-Cl-F]{FreeAcid} | Mercaptopropionyl-[hArg68;Cha69;Oic74;Nle75;4-Cl-F77]Apelin-17(65-77) | 907 |
| {Mercaptopropionyl}LRP[hArg][Cha]SH KG[Oic][Nle]P[4-Cl-F]{FreeAcid} | Mercaptopropionyl-[Leu65;hArg68;Cha69;Oic74;Nle75;4-Cl-F77]Apelin-17(65-77) | 908 |
| {Mercaptopropionyl}LLRP[hArg][Cha]S HKG[Oic][Nle]P[4-Cl-F]{FreeAcid} | Mercaptopropionyl-[Leu65;hArg68;Cha69;Oic74;Nle75;4-Cl-F77]Apelin-17(65-77) | 909 |
| {Pra}LRP[hArg][Cha]SHKG[Oic][Nle]P[ 4-C1-F]{FreeAcid} | Pra-[Leu65;hArg68;Cha69;Oic74;Nle75;4-Cl-F77]Apelin-17(65-77) | 910 |
| [PE]RP[hArg][Cha][Pra]HKG[Oic][Nle]P [4-C1-F]{FreeAcid} | [PE65;hArg68;Cha69;Pra70;Oic74;Nle75;4-Cl-F77]Apelin-13(65-77) | 911 |
| [PE]RP[hArg][Cha]S[Pra]KG[Oic][Nle]P [4-C1-F]{FreeAcid} | [PE65;hArg68;Cha69;Pra71;Oic74;Nle75;4-Cl-F77]Apelin-13(65-77) | 912 |
| {MeS-Propionyl }KFRRLRP[hArg][Cha]SHKG[ Oic][Nle]P[4-Cl-F] | 3-(methylthio)propionyl-[Leu65;hArg68;Cha69;Oic74;Nle75;4-Cl-F77]Apelin-17(61-77) | 913 |
| {MeS-Propionyl }KFRRARP[hArg][Cha]SHKG [Oic][Nle]P[4-Cl-F] | 3-(methylthio)propionyl-[Ala65;hArg68;Cha69;Oic74;Nle75;4-Cl-F77]Apelin-17(61-77) | 914 |
| {MeS-Propionyl }KFRRVRP[hArg][Cha]SHKG [Oic][Nle]P[4-Cl-F] | 3-(methylthio)propionyl-[Val65;hArg68;Cha69;Oic74;Nle75;4-Cl-F77]Apelin-17(61-77) | 915 |
| {MeS-Propionyl }KFRRIRP[hArg][Cha]SHKG[ Oic][Nle]P[4-Cl-F] | 3-(methylthio)propionyl-[Ile65;hArg68;Cha69;Oic74;Nle75;4-Cl-F77]Apelin-17(61-77) | 916 |
| {MeS-Propionyl }KFRRFRP[hArg][Cha]SHKG[ Oic][Nle]P[4-Cl-F] | 3-(methylthio)propionyl-[Phe65;hArg68;Cha69;Oic74;Nle75;4-Cl-F77]Apelin-17(61-77) | 917 |
| {MeS-Propionyl }KFRRYRP[hArg][Cha]SHKG [Oic][Nle]P[4-Cl-F] | 3-(methylthio)propionyl-[Tyr65;hArg68;Cha69;Oic74;Nle75;4-Cl-F77]Apelin-17(61-77) | 918 |
| {MeS-Propionyl}KFRRL[hArg]P[hArg][Cha]S HKG[Oic][Nle]P[4-Cl-F] | 3-(methylthio)propionyl-[Leu65;hArg66,68;Cha69;Oic74;Nle75;4-Cl-F77]Apelin-17(61-77) | 919 |
| {MeS-Propionyl }KFRKLRP[hArg][Cha]SHKG[ Oic][Nle]P[4-Cl-F] | 3-(methylthio)propionyl-[Lys64;Leu65;hArg68;Cha69;Oic74;Nle75; 4-Cl-F77]Apelin-17(61-77) | 920 |
| {MeS-Propionyl }KFR[Orn]LRP[hArg][Cha]SH KG[Oic] [Nle]P[4-Cl-F] | 3-(methylthio)propionyl-[Orn64;Leu65;hArg68;Cha69;Oic74;Nle75; 4-Cl-F77]Apelin-17(61-77) | 921 |
| {MeS-Propionyl }KFRHLRP[hArg][Cha]SHKG[ Oic][Nle]P[4-Cl-F] | 3-(methylthio)propionyl-[His64;Leu65;hArg68;Cha69;Oic74;Nle75; 4-Cl-F77]Apelin-17(61-77) | 922 |
| {MeS-Propionyl }KFRWLRP[hArg][Cha]SHKG [Oic][Nle]P[4-Cl-F] | 3-(methylthio)propionyl-[Trp64;Leu65;hArg68;Cha69;Oic74;Nle75; 4-Cl-F77]Apelin-17(61-77) | 923 |
| {MeS-Propionyl }KFRFLRP[hArg][Cha]SHKG[ Oic][Nle]P[4-Cl-F] | 3-(methylthio)propionyl-[Phe64;Leu65;hArg68;Cha69;Oic74;Nle75; 4-Cl-F77]Apelin-17(61-77) | 924 |
| {MeS-Propionyl }KFRYLRP[hArg][Cha]SHKG[ Oic][Nle]P[4-Cl-F] | 3-(methylthio)propionyl-[Tyr64;Leu65;hArg68;Cha69;Oic74;Nle75; 4-Cl-F77]Apelin-17(61-77) | 925 |
| {MeS-Propionyl }KFR[3Pa1]LRP[hArg][Cha]SH KG[Oic] [Nle]P[4-Cl-F] | 3-(methylthio)propionyl-[3Pal64;Leu65;hArg68;Cha69;Oic74;Nle75; 4-Cl-F77]Apelin-17(61-77) | 926 |
| {MeS-Propionyl}KFR[2Pal]LRP[hArg][Cha]SH KG[Oic] [Nle]P[4-Cl-F] | 3-(methylthio)propionyl-[2Pal64;Leu65;hArg68;Cha69;Oic74;Nle75; 4-Cl-F77]Apelin-17(61-77) | 927 |
| {MeS-Propionyl }KFHRLRP[hArg][Cha]SHKG[ Oic][Nle]P[4-Cl-F] | 3-(methylthio)propionyl-[His63;Leu65;hArg68;Cha69;Oic74;Nle75; 4-Cl-F77]Apelin-17(61-77) | 928 |
| {MeS-Propionyl }KFWRLRP[hArg][Cha]SHKG [Oic][Nle]P[4-Cl-F] | 3-(methylthio)propionyl-[Trp63;Leu65;hArg68;Cha69;Oic74;Nle75; 4-Cl-F77]Apelin-17(61-77) | 929 |
| {MeS-Propionyl }KFFRLRP[hArg][Cha]SHKG[ Oic][Nle]P[4-Cl-F] | 3-(methylthio)propionyl-[Phe63;Leu65;hArg68;Cha69;Oic74;Nle75; 4-Cl-F77]Apelin-17(61-77) | 930 |
| {MeS-Propionyl }KFYRLRP[hArg][Cha]SHKG[ Oic][Nle]P[4-Cl-F] | 3-(methylthio)propionyl-[Tyr63;Leu65;hArg68;Cha69;Oic74;Nle75; 4-Cl-F77]Apelin-17(61-77) | 931 |
| {MeS-Propionyl }KF[3Pal]RLRP[hArg][Cha]SH KG[Oic] [Nle]P[4-Cl-F] | 3-(methylthio)propionyl-[3Pal63;Leu65;hArg68;Cha69;Oic74;Nle75; 4-Cl-F77]Apelin-17(61-77) | 932 |
| {MeS-Propionyl }KF[2Pal]RLRP[hArg][Cha]SH KG[Oic] [Nle]P[4-Cl-F] | 3-(methylthio)propionyl-[2Pal63;Leu65;hArg68;Cha69;Oic74;Nle75; 4-Cl-F77]Apelin-17(61-77) | 933 |
| {MeS-Propionyl }FRKLRP[hArg][Cha]SHKG[O ic] [Nle]P[4-Cl-F] | 3-(methylthio)propionyl-[Lys64;Leu65;hArg68;Cha69;Oic74;Nle75; 4-Cl-F77]Apelin-17(62-77) | 934 |
| {MeS-Propionyl }FR[Orn]LRP[hArg][Cha]SHK G[Oic][Nle]P[4-Cl-F] | 3-(methylthio)propionyl-[Orn64;Leu65;hArg68;Cha69;Oic74;Nle75; 4-Cl-F77]Apelin-17(62-77) | 935 |
| {MeS-Propionyl}FRHLRP[hArg][Cha]SHKG[O ic][Nle]P[4-Cl-F] | 3-(methylthio)propionyl-[His64;Leu65;hArg68;Cha69;Oic74;Nle75; 4-Cl-F77]Apelin-17(62-77) | 936 |
| {MeS-Propionyl }FRWLRP[hArg][Cha]SHKG[ Oic][Nle]P[4-Cl-F] | 3-(methylthio)propionyl-[Trp64;Leu65;hArg68;Cha69;Oic74;Nle75; 4-Cl-F77]Apelin-17(62-77) | 937 |
| {MeS-Propionyl}FRFLRP[hArg][Cha]SHKG[O ic][Nle]P[4-Cl-F] | 3-(methylthio)propionyl-[Phe64;Leu65;hArg68;Cha69;Oic74;Nle75; 4-Cl-F77]Apelin-17(62-77) | 938 |
| {MeS-Propionyl}FRYLRP[hArg][Cha]SHKG[O ic][Nle]P[4-Cl-F] | 3-(methylthio)propionyl-[Tyr64;Leu65;hArg68;Cha69;Oic74;Nle75; 4-Cl-F77]Apelin-17(62-77) | 939 |
| {MeS-Propionyl}FKRLRP[hArg][Cha]SHKG[O ic][Nle]P[4-Cl-F] | 3-(methylthio)propionyl-[Lys63;Leu65;hArg68;Cha69;Oic74;Nle75; 4-Cl-F77]Apelin-17(62-77) | 940 |
| {MeS-Propionyl }F[Orn]RLRP[hArg][Cha]SHK G[Oic][Nle]P[4-Cl-F] | 3-(methylthio)propionyl-[Orn63;Leu65;hArg68;Cha69;Oic74;Nle75; 4-Cl-F77]Apelin-17(62-77) | 941 |
| {MeS-Propionyl}FHRLRP[hArg][Cha]SHKG[O ic][Nle]P[4-Cl-F] | 3-(methylthio)propionyl-[His63;Leu65;hArg68;Cha69;Oic74;Nle75; 4-Cl-F77]Apelin-17(62-77) | 942 |
| {MeS-Propionyl }FWRLRP[hArg][Cha]SHKG[ Oic][Nle]P[4-Cl-F] | 3-(methylthio)propionyl-[Trp63;Leu65;hArg68;Cha69;Oic74;Nle75; 4-Cl-F77]Apelin-17(62-77) | 943 |
| {MeS-Propionyl}FFRLRP[hArg][Cha]SHKG[O ic][Nle]P[4-Cl-F] | 3-(methylthio)propionyl-[Phe63;Leu65;hArg68;Cha69;Oic74;Nle75; 4-Cl-F77]Apelin-17(62-77) | 944 |
| {MeS-Propionyl }FYRLRP[hArg][Cha]SHKG[O ic] [Nle]P[4-Cl-F] | 3-(methylthio)propionyl-[Tyr63;Leu65;hArg68;Cha69;Oic74;Nle75; 4-Cl-F77]Apelin-17(62-77) | 945 |
| {MeS-Propionyl}F[3Pal]RLRP[hArg][Cha]SHK G[Oic][Nle]P[4-Cl-F] | 3-(methylthio)propionyl-[3Pal63;Leu65;hArg68;Cha69;Oic74;Nle75; 4-Cl-F77]Apelin-17(62-77) | 946 |
| {MeS-Propionyl }F[2Pal]RLRP[hArg][Cha]SHK G[Oic][Nle]P[4-Cl-F] | 3-(methylthio)propionyl-[2Pal63;Leu65;hArg68;Cha69;Oic74;Nle75; 4-Cl-F77]Apelin-17(62-77) | 947 |
| {MeS-Propionyl}KFRRL[hArg]P[hArg][Cha]S HKG[Oic][Nle]P[4-Cl-F] | 3-(methylthio)propionyl-[Leu65;hArg66,68;Cha69;Oic74;Nle75;4-Cl-F77]Apelin-17(61-77) | 948 |
| {MeS-Propionyl }FQQL[hArg]P[hArg][Cha]SH KG[Oic] [Nle]P[4-Cl-F] | 3-(methylthio)propionyl-[Gln63,64;Leu65;hArg66,68;Cha69;Oic74; Nle75;4-Cl-F77]Apelin-17(62-77) | 949 |
| {MeS-Propionyl}KF[Cit] [Cit]L[hArg]P[hArg][ Cha]SHKG[Oic][Nle]P[4-Cl-F] | 3-(methylthio)propionyl-[Cit63,64;Leu65;hArg66,68;Cha69;Oic74;N le75;4-Cl-F77]Apelin-17(61-77) | 950 |
| {MeS-Propionyl}F[Cit][Cit]L[hArg]P[hArg][Ch a]SHKG[Oic][Nle]P[4-Cl-F] | 3-(methylthio)propionyl-[Cit63,64;Leu65;hArg66,68;Cha69;Oic74;N le75;4-Cl-F77]Apelin-17(62-77) | 951 |
| {MeS-Propionyl}KFARL[hArg]P[hArg][Cha]S HKG[Oic][Nle]P[4-Cl-F] | 3-(methylthio)propionyl-[Ala63;Leu65;hArg66,68;Cha69;Oic74;Nle 75;4-Cl-F77]Apelin-17(61-77) | 952 |
| {MeS-Propionyl }KFERL[hArg]P[hArg][Cha]S HKG[Oic][Nle]P[4-Cl-F] | 3-(methylthio)propionyl-[Glu63;Leu65;hArg66,68;Cha69;Oic74;Nle 75;4-Cl-F77]Apelin-17(61-77) | 953 |
| {MeS-Propionyl}KFTRL[hArg]P[hArg][Cha]S HKG[Oic][Nle]P[4-Cl-F] | 3-(methylthio)propionyl-[Thr63;Leu65;hArg66,68;Cha69;Oic74;Nle 75;4-Cl-F77]Apelin-17(61-77) | 954 |
| {MeS-Propionyl}KFYRL[hArg]P[hArg][Cha]S HKG[Oic][Nle]P[4-Cl-F] | 3-(methylthio)propionyl-[Tyr63;Leu65;hArg66,68;Cha69;Oic74;Nle 75;4-Cl-F77]Apelin-17(61-77) | 955 |
| {MeS-Propionyl }KFIRL[hArg]P[hArg][Cha]SH KG[Oic] [Nle]P[4-Cl-F] | 3-(methylthio)propionyl-[Ile63;Leu65;hArg66,68;Cha69;Oic74;Nle7 5;4-Cl-F77]Apelin-17(61-77) | 956 |
| {MeS-Propionyl}KFPRL[hArg]P[hArg][Cha]S HKG[Oic][Nle]P[4-Cl-F] | 3-(methylthio)propionyl-[Pro63;Leu65;hArg66,68;Cha69;Oic74;Nle7 5;4-Cl-F77]Apelin-17(61-77) | 957 |
| {MeS-Propionyl}KFSRL[hArg]P[hArg][Cha]S HKG[Oic][Nle]P[4-Cl-F] | 3-(methylthio)propionyl-[Ser63;Leu65;hArg66,68;Cha69;Oic74;Nle7 5;4-Cl-F77]Apelin-17(61-77) | 958 |
| {MeS-Propionyl}KFFRL[hArg]P[hArg][Cha]S HKG[Oic][Nle]P[4-Cl-F] | 3-(methylthio)propionyl-[Phe63;Leu65;hArg66,68;Cha69;Oic74;Nle 75;4-Cl-F77]Apelin-17(61-77) | 959 |
| {MeS-Propionyl }KFLRL[hArg]P[hArg][Cha]S HKG[Oic][Nle]P[4-Cl-F] | 3-(methylthio)propionyl-[Leu63,65;hArg66,68;Cha69;Oic74;Nle75;4 -Cl-F77]Apelin-17(61-77) | 960 |
| {MeS-Propionyl}KFVRL[hArg]P[hArg][Cha]S HKG[Oic][Nle]P[4-Cl-F] | 3-(methylthio)propionyl-[Val63;Leu65;hArg66,68;Cha69;Oic74;Nle 75;4-Cl-F77]Apelin-17(61-77) | 961 |
| {MeS-Propionyl }KFRQL[hArg]P[hArg][Cha]S HKG[Oic][Nle]P[4-Cl-F] | 3-(methylthio)propionyl-[Gln64;Leu65;hArg66,68;Cha69;Oic74;Nle 75;4-Cl-F77]Apelin-17(61-77) | 962 |
| {MeS-Propionyl}KFRAL[hArg]P[hArg][Cha]S HKG[Oic][Nle]P[4-Cl-F] | 3-(methylthio)propionyl-[Ala64;Leu65;hArg66,68;Cha69;Oic74;Nle 75;4-Cl-F77]Apelin-17(61-77) | 963 |
| {MeS-Propionyl }KFREL[hArg]P[hArg][Cha]S HKG[Oic][Nle]P[4-Cl-F] | 3-(methylthio)propionyl-[Glu64;Leu65;hArg66,68;Cha69;Oic74;Nle 75;4-Cl-F77]Apelin-17(61-77) | 964 |
| {MeS-Propionyl }KFRTL[hArg]P[hArg][Cha]S HKG[Oic][Nle]P[4-Cl-F] | 3-(methylthio)propionyl-[Thr64;Leu65;hArg66,68;Cha69;Oic74;Nle 75;4-Cl-F77]Apelin-17(61-77) | 965 |
| {MeS-Propionyl}KFRYL[hArg]P[hArg][Cha]S HKG[Oic][Nle]P[4-Cl-F] | 3-(methylthio)propionyl-[Tyr64;Leu65;hArg66,68;Cha69;Oic74;Nle 75;4-Cl-F77]Apelin-17(61-77) | 966 |
| {MeS-Propionyl }KFRIL[hArg]P[hArg][Cha]SH KG[Oic] [Nle]P[4-Cl-F] | 3-(methylthio)propionyl-[Ile64;Leu65;hArg66,68;Cha69;Oic74;Nle7 5;4-Cl-F77]Apelin-17(61-77) | 967 |
| {MeS-Propionyl}KFRPL[hArg]P[hArg][Cha]S HKG[Oic][Nle]P[4-Cl-F] | 3-(methylthio)propionyl-[Pro64;Leu65;hArg66,68;Cha69;Oic74;Nle7 5;4-Cl-F77]Apelin-17(61-77) | 968 |
| {MeS-Propionyl }KFRSL[hArg]P[hArg][Cha]S HKG[Oic][Nle]P[4-Cl-F] | 3-(methylthio)propionyl-[Ser64;Leu65;hArg66,68;Cha69;Oic74;Nle7 5;4-Cl-F77]Apelin-17(61-77) | 969 |
| {MeS-Propionyl}KFRFL[hArg]P[hArg][Cha]S HKG[Oic][Nle]P[4-Cl-F] | 3-(methylthio)propionyl-[Phe64;Leu65;hArg66,68;Cha69;Oic74;Nle 75;4-Cl-F77]Apelin-17(61-77) | 970 |
| {MeS-Propionyl}KFRLL[hArg]P[hArg][Cha]S HKG[Oic][Nle]P[4-Cl-F] | 3-(methylthio)propionyl-[Leu64,65;hArg66,68;Cha69;Oic74;Nle75;4 -Cl-F77]Apelin-17(61-77) | 971 |
| {MeS-Propionyl}KFRVL[hArg]P[hArg][Cha]S HKG[Oic][Nle]P[4-Cl-F] | 3-(methylthio)propionyl-[Val64;Leu65;hArg66,68;Cha69;Oic74;Nle 75;4-Cl-F77]Apelin-17(61-77) | 972 |
| {MeS-Propionyl }FARL[hArg]P[hArg][Cha]SH KG[Oic] [Nle]P[4-Cl-F] | 3-(methylthio)propionyl-[Ala63;Leu65;hArg66,68;Cha69;Oic74;Nle 75;4-Cl-F77]Apelin-17(62-77) | 973 |
| {MeS-Propionyl }FERL[hArg]P[hArg][Cha]SH KG[Oic] [Nle]P[4-Cl-F] | 3-(methylthio)propionyl-[Glu63;Leu65;hArg66,68;Cha69;Oic74;Nle 75;4-Cl-F77]Apelin-17(62-77) | 974 |
| {MeS-Propionyl }FTRL[hArg]P[hArg][Cha]SH KG[Oic] [Nle]P[4-Cl-F] | 3-(methylthio)propionyl-[Thr63;Leu65;hArg66,68;Cha69;Oic74;Nle 75;4-Cl-F77]Apelin-17(62-77) | 975 |
| {MeS-Propionyl }FYRL[hArg]P[hArg][Cha]SH KG[Oic] [Nle]P[4-Cl-F] | 3-(methylthio)propionyl-[Tyr63;Leu65;hArg66,68;Cha69;Oic74;Nle 75;4-Cl-F77]Apelin-17(62-77) | 976 |
| {MeS-Propionyl}FIRL[hArg]P[hArg][Cha]SHK G[Oic][Nle]P[4-Cl-F] | 3-(methylthio)propionyl-[Ile63;Leu65;hArg66,68;Cha69;Oic74;Nle7 5;4-Cl-F77]Apelin-17(62-77) | 977 |
| {MeS-Propionyl }FPRL[hArg]P[hArg][Cha]SH KG[Oic] [Nle]P[4-Cl-F] | 3-(methylthio)propionyl-[Pro63;Leu65;hArg66,68;Cha69;Oic74;Nle7 5;4-Cl-F77]Apelin-17(62-77) | 978 |
| {MeS-Propionyl }FSRL[hArg]P[hArg][Cha]SH KG[Oic] [Nle]P[4-Cl-F] | 3-(methylthio)propionyl-[Ser63;Leu65;hArg66,68;Cha69;Oic74;Nle7 5;4-Cl-F77]Apelin-17(62-77) | 979 |
| {MeS-Propionyl }FFRL[hArg]P[hArg][Cha]SH KG[Oic] [Nle]P[4-Cl-F] | 3-(methylthio)propionyl-[Phe63;Leu65;hArg66,68;Cha69;Oic74;Nle 75;4-Cl-F77]Apelin-17(62-77) | 980 |
| {MeS-Propionyl}FLRL[hArg]P[hArg][Cha]SH KG[Oic] [Nle]P[4-Cl-F] | 3-(methylthio)propionyl-[Leu63;Leu65;hArg66,68;Cha69;Oic74;Nle 75;4-Cl-F77]Apelin-17(62-77) | 981 |
| {MeS-Propionyl}FVRL[hArg]P[hArg][Cha]SH KG[Oic] [Nle]P[4-Cl-F] | 3-(methylthio)propionyl-[Val63;Leu65;hArg66,68;Cha69;Oic74;Nle 75;4-Cl-F77]Apelin-17(62-77) | 982 |
| {MeS-Propionyl }FRQL[hArg]P[hArg][Cha]SH KG[Oic] [Nle]P[4-Cl-F] | 3-(methylthio)propionyl-[Gln64;Leu65;hArg66,68;Cha69;Oic74;Nle 75;4-Cl-F77]Apelin-17(62-77) | 983 |
| {MeS-Propionyl }FRAL[hArg]P[hArg][Cha]SH KG[Oic] [Nle]P[4-Cl-F] | 3-(methylthio)propionyl-[Ala64;Leu65;hArg66,68;Cha69;Oic74;Nle 75;4-Cl-F77]Apelin-17(62-77) | 984 |
| {MeS-Propionyl}FREL[hArg]P[hArg][Cha]SH KG[Oic] [Nle]P[4-Cl-F] | 3-(methylthio)propionyl-[Glu64;Leu65;hArg66,68;Cha69;Oic74;Nle 75;4-Cl-F77]Apelin-17(62-77) | 985 |
| {MeS-Propionyl}FRTL[hArg]P[hArg][Cha]SH KG[Oic] [Nle]P[4-Cl-F] | 3-(methylthio)propionyl-[Thr64;Leu65;hArg66,68;Cha69;Oic74;Nle 75;4-Cl-F77]Apelin-17(62-77) | 986 |
| {MeS-Propionyl }FRYL[hArg]P[hArg][Cha]SH KG[Oic] [Nle]P[4-Cl-F] | 3-(methylthio)propionyl-[Tyr64;Leu65;hArg66,68;Cha69;Oic74;Nle 75;4-Cl-F77]Apelin-17(62-77) | 987 |
| {MeS-Propionyl}FRIL[hArg]P[hArg][Cha]SHK G[Oic][Nle]P[4-Cl-F] | 3-(methylthio)propionyl-[Ile64;Leu65;hArg66,68;Cha69;Oic74;Nle7 5;4-Cl-F77]Apelin-17(62-77) | 988 |
| {MeS-Propionyl}FRPL[hArg]P[hArg][Cha]SH KG[Oic][Nle]P[4-Cl-F] | 3-(methylthio)propionyl-[Pro64;Leu65;hArg66,68;Cha69;Oic74;Nle7 5;4-Cl-F77]Apelin-17(62-77) | 989 |
| {MeS-Propionyl}FRSL[hArg]P[hArg][Cha]SH KG[Oic][Nle]P[4-Cl-F] | 3-(methylthio)propionyl-[Ser64;Leu65;hArg66,68;Cha69;Oic74;Nle7 5;4-Cl-F77]Apelin-17(62-77) | 990 |
| {MeS-Propionyl}FRFL[hArg]P[hArg][Cha]SH KG[Oic][Nle]P[4-Cl-F] | 3-(methylthio)propionyl-[Phe64;Leu65;hArg66,68;Cha69;Oic74;Nle 75;4-Cl-F77]Apelin-17(62-77) | 991 |
| {MeS-Propionyl }FRLL[hArg]P[hArg][Cha]SH KG[Oic] [Nle]P[4-Cl-F] | 3-(methylthio)propionyl-[Leu64,65;hArg66,68;Cha69;Oic74;Nle75;4 -Cl-F77]Apelin-17(62-77) | 992 |
| {MeS-Propionyl }FRVL[hArg]P[hArg][Cha]SH KG[Oic] [Nle]P[4-Cl-F] | 3-(methylthio)propionyl-[Val64;Leu65;hArg66,68;Cha69;Oic74;Nle 75;4-Cl-F77]Apelin-17(62-77) | 993 |
| {MeS-Propionyl}KFRRQRP[hArg][Cha]SHKG [Oic][Nle]P{Amide} | 3-(methylthio)propionyl-[hArg68;Cha69;Oic74;Nle75]Apelin-17(61-76)-Amide | 994 |
| {MeS-Propionyl}KF[Cit][Cit]L[hArg]P[hArg][ Cha]SHKG[Oic][Nle]P{Amide} | 3-(methylthio)propionyl-[Cit63,64;Leu65;hArg66,68;Cha69;Oic74;N le75]Apelin-17(61-76)-Amide | 995 |
| {MeS-Propionyl}F[Cit][Cit]L[hArg]P[hArg][Ch a]SHKG[Oic][Nle]P{Amide} | 3-(methylthio)propionyl-[Cit63,64;Leu65;hArg66,68;Cha69;Oic74;N le75]Apelin-17(62-76)-Amide | 996 |
| {MeS-Propionyl }KFRRL[hArg]P[hArg][Cha]S HKG[Oic][Nle]P{Amide} | 3-(methylthio)propionyl-[Leu65;hArg66,68;Cha69;Oic74;Nle75]Ape lin-17(61-76)-Amide | 997 |
| {MeS-Propionyl }FQQL[hArg]P[hArg][Cha]SH KG[Oic][Nle]P{Amide} | 3-(methylthio)propionyl-[Gln63,64;Leu65;hArg66,68;Cha69;Oic74; Nle75]Apelin-17(62-76)-Amide | 998 |
| {MeS-Propionyl}FRRQRP[hArg][Cha]SHKG[ Oic][Nle]P[4-Cl-F]{COOH} | 3-(methylthio)propionyl-[hArg68;Cha69;Oic74;Nle75;4-Cl-F77]Apelin-17(62-77) | 999 |
| {MeS-Propionyl }F[hArg][hArg]Q[hArg]P[hArg ][Cha]SHKG[Oic][Nle]P[4-Cl-F]{COOH} | 3-(methylthio)propionyl-[hArg63,64,66,68;Cha69;Oic74;Nle75;4-Cl-F77]Apelin-17(62-77) | 1000 |
| {MeS-Propionyl }A[hArg][hArg]Q[hArg]P[hArg ][Cha]SHKG[Oic][Nle]P[4-Cl-F] {COOH} | 3-(methylthio)propionyl-[Ala62;hArg63,64,66,68;Cha69;Oic74;Nle7 5;4-Cl-F77]Apelin-17(62-77) | 1001 |
| {MeS-Propionyl}FAQ[hArg]P[hArg][Cha]SHK G[Oic][Nle]P[4-Cl-F]{COOH} | 3-(methylthio)propionyl-[Phe63;Ala64;hArg66,68;Cha69;Oic74;Nle7 5;4-Cl-F77]Apelin-17(63-77) | 1002 |
| {MeS-Propionyl}F[hArg]AQ[hArg]P[hArg][Ch a]SHKG[Oic][Nle]P[4-Cl-F]{COOH} | 3-(methylthio)propionyl-[hArg63,66,68;Ala64;Cha69;Oic74;Nle75;4 -Cl-F77]Apelin-17(62-77) | 1003 |
| {MeS-Propionyl }F[hArg][hArg] A[hArg]P [hArg ][Cha]SHKG[Oic][Nle]P[4-Cl-F] {COOH} | 3-(methylthio)propionyl-[hArg63,64,66,68;Ala65;Cha69;Oic74;Nle7 5;4-Cl-F77]Apelin-17(62-77) | 1004 |
| {MeS-Propionyl}F[hArg][hArg]QAP[hArg][Ch a]SHKG[Oic][Nle]P[4-Cl-F]{COOH} | 3-(methylthio)propionyl-[hArg63,64,68;Ala66;Cha69;Oic74;Nle75;4 -Cl-F77]Apelin-17(62-77) | 1005 |
| {MeS-PropionylF[hArg][harg]Q[hArg][Oic][h Arg][Cha]SHKG[Oic][Nle]P[4-Cl-F]{COOH} | 3-(methylthio)propionyl-[hArg63,64,66,68;Oic67,74;Cha69;Nle75;4-Cl-F77]Apelin-17(62-77) | 1006 |
| {MeS-Propionyl}F[hArg][hArg]Q[hArg]P[hArg ][Cha]SHKG[Oic][Nle][Oic][4-Cl-F]{COOH} | 3-(methylthio)propionyl-[hArg63,64,66,68;Cha69;Oic74,76;Nle75;4-Cl-F77]Apelin-17(62-77) | 1007 |
| {MeS-Propionyl}F[hArg][hArg]Q[hArg][Oic][h Arg][Cha]SHKG[Oic][Nle][Oic][4-Cl-F]{COOH} | 3-(methylthio)propionyl-[hArg63,64,66,68;Oic67,74,76;Cha69;Nle75 ;4-Cl-F77]Apelin-17(62-77) | 1008 |
| {MeS-Propionyl}F[hArg][hArg]Q[hArg]P[hArg ][Cha][K(NPeg11)]HKG[Oic][Nle]P[4-Cl-F]{COOH} | 3-(methylthio)propionyl-[hArg63,64,66,68;Cha69;K(NPeg11)70;Oic 74;Nle75;4-Cl-F77]Apelin-17(62-77) | 1009 |
| {MeS-Propionyl }F[hArg][hArg]Q[hArg]P[hArg ][Cha]S[K(NPeg11)]KG[Oic][Nle]P[4-C1-F]{COOH} | 3-(methylthio)propionyl-[hArg63,64,66,68;Cha69;K(NPeg11)71;Oic 74;Nle75;4-Cl-F77]Apelin-17(62-77) | 1010 |
| {MeS-Propionyl }F[hArg][hArg]Q[hArg]P[hArg ][Cha]SH[K(NPeg11)]G[Oic][Nle]P[4-Cl-F]{COOH} | 3-(methylthio)propionyl-[hArg63,64,66,68;Cha69;K(NPeg11)72;Oic 74;Nle75;4-Cl-F77]Apelin-17(62-77) | 1011 |
| {MeS-Propionyl }F[hArg][hArg]Q[hArg]P[hArg ][Cha][Atz(PEG10)]HKG[Oic][Nle]P[4-Cl-F]{COOH} | 3-(methylthio)propionyl-[hArg63,64,66,68;Cha69;Atz(PEG10)70;Oi | 1012 |
| {MeS-Propionyl}F[hArg][hArg]Q[hArg]P[hArg ][Cha]S[Atz(PEG10)]KG[Oic][Nle]P[4-Cl-F]{COOH} | 3-(methylthio)propionyl-[hArg63,64,66,68;Cha69;Atz(PEG10)71;Oi c74;Nle75;4-Cl-F77]Apelin-17(62-77) | 1013 |
| {MeS-Propionyl }F[hArg][hArg]Q[hArg]P[hArg ][Cha]THKG[Oic][Nle]P[4-Cl-F] {COOH} | 3-(methylthio)propionyl-[hArg63,64,66,68;Cha69;Thr70;Oic74;Nle7 5;4-Cl-F77]Apelin-17(62-77) | 1015 |
| {MeS-Propionyl}F[hArg][hArg]Q[hArg]P[hArg ][Cha]AHKG[Oi][Nle]P[4-Cl-F]{COOH} | 3-(methylthio)propionyl-[hArg63,64,66,68;Cha69;Ala70;Oic74;Nle7 5;4-Cl-F77]Apelin-17(62-77) | 1016 |
| {MeS-Propionyl}F[hArg][hArg]Q[hArg]P[hArg ][Cha][Abu]HKG[Oic][Nle]P[4-Cl-F]{COOH} | 3-(methylthio)propionyl-[hArg63,64,66,68;Cha69;Abu70;Oic74;Nle7 5;4-Cl-F77]Apelin-17(62-77) | 1017 |
| {MeS-Propionyl}F[hArg][hArg]Q[hArg]P[hArg][Cha][Nle]HKG[Oic][Nle]P[4-Cl-F]{COOH} | 3-(methylthio)propionyl-[hArg63,64,66,68;Cha69;Nle70,75;Oic74;4-Cl-F77]Apelin-17(62-77) | 1018 |
| {MeS-Propionyl }F[hArg][hArg]Q[hArg]P[hArg ][Cha][Nva]HKG[Oic][Nle]P[4-Cl-F] {COOH} | 3-(methylthio)propionyl-[hArg63,64,66,68;Cha69;Nva70;Oic74;Nle7 5;4-Cl-F77]Apelin-17(62-77) | 1019 |
| {MeS-Propionyl}F[hArg][harg]Q[hArg]P[harg ][Cha]VHKG[Oic][Nle]P[4-Cl-F]{COOH} | 3-(methylthio)propionyl-[hArg63,64,66,68;Cha69;Val70;Oic74;Nle7 5;4-Cl-F77]Apelin-17(62-77) | 1020 |
| {MeS-Propionyl }F[hArg][hArg]Q[hArg]P[hArg ][Cha]GHKG[Oic][Nle]P[4-Cl-F] {COOH} | 3-(methylthio)propionyl-[hArg63,64,66,68;Cha69;Gly70;Oic74;Nle7 5;4-Cl-F77]Apelin-17(62-77) | 1021 |
| {MeS-Propionyl }F[hArg][hArg]Q[hArg]P[hArg ][Cha]LHKG[Oic][Nle]P[4-Cl-F] {COOH} | 3-(methylthio)propionyl-[hArg63,64,66,68;Cha69;Leu70;Oic74;Nle7 5;4-Cl-F77]Apelin-17(62-77) | 1022 |
| {MeS-Propionyl }F[hArg][hArg]Q[hArg]P[hArg ][Cha]IHKG[Oic][Nle]P[4-Cl-F]{COOH} | 3-(methylthio)propionyl-[hArg63,64,66,68;Cha69;Ile70;Oic74;Nle75 ;4-Cl-F77]Apelin-17(62-77) | 1023 |
| {MeS-Propionyl }F[hArg][hArg]Q[hArg]P[hArg ][Cha][CPG]HKG[Oic][Nle]P[4-Cl-F]{COOH} | 3-(methylthio)propionyl-[hArg63,64,66,68;Cha69;CPG70;Oic74;Nle 75;4-Cl-F77]Apelin-17(62-77) | 1024 |
| {MeS-Propionyl }F[hArg][hArg]Q[hArg]P[hArg ][Cha][Chg]HKG[Oic][Nle]P[4-Cl-F] {COOH} | 3-(methylthio)propionyl-[hArg63,64,66,68;Cha69;Chg70;Oic74;Nle7 5;4-Cl-F77]Apelin-17(62-77) | 1025 |
| {MeS-Propionyl }F[hArg][hArg]Q[hArg]P[hArg ][Cha][Cha]HKG[Oic][Nle]P[4-Cl-F] {COOH} | 3-(methylthio)propionyl-[hArg63,64,66,68;Cha69,70;Oic74;Nle75;4-Cl-F77]Apelin-17(62-77) | 1026 |
| {MeS-Propionyl}F[hArg][hArg]Q[hArg]P[hArg ][Cha]HHKG[Oic][Nle]P[4-Cl-F]{COOH} | 3-(methylthio)propionyl-[hArg63,64,66,68;Cha69;His70;Oic74;Nle7 5;4-Cl-F77]Apelin-17(62-77) | 1027 |
| {MeS-Propionyl}F[hArg][hArg]Q[hArg]P[hArg ][Cha]YHKG[Oic][Nle]P[4-Cl-F]{COOH} | 3-(methylthio)propionyl-[hArg63,64,66,68;Cha69;Tyr70;Oic74;Nle7 5;4-Cl-F77]Apelin-17(62-77) | 1028 |
| {MeS-Propionyl}F[hArg][hArg]Q[hArg]P[hArg ][Cha]WHKG[Oic][Nle]P[4-C1-F]{COOH} | 3-(methylthio)propionyl-[hArg63,64,66,68;Cha69;Trp70;Oic74;Nle7 5;4-Cl-F77]Apelin-17(62-77) | 1029 |
| {MeS-Propionyl }F[hArg][hArg]Q[hArg]P[hArg ][Cha]FHKG[Oic][Nle]P[4-Cl-F] {COOH} | 3-(methylthio)propionyl-[hArg63,64,66,68;Cha69;Phe70;Oic74;Nle7 5;4-Cl-F77]Apelin-17(62-77) | 1030 |
| {MeS-Propionyl }F[hArg][hArg]Q[hArg]P[hArg ][Cha][NMePhe]HKG[Oic][Nle]P[4-Cl-F] {COOH} | 3-(methylthio)propionyl-[hArg63,64,66,68;Cha69;NMePhe70;Oic74; Nle75;4-Cl-F77]Apelin-17(62-77) | 1031 |
| {MeS-Propionyl}F[hArg][hArg]Q[hArg]P[hArg ][Cha][1-Nal]HKG[Oic][Nle]P[4-Cl-F]{COOH} | 3-(methylthio)propionyl-[hArg63,64,66,68;Cha69;1-Nal70;Oic74;Nle75;4-Cl-F77]Apelin-17(62-77) | 1032 |
| {MeS-Propionyl}F[hArg][hArg]Q[hArg]P[hArg ][Cha][2-Nal]HKG[Oic][Nle]P[4-Cl-F]{COOH} | 3-(methylthio)propionyl-[hArg63,64,66,68;Cha69;2-Nal70;Oic74;Nle75;4-Cl-F77]Apelin-17(62-77) | 1033 |
| {MeS-Propionyl }F[hArg][hArg]Q[hArg]P[hArg ][Cha][Bip]HKG[Oic][Nle]P[4-Cl-F]{COOH} | 3-(methylthio)propionyl-[hArg63,64,66,68;Cha69;Bip70;Oic74;Nle7 5;4-Cl-F77]Apelin-17(62-77) | 1034 |
| {MeS-Propionyl }F[hArg][hArg]Q[hArg]P[hArg ][Cha][Igl]HKG[Oic][Nle]P[4-Cl-F]{COOH} | 3-(methylthio)propionyl-[hArg63,64,66,68;Cha69;Igl70;Oic74;Nle75 ;4-Cl-F77]Apelin-17(62-77) | 1035 |
| {MeS-Propionyl }F[hArg][hArg]Q[hArg]P[hArg ][Cha][4-Cl-F]HKG[Oic][Nle]P[4-Cl-F] {COOH} | 3-(methylthio)propionyl-[hArg63,64,66,68;Cha69;4-Cl-F70,77;Oic74;Nle75]Apelin-17(62-77) | 1036 |
| {MeS-Propionyl }F[hArg][hArg]Q[hArg]P[hArg ][Cha][4-F-F]HKG[Oic][Nle]P[4-Cl-F] {COOH} | 3-(methylthio)propionyl-[hArg63,64,66,68;Cha69;4-F-F70;Oic74;Nle75;4-Cl-F77]Apelin-17(62-77) | 1037 |
| {MeS-Propionyl}F[hArg][hArg]Q[hArg]P[hArg ][Cha][pI-Phe]HKG[Oic][Nle]P[4-Cl-F]{COOH} | 3-(methylthio)propionyl-[hArg63,64,66,68;Cha69;pI-Phe70;Oic74;Nle75;4-Cl-F77]Apelin-17(62-77) | 1038 |
| {MeS-Propionyl }F[hArg][hArg]Q[hArg]P[hArg ][Cha][2Pal]HKG[Oic][Nle]P[4-Cl-F] {COOH} | 3-(methylthio)propionyl-[hArg63,64,66,68;Cha69;2Pal70;Oic74;Nle 75;4-Cl-F77]Apelin-17(62-77) | 1039 |
| {MeS-Propionyl }F[hArg][hArg]Q[hArg]P[hArg ][Cha][3Pal]HKG[Oic][Nle]P[4-Cl-F] {COOH} | 3-(methylthio)propionyl-[hArg63,64,66,68;Cha69;3Pal70;Oic74;Nle 75;4-Cl-F77]Apelin-17(62-77) | 1040 |
| {MeS-Propionyl}F[hArg][harg]Q[hArg]P[harg ][Cha][Tic]HKG[Oic][Nle]P[4-Cl-F]{COOH} | 3-(methylthio)propionyl-[hArg63,64,66,68;Cha69;Tic70;Oic74;Nle7 5;4-Cl-F77]Apelin-17(62-77) | 1041 |
| {MeS-Propionyl }F[hArg][hArg]Q[hArg]P[hArg ][Cha][Orn]HKG[Oic][Nle]P[4-Cl-F] {COOH} | 3-(methylthio)propionyl-[hArg63,64,66,68;Cha69;Orn70;Oic74;Nle7 5;4-Cl-F77]Apelin-17(62-77) | 1042 |
| {MeS-Propionyl }F[hArg][hArg]Q[hArg]P[hArg ][Cha][Dab]HKG[Oic][Nle]P[4-Cl-F] {COOH} | 3-(methylthio)propionyl-[hArg63,64,66,68;Cha69;Dab70;Oic74;Nle7 5;4-Cl-F77]Apelin-17(62-77) | 1043 |
| {MeS-Propionyl}F[hArg][harg]Q[hArg]P[harg ][Cha][Dap]HKG[Oic][Nle]P[4-Cl-F]{COOH} | 3-(methylthio)propionyl-[hArg63,64,66,68;Cha69;Dap70;Oic74;Nle7 5;4-Cl-F77]Apelin-17(62-77) | 1044 |
| {MeS-Propionyl }F[hArg][hArg]Q[hArg]P[hArg ][Cha]STKG[Oic][Nle]P[4-Cl-F]{COOH} | 3-(methylthio)propionyl-[hArg63,64,66,68;Cha69;Thr71;Oic74;Nle7 5;4-Cl-F77]Apelin-17(62-77) | 1045 |
| {MeS-Propionyl }F[hArg][hArg]Q[hArg]P[hArg ][Cha]SAKG[Oic][Nle]P[4-Cl-F]{COOH} | 3-(methylthio)propionyl-[hArg63,64,66,68;Cha69;Ala71;Oic74;Nle7 5;4-Cl-F77]Apelin-17(62-77) | 1046 |
| {MeS-Propionyl }F[hArg][hArg]Q[hArg]P[hArg ][Cha]S[Abu]KG[Oic][Nle]P[4-Cl-F] {COOH} | 3-(methylthio)propionyl-[hArg63,64,66,68;Cha69;Abu71;Oic74;Nle7 5;4-Cl-F77]Apelin-17(62-77) | 1047 |
| {MeS-Propionyl }F[hArg][hArg]Q[hArg]P[hArg ][Cha]S[Nle]KG[Oic][Nle]P[4-Cl-F] {COOH} | 3-(methylthio)propionyl-[hArg63,64,66,68;Cha69;Nle71,75;Oic74;4-Cl-F77]Apelin-17(62-77) | 1048 |
| {MeS-Propionyl}F[hArg][hArg]Q[hArg]P[hArg ][Cha]S[Nva]KG[Oi][Nle]P[4-Cl-F]{COOH} | 3-(methylthio)propionyl-[hArg63,64,66,68;Cha69;Nva71;Oic74;Nle7 5;4-Cl-F77]Apelin-17(62-77) | 1049 |
| {MeS-Propionyl}F[hArg][harg]Q[hArg]P[harg ][Cha]SVKG[Oic][Nle]P[4-Cl-][Cha]SVKG[Oic][Nle]P[4-Cl-F]{COOH} | 3-(methylthio)propionyl-[hArg63,64,66,68;Cha69;Val71;Oic74;Nle7 5;4-Cl-F77]Apelin-17(62-77) | 1050 |
| {MeS-Propionyl }F[hArg][hArg]Q[hArg]P[hArg ][Cha]SGKG[Oic][Nle]P[4-Cl-F] {COOH} | 3-(methylthio)propionyl-[hArg63,64,66,68;Cha69;Gly71;Oic74;Nle7 5;4-Cl-F77]Apelin-17(62-77) | 1051 |
| {MeS-Propionyl}F[hArg][hArg]Q[hArg]P[hArg ][Cha]SLKG[Oic][Nle]P[4-Cl-F]{COOH} | 3-(methylthio)propionyl-[hArg63,64,66,68;Cha69;Leu71;Oic74;Nle7 5;4-Cl-F77]Apelin-17(62-77) | 1052 |
| {MeS-Propionyl }F[hArg][hArg]Q[hArg]P[hArg ][Cha]SIKG[Oic][Nle]P[4-Cl-F] {COOH} | 3-(methylthio)propionyl-[hArg63,64,66,68;Cha69;Ile71;Oic74;Nle75 ;4-Cl-F77]Apelin-17(62-77) | 1053 |
| {MeS-Propionyl }F[hArg][hArg]Q[hArg]P[hArg ][Cha]S[CPG]KG[Oic][Nle]P[4-Cl-F] {COOH} | 3-(methylthio)propionyl-[hArg63,64,66,68;Cha69;CPG71;Oic74;Nle 75;4-Cl-F77]Apelin-17(62-77) | 1054 |
| {MeS-Propionyl }F[hArg][hArg]Q[hArg]P[hArg ][Cha]S[Chg]KG[Oic][Nle]P[4-Cl-F]{COOH} | 3-(methylthio)propionyl-[hArg63,64,66,68;Cha69;Chg71;Oic74;Nle7 5;4-Cl-F77]Apelin-17(62-77) | 1055 |
| {MeS-Propionyl }F[hArg][hArg]Q[hArg]P[hArg ][Cha]S[Cha]KG[Oic][Nle]P[4-Cl-F]{COOH} | 3-(methylthio)propionyl-[hArg63,64,66,68;Cha69,71;Oic74;Nle75;4-Cl-F77]Apelin-17(62-77) | 1056 |
| {MeS-Propionyl }F[hArg][hArg]Q[hArg]P[hArg ][Cha]SHKG[Oic][Nle]P[4-Cl-F]{COOH} | 3-(methylthio)propionyl-[hArg63,64,66,68;Cha69;Oic74;Nle75;4-Cl-F77]Apelin-17(62-77) | 1057 |
| {MeS-Propionyl }F[hArg][hArg]Q[hArg]P[hArg ][Cha]SYKG[Oic][Nle]P[4-Cl-F] {COOH} | 3-(methylthio)propionyl-[hArg63,64,66,68;Cha69;Tyr71;Oic74;Nle7 5;4-Cl-F77]Apelin-17(62-77) | 1058 |
| {MeS-Propionyl }F[hArg][hArg]Q[hArg]P[hArg ][Cha]SWKG[Oic][Nle]P[4-Cl-F] {COOH} | 3-(methylthio)propionyl-[hArg63,64,66,68;Cha69;Trp71;Oic74;Nle7 5;4-Cl-F77]Apelin-17(62-77) | 1059 |
| {MeS-Propionyl }F[hArg][hArg]Q[hArg]P[hArg ][Cha]SFKG[Oic][Nle]P[4-Cl-F] {COOH} | 3-(methylthio)propionyl-[hArg63,64,66,68;Cha69;Phe71;Oic74;Nle7 5;4-Cl-F77]Apelin-17(62-77) | 1060 |
| {MeS-Propionyl }F[hArg][hArg]Q[hArg]P[hArg ][Cha]S[NMePhe]KG[Oic][Nle]P[4-Cl-F] {COOH} | 3-(methylthio)propionyl-[hArg63,64,66,68;Cha69;NMePhe71;Oic74; Nle75;4-Cl-F77]Apelin-17(62-77) | 1061 |
| {MeS-Propionyl}F[hArg][hArg]Q[hArg]P[hArg ][Cha]S[1-Nal]KG[Oic][Nle]P[4-C1-F]{COOH} | 3-(methylthio)propionyl-[hArg63,64,66,68;Cha69;1-Nal71;Oic74;Nle75;4-Cl-F77]Apelin-17(62-77) | 1062 |
| {MeS-Propionyl}F[hArg][hArg]Q[hArg]P[hArg ][Cha]S[2-Nal]KG[Oic][Nle]P[4-Cl-F]{COOH} | 3-(methylthio)propionyl-[hArg63,64,66,68;Cha69;2-Nal71;Oic74;Nle75;4-Cl-F77]Apelin-17(62-77) | 1063 |
| {MeS-Propionyl }F[hArg][hArg]Q[hArg]P[hArg ][Cha]S[Bip]KG[Oic][Nle]P[4-Cl-F] {COOH} | 3-(methylthio)propionyl-[hArg63,64,66,68;Cha69;Bip71;Oic74;Nle7 5;4-Cl-F77]Apelin-17(62-77) | 1064 |
| {MeS-Propionyl }F[hArg][hArg]Q[hArg]P[hArg ][Cha]S[Igl]KG[Oic][Nle]P[4-Cl-F] {COOH} | 3-(methylthio)propionyl-[hArg63,64,66,68;Cha69;Igl71;Oic74;Nle75 ;4-Cl-F77]Apelin-17(62-77) | 1065 |
| {MeS-Propionyl }F[hArg][hArg]Q[hArg]P[hArg ][Cha]S[4-Cl-F]KG[Oic][Nle]P[4-Cl-F] {COOH} | 3-(methylthio)propionyl-[hArg63,64,66,68;Cha69;4-Cl-F71,77;Oic74;Nle75]Apelin-17(62-77) | 1066 |
| {MeS-Propionyl }F[hArg][hArg]Q[hArg]P[hArg ][Cha]S[4-F-F]KG[Oic][Nle]P[4-Cl-F]{COOH} | 3-(methylthio)propionyl-[hArg63,64,66,68;Cha69;4-F-F71;Oic74;Nle75;4-Cl-F77]Apelin-17(62-77) | 1067 |
| {MeS-Propionyl}F[hArg][hArg]Q[hArg]P[hArg ][Cha]S[4-I-F]KG[Oic][Nle]P[4-Cl-F]{COOH} | 3-(methylthio)propionyl-[hArg63,64,66,68;Cha69;4-I-F71;Oic74;Nle75;4-Cl-F77]Apelin-17(62-77) | 1068 |
| {MeS-Propionyl}F[hArg][hArg]Q[hArg]P[hArg ][Cha]S[2Pal]KG[Oic][Nle]P[4-Cl-F]{COOH} | 3-(methylthio)propionyl-[hArg63,64,66,68;Cha69;2Pal71;Oic74;Nle 75;4-Cl-F77]Apelin-17(62-77) | 1069 |
| {MeS-Propionyl }F[hArg][hArg]Q[hArg]P[hArg ][Cha]S[3Pal]KG[Oic][Nle]P[4-Cl-F] {COOH} | 3-(methylthio)propionyl-[hArg63,64,66,68;Cha69;3Pal71;Oic74;Nle 75;4-Cl-F77]Apelin-17(62-77) | 1070 |
| {MeS-Propionyl }F[hArg][hArg]Q[hArg]P[hArg ][Cha]S[Tic]KG[Oic][Nle]P[4-Cl-F] {COOH} | 3-(methylthio)propionyl-[hArg63,64,66,68;Cha69;Tic71;Oic74;Nle7 5;4-Cl-F77]Apelin-17(62-77) | 1071 |
| {MeS-Propionyl }F[hArg][hArg]Q[hArg]P[hArg ][Cha]S[Orn]KG[Oic][Nle]P[4-Cl-F] {COOH} | 3-(methylthio)propionyl-[hArg63,64,66,68;Cha69;Orn71;Oic74;Nle7 5;4-Cl-F77]Apelin-17(62-77) | 1072 |
| {MeS-Propionyl }F[hArg][hArg]Q[hArg]P[hArg ][Cha]S[Dab]KG[Oic][Nle]P[4-Cl-F] {COOH} | 3-(methylthio)propionyl-[hArg63,64,66,68;Cha69;Dab71;Oic74;Nle7 5;4-Cl-F77]Apelin-17(62-77) | 1073 |
| {MeS-Propionyl}F[hArg][harg]Q[hArg]P[harg ][Cha]S[Dap]KG[Oic][Nle]P[4-Cl-F]{COOH} | 3-(methylthio)propionyl-[hArg63,64,66,68;Cha69;Dap71;Oic74;Nle7 5;4-Cl-F77]Apelin-17(62-77) | 1074 |
| | | |
| {MeS-Propionyl }F[hArg][hArg]Q[hArg]P[hArg ][Cha]SH[Nle]G[Oic][Nle]P[4-Cl-F] {COOH} | 3-(methylthio)propionyl-[hArg63,64,66,68;Cha69;Nle72,75;Oic74;4-Cl-F77]Apelin-17(62-77) | 1076 |
| {MeS-Propionyl}F[hArg][hArg]Q[hArg]P[hArg ][Cha]SH[Nva]G[Oic][Nle]P[4-Cl-F]{COOH} | 3-(methylthio)propionyl-[hArg63,64,66,68;Cha69;Nva72;Oic74;Nle7 5;4-Cl-F77]Apelin-17(62-77) | 1077 |
| {MeS-Propionyl }F[hArg][hArg]Q[hArg]P[hArg ][Cha]SHVG[Oic][Nle]P[4-Cl-F]{COOH} | 3-(methylthio)propionyl-[hArg63,64,66,68;Cha69;Val72;Oic74;Nle7 5;4-Cl-F77]Apelin-17(62-77) | 1078 |
| {MeS-Propionyl}F[hArg][harg]Q[hArg]P[harg ][Cha]SHGG[Oic][Nle]P[4-Cl-F]{COOH} | 3-(methylthio)propionyl-[hArg63,64,66,68;Cha69;Gly72;Oic74;Nle7 5;4-Cl-F77]Apelin-17(62-77) | 1079 |
| {MeS-Propionyl}F[hArg][hArg]Q[hArg]P[hArg ][Cha]SH[CPG]G[Oic][Nle]P[4-Cl-F]{COOH} | 3-(methylthio)propionyl-[hArg63,64,66,68;Cha69;CPG72;Oic74;Nle 75;4-Cl-F77]Apelin-17(62-77) | 1080 |
| {MeS-Propionyl }F[hArg][hArg]Q[hArg]P[hArg ][Cha]SH[Chg]G[Oic][Nle]P[4-Cl-F] {COOH} | 3-(methylthio)propionyl-[hArg63,64,66,68;Cha69;Chg72;Oic74;Nle7 5;4-Cl-F77]Apelin-17(62-77) | 1081 |
| {MeS-Propionyl}F[hArg][harg]Q[hArg]P[harg ][Cha]SH[Cha]G[Oic][Nle]P[4-Cl-F]{COOH} | 3-(methylthio)propionyl-[hArg63,64,66,68;Cha69,72;Oic74;Nle75;4-Cl-F77]Apelin-17(62-77) | 1082 |
| {MeS-Propionyl}F[hArg][hArg]Q[hArg]P[hArg ][Cha]SHWG[Oic][Nle]P[4-Cl-F]{COOH} | 3-(methylthio)propionyl-[hArg63,64,66,68;Cha69;Trp72;Oic74;Nle7 5;4-Cl-F77]Apelin-17(62-77) | 1083 |
| {MeS-Propionyl}F[hArg][hArg]Q[hArg]P[hArg ][Cha]SH[1-Nal]G[Oic][Nle]P[4-Cl-F]{COOH} | 3-(methylthio)propionyl-[hArg63,64,66,68;Cha69;1-Nal72;Oic74;Nle75;4-Cl-F77]Apelin-17(62-77) | 1084 |
| {MeS-Propionyl}F[hArg][hArg]Q[hArg]P[hArg ][Cha]SH[2-Nal]G[Oic][Nle]P[4-Cl-F]{COOH} | 3-(methylthio)propionyl-[hArg63,64,66,68;Cha69;2-Nal72;Oic74;Nle75;4-Cl-F77]Apelin-17(62-77) | 1085 |
| {MeS-Propionyl }F[hArg][hArg]Q[hArg]P[hArg ][Cha]SH[Bip]G[Oic][Nle]P[4-Cl-F] {COOH} | 3-(methylthio)propionyl-[hArg63,64,66,68;Cha69;Bip72;Oic74;Nle7 5;4-Cl-F77]Apelin-17(62-77) | 1086 |
| {MeS-Propionyl }F[hArg][hArg]Q[hArg]P[hArg ][Cha]SH[Igl]G[Oic][Nle]P[4-Cl-F] {COOH} | 3-(methylthio)propionyl-[hArg63,64,66,68;Cha69;Igl72;Oic74;Nle75 ;4-Cl-F77]Apelin-17(62-77) | 1087 |
| {MeS-Propionyl }F[hArg][hArg]Q[hArg]P[hArg ][Cha]SH[4-Cl-F]G[Oic][Nle]P[4-Cl-F] {COOH} | 3-(methylthio)propionyl-[hArg63,64,66,68;Cha69;4-Cl-F72,77;Oic74;Nle75]Apelin-17(62-77) | 1088 |
| {MeS-Propionyl }F[hArg][hArg]Q[hArg]P[hArg ][Cha]SH[4-F-F]G[Oic][Nle]P[4-Cl-F]{COOH} | 3-(methylthio)propionyl-[hArg63,64,66,68;Cha69;4-F-F72;Oic74;Nle75;4-Cl-F77]Apelin-17(62-77) | 1089 |
| {MeS-Propionyl}F[hArg][hArg]Q[hArg]P[hArg ][Cha]SH[pI-Phe]G[Oic][Nle]P[4-Cl-F]{COOH} | 3-(methylthio)propionyl-[hArg63,64,66,68;Cha69;pI-Phe72;Oic74;Nle75;4-Cl-F77]Apelin-17(62-77) | 1090 |
| {MeS-Propionyl }F[hArg][hArg]Q[hArg]P[hArg ][Cha]SH[2Pal]G[Oic][Nle]P[4-Cl-F] {COOH} | 3-(methylthio)propionyl-[hArg63,64,66,68;Cha69;2Pal72;Oic74;Nle 75;4-Cl-F77]Apelin-17(62-77) | 1091 |
| {MeS-Propionyl }F[hArg][hArg]Q[hArg]P[hArg ][Cha]SH[3Pal]G[Oic][Nle]P[4-Cl-F] {COOH} | 3-(methylthio)propionyl-[hArg63,64,66,68;Cha69;3Pal72;Oic74;Nle 75;4-Cl-F77]Apelin-17(62-77) | 1092 |
| {MeS-Propionyl}F[hArg][hArg]Q[hArg]P[hArg ][Cha]SH[Orn]G[Oic][Nle]P[4-Cl-F]{COOH} | 3-(methylthio)propionyl-[hArg63,64,66,68;Cha69;Orn72;Oic74;Nle7 5;4-Cl-F77]Apelin-17(62-77) | 1093 |
| {MeS-Propionyl}F[hArg][hArg]Q[hArg]P[hArg ][Cha]SH[Dab]G[Oic][Nle]P[4-Cl-F]{COOH} | 3-(methylthio)propionyl-[hArg63,64,66,68;Cha69;Dab72;Oic74;Nle7 5;4-Cl-F77]Apelin-17(62-77) | 1094 |
| {MeS-Propionyl }F[hArg][hArg]Q[hArg]P[hArg ][Cha]SH[Dap]G[Oic][Nle]P[4-Cl-F] {COOH} | 3-(methylthio)propionyl-[hArg63,64,66,68;Cha69;Dap72;Oic74;Nle7 5;4-Cl-F77]Apelin-17(62-77) | 1095 |

Table 9 shows examples of peptides. The examples can include a PEG derivative or serum half-life prolonging group. In the table, the free acid can be a carboxylic acid and the amide can be at the C-terminus.

**TABLE 9.**

| AMINO ACID SEQUENCE | DESIGNATION | SEQ ID NO. |
|---|---|---|
| {Acetyl}[Atz(PEG10)],GGLRP[hArg ][NMeLeu]SHKGP[Nle]P[4-Cl-F]{FreeAcid} | Ac-Atz(PEG10)-Gly-Gly-[Leu65;hArg68;NMeLeu69;Nle75;4-Cl-F77]Apelin-13(65-77) | 1096 |
| {Acetyl}[Atz(PEG10)],GLR-P[hArg][ NMeLeu]SHKGP[Nle]P[4-Cl-F]{FreeAcid} | Ac-Atz(PEG10)-Gly-[Leu65;hArg68;NMeLeu69;Nle75;4-Cl-F77]Apelin-13(65-77) | 1097 |
| {Acetyl} [Atz(PEG1 0)],LRP[hArg][N MeLeu]SHKGP[Nle]P[4-Cl-F] {FreeAcid} | Ac-Atz(PEG10)-[Leu65;hArg68;NMeLeu69;Nle75;4-Cl-F77]Apelin-13(65-77) | 1098 |
| {Hydrogen}[PE][Atz(PEG10)],P[hAr g][NMeLeu]SHKGP[Nle]P[4-Cl-F]{FreeAcid} | [PE65;Atz(PEG10)66;hArg68;NMeLeu69 ;Nle75;4-Cl-F77]Apelin-13(65-77) | 1099 |
| {Hydrogen}[PE]R[Atz(PEG10)],[hA rg][NMeLeu]SHKGP[Nle]P[4-Cl-F] {FreeAcid} | [PE65;Atz(PEG10)67;hArg68;NMeLeu69 ;Nle75;4-Cl-F77]Apelin-13(65-77) | 1100 |
| {Hydrogen} [PE]RP[Atz(PEG10)][N MeLeu]SHKGP[Nle]P[4-Cl-F]{FreeAcid} | [PE65;Atz(PEG10)68;NMeLeu69;Nle75; 4-Cl-F77]Apelin-13(65-77) | 1101 |
| {Hydrogen} [PE]RP[hArg][Atz(PEG 10)]SHKGP[Nle]P[4-Cl-F]{FreeAcid} | PE65; [PE65;hArg68;Atz(PEG10)69;Nle75;4-Cl-F77]Apelin-13(65-77) | 1102 |
| {Hydrogen}[PE]RP[hArg][NMeLeu] [Atz(PEG10)]HKGP[Nle]P[4-Cl-F]{FreeAcid} | [PE65;hArg68;NMeLeu69;Atz(PEG10)70 ;Nle75;4-Cl-F77]Apelin-13(65-77) | 1103 |
| {Hydrogen}[PE]RP[hArg][NMeLeu] S[Atz(PEG10)]KGP[Nle]P[4-Cl-F]{FreeAcid} | [PE65;hArg68;NMeLeu69;Atz(PEG10)71 ;Nle75;4-Cl-F77]Apelin-13(65-77) | 1104 |
| {Hydrogen}[PE]RP[hArg][NMeLeu] SH[Atz(PEG10)]GP[Nle]P[4-Cl-F]{FreeAcid} | [PE65;hArg68;NMeLeu69;Atz(PEG10)72 ;Nle75;4-Cl-F77]Apelin-13(65-77) | 1105 |
| {Hydrogen} [PE]RP[hArg] [NMeLeu] SHK[Atz(PEG10)]P[Nle]P[4-Cl-F] {FreeAcid} | [PE65;hArg68;NMeLeu69;Atz(PEG10)73 ;Nle75;4-Cl-F77]Apelin-13(65-77) | 1106 |
| {Hydrogen}[PE]RP[hArg][NMeLeu] SHKGP[Atz(PEG10)]P[4-Cl-F]{FreeAcid} | [PE65;hArg68;NMeLeu69;Atz(PEG10)75 ;4-Cl-F77]Apelin-13(65-77) | 1107 |
| {Hydrogen}[PE]RP[hArg][NMeLeu] SHKGP[Nle][Atz(PEG10)][4-Cl-F]{FreeAcid} | [PE65;hArg68;NMeLeu69;Nle75;Atz(PE G10)76;4-Cl-F77]Apelin-13(65-77) | 1108 |
| {Hydrogen}[PE]RP[hArg][NMeLeu] SHKGP[Nle]P[Atz(PEG10)],{Amide | [PE65;hArg68;NMeLeu69;Nle75;Atz(PE G10)77]Apelin-13(65-77)-amide | 1109 |
| {Hydrogen}[PE]RP[hArg][NMeLeu] SHKGP[Nle]P[4-Cl-F][Atz(PEG10)],{Amide} | [PE65;hArg68;NMeLeu69;Nle75;4-Cl-F77]Apelin-13(65-77)-Atz(PEG10)-amide | 1110 |
| {Hydrogen}[PE]RP[hArg][NMeLeu] SHKGP[Nle]P[4-Cl-F]G[Atz(PEG10)],{Amide} | [PE65;hArg68;NMeLeu69;Nle75;4-Cl-F77]Apelin-13(65-77)-Gly-Atz(PEG10)-amide | 1111 |
| {Hydrogen}[PE]RP[hArg][NMeLeu] SHKGP[Nle]P[4-Cl-F]GG[Atz(PEG10)],{Amide} | [PE65;hArg68;NMeLeu69;Nle75;4-Cl-F77]Apelin-13 (65-77)-Gly-Gly-Atz(PEG10)-amide | 1112 |
| {Acetyl}[Atz(PEG10)],GGLRP[hArg ][Cha]SHKGP[Nle]f{FreeAeid} | Ac-Atz(PEG10)-[Gly65,66,75;Leu67;Pro69;hArg70;Cha71 ;Ser72;His73;Lys74;Nle77]Apelin-13(65-77)-D-Phe | 1113 |
| {Acetyl}[Atz(PEG10)],GLRP[hArg][ Cha]SHKGP[Nle]f{FreeAcid} | Ac-[Atz(PEG10)65;Gly66,75;Leu67;Pro69;h Arg70;Cha71;Ser72;His73;Lys74;Nle77] Apelin-13(65-77)-D-Phe | 1114 |
| {Acetyl}[Atz(PEG10)],LRP[hArg][C ha]SHKGP[Nle]f{FreeAcid} | Ac-Atz(PEG10)-[Leu65;hArg68;Cha69;Nle75;D-Phe76]Apelin-13(65-76) | 1115 |
| {Acetyl}[Atz(PEG10)],RP[hArg][Ch a]SHKGP[Nle]f{FreeAeid} | Ac-[Atz(PEG10)65;hArg68;Cha69;Nle75;D-Phe76]Apelin-13(65-76) | 1116 |
| {Hydrogen}[PE][Atz(PEG10)],P[hAr g][Cha]SHKGP[Nle]f{FreeAcid} | [PE65;Atz(PEG10)66;hArg68;Cha69;Nle 75;D-Phe76]Apelin-13(65-76) | 1117 |
| {Hydrogen}[PE]R[Atz(PEG10)],[hA rg][Cha]SHKGP[Nle]f{FreeAcid} | [PE65;Atz(PEG10)67;hArg68;Cha69;Nle 75;D-Phe76]Apelin-13(65-76) | 1118 |
| {Hydrogen}[PE]RP[Atz(PEG10)],[C ha]SHKGP[Nle]f{FreeAcid} | [PE65;Atz(PEG10)68;Cha69;Nle75;D-Phe76]Apelin-13(65-76) | 1119 |
| {Hydrogen} [PE]RP[hArg][Atz(PEG 10)],SHKGP[Nle]f{FreeAcid} | [PE65;hArg68;Atz(PEG10)69;Nle75;D-Phe76]Apelin-13(65-76) | 1120 |
| {Hydrogen} [PE]RP[hArg] [Cha] [Atz( PEG10)],HKGP[Nle]f{FreeAcid} | [PE65;hArg68;Cha69;Atz(PEG10)70;Nle 75;D-Phe76]Apelin-13(65-76) | 1121 |
| {Hydrogen}[PE]RP[hArg][Cha]S[At z(PEG10)],KGP[Nle]f{FreeAcid} | [PE65;hArg68;Cha69;Atz(PEG10)71;Nle 75;D-Phe76]Apelin-13(65-76) | 1122 |
| {Hydrogen} [PE]RP[hArg][Cha]SH[ Atz(PEG10)],GP[Nle]f{FreeAcid} | [PE65;hArg68;Cha69;Atz(PEG10)72;Nle 75;D-Phe76]Apelin-13(65-76) | 1123 |
| {Hydrogen} [PE]RP[hArg][Cha]SHK [Atz(PEG10)],P[Nle]f{FreeAcid} | [PE65;hArg68;Cha69;Atz(PEG10)73;Nle 75;D-Phe76]Apelin-13(65-76) | 1124 |
| {Hydrogen} [PE]RP[hArg][Cha]SHK G[Atz(PEG10)],[Nle]f{FreeAcid} | [PE65;hArg68;Cha69;Atz(PEG10)74;Nle 75;D-Phe76]Apelin-13(65-76) | 1125 |
| {Hydrogen}[PE]RP[hArg][Cha]SHK GP[Atz(PEG10)],f{FreeAcid} | [PE65;hArg68;Cha69;Atz(PEG10)75;D-Phe76]Apelin-13(65-76) | 1126 |
| {Hydrogen} [PE]RP[hArg][Cha]SHK GP[Nle][Atz(PEG10)],{Amide} | [PE65;hArg68;Cha69;Nle75;Atz(PEG10) 76]Apelin-13(65-76)-amide | 1127 |
| {Hydrogen}[PE]RP[hArg] [Cha]SHK GP[Nle]f[Atz(PEG10)], {Amide} | [PE65;hArg68;Cha69;Nle75;D-Phe76;Atz(PEG10)77]Apelin-13 (65-77)-amide | 1128 |
| {Hydrogen}[PE]RP[hArg] [Cha]SHK GP[Nle]fG[Atz(PEG10)], {Amide} | [PE65;hArg68;Cha69;Nle75;D-Phe76;Gly77]Apelin-13(65-77)-Atz(PEG10)-amide | 1129 |
| {Hydrogen}[PE]RP[hArg][Cha]SHK GP[Nle]fGG[Atz(PEG10)],{Amide} {Acetyl}[Atz(PEG10)]LRP[hArg][N MeLeu][Atz(PEG10)]HKGP[Nle][4-Cl-F]{FreeAcid} | [PE65;hArg68;Cha69;Nle75;D-Phe76;Gly77]Apelin-13(65-77)-Gly-Atz(PEG10)-amide Ac-Atz(PEG10)-[Leu65;hArg68;NMeLeu69;Atz(PEG10)7 0;Nle75;4-Cl-F76]Apelin-13(65-76) | 1130 1131 |
| {Hydrogen}[PE]RP[hArg] [Cha][Atz( PEG10)]HKGP[Nle][4-Cl-F]{FreeAcid} | [PE65;hArg68;Cha69;Atz(PEG10)70;Nle 75;4-Cl-F76]Apelin-13(65-76) | 1132 |
| {Hydrogen}[PE]RP[hArg][Cha][Atz( PEG10)]HKG[Oic][Nle][4-Cl-F]{FreeAcid} | 74;Nle75,4-Cl-F76]Apelin-13(65-76) | 1133 |
| {Hydrogen}[PE]RP[hArg] [Cha][Atz( PEG10)]HKG[Oic][Nle][4-Cl-F]{Amide} | [PE65;hArg68;Cha69;Atz(PEG10)70;Oic 74;Nle75;4-Cl-F76]Apelin-13(65-76)-amide | 1134 |
| {Hydrogen}[PE]RP[hArg][Cha]F[At z(PEG10)]KG[Oic][Nle][4-Cl-F]{FreeAcid} | [PE65;hArg68;Cha69;Phe70;Atz(PEG10) 71;Oic74;Nle75;4-Cl-F76]Apelin-13(65-76) | 1135 |
| {Hydrogen}[PE]RP[hArg][Cha]F[At z(PEG10)]KG[Oic][Nle][4-Cl-F]{Amide} | [PE65;hArg68;Cha69;Phe70;Atz(PEG10) 71;Oic74;Nle75;4-Cl-F76]Apelin-13(65-76)-amide | 1136 |
| {Hydrogen}[PE]RP[hArg][NMeLeu] F[Atz(PEG10)]KG[Oic][Nle][4-Cl-F]{FreeAcid} | [PE65;hArg68;NMeLeu69;Phe70;Atz(PE G10)71;Oic74;Nle75;4-Cl-F76]Apelin-13(65-76) | 1137 |
| {Hydrogen}[PE]RP[hArg][NMeLeu] F[Atz(PEG10)]KG[Oic][Nle][4-Cl-F]{Amide} | [PE65;hArg68;NMeLeu69;Phe70;Atz(PE G10)71;Oic74;Nle75;4-Cl-F76]Apelin-13(65-76)-amide | 1138 |
| {Hydrogen}[PE]RP[hArg][NMeLeu] [Atz(PEG10)]HKGP[Nle][4-Cl-F]{FreeAcid} | [PE65;hArg68;NMeLeu69;Atz(PEG10)70 ;Nle75;4-Cl-F76]Apelin-13(65-76) | 1139 |
| {Hydrogen}[PE]RP[hArg][NMeLeu] [Atz(PEG10)]HKG[Oic][Nle][4-Cl-F]{FreeAcid} | [PE65;hArg68;NMeLeu69;Atz(PEG10)70 ;Oic74;Nle75;4-Cl-F76]Apelin-13(65-76) | 1140 |
| {Hydrogen}[PE]RP[hArg][NMeLeu] [Atz(PEG10)]HKG[Oic][Nle][4-Cl-F]{Amide} {Acetyl}[Atz(PEG10)]LRP[hArg][N MeLeu][Atz(PEG10)]HKGP[Nle]f{F reeAcid} | [PE65;hArg68;NMeLeu69;Atz(PEG10)70 ;Oic74;Nle75;4-Cl-F76]Apelin-13(65-76)-amide Ac-Atz(PEG10)-[Leu65;hArg68;NMeLeu69;Atz(PEG10)7 0;Nle75;D-Phe76]Apelin-13(65-76) | 1141 1142 |
| {Hydrogen} [PE]RP[hArg] [Cha] [Atz( PEG10)]HKGP[Nle]f{FreeAcid} | [PE65;hArg68;Cha69;Atz(PEG10)70;Nle 75;D-Phe76]Apelin-13(65-76) | 1143 |
| {Hydrogen} [PE]RP[hArg] [Cha] [Atz( PEG10)]HKG[Oic][Nle]f{FreeAcid} | [PE65;hArg68;Cha69;Atz(PEG10)70;Oic 74;Nle75;D-Phe76]Apelin-13(65-76) | 1144 |
| {Hydrogen}[PE]RP[hArg][Cha]F[At z(PEG10)]KGP[Nle]f{FreeAcid} | [PE65;hArg68;Cha69;Phe70;Atz(PEG10) 71;Nle75;D-Phe76]Apelin-13(65-76) | 1145 |
| {Hydrogen}[PE]RP[hArg][Cha]F[At z(PEG10)]KG[Oic][Nle]f{FreeAcid} | [PE65;hArg68;Cha69;Phe70;Atz(PEG10) 71;Oic74;Nle75;D-Phe76]Apelin-13(65-76) | 1146 |
| {Hydrosen}[PE]RP[hArg][Cha]F[At z(PEG10)]KGP[Nle]f{Amide} | [PE65;hArg68;Cha69;Phe70;Atz(PEG10) 71;Nle75;D-Phe76]Apelin-13(65-76)-amide | 1147 |
| {Hydrosen}[PE]RP[hArg][Cha]F[At z(PEG10)]KG[Oic][Nle]f{Amide} | [PE65;hArg68;Cha69;Phe70;Atz(PEG10) 71;Oic74;Nle75;D-Phe76]Apelin-13(65-76)-amide | 1148 |
| {Hydrogen}[PE]RP[hArg][NMeLeu] F[Atz(PEG10)]KGP[Nle]f{FreeAcid} | [PE65;hArg68;NMeLeu69;Phe70;Atz(PE G10)71;Nle75;D-Phe76]Apelin-13(65-76) | 1149 |
| {Hydrogen}[PE]RP[hArg][NMeLeu] F[Atz(PEG10)]KG[Oic][Nle]f{FreeA cid} | [PE65;hArg68;NMeLeu69;Phe70;Atz(PE G10)71;Oic74;Nle75;D-Phe76]Apelin-13(65-76) | 1150 |
| {Hydrogen}[PE]RP[hArg][NMeLeu] F[Atz(PEG10)]KGP[Nle]f{Amide} | [PE65;hArg68;NMeLeu69;Phe70;Atz(PE G10)71;Nle75;D-Phe76]Apelin-13(65-76)-amide | 1151 |
| {Hydrogen}[PE]RP[hArg][NMeLeu] F[Atz(PEG10)]KG[Oic][Nle]f{Amid e} | [PE65;hArg68;NMeLeu69;Phe70;Atz(PE G10)71;Oic74;Nle75;D-Phe76]Apelin-13(65-76)-amide | 1152 |
| {Hydrogen}[PE]RP[hArg][NMeLeu] [Atz(PEG10)]HKGP[Nle]f{FreeAcid} | [PE65;hArg68;NMeLeu69;Atz(PEG10)70 ;Nle75;D-Phe76]Apelin-13(65-76) | 1153 |
| {Hydrogen}[PE]RP[hArg][NMeLeu] [Atz(PEG10)]HKG[Oic][Nle]f{Free Acid} {Hydrogen}[PE]RP[hArg][NMeLeu] [Atz(20K-mPEG)]HKG[Oic][Nle][4-Cl-F] {FreeAcid} | [PE65;hArg68;NMeLeu69;Atz(PEG10)70 ;Oic74;Nle75;D-Phe76]Apelin-13(65-76) [PE65;hArg68;NMeLeu69;[Atz(20K-mPEG)]70;Oic74;Nle75;4-Cl-F76]Apelin-13(65-76) | 1155 1156 |
| {Hydrogen} [PE]RP[hArg] [Cha] [Atz( 20K-mPEG)]HKG[Oic][Nle][4-Cl-F] {FreeAcid} | [PE65;hArg68;Cha69;[Atz(20K-mPEG)]70;Oic74;Nle75;4-Cl-F76]Apelin-13(65-76) | 1157 |
| {Hydrogen} [PE]RP[hArg] [NMeLeu] F[Atz(20K-mPEG)]KG[Oic][Nle]f{FreeAcid} {Acetyl}[Atz(20K-mPEG)]LRP[hArg][Cha]SHKGP[Nle ][D-1Nal]{FreeAcid} | [PE65;hArg68;NMeLeu69;Phe70;[Atz(20 K-mPEG)]71;Oic74;Nle75;D-Phe76]Apelin-13(65-76) [Ac-Atz(20K-mPEG)64;hArg68;Cha69;Nle75;D-1-Na176]Apelin-13(64-76) | 1158 1159 |
| {Acetyl}[Atz(20K-mPEG)] [Ahx]LRP[hArg][Cha]SHK GP[Nle][D-1Nal]{FreeAcid} | [Ac-Atz(20K-mPEG)63;Ahx64;hArg68;Cha69;Nle75;D -1-Nal76]Apelin-13(63-76) | 1160 |
| {Acetyl}[Atz(PEG10)]LRP[hArg][N MeLeu][Atz(PEG10)]HKGP[Nle]P[4 -Cl-F]{FreeAcid} | Ac-Atz(PEG10)-[Leu65;hArg68;NMeLeu69;Atz(PEG10)7 0;Nle75;4-Cl-F77]Apelin-13(65-77) | 1161 |
| {Hydrogen} [PE]RP[hArg] [Cha] [Atz( PEG10)]HKGP[Nle]P[4-Cl-F]{FreeAcid} | [PE65;hArg68;Cha69;Atz(PEG10)70;Nle 75;4-Cl-F77]Apelin-13(65-77) | 1162 |
| {Hydrogen} [PE]RP[hArg] [Cha] [Atz( PEG10)]HKG[Oic][Nle]P[4-Cl-F]{FreeAcid} | [PE65;hArg68;Cha69;Atz(PEG10)70;Oic 74,Nle75,4-Cl-F77]Apelin-13(65-77) | 1163 |
| {Hydrogen} [PE]RP[hArg] [Cha] [Atz( PEG10)]HKG[Oic][Nle]P[4-Cl-F]{Amide} | [PE65;hArg68;Cha69;Atz(PEG10)70;Oic 74;Nle75;4-Cl-F77]Apelin-13(65-77)-amide | 1164 |
| {Hydrogen}[PE]RP[hArg][NMeLeu] [Atz(PEG10)]HKGP[Nle]P[4-Cl-F]{FreeAcid} | [PE65;hArg68;NMeLeu69;Atz(PEG10)70 ;Nle75;4-Cl-F77]Apelin-13(65-77) | 1165 |
| {Hydrogen}[PE]RP[hArg][NMeLeu] [Atz(PEG10)]HKG[Oic][Nle]P[4-Cl-F]{FreeAcid} | [PE65;hArg68;NMeLeu69;Atz(PEG10)70 ;Oic74;Nle75;4-Cl-F77]Apelin-13(65-77) | 1166 |
| {Hydrogen}[PE]RP[hArg][NMeLeu] [Atz(PEG10)]HKG[Oic][Nle]P[4-Cl-F]{Amide} {Hydrogen} [PE]RP[hArg] [Cha] [Atz( 1K-mPEG)]HKG[Oic][Nle]P[4-Cl-F]{FreeAcid} | [PE65;hArg68;NMeLeu69;Atz(PEG10)70 ;Oic74;Nle75;4-Cl-F77]Apelin-13(65-77)-amide [PE65;hArg68;Cha69;Atz(1K-mPEG)70;Oic74;Nle75;4-Cl-F77]Apelin-13(65-77) | 1167 1168 |
| {Hydrogen} [PE]RP[hArg] [Cha] [Atz( 2K-mPEG)]HKG[Oic][Nle]P[4-Cl-F]{FreeAcid} | [PE65;hArg68;Cha69;Atz(2K-mPEG)70;Oic74;Nle75;4-Cl-F77]Apelin-13(65-77) | 1169 |
| {Hydrogen} [PE]RP[hArg] [Cha] [Atz( 5K-mPEG)]HKG[Oic][Nle]P[4-Cl-F]{FreeAcid} | [PE65;hArg68;Cha69;Atz(5K-mPEG)70;Oic74;Nle75;4-Cl-F77]Apelin-13(65-77) | 1170 |
| {Hydrogen} [PE]RP[hArg] [Cha] [Atz( 1 0K-mPEG)]HKG[Oic][Nle]P[4-Cl-F]{FreeAcid} | [PE65;hArg68;Cha69;Atz(10K-mPEG);Oic74;Nle75;4-Cl-F77]Apelin-13(65-77) | 1171 |
| {Hydrogen} [PE]RP[hArg] [Cha] [Atz( 20K-mPEG)]HKG[Oic][Nle]P[4-Cl-F]{FreeAcid} | [PE65;hArg68;Cha69;Atz(20K-mPEG)70;Oic74;Nle75;4-Cl-F77]Apelin-13(65-77) | 1178 |
| {Hydrogen}[PE]RP[hArg][NMeLeu] [Atz(20K-mPEG)]HKG[Oic][Nle][4-Cl-F]{FreeAcid} | [PE65;hArg68;NMeLeu69;[Atz(20K-mPEG)]70;Oic74;Nle75;4-Cl-F76]Apelin-13(65-76) | 1179 |
| {Hydrogen} [PE]RP[hArg] [Cha] [Atz( 20K-mPEG)]HKG[Oic][Nle][4-Cl-F]{FreeAcid} | [PE65;hArg68;Cha69;[Atz(20K-mPEG)]70;Oic74;Nle75;4-Cl-F76]Apelin-13(65-76) | 1180 |
| {Hydrogen}[PE]RP[hArg][NMeLeu] [Atz(20K-mPEG)]HKG[Oic][Nle]P[4-Cl-F]{FreeAcid} | [PE65;hArg68;NMeLeu69;[Atz(20K-mPEG)]70;Oic74;Nle75;4-Cl-F77]Apelin-13(65-77) | 1181 |
| {Hydrogen}[PE]RP[hArg][Cha]S[At z(1K-mPEG)]KG[Oic][Nle]P[4-Cl-F]{FreeAcid} | [PE65;hArg68;Cha69;Atz(1K-mPEG)71;Oic74;Nle75;4-Cl-F77]Apelin-13(65-77) | 1182 |
| {Hydrogen}[PE]RP[hArg][Cha]S[At z(2K-mPEG)]KG[Oic][Nle]P[4-Cl-F]{FreeAcid} | [PE65;hArg68;Cha69;Atz(2K-mPEG)K71;Oic74;Nle75;4-Cl-F77]Apelin-13(65-77) | 1183 |
| {Hydrogen}[PE]RP[hArg][Cha]S[At z(5K-mPEG)]KG[Oic][Nle]P[4-Cl-F]{FreeAcid} | [PE65;hArg68;Cha69;Atz(5K-mPEG)71;Oic74;Nle75;4-Cl-F77]Apelin-13(65-77) | 1184 |
| {Hydrogen}[PE]RP[hArg][Cha]S[At z(10K-mPEG)]KG[Oic][Nle]P[4-Cl-F]{FreeAcid} | [PE65;hArg68;Cha69;Atz(10K-mPEG);Oic74;Nle75;4-Cl-F77]Apelin-13(65-77) | 1185 |
| {Hydrogen}[PE]RP[hArg][Cha]S[At z(20K-mPEG)]KG[Oic][Nle]P[4-Cl-F]{FreeAcid} | [PE65;hArg68;Cha69;Atz(20K-mPEG)71;Oic74;Nle75;4-Cl-F77]Apelin-13(65-77) | 1186 |
| {Hydrogen}[PE]RP[hArg][NMeLeu] F[Atz(20K-mPEG)]KG[Oic][Nle]f{FreeAcid} {Acetyl}[Atz(20K-mPEG)]LRP[hArg][NMeLeu][Atz(2 0K-mPEG)]HKGP[Nle]P[4-Cl-F]{FreeACid} {Hydrogen}[PE]RP[hArg][NMeLeu] SHKGP[Nle]P[D-Bip]K(Ac-Atz(20K-mPEG)) | [PE65;hArg68;NMeLeu69;Phe70;[Atz(20 K-mPEG)]71;Oic74;Nle75;D-Phe76]Apelin-13(65-76) Ac-[Atz(20K-mPEG)]-[Leu65;hArg68;NMeLeu69;[Atz(20K-mPEG)]70;Nle75;4-Cl-F77]Apelin-13(65-77) PE65;hArg68;NMeLeu69;Nle75;D-Bip77;K(Ac-Atz(20K-mPEG))78]Apelin-13(65-78)-amide | 1187 1188 1189 |
| {Acetyl}[Atz(1K-mPEG)]LRP[hArg] [Cha]SHKG[Oic] [Nle]P[4-Cl-F]{FreeAcid} | Ac-Atz(1K-mPEG)-[Leu65;hArg68;Cha69;Oic74;Nle75;4-Cl-F77]Apelin-13(65-77) | 1190 |
| {Acetyl}[Atz(2K-mPEG)]LRP[hArg] [Cha]SHKG[Oic] [Nle]P[4-Cl-F] {FreeAcid} | Ac-Atz(2K-mPEG)-[Leu65;hArg68;Cha69;Oic74;Nle75;4-Cl-F77]Apelin-13(65-77) | 1191 |
| {Acetyl}[Atz(5K-mPEG)]LRP[hArg] [Cha]SHKG[Oic] [Nle]P[4-Cl-F] {FreeAcid} | Ac-Atz(5K-mPEG)-[Leu65;hArg68;Cha69;Oic74;Nle75;4-Cl-F77]Apelin-13(65-77) | 1192 |
| {Acetyl}[Atz(10K-mPEG)]LRP[hArg] [Cha]SHKG[Oic] [Nle]P[4-Cl-F]{FreeAcid} | Ac-Atz(10K-mPEG)-[Leu65;hArg68;Cha69;Oic74;Nle75;4-Cl-F77]Apelin-13(65-77) | 1193 |
| {Acetyl}[Atz(20K-mPEG)]LRP[hArg] [Cha]SHKG[Oic] [Nle]P[4-Cl-F] {FreeAcid} | Ac-Atz(20K-mPEG)-[Leu65;hArg68;Cha69;Oic74;Nle75;4-Cl-F77]Apelin-13(65-77) | 1194 |
| {Hydrogen}[Atz(20K-mPEG)]KFRRQRP[hArg] [Cha]SHK G[Oic][Nle]P[4-Cl-F]{FreeAcid} | Atz(20K-mPEG)-[hArg68;Cha69;Oic74;Nle75;4-Cl-F77]Apelin-17(61-77) | 1195 |
| {Acetyl}[Atz(20K-mPEG)]LRP[hArg][Cha]SHKGP[Nle ][D-1Nal]{FreeAcid} | [Ac-Atz(20K-mPEG)64;hArg68;Cha69;Nle75;D-1-Nal76]Apelin-13(64-76) | 1196 |
| {Acetyl}[Atz(20K-mPEG)] [Ahx]LRP[hArg][Cha]SHK GP[Nle][D-1Nal]{FreeAcid} | [Ac-Atz(20K-mPEG)63;Ahx64;hArg68;Cha69;Nle75;D -1-Nal76]Apelin-13(63-76) | 1197 |
| {Hydrogen}[Atz(20K-mPEG)]LRP[hArg] [Cha]SHKG[Oic] [Nle]P[4-Cl-F]{FreeAcid} { Thiopropionyl(20K-mPEG)}LRP[hArg][Cha]SHKG[Oic] [Nle]P[4-Cl-F] {FreeAcid} | Atz(20K-mPEG)-[Leu65;hArg68;Cha69;Oic74;Nle75;4-Cl-F77]Apelin-13(65-77) Thiopropionyl(20K-mPEG)-[Leu65;hArg68;Cha69;Oic74;Nle75;4-Cl-F77]Apelin-13(65-77) | 1198 1199 |
| {Thiopropionyl(20K-mPEG)}KFRRQRP[hArg] [Cha]SHK G[Oic][Nle]P[4-Cl-F]{FreeAcid} | Thiopropionyl(20K-mPEG)-[hArg68;Cha69;Oic74;Nle75;4-Cl-F77]Apelin-17(61-77) | 1200 |
| {Thiopropionyl(40K-mPEG)}KFRRQRP[hArg] [Cha]SHK G[Oic][Nle]P[4-Cl-F]{FreeAcid} | Thiopropionyl(40K-mPEG)-[hArg68;Cha69;Oic74;Nle75;4-Cl-F77]Apelin-17(61-77) | 1201 |
| {Thiopropionyl(20K-mPEG)}LLRP[hArg][Cha]SHKG[Oi c][Nle]P[4-Cl-F]{FreeAcid} | Thiopropionyl(20K-mPEG)-Leu-[Leu65;hArg68;Cha69;Oic74;Nle75;4-Cl-F77]Apelin-13(65-77) | 1202 |
| Thiopropionyl(20K-mPEG)}FRRQRP[hArg][Cha]SHKG [Oic][Nle]P[4-Cl-F]{COOH} | Thiopropionyl(20K-mPEG)-[hArg68;Cha69;Oic74;Nle75;4-Cl-F77]Apelin-17(62-77) | 1203 |
| {Thiopropionyl(20K-mPEG)}RRQRP[hArg] [Cha]SHKG[ Oic][Nle]P[4-Cl-F]{COOH} | Thiopropionyl(20K-mPEG)-[hArg68;Cha69;Oic74;Nle75;4-Cl-F77]Apelin-17(63-77) | 1204 |
| {Thiopropionyl(20K-mPEG)}RQRP[hArg] [Cha]SHKG[Oi c][Nle]P[4-Cl-F]{COOH} | Thiopropionyl(20K-mPEG)-[hArg68;Cha69;Oic74;Nle75;4-Cl-F77]Apelin-17(64-77) | 1205 |
| {Thiopropionyl(20K-mPEG)}QRP[hArg][Cha]SHKG[Oic ][Nle]P[4-Cl-F] {COOH} | Thiopropionyl(20K-mPEG)-[hArg68;Cha69;Oic74;Nle75;4-Cl-F77]Apelin-17(65-77) | 1206 |
| {Thiopropionyl(1K-mPEG)}QRP[hArg][Cha]SHKG[Oic ][Nle]P[4-Cl-F] {FreeAcid} | Thiopropionyl(1K-mPEG)-[hArg68;Cha69;Oic74;Nle75;4-Cl-F77]Apelin-17(65-77) | 1207 |
| {Thiopropionyl(2K-mPEG)}QRP[hArg][Cha]SHKG[Oic ][Nle]P[4-Cl-F] {FreeAcid} | Thiopropionyl(2K-mPEG)-[hArg68;Cha69;Oic74;Nle75;4-Cl-F77]Apelin-17(65-77) | 1208 |
| {Thiopropionyl(5K-mPEG)}QRP[hArg][Cha]SHKG[Oic ][Nle]P[4-Cl-F]{FreeAcid} | Thiopropionyl(5K-mPEG)-[hArg68;Cha69;Oic74;Nle75;4-Cl-F77]Apelin-17(65-77) | 1209 |
| {Thiopropionyl(10K-mPEG)}QRP[hArg][Cha]SHKG[Oic ][Nle]P[4-Cl-F] {FreeAcid} | Thiopropionyl(10K-mPEG)-[hArg68;Cha69;Oic74;Nle75;4-Cl-F77]Apelin-17(65-77) | 1210 |
| {Thiopropionyl(40K-mPEG)}QRP[hArg][Cha]SHKG[Oic ][Nle]P[4-Cl-F]{FreeAcid} {Thiopropionyl(1K-mPEG)}KFRRQRP[hArg][Cha]SHK G[Oic][Nle]P[4-Cl-F]{FreeAcid} | Thiopropionyl(40K-mPEG)-[hArg68;Cha69;Oic74;Nle75;4-Cl-F77]Apelin-17(65-77) Thiopropionyl(1K-mPEG)-[hArg68;Cha69;Oic74;Nle75;4-Cl-F77]Apelin-17(61-77) | 1211 1212 |
| {Thiopropionyl(2K-mPEG)}KFRRQRP[hArg][Cha]SHK G[Oic][Nle]P[4-Cl-F]{FreeAcid} | Thiopropionyl(2K-mPEG)-[hArg68;Cha69;Oic74;Nle75;4-Cl-F77]Apelin-17(61-77) | 1213 |
| {Thiopropionyl(5K-mPEG)}KFRRQRP[hArg] [Cha]SHK G[Oic][Nle]P[4-Cl-F]{FreeAcid} | Thiopropionyl(5K-mPEG)-[hArg68;Cha69;Oic74;Nle75;4-Cl-F77]Apelin-17(61-77) | 1214 |
| {Thiopropionyl(10K-mPEG)}KFRRQRP[hArg][Cha]SHK G[Oic][Nle]P[4-Cl-F]{FreeAcid} | Thiopropionyl(10K-mPEG)-[hArg68;Cha69;Oic74;Nle75;4-Cl-F77]Apelin-17(61-77) | 1215 |
| {Thiopropionyl(40K-mPEG)}KFRRQRP[hArg][Cha]SHK G[Oic][Nle]P[4-Cl-F]{FreeAcid} | Thiopropionyl(40K-mPEG)-[hArg68;Cha69;Oic74;Nle75;4-Cl-F77]Apelin-17(61-77) | 1216 |
| {Thiopropionyl(20K-mPEG)}F[hArg][hArg]Q[hArg]P[hA rg][Cha]SHKG[Oic][Nle]P[4-Cl-F] | Thiopropionyl(20K-mPEG)-[hArg63,64,66,68;Cha69;Oic74;Nle75;4-Cl-F77]Apelin-17(62-77) | 1217 |
| {Thiopropionyl(20K-mPEG) }OF[hArg][hArg]Q[hArg]P[h Arg] [Cha]SHKG[Oic][Nle]P[4-Cl-F] | Thiopropionyl(20K-mPEG)-[Orn61;hArg63,64,66,68;Cha69;Oic 74;Nle75;4-Cl-F77]Apelin-17(61-77) | 1218 |
| {Thiopropionyl(20K-mPEG)}OF[hArg][hArg]Q[Cit]P[hA rg] [Cha]SHKG[Oic][Nle]P[4-Cl-F] | Thiopropionyl(20K-mPEG)-[Orn61;hArg63,64,68;Cit66;Cha69; Oic74;Nle75;4-Cl-F77]Apelin-17(61-77) | 1219 |
| {Thiopropionyl(20K-mPEG)}F[hArg][hArg]Q[Cit]P[hArg ][Cha]SHKG[Oic][Nle]P[4-Cl-F] | Thiopropionyl(20K-mPEG)-[hArg63,64,68;Cit66;Cha6 9;Oic74;Nle75;4-Cl-F77]Apelin-17(62-77) | 1220 |
| {Thiopropionyl(20K-mPEG)}F[Cit][Cit]Q[hArg]P[hArg][ Cha]SHKG[Oic][Nle]P[4-Cl-F] [NPeg11]KFRRQRP[hArg][Cha]SH KG[Oic] [Nle]P[4-Cl-F] | Thiopropionyl(20K-mPEG)-[Cit63,64;hArg66,68;Cha6 9;Oic74;Nle75;4-Cl-F77]Apelin-17(62-77) NPeg11-[hArg68;Cha69;Oic74;Nle75;4-Cl-F77]Apelin-17(61-77) | 1221 1222 |
| [Atz(PEG10)]KFRRQRP[hArg][Cha] SHKG[Oic][Nle]P[4-Cl-F] | Atz(Peg10)-[hArg68;Cha69;Oic74;Nle75;4-Cl-F77]Apelin-17(61-77) | 1223 |
| [Atz(PEG10)]KF[hArg][hArg]QRP[h Arg][Cha]SHKG[Oic][Nle]P[4-Cl-F] | Atz(Peg10)-[hArg63,64,68;Cha69;Oic74;Nle75;4-Cl-F77]Apelin-17(61-77) | 1224 |
| [Atz(PEG10)]KF[hArg][hArg]Q[hAr g]P[hArg][Cha]SHKG[Oic][Nle]P[4-Cl-F] | Atz(Peg10)-[hArg63,64,66,68;Cha69;Oic74;Nle75;4-Cl-F77]Apelin-17(61-77) | 1225 |
| [Atz(PEG10)]KF[hArg][hArg]QQP[h Arg] [Cha]SHKG[Oic][Nle]P[4-Cl-F] | Atz(Peg10)-[hArg63,64,68;Gln66;Cha69;Oic74;Nle75 ;4-Cl-F77]Apelin-17(61-77) | 1226 |
| [Atz(PEG10)]KF[hArg][hArg]Q[Cit] P[hArg] [Cha]SHKG[Oic][Nle]P[4-Cl-F] | Atz(Peg10)-[hArg63,64,68;Cit66;Cha69;Oic74;Nle75; 4-Cl-F77]Apelin-17(61-77) | 1227 |
| [Atz(PEG10)]KF[hArg][Cit]Q[Cit]P[ hArg][Cha]SHKG[Oic][Nle]P[4-Cl-F] | Atz(Peg10)-[hArg63,68;Cit64,66;Cha69;Oic74;Nle75; 4-Cl-F77]Apelin-17(61-77) | 1228 |
| [Atz(PEG10)]KF[Cit][Cit]Q[hArg]P[ hArg][Cha]SHKG[Oic][Nle]P[4-Cl-F] | Atz(Peg10)-[Cit63,64;hArg66,68;Cha69;Oic74;Nle75; 4-Cl-F77]Apelin-17(61-77) | 1229 |
| [Atz(PEG10)]KF[Cit][Cit]Q[Cit]P[h Arg] [Cha]SHKG[Oic][Nle]P[4-Cl-F] | Atz(Peg10)-[Cit63,64,66;hArg68;Cha69;Oic74;Nle75; 4-Cl-F77]Apelin-17(61-77) | 1230 |
| [Atz(PEG10)]OFRRQRP[hArg][Cha] SHKG[Oic][Nle]P[4-Cl-F] | Atz(Peg10)-[Orn61;hArg68;Cha69;Oic74;Nle75;4-Cl-F77]Apelin-17(61-77) | 1231 |
| [Atz(PEG10)]OF[hArg][hArg]Q[hAr g]P[hArg][Cha]SHKG[Oic][Nle]P[4-Cl-F] | Atz(Peg10)-[Orn61;hArg63,64,66,68;Cha69;Oic74;Nl e75;4-Cl-F77]Apelin-17(61-77) | 1232 |
| [Atz(PEG10)]FRRQRP[hArg][Cha]S HKG[Oic][Nle]P[4-Cl-F] | Atz(Peg10)-[hArg68;Cha69;Oic74;Nle75;4-Cl-F77]Apelin-17(62-77) | 1233 |
| [Atz(PEG10)]LRP[hArg][Cha]SHKG [Oic][Nle]P[4-Cl-F] | Atz(Peg10)-[Leu65;hArg68;Cha69;Oic74;Nle75;4-Cl-F77]Apelin-17(65-77) | 1234 |
| {Palmitoyl}LRP[hArg][Cha]FHKGP [Nle][4-Cl-F]{FreeAcid} | Palmitoyl-[Leu65;hArg68;Cha69;Phe70;Nle75;4-Cl-F76]Apelin-13(65-76) | 1235 |
| {Palmitoyl}LRP[hArg][Cha]FHKPP[ Nle][D-1Nal]{FreeAcid} | Palmitoyl-[Leu65;hArg68;Cha69;Phe70;Pro73;Nle7 5;D-1Nal76]Apelin-13(65-76) | 1236 |
| {Palmitoyl}LRP[hArg][Cha]SHKGP [Nle][4-Cl-F] | Palmitoyl-Leu65;hArg68;Cha69;Nle75;4-Cl-F76]Apelin-13(65-76) | 1237 |
| {Palmitoyl}LRP[hArg][NMeLeu]FA [1-Nal]G[1-Nal][Nle][4-Cl-F]{Amide} | Palmitoyl-[Leu65;hArg68;NMeLeu69;Phe70;Ala71; 1-Nal72,74;Nle75;4-Cl-F76]Apelin-13(65-76) | 1238 |
| {Palmitoyl}LRP[hArg][NMeLeu]FA [1-Nal]G[Oic][Nle][4-Cl-F]{Amide} | Palmitoyl-[Leu65;hArg68;NMeLeu69;Phe70;Ala71; 1-Nal72,Oic74;Nle75;4-Cl-F76]Apelin-13(65-76) | 1239 |
| {Palmitoyl}LRP[hArg][NMeLeu]FA [1-Nal]G[1-Nal][Nle][4-Cl-F]{Amide} | Palmitoyl-[Leu65;hArg68;NMeLeu69;Phe70;Ala71; 1-Nal72,74;Nle75;4-Cl-F76]Apelin-13(65-76) | 1240 |

Table 10 shows examples ofpeptides. The examples can include a free acid that can be a carboxylic acid and an amide that can be at the C-terminus.

**TABLE 10.**

| AMINO ACID SEQUENCE | DESIGNATION | SEQ ID NO |
|---|---|---|
| {Hydrogen}[PE]RP[hArg][Cha]SHKGP[Nle][4 -F-F]{FreeAcid} | [PE65;hArg68;Cha69;Nle75;4-F-F76]Apelin-13(65-76) | 1241 |
| {Hydrogen}[PE]RP[hArg][Cha]SHKGP[Nle][4 -Me-F] {FreeAcid} | [PE65;hArg68;Cha69;Nle75;4-Me-F76]Apelin-13(65-76) | 1242 |
| {Hydrogen}[PE]RP[hArg][Cha]SHKGP[Nle][D -Bip] {FreeAcid} | [PE65;hArg68;Cha69;Nle75;D-Bip76]Apelin-13(65-76) | 1243 |
| {Hydrogen}[PE]RP[hArg][Cha]SHKGP[Nle]y{ FreeAcid} | [PE65;hArg68;Cha69;Nle75;D-Tyr76]Apelin-13(65-76) | 1244 |
| {Hydrogen}[PE]RPR[Cha]SHKGP[Nle]F{Free Acid} | [PE65;Cha69;Nle75;Phe76]Apelin -13(65-76) | 1245 |
| {Hydrogen} [PE]RP[hArg][NMeLeu]SHKGP[N le]F{FreeAcid} | [PE65;hArg68;NMeLeu69;Nle75; Phe76]Apelin-13(65-76) | 1246 |
| {Hydrogen} [PE]RP[hArg][Cha]SHKGP[pI-Phe]F{FreeAcid} | [PE65;hArg68;Cha69;pI-Phe75;Phe76]Apelin-13(65-76) | 1247 |
| {Hydrogen}[PE]RPR[Cha]SHKGP[pI-Phe]F{FreeAcid} | [PE65;Cha69;pI-Phe75;Phe76]Apelin-13(65-76) | 1248 |
| {Hydrogen} [PE]RP[hArg][NMeLeu]SHKGP[pI -Phe]F{FreeAcid} | [PE65;hArg68;NMeLeu69;pI-Phe75;Phe76]Apelin-13(65-76) | 1249 |
| {Hydrogen}[PE]RP[hArg][Cha]SHKGP[Nle][p I-Phe]{FreeAcid} | [PE65;hArg68;Cha69;Nle75;pI-Phe76]Apelin-13(65-76) | 1250 |
| {Hydrogen}[PE]RP[hArg][Cha]SHKPP[Nle][D -1Nal]{Amide} | [PE65;hArg68;Cha69;Pro73;Nle75 ;D-1Nal76]Apelin-13(65-76)-amide | 1251 |
| {Hydrogen}[PE]RP[hArg][Cha]FHKPP[Nle][4-Cl-F]{FreeAcid} | [PE65;hArg68;Cha69;Phe70;Pro73 ;Nle75;4-Cl-F76]Apelin-13(65-76) | 1252 |
| {Hydrogen} [PE]RP[hArg][Cha]FHKGP[Nle][4 -Cl-F]{FreeAcid} | [PE65;hArg68;Cha69;Phe70;Nle75 ;4-Cl-F76]Apelin-13(65-76) | 1253 |
| {Hydrogen}[PE]RP[hArg][Cha]SHKPP[Nle][4-Cl-F]{FreeAcid} | [PE65;hArg68;Cha69;Pro73;Nle75 ;4-Cl-F76]Apelin-13(65-76) | 1254 |
| {Hydrogen} [PE]RP[hArg][Cha]FHKPP[Nle][D -1Nal]{FreeAcid} | [PE65;hArg68;Cha69;Phe70;Pro73 ;Nle75;D-1Nal76]Apelin-13(65-76) | 1255 |
| {Hydrogen}[PE]RP[hArg][Cha]FHKGP[Nle][D -1Nal]{FreeAcid} | [PE65;hArg68;Cha69;Phe70;Nle75 ;D-1Nal76]Apelin-13(65-76) | 1256 |
| {Hydroge}[PE]RP[hArg][Cha]FHKPP[Nle][D -1Nal]{Amide} | [PE65;hArg68;Cha69;Phe70;Pro73 ;Nle75;D-1Nal76]Apelin-13(65-76)-amide | 1257 |
| {Hydrogen}[PE]RP[hArg][Cha]FHKGP[Nle][D -1Nal]{Amide} | [PE65;hArg68;Cha69;Phe70;Nle75 ;D-1Nal76]Apelin-13(65-76)-amide | 1258 |
| {Hydrogen}[PE]RP[hArg][Cha]FHKPP[Nle][4-Cl-F]{Amide} | [PE65;hArg68;Cha69;Phe70;Pro73 ;Nle75;4-Cl-F76]Apelin-13(65-76)-amide | 1259 |
| {Hydrogen}[PE]RP[hArg][Cha]FHKGP[Nle][4 -Cl-F] {Amide} | [PE65;hArg68;Cha69;Phe70;Nle75 ;4-Cl-F76]Apelin-13(65-76)-amide | 1260 |
| {Hydrogen}[PE]RP[hArg][Cha]SHKGP[Nle]f{ Amide} | [PE65;hArg68;Cha69;Nle75;D-Phe76]Apelin-13(65-76)-amide | 1261 |
| {Hydrogen} [PE]RP[hArg][Cha]SHKGP[Nle]w {Amide} | [PE65;hArg68;Cha69;Nle75;D-Trp76]Apelin-13(65-76)-amide | 1262 |
| {Hydrogen}[PE]RP[hArg][Cha]SHKGP[Nle][D -2Nal] {Amide} | [PE65;hArg68;Cha69;Nle75;D-2Nal76]Apelin-13(65-76)-amide | 1263 |
| {Hydrogen} [PE]RP[hArg][NMeLeu][NMePhe] AKG[1-Nal][Nle]f{FreeAcid} | [PE65;hArg68;NMeLeu69;NMePh e70;Ala71;1-Nal74;Nle75;D-Phe76]Apelin-13(65-76) | 1264 |
| {Hydrogen}[PE]R-P[hArg][NMeLeu]FHKG[1-Nal][Nle]f{FreeAcid} | [PE65;hArg68;NMeLeu69;Phe70; 1-Nal74;Nle75;D-Phe76]Apelin-13(65-76) | 1265 |
| {Hydrogen}[PE]RP[hArg][Cha]SHKGP[Nle][4 -Cl-F] {Amide} | [PE65;hArg68;Cha69;Nle75;4-Cl-F76]Apelin-13(65-76)-amide | 1266 |
| {Hydrogen} [PE]RP[hArg][Cha]SHKG[1- Nal][Nle][4-Cl-F]{Amide} | [PE65;hArg68;Cha69;1-Nal74;Nle75;4-Cl-F76]Apelin-13(65-76)-amide | 1267 |
| {Hydrogen} [PE]RP[hArg][Cha]FHKG[1- Nal][Nle][4-Cl-F]{Amide} | [PE65;hArg68;Cha69;Phe70;1-Nal74;Nle75;4-Cl-F76]Apelin-13(65-76)-amide | 1268 |
| {Hydrogen}[PE]RP[hArg][Cha]FHKGP[Nle][4 -Cl-F] {Amide} | [PE65;hArg68;Cha69;Phe70;Nle75 ;4-Cl-F76]Apelin-13(65-76)-amide | 1269 |
| {Hydrogen} [PE]RP[hArg][Cha]SAKG[1- Nal][Nle][4-Cl-F]{Amide} | [PE65;hArg68;Cha69;Ala71;1-Nal74;Nle75;4-Cl-F76]Apelin-13(65-76)-amide | 1270 |
| {Hydrogen} [PE]RP[hArg][Cha]FAKG[1- Nal][Nle][4-Cl-F]{Amide} | [PE65;hArg68;Cha69;Phe70;Ala71 ;1-Nal74;Nle75;4-Cl-F76]Apelin-13(65-76)-amide | 1271 |
| {Hydrogen} [PE]RP[hArg][Cha][NMePhe] [2-Nal]KG[1-Nal][Nle][4-Cl-F]{FreeAcid} | [PE65;hArg68;Cha69;NMePhe70; 2-Nal71;1-Nal74;Nle75 ;4-Cl-F76]Apelin-13(65-76) | 1272 |
| {Hydrogen}[PE]R-P[hArg][Cha]FAKGP[Nle][4 -Cl-F]{Amide} | [PE65;hArg68;Cha69;Phe70;Ala71 ;Nle75;4-Cl-F76]Apelin-13(65-76)-amide | 1273 |
| {Hydrogen}[PE]RP[hArg][Cha]S[1-Nal]KG[1 - Nal][Nle][4-Cl-F]{Amide} | [PE65;hArg68;Cha69;1-Nal71,74;Nle75;4-Cl-F76]Apelin-13(65-76)-amide | 1274 |
| {Hydrogen} [PE]RP[hArg][Cha]F[1-Nal]KG[1- Nal][Nle][4-Cl-F]{Amide} | [PE65;hArg68;Cha69;Phe70;1-Nal71,74;Nle75;4-Cl-F76]Apelin-13(65-76)-amide | 1275 |
| {Hydrogen}[PE]RP[hArg][Cha]F[1 - Nal]KGP[Nle][4-Cl-F]{Amide} | [PE65;hArg68;Cha69;Phe70;1-Nal71;Nle75;4-Cl-F76]Apelin-13(65-76)-amide | 1276 |
| {Hydrogen} [PE]RP[hArg][Cha][NMePhe] [1 - Nal]KG[1-Nal][Nle][4-Cl-F]{Amide} | [PE65;hArg68;Cha69;NMePhe70; 1-Nal71,74;Nle75;4-Cl-F76]Apelin-13(65-76)-amide | 1277 |
| {Hydrogen}[PE]RP[hArg][Cha][NMePhe][1-Nal]KG[1-Nal][Nle][4-Cl-F]{Amide} | [PE65;hArg68;Cha69;NMePhe70; 1-Nal71,74;Nle75;4-Cl-F76]Apelin-13(65-76)-amide | 1278 |
| {Hydrogen} [PE]RP[hArg][Cha][NMePhe][1 - Nal]KGP[Nle][4-Cl-F]{Amide} | [PE65;hArg68;Cha69;NMePhe70; 1-Nal71;Nle75;4-Cl-F76]Apelin-13(65-76)-amide | 1279 |
| {Hydrogen} [PE]RP[hArg][Cha][NMePhe]AKG [1-Nal][Nle][4-Cl-F] {Amide} | [PE65;hArg68;Cha69;NMePhe70; Ala71;1-Nal74;Nle75 ;4-Cl-F76]Apelin-13(65-76)-amide | 1280 |
| {Hydrogen}[PE]RP[hArg][Cha][NMePhe]AKG [1-Nal][Nle][4-Cl-F]{Amide} | [PE65;hArg68;Cha69;NMePhe70; Ala71;1-Nal74;Nle75;4-Cl-F76]Apelin-13(65-76)-amide | 1281 |
| {Hydrogen}[PE]RP[hArg][Cha][NMePhe]AKG P[Nle][4-Cl-F]{Amide} | [PE65;hArg68;Cha69;NMePhe70; Ala71;Nle75;4-Cl-F76]Apelin-13(65-76)-amide | 1282 |
| {Hydrogen} [PE]RP[hArg][Cha]S[2-Nal]KG[1 - Nal][Nle][4-Cl-F]{Amide} | [PE65;hArg68;Cha69;2-Nal71;1-Nal74;Nle75;4-Cl-F76]Apelin-13(65-76)-amide | 1283 |
| {Hydrogen} [PE]RP[hArg][Cha]F[2-Nal]KG[1-Nal][Nle][4-Cl-F]{Amide} | [PE65;hArg68;Cha69;Phe70;2-Na171;1-Nal74;Nle75;4-Cl-F76]Apelin-13(65-76)-amide | 1284 |
| {Hydrogen}[PE]RP[hArg][Cha]F[2-Nal]KGP[Nle][4-Cl-F]{Amide} | [PE65;hArg68;Cha69;Phe70;2-Nal71;Nle75;4-Cl-F76]Apelin-13(65-76)-amide | 1285 |
| {Hydrogen} [PE]RP[hArg][Cha][NMePhe][2-Nal]KG[1-Nal][Nle][4-Cl-F]{Amide} | [PE65;hArg68;Cha69;NMePhe70; 2-Nal71;1-Nal74;Nle75 ;4-Cl-F76]Apelin-13(65-76)-amide | 1286 |
| {Hydrogen} [PE]RP[hArg][Cha][NMePhe] [2-Nal]KG[1-Nal][Nle][4-Cl-F]{Amide} | [PE65;hArg68;Cha69;NMePhe70; 2-Nal71;1-Nal74;Nle75 ;4-Cl-F76]Apelin-13(65-76)-amide | 1287 |
| {Hydrogen} [PE]RP[hArg][Cha][NMePhe][2-Nal]KGP[Nle][4-Cl-F]{Amide} | [PE65;hArg68;Cha69;NMePhe70; 2-Nal71;Nle75;4-Cl-F76]Apelin-13(65-76)-amide | 1288 |
| {Hydrogen}[PE]R-P[hArg][NMeLeu]F[1-Nal]KG[1-Nal][Nle]f{Amide} | [PE65;hArg68;NMeLeu69;Phe70; 1-Nal71,74;Nle75;D-Phe76]Apelin-13(65-76)-amide | 1289 |
| {Hydrogen} [PE]RP[hArg][NMeLeu]F[2-Nal]KG[1-Nal][Nle]f{Amide} | [PE65;hArg68;NMeLeu69;Phe70; 2-Nal71;1-Nal74;Nle75 ;D-Phe76]Apelin-13(65-76)-amide | 1290 |
| {Hydrogen}[PE]RP[hArg][Cha][NMePhe][1-Nal]KG[1-Nal][Nle][4-Cl-F]{FreeAcid} | [PE65;hArg68;Cha69;NMePhe70; 1-Nal71,74;Nle75;4-Cl-F76]Apelin-13(65-76) | 1291 |
| {Hydrogen}[PE]RP[hArg][NMeLeu]SHKGP[N le]f{Amide} | [PE65;hArg68;NMeLeu69;Nle75; D-Phe76]Apelin-13(65-76)-amide | 1292 |
| {Hydrogen}[PE]RP[hArg][NMeLeu]SHKG[1-Nal][Nle]f{Amide} | [PE65;hArg68;NMeLeu69;1-Nal74;Nle75;D-Phe76]Apelin-13(65-76)-amide | 1293 |
| {Hydrogen}[PE]RP[hArg][NMeLeu]FHKG[1-Nal][Nle]f{Amide} | [PE65;hArg68;NMeLeu69;Phe70; 1-Nal74;Nle75;D-Phe76]Apelin-13(65-76)-amide | 1294 |
| {Hydrogen} [PE]RP[hArg][NMeLeu]FHKGP[N le]f{Amide} | [PE65;hArg68;NMeLeu69;Phe70; Nle75;D-Phe76]Apelin-1 3(65-76)-amide | 1295 |
| {Hydrogen}[PE]RP[hArg][NMeLeu]SAKG[1-Nal][Nle]f{Amide} | [PE65;hArg68;NMeLeu69;Ala71;1 -Nal74;Nle75;D-Phe76]Apelin-13(65-76)-amide | 1296 |
| {Hydrogen}[PE]RP[hArg][NMeLeu]FAKG[1-Nal][Nle]f{Amide} | [PE65;hArg68;NMeLeu69;Phe70; Ala71; 1-Nal74;Nle75;D-Phe76]Apelin-13(65-76)-amide | 1297 |
| {Hydrogen}[PE]R-P[hArg][NMeLeu]FA[1-Nal]G[1-Nal][Nle]f{Amide} | [PE65;hArg68;NMeLeu69;Phe70; Ala71; 1-Nal72,74;Nle75;D-Phe76]Apelin-13(65-76)-amide | 1298 |
| {Hydrogen} [PE]RP[hArg][NMeLeu]FAKGP[N le]f{Amide} | [PE65;hArg68;NMeLeu69;Phe70; Ala71;Nle75;D-Phe76]Apelin-13(65-76)-amide | 1299 |
| {Hydrogen}[PE]R-P[hArg][NMeLeu]S[1-Nal]KG[1-Nal][Nle]f{Amide} | [PE65;hArg68;NMeLeu69;1-Nal71,74;Nle75;D-Phe76]Apelin-13(65-76)-amide | 1300 |
| {Hydrogen}[PE]RP[hArg][NMeLeu]F[1-Nal]KG[1-Nal][Nle]f{Amide} | [PE65;hArg68;NMeLeu69;Phe70; 1-Nal71,74;Nle75;D-Phe76]Apelin-13(65-76)-amide | 1301 |
| {Hydrogen}[PE]R-P[hArg][NMeLeu]F[1-Nal]KGP[Nle]f{Amide} | [PE65;hArg68;NMeLeu69;Phe70; 1-Nal71;Nle75;D-Phe76]Apelin-13(65-76)-amide | 1302 |
| {Hydrogen} [PE]RP[hArg][NMeLeu][NMePhe] [1-Nal]KGP[Nle]f{Amide} | [PE65;hArg68;NMeLeu69;NMePh e70;1-Nal71;Nle75;D-Phe76]Apelin-13(65-76)-amide | 1303 |
| {Hydrogen} [PE]RP[hArg][NMeLeu][NMePhe] AKG[1-Nal][Nle]f{Amide} | [PE65;hArg68;NMeLeu69;NMePh e70;Ala71;1-Nal74;Nle75 ;D-Phe76]Apelin-13(65-76)-amide | 1304 |
| {Hydrogen} [PE]RP[hArg][NMeLeu][NMePhe] AKG[1-Nal][Nle]f{Amide} | [PE65;hArg68;NMeLeu69;NMePh e70;Ala71; 1-Nal74;Nle75 ;D-Phe76]Apelin-13(65-76)-amide | 1305 |
| {Hydrogen}[PE]RP[hArg][NMeLeu][NMePhe] A[1-Nal]G[1-Nal][Nle]f{Amide} | [PE65;hArg68;NMeLeu69;NMePh e70;Ala71; 1-Nal72,74;Nle75;D-Phe76]Apelin-13(65-76)-amide | 1306 |
| {Hydrogen} [PE]RP[hArg][NMeLeu][NMePhe] AKGP[Nle]f{Amide} | [PE65;hArg68;NMeLeu69;NMePh e70;Ala71;Nle75;D-Phe76]Apelin-13(65-76)-amide | 1307 |
| {Hydrogen}[PE]RP[hArg][NMeLeu]S[2-Nal]KG[1-Nal][Nle]f{Amide} | [PE65;hArg68;NMeLeu69;2-Nal71;1-Nal74;Nle75;D-Phe76]Apelin-13(65-76)-amide | 1308 |
| {Hydrogen}[PE]RP[hArg][NMeLeu]F[2-Nal]KG[1-Nal][Nle]f{Amide} | [PE65;hArg68;NMeLeu69;Phe70; 2-Nal71;1-Nal74;Nle75 ;D-Phe76]Apelin-13(65-76)-amide | 1309 |
| {Hydrogen} [PE]RP[hArg][NMeLeu]F[2-Nal]KGP[Nle]f{Amide} | [PE65;hArg68;NMeLeu69;Phe70; 2-Nal71;Nle75;D-Phe76]Apelin-13(65-76)-amide | 1310 |
| {Hydrogen}[PE]RP[hArg][NMeLeu][NMePhe] [2-Nal]KGP[Nle]f{Amide} | [PE65;hArg68;NMeLeu69;NMePh e70;2-Nal71;Nle75;D-Phe76]Apelin-13(65-76)-amide | 1311 |
| [PE]RP[hArg][Cha]SHKGP[Nle][D-1Nal] | [PE65;hArg68;Cha69;Nle75;D-1Nal76]Apelin-13(65-76) | 1312 |
| [PE]RP[hArg][Cha]SHKGP[Nle][4-Cl-F] | [PE65;hArg68,Cha69;Nle75,4-Cl-F76]Apelin-13(65-76) | 1313 |
| [PE]RP[hArg][Cha]SHKGP[Nle][2-Nal] | [PE65;hArg68;Cha69;Nle75;2-Nal76]Apelin-13(65-76) | 1314 |
| [PE]RP[hArg][Cha]SHKGP[Nle][NMePhe] {A mide} | [PE65;hArg68;Cha69;Nle75;NMe Phe76]Apelin-13(65-76)-amide | 1315 |
| [PE]RP[hArg][Cha]SHKGP[Nle]f | [PE65;hArg68;Cha69;Nle75;D-Phe76]Apelin-13(65-76) | 1316 |
| [PE]RP[hArg][Cha]SHKGP[Nle]w | [PE65;hArg68;Cha69;Nle75;D-Trp76]Apelin-13(65-76) | 1317 |
| [PE]RP[hArg][Cha]SHKGP[Nle][1-Nal] | [PE65;hArg68;Cha69;Nle75;1-Nal76]Apelin-13(65-76) | 1318 |
| [PE]RP[hArg][Cha]F[1-Nal]KG[1-Nal][Nle] [4-Cl-F] {Amide} | [PE65;hArg68;Cha69;Phe70;1-Nal71,74;Nle75;4-Cl-F76]Apelin-13(65-76)-amide | 1319 |
| [PE]RP[hArg][NMeLeu]FA[1-Nal]G[1-Nal][Nle]f{Amide} | [PE65;hArg68;NMeLeu69;Phe70; Ala71;1-Nal72,74;Nle75;D-Phe76]Apelin-13(65-76)-amid | 1320 |
| [PE]RP[hArg][Cha]F[1-Nal]KG[Oic][Nle][4-Cl-F] {Amide} | [PE65;hArg68;Cha69;Phe70;1-Nal71,Oic74;Nle75;4-Cl-F76]Apelin-13(65-76)-amide | 1321 |
| [PE]RP[hArg][NMeLeu]FA[1-Nal]G[Oic][Nle]f{Amide} | [PE65;hArg68;NMeLeu69;Phe70; Ala71;1-Nal72,Oic74;Nle75;D-Phe76]Apelin-13(65-76)-a | 1322 |
| [PE]RP[hArg][Cha]F[1-Nal]KG[1-Nal][Nle] [4-Cl-F] {Amide} | [PE65;hArg68;Cha69;Phe70;1-Nal71,74;Nle75;4-Cl-F76]Apelin-13(65-76)-amide | 1323 |
| [PE]RP[hArg][NMeLeu]FA[1-Nal]G[1-Nal][Nle]f{Amide} | [PE65;hArg68;NMeLeu69;Phe70; Ala71;1-Nal72,74;Nle75;D-Phe76]Apelin-13(65-76)-amid | 1324 |
| {Hydrogen}[PE]R-P[hArg][NMeLeu][NMePhe] A[1-Nal]G[1-Nal][Nle]f{Amide} | [PE65;hArg68;NMeLeu69;NMePh e70;Ala71; 1-Nal72,74;Nle75;D-Phe76]Apelin-13(65-76)-amide | 1325 |
| {Hydrogen}[PE]R-P[hArg][NMeLeu]FA[1-Nal]G[l-Nal][Nle]f{Amide} | [PE65;hArg68;NMeLeu69;Phe70; Ala71; 1-Nal72,74;Nle75;D-Phe76]Apelin-13(65-76)-amide | 1326 |

Table 11 shows examples of peptides. The examples can include a free acid that can be a carboxylic acid and an amide that can be at the C-terminus and can also include a PEG derivative or serum half-life prolonging group.

**TABLE 11**

| AMINO ACID SEQUENCE | DESIGNATION | SEQ ID NO. |
|---|---|---|
| {Acetyl}[Atz(PEG10)]GGLRP[hArg][Cha]SH KGP[Nle]f{FeeAcid} | Ac-Atz(PEG10)-GG-[Leu65;hArg68;Cha69;Nle75;D-Phe76]Apelin-13(65-76) | 1327 |
| {Acetyl}[Atz(PEG10)]GLRP[hArg][Cha]SHK GP[Nle]f{FreeAcid} | Ac-[Atz(PEG10)-G-[Leu65;hArg68;Cha69;Nle75;D-Phe76]Apelin-13(65-76) | 1328 |
| {Acetyl}[Atz(PEG10)]LRP[hArg][Cha]SHKG P[Nle]f{FreeAcid} | Ac-Atz(PEG10)-[Leu65;hArg68;Cha69;Nle75;D-Phe76]Apelin-13(65-76) | 1329 |
| {Acetyl}[Atz(PEG10)]RP[hArg][Cha] SHKGP[ Nle]f{FreeAcid} | Ac-[Atz(PEG10)65;hArg68;Cha69; Nle75;D-Phe76]Apelin-13(65-76) | 1330 |
| {Hydrogen}[PE][Atz(PEG10)]P[hArg][Cha]S HKGP[Nle]f{FreeAcid} | [PE65;Atz(PEG10)66;hArg68;C ha69;Nle75;D-Phe76]Apelin-13(65-76) | 1331 |
| {Hydrogen}[PE]R[Atz(PEG10)][hArg][Cha]S HKGP[Nle]f{FreeAcid} | [PE65;Atz(PEG10)67;hArg68;C ha69;Nle75;D-Phe76]Apelin-13(65-76) | 1332 |
| {Hydrogen} [PE]RP[Atz(PEG10)][Cha]SHKGP [Nle ]f{FreeAcid} | [PE65;Atz(PEG10)68;Cha69;Nle 75;D-Phe76]Apelin-13(65-76) | 1333 |
| {Hydrogen}[PE]RP[hArg][Atz(PEG10)]SHKG P[N1e]f{FreeAcid} | [PE65;hArg68;Atz(PEG10)69;Nl e75;D-Phe76] Apelin-13(65-76) | 1334 |
| {Hydrogen}[PE]RP[hArg][Cha][Atz(PEG10)] HKGP[Nle]f{FreeAcid} | [PE65;hArg68;Cha69;Atz(PEG1 0)70;Nle75;D-Phe76]Apelin-13(65-76) | 1335 |
| {Hydrogen}[PE]RP[hArg][Cha]S[Atz(PEG10)] KGP[Nle]f{FreeAcid} | [PE65;hArg68;Cha69;Atz(PEG1 0)71;Nle75;D-Phe76]Apelin-13(65-76) | 1336 |
| {Hydrogen} [PE]RP[hArg] [Cha] SH[Atz(PEG1 0)]GP[Nle]f{FreeAcid} | [PE65;hArg68;Cha69;Atz(PEG1 0)72;Nle75;D-Phe76]Apelin-13(65-76) | 1337 |
| {Hydrogen} [PE]RP[hArg][Cha]SHK[Atz(PEG 10)]P[Nle]f{FreeAcid} | [PE65;hArg68;Cha69;Atz(PEG1 0)73;Nle75;D-Phe76]Apelin-13(65-76) | 1338 |
| {Hydrogen} [PE]RP[hArg][Cha]SHKG[Atz(PE G10)][Nle]f{FreeAcid} | [PE65;hArg68;Cha69;Atz(PEG1 0)74;Nle75;D-Phe76]Apelin-13(65-76) | 1339 |
| {Hydrogen} [PE]RP[hArg] [Cha] SHKGP[Atz(P EG10)]f{FreeAcid} | [PE65;hArg68;Cha69;Atz(PEG1 0)75;D-Phe76]Apelin-13(65-76) | 1340 |
| {Hydrogen}[PE]RP[hArg][Cha]SHKGP[Nle][ Atz(PEG10)]{Amide} | [PE65;hArg68;Cha69;Nle75;Atz (PEG10)76]Apelin-13(65-76)-amide | 1341 |
| {Hydrogen} [PE]RP[hArg][Cha]SHKGP[Nle]f[ Atz(PEG10)]{Amide} | [PE65;hArg68;Cha69;Nle75;D-Phe76;Atz(PEG10)77]Apelin-13(65-77)-amide | 1342 |
| {Hydrogen} [PE]RP[hArg][Cha]SHKGP[Nle]f G[Atz(PEG10)] {Amide} | [PE65;hArg68;Cha69;Nle75;D-Phe76;Gly77]Apelin-13(65-77)-Atz(PEG10)-amide | 1343 |
| {Hydrogen} [PE]RP[hArg][Cha]SHKGP[Nle]f GG[Atz(PEG10)] {Amide} { Acetyl}[Atz(PEG10)]LRP[hArg][NMeLeu] [A tz(PEG10)]HKGP[Nle][4-Cl-F]{FreeAcid} | [PE65;hArg68;Cha69;Nle75;D-Phe76;Gly77]Apelin-13(65-77)-Gly-Atz(PEG10)-amide Ac-Atz(PEG10)-[Leu65;hArg68;NMeLeu69;Atz( PEG 10)70;Nle75;4-Cl-F76]Apelin-13(65-76) | 1344 1345 |
| {Hydrogen}[PE]RP[hArg][Cha][Atz(PEG10)] HKGP[Nle][4-Cl-F]{FreeAcid} | [PE65;hArg68;Cha69;Atz(PEG1 0)70;Nle75;4-Cl-F76]Apelin-13(65-76) | 1346 |
| {Hydrogen}[PE]RP[hArg][Cha][Atz(PEG10)] HKG[Oic][Nle][4-Cl-F]{FreeAcid} | [PE65;hArg68;Cha69;Atz(PEG1 0)70;Oic74;Nle75;4-Cl-F76]Apelin-13(65-76) | 1347 |
| {Hydrogen}[PE]RP[hArg][Cha][Atz(PEG10)] HKG[Oic][Nle][4-Cl-F]{Amide} | [PE65;hArg68;Cha69;Atz(PEG1 0)70;Oic74;Nle75;4-Cl-F76]Apelin-13 (65-76)-amide | 1348 |
| {Hydrogen}[PE]RP[hArg][Cha]F[Atz(PEG10)] KG[Oic][Nle][4-Cl-F]{FreeAcid} | [PE65;hArg68;Cha69;Phe70;Atz (PEG10)71;Oic74;Nle75;4-Cl-F76]Apelin-13(65-76) | 1349 |
| {Hydrogen}[PE]RP[hArg][Cha]F[Atz(PEG10)] KG[Oic][Nle][4-Cl-F]{Amide} | [PE65;hArg68;Cha69;Phe70;Atz (PEG10)71;Oic74;Nle75;4-Cl-F76]Apelin-13(65-76)-amide | 1350 |
| {Hydrogen} [PE]RP[hArg][NMeLeu]F[Atz(PE G10)]KG[Oic][Nle][4-Cl-F] {FreeAcid} | [PE65;hArg68;NMeLeu69;Phe7 0;Atz(PEG10)71;Oic74;Nle75;4-Cl-F76]Apelin-13(65-76) | 1351 |
| {Hydrogen} [PE]RP[hArg][NMeLeu]F[Atz(PE G10)]KG[Oic][Nle][4-Cl-F] {Amide} | [PE65;hArg68;NMeLeu69;Phe7 0;Atz(PEG10)71;Oic74;Nle75;4-Cl-F76]Apelin-13(65-76)-amide | 1352 |
| {Hydrogen} [PE]RP[hArg][NMeLeu][Atz(PEG 10)]HKGP[Nle][4-Cl-F]{FreeAcid} | [PE65;hArg68;NMeLeu69;Atz(P EG10)70;Nle75;4-Cl-F76]Apelin-13(65-76) | 1353 |
| {Hydrogen} [PE]RP[hArg][NMeLeu][Atz(PEG 10)]HKG[Oic][Nle][4-Cl-F] {FreeAcid} | [PE65;hArg68;NMeLeu69;Atz(P EG10)70;Oic74;Nle75;4-Cl-F76]Apelin-13(65-76) | 1354 |
| {Hydrogen}[PE]RP[hArg][NMeLeu][Atz(PEG 10)]HKG[Oic][Nle][4-Cl-F]{Amide} | [PE65;hArg68;NMeLeu69;Atz(P EG10)70;Oic74;Nle75;4-Cl-F76]Apelin-13 (65-76)-amide | 1355 |
| {Acetyl}[Atz(PEG10)]LRP[hArg][NMeLeu][A tz(PEG10)]HKGP[Nle]f{FreeAcid} | Ac-Atz(PEG10)-[Leu65;hArg68;NMeLeu69;Atz( PEG10)70;Nle75;D-Phe76]Apelin-13(65-76) | 1356 |
| {Hydrogen}[PE]RP[hArg][Cha][Atz(PEG10)] F[KGP[Nle]f{FreeAcid} | [PE65;hArg68;Cha69;Atz(PEG1 0)70;Nle75;D-Phe76]Apelin-13(65-76) | 1357 |
| {Hydrogen}[PE]RP[hArg][Cha][Atz(PEG10)] HKG[Oic][Nle]f{FreeAcid} | [PE65;hArg68;Cha69;Atz(PEG1 0)70;Oic74;Nle75;D-Phe76]Apelin-13(65-76) | 1358 |
| {Hydrogen}[PE]RP[hArg][Cha]F[Atz(PEG10)] KGP[Nle]f{FreeAcid} | [PE65;hArg68;Cha69;Phe70;Atz (PEG 10)71;Nle75;D-Phe76]Apelin-13(65-76) | 1359 |
| {Hydrogen}[PE]RP[hArg][Cha]F[Atz(PEG10)] KG[Oic][Nle]f{FreeAcid} | [PE65;hArg68;Cha69;Phe70;Atz (PEG10)71;Oic74;Nle75;D-Phe76]Apelin-13(65-76) | 1360 |
| {Hydrogen}[PE]RP[hArg][Cha]F[Atz(PEG10)] KGP[Nle]f{Amide} | [PE65;hArg68;Cha69;Phe70;Atz (PEG10)71;Nle75;D-Phe76]Apelin-13 (65-76)-amide | 1361 |
| {Hydrogen}[PE]RP[hArg][Cha]F[Atz(PEG10)] KG[Oic][Nle]f{Amide} | [PE65;hArg68;Cha69;Phe70;Atz (PEG10)71;Oic74;Nle75;D-Phe76]Apelin-13(65-76)-amide | 1362 |
| {Hydrogen} [PE]RP[hArg][NMeLeu]F[Atz(PE G10)]KGP[Nle]f{FreeAcid} | [PE65;hArg68;NMeLeu69;Phe7 0;Atz(PEG10)71;Nle75;D-Phe76]Apelin-13(65-76) | 1363 |
| {Hydrogen} [PE]RP[hArg][NMeLeu]F[Atz(PE G10)]KG[Oic][Nle]f{FreeAcid} | [PE65;hArg68;NMeLeu69;Phe7 0;Atz(PEG10)71;Oic74;Nle75;D -Phe76]Apelin-13(65-76) | 1364 |
| {Hydrogen} [PE]RP[hArg][NMeLeu]F[Atz(PE G10)]KGP[Nle] f{Amide} | [PE65;hArg68;NMeLeu69;Phe7 0;Atz(PEG10)71;Nle75;D-Phe76]Apelin-13(65-76)-amide | 1365 |
| {Hydrogen} [PE]RP[hArg][NMeLeu]F[Atz(PE G 10)]KG[Oic][Nle]f{Amide} | [PE65;hArg68;NMeLeu69;Phe7 0;Atz(PEG10)71;Oic74;Nle75;D -Phe76]Apelin-13(65-76)-amide | 1366 |
| {Hydrogen} [PE]RP[hArg][NMeLeu][Atz(PEG 10)]HKGP[Nle]f{FreeAcid} | [PE65;hArg68;NMeLeu69;Atz(P EG10)70;Nle75;D-Phe76]Apelin-13(65-76) | 1367 |
| {Hydrogen} [PE]RP[hArg][NMeLeu][Atz(PEG 10)]HKG[Oic][Nle]f{FreeAcid} | [PE65;hArg68;NMeLeu69;Atz(P EG 10)70;Oic74;Nle75;D-Phe76]Apelin-13(65-76) | 1368 |
| {Hydrogen} [PE]RP[hArg][NMeLeu][Atz(20K-mPEG)]HKG[Oic][Nle][4-Cl-F]{FreeAcid} | [PE65;hArg68;NMeLeu69;[Atz( 20K-mPEG)]70;Oic74;Nle75;4-Cl-F76]Apelin-13(65-76) | 1369 |
| {Hydrogen} [PE]RP[hArg] [Cha] [Atz(20K-mPEG)]HKG[Oic][Nle][4-Cl-F]{FreeAcid} | [PE65;hArg68;Cha69;[Atz(20K-mPEG)]70;Oic74;Nle75;4-Cl-F76]Apelin-13(65-76) | 1370 |
| {Hydrogen} [PE]RP[hArg][NMeLeu]F[Atz(20 K-mPEG)]KG[Oic][Nle]f{FreeAcid} | [PE65;hArg68;NMeLeu69;Phe7 0;[Atz(20K-mPEG)]71;Oic74;Nle75;D-Phe76]Apelin-13(65-76) | 1371 |
| {Acetyl}[Atz(20K-mPEG)]LRP[hArg][Cha]SHKGP[Nle][D-1Nal]{FreeAcid} | [Ac-Atz(20K-mPEG)64;hArg68;Cha69;Nle75; D-1-Nal76]Apelin-13(64-76) | 1372 |
| {Acetyl}[Atz(20K-mPEG)] [Ahx]LRP[hArg][Cha]SHKGP[Nle][D -1Nal] {FreeAcid} | [Ac-Atz(20K-mPEG)63;Ahx64;hArg68;Cha69 ;Nle75;D-1-Nal76]Apelin-13(63-76) | 1373 |
| {Palmitoyl}LRP[hArg][Cha]FHKGP[Nle][4-Cl-F] {FreeAcid} | Palmitoyl-[Leu65;hArg68;Cha69;Phe70;Nl e75;4-Cl-F76]Apelin-13(65-76) | 1374 |
| {Palmitoyl}LRP[hArg][Cha]FHKPP[Nle][D-1Nal]{FreeAcid} | Palmitoyl-[Leu65;hArg68;Cha69;Phe70;Pr o73;Nle75;D-1Nal76]Apelin-13(65-76) | 1375 |
| {Palmitoyl}LRP[hArg][Cha]SHKGP[Nle][4-Cl-F] | Palmitoyl-Leu65;hArg68;Cha69;Nle75;4-Cl-F76]Apelin-13(65-76) | 1376 |
| {Palmitoyl}LRP[hArg][NMeLeu]FA[1-Nal]G[1-Nal][Nle][4-C1-F]{Amide} | Palmitoyl-[Leu65;hArg68;NMeLeu69;Phe7 0;Ala71;1-Nal72,74;Nle75;4-Cl-F76]Apelin-13(65-76) | 1377 |
| {Palmitoyl}LRP[hArg][NMeLeu]FA[1-Nal]G[Oic][Nle][4-Cl-F]{Amide} | Palmitoyl-[Leu65;hArg68;NMeLeu69;Phe7 0;Ala71;1-Nal72,Oic74;Nle75;4-Cl-F76]Apelin-13(65-76) | 1378 |
| {Palmitoyl}LRP[hArg][NMeLeu]FA[1-Nal]G[1-Nal][Nle][4-Cl-F]{Amide} | Palmitoyl-[Leu65;hArg68;NMeLeu69;Phe7 0;Ala71;1-Nal72,74;Nle75;4-Cl-F76]Apelin-13(65-76) | 1379 |

**TABLE 12.**

| AMINO ACID SEQUENCE | DESIGNATION | SEQ ID NO. |
|---|---|---|
| KFRRQRP[hArg][Cha]SHKG[Oic]EP[4-Cl-F]K{COOH} | [hArg68;Cha69;Oic 74;EI75;4-Cl-F77]Apelin-17(61-77)-K1 | 1380 |
| KFRRQRP[hArg][Cha]SHKGE[Nle]PK{COOH} | [hArg68;Cha69;E17 4;Nle75;K177]Apel in-17(61-77) | 1381 |
| 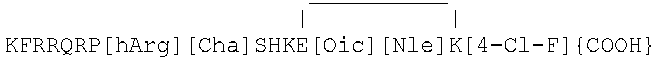 KFRRQRP[hArg][Cha]SHKE[Oic][Nle]K[4-Cl-F]{COOH} | [hArg68;Cha69;E17 3;Oic74;Nle75;K17 6;4-Cl-F77]Apelin-17(61-77) | 1382 |
| 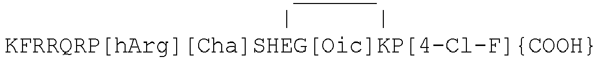 KFRRQRP[hArg][Cha]SHEG[Oic]KP[4-Cl-F]{COOH} | [hArg68;Cha69;E17 2;Oic74;K175;4-Cl-F77]Apelin-17(61-77) | 1383 |
|                     ___ ;   ; KFRRQRP[hArg][Cha]SEKGK[Nle]P[4-Cl-F]{COOH} | [hArg68;Cha69;E17 1;K174;Nle75;4-Cl-F77]Apelin-17(61-77) | 1384 |
|                    __ ;  ; KFRRQRP[hArg][Cha]EHKK[Oic][Nle]P[4-Cl-F]{COOH} | [hArg68;Cha69;E17 0;K173;Oic74;Nle7 5;4-Cl-F77]Apelin-17(61-77) | 1385 |
|               __ ;  ; KFRRQRP[hArg]ESHKG[Oic][Nle]P[4-Cl-F]{COOH} | [hArg68;E169;K17 2;Oic74;Nle75;4-Cl-F77]Apelin-17(61-77) | 1386 |
| 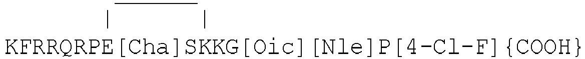 KFRRQRPE[Cha]SKKG[Oic][Nle]P[4-Cl-F]{COOH} | [E168;Cha69;K171; Oic74;Nle75;4-Cl-F77]Apelin-17(61-77) | 1387 |
| 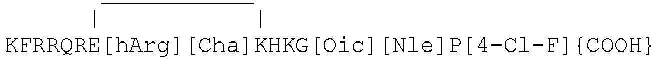 KFRRQRE[hArg][Cha]KHKG[Oic][Nle]P[4-Cl-F]{COOH} | [E167;hArg68;Cha6 9;K170;Oic74;Nle7 5;4-Cl-F77]Apelin-17(61-77) | 1388 |
|       ________ ;        ; KFRRQEP[hArg]KSHKG[Oic][Nle]P[4-Cl-F]{COOH} | [E166;hArg68;K16 9;Oic74;Nle75;4-Cl-F77]Apelin-17(61-77) | 1389 |
| 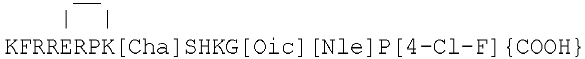 KFRRERPK[Cha]SHKG[Oic][Nle]P[4-Cl-F]{COOH} | [E165;K168;Cha69; Oic74;Nle75;4-Cl-F77]Apelin-17(61-77) | 1390 |
|      ___ ;   ; KFREQRK[hArg][Cha]SHKG[Oic][Nle]P[4-Cl-F]{COOH} | [E164;K167;hArg6 8;Cha69;Oic74;Nle 75;4-Cl-F77]Apelin-17(61-77) | 1391 |
| 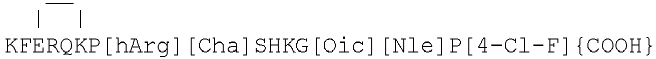 KFERQKP[hArg][Cha]SHKG[Oic][Nle]P[4-Cl-F]{COOH} | [E163;K166;hArg6 8;Cha69;Oic74;Nle 75;4-Cl-F77]Apelin-17(61-77) | 1392 |
|   __ ;  ; KERRKRP[hArg][Cha]SHKG[Oic][Nle]P[4-Cl-F]{COOH} | [E162;K165;hArg6 8;Cha69;Oic74;Nle 75;4-Cl-F77]Apelin-17(61-77) | 1393 |
|  __ ;   ; EFRKQRP[hArg][Cha]SHKG[Oic][Nle]P[4-Cl-F]{COOH} | [E161;K164;hArg6 8;Cha69;Oic74;Nle 75;4-Cl-F77]Apelin-17(61-77) | 1394 |
| 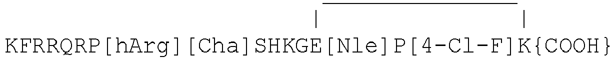 KFRRQRP[hArg][Cha]SHKGE[Nle]P[4-Cl-F]K{COOH} | [hArg68;Cha69;E17 4;Nle75;4-Cl-F77]Apelin-17(61-77)-K1 | 1395 |
| KFRRQRP[hArg][Cha]SHKE[Oic][Nle]PK{COOH} | [hArg68;Cha69;E17 3;Oic74;Nle75;K17 7]Apelin-17(61-77) | 1396 |
| 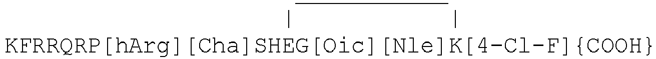 KFRRQRP[hArg][Cha]SHEG[Oic][Nle]K[4-Cl-F]{COOH} | [hArg68;Cha69;E17 2;Oic74;Nle75;K17 6;4-Cl-F77]Apelin-17(61-77) | 1397 |
| 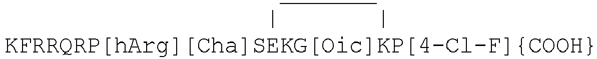 KFRRQRP[hArg][Cha]SEKG[Oic]KP[4-Cl-F]{COOH} | [hArg68;Cha69;E17 1;Oic74;K175;4-Cl-F77]Apelin-17(61-77) | 1398 |
|  ____ ;    ; KFRRQRP[hArg][Cha]EHKGK[Nle]P[4-Cl-F]{COOH} | [hArg68;Cha69;E17 0;K174;Nle75;4-Cl-F77]Apelin-17(61-77) | 1399 |
|               ___ ;   ; KFRRQRP[hArg]ESHKK[Oic][Nle]P[4-Cl-F]{COOH} | [hArg68;E169;K17 3;Oic74;Nle75;4-Cl-F77]Apelin-17(61-77) | 1400 |
| 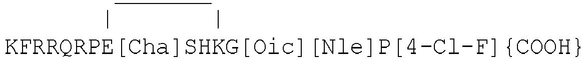 KFRRQRPE[Cha]SHKG[Oic][Nle]P[4-Cl-F]{COOH} | [E168;Cha69;K172; Oic74;Nle75;4-Cl-F77]Apelin-17(61-77) | 1401 |
| 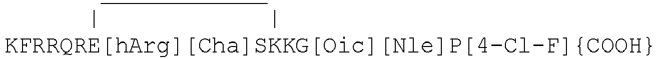 KFRRQRE[hArg][Cha]SKKG[Oic][Nle]P[4-Cl-F]{COOH} | [E167;hArg68;Cha6 9;K171;Oic74;Nle7 5;4-Cl-F77]Apelin-17(61-77) | 1402 |
| 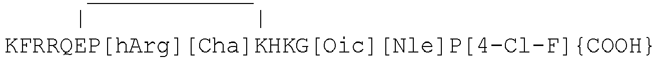 ;                    ; KFRRQEP[hArg][Cha]KHKG[Oic][Nle]P[4-Cl-F]{COOH} | [E166;hArg68;Cha6 9;K170;Oic74;Nle7 5;4-Cl-F77]Apelin-17(61-77) | 1403 |
| 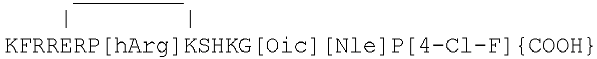 KFRRERP[hArg]KSHKG[Oic][Nle]P[4-Cl-F]{COOH} | [E165;hArg68;K16 9;Oic74;Nle75;4-Cl-F77]Apelin-17(61-77) | 1404 |
|     ___ ;   ; KFREQRPK[Cha]SHKG[Oic][Nle]P[4-Cl-F]{COOH} | [E164;K168;Cha69; Oic74;Nle75;4-Cl-F77]Apelin-17(61-77) | 1405 |
|     ____ ;    ; KFERQRK[hArg][Cha]SHKG[Oic][Nle]P[4-Cl-F]{COOH} | [E163;K167;hArg6 8;Cha69;Oic74;Nle 75;4-Cl-F77]Apelin-17(61-77) | 1406 |
| 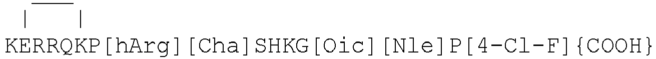 KERRQKP[hArg][Cha]SHKG[Oic][Nle]P[4-Cl-F]{COOH} | [E162;K166;hArg6 8;Cha69;Oic74;Nle 75;4-Cl-F77]Apelin-17(61-77) | 1407 |
|  ____ ;    ; EFRRKRP[hArg][Cha]SHKG[Oic][Nle]P[4-Cl-F]{COOH} | [E161;K165;hArg6 8;Cha69;Oic74;Nle 75;4-Cl-F77]Apelin-17(61-77) | 1408 |
| 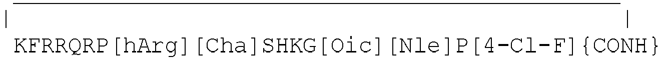 KFRRQRP[hArg][Cha]SHKG[Oic][Nle]P[4-Cl-F]{CONH} | N-to-C cyclic[hArg68;Cha6 9;Oic74;Nle75;4-Cl-F77]Apelin-17(61-77) | 1409 |
| KFRRQRP[hArg][Cha]SHKG[Oic][Nle]P[4-Cl-F]E{COOH} | N-to-Ecyclic[hArg68;Ch a69;Oic74;Nle75;4-Cl-F77]Apelin-17(61-77)-Glu | 1410 |
| KFRRQRP[hArg][Cha]SHKG[Oic][Nle]P[4-Cl-F]D{COOH} | N-to-Dcyclic[hArg68;Ch a69;Oic74;Nle75;4-Cl-F77]Apelin-17(61-77)-Asp | 1411 |

Various embodiments presented above provide a specific formula for a peptide wherein "z" can be defined as PEG or PE. Disclosed herein are peptides comprising the same formulaes wherein "z" can be any half-life prolonging group.

The previous description and the following working examples are illustrative and not to be construed in any way as limiting the invention.

### EXAMPLES

### EXAMPLE 1

Various polypeptides have been prepared that act as APJ agonists. The polypeptides were screened for potency and metabolic stability. The sequences were randomized through techniques known in the art in which one or more amino acids may be changed while maintaining or improving the affinity, functional activity, or stability of the peptide. Nishizawa et al., Peptide Science 37: 151-157 2001; Murza et al., ChemMeDChem., 7: 318-325 2012

Approximately >800 modified polypeptides were used in structure-activity relationship assays to optimize for potency and metabolic stability relative to endogenous ligand pyr apelin-13. All the compounds were screened up to 10uM along with pyr apelin-13 used as positive control. Compounds that showed functional activity in all the SAR assays were deemed positive and re-screened. The iterative process was continued and polypeptides were further modified for optimization.

Several APJ agonist sequences are provided in Tables 12, and 13 below and in Figures 3 and 4. In Table 13, the brackets indicate the residue is a non-conical amino acid. Also, in Table 12, the symbols "⇔" and "⇑" may be any PEG derivative covalently attached to the APJ agonist via any of the linkers described herein or may simply be attached by a normal peptide bond (i.e., so that no linker is present). PE is pyroglutamate.

**TABLE 13. APJ AGONISTS**

| MOLECULE SEQUENCE / PEPTIDE NAME | SEQ ID NO. |
|---|---|
| [PE]RP[hArg][Cha][⇑]HKG[Oic][Nle]P[4-Cl-F] [PE65;hArg68;Cha69;PEG70;Oic74;Nle75;4-Cl-F77]Apelin-13(65-77) | 3 |
| [PE]RP[hArg][NMeLeu][⇑]HKG[Oic][Nle]P[4-Cl-F] [PE65;hArg68;NMeLeu69;PEG70;Oic74;Nle75;4-Cl-F77]Apelin-13(65-77) | 4 |
| [PE]RP[hArg][Cha]S[⇑]KG[Oic][Nle]P[4-Cl-F] [PE65;hArg68;Cha69;PEG71;Oic74;Nle75;4-Cl-F77]Apelin-13(65-77) | 5 |
| [PE]RP[hArg][NMeLeu]S[⇑]KG[Oic][Nle]P[4-Cl-F] [PE65;hArg68;NMeLeu69;PEG71;Oic74;Nle75;4-Cl-F77]Apelin-13(65-77) | 6 |
| ⇔LRP[hArg][Cha][⇑]HKG[Oic][Nle]P[4-Cl-F] PEG-[Leu65;hArg68;Cha69;PEG70;Oic74;Nle75;4-Cl-F77]Apelin-13(65-77) | 7 |
| ⇔LRP[hArg][NMeLeu][⇑]HKG[Oic][Nle]P[4-Cl-F] PEG-[Leu65;hArg68;NMeLeu69;PEG70;Oic74;Nle75;4-Cl-F77]Apelin-13(65-77) | 8 |
| ⇔ LRP[hArg][Cha]S[⇑]KG[Oic][Nle]P[4-Cl-F] PEG-[Leu65;hArg68;Cha69;PEG71;Oic74;Nle75;4-Cl-F77]Apelin-13(65-77) | 9 |
| ⇔ LRP[hArg][NMeLeu]S[⇑]KG[Oic][Nle]P[4-Cl-F] PEG-[Leu65;hArg68;NMeLeu69;PEG71;Oic74;Nle75;4-Cl-F77]Apelin-13(65-77) | 10 |
| ⇔KFRRQRP[hArg][Cha]SHKG[Oic][Nle]P[4-Cl-F] PEG-[hArg68;Cha69;Oic74;Nle75;4-Cl-Phe77]Apelin-17(61-77) | 11 |
| ⇔KFRRQRP[hArg][NMeLeu]SHKG[Oic][Nle]P[4-Cl-F] PEG-[hArg68;NMeLeu69;Oic74;Nle75;4-Cl-Phe77]Apelin-17(61-77) | 12 |
| ⇔FRRQRP[hArg][Cha]SHKG[Oic][Nle]P[4-Cl-F] PEG-[hArg68;Cha69;Oic74;Nle75;4-Cl-Phe77]Apelin-17(62-77) | 13 |
| ⇔FRRQRP[hArg][NMeLeu]SHKG[Oic][Nle]P[4-Cl-F] PEG-[hArg68;NMeLeu69;Oic74;Nle75;4-Cl-Phe77]Apelin-17(62-77) | 14 |
| ⇔RRQRP[hArg][Cha]SHKG[Oic][Nle]P[4-Cl-F] PEG-[hArg68;Cha69;Oic74;Nle75;4-Cl-Phe77]Apelin-17(63-77) | 15 |
| ⇔RRQRP[hArg][NMeLeu]SHKG[Oic][Nle]P[4-Cl-F] PEG-[hArg68;NMeLeu69;Oic74;Nle75;4-Cl-Phe77]Apelin-17(63-77) | 16 |
| ⇔RQRP[hArg][Cha]SHKG[Oic][Nle]P[4-Cl-F] PEG-[hArg68;Cha69;Oic74;Nle75;4-Cl-Phe77]Apelin-17(64-77) | 17 |
| ⇔RQRP[hArg][NMeLeu]SHKG[Oic][Nle]P[4-Cl-F] PEG-[hArg68;NMeLeu69;Oic74;Nle75;4-Cl-Phe77]Apelin-17(64-77) | 18 |
| ⇔QRP[hArg][Cha]SHKG[Oic][Nle]P[4-Cl-F] PEG-[hArg68;Cha69;Oic74;Nle75;4-Cl-Phe77]Apelin-17(65-77) | 20 |
| ⇔QRP[hArg][NMeLeu]SHKG[Oic][Nle]P[4-Cl-F] PEG-[hArg68;NMeLeu69;Oic74;Nle75;4-Cl-Phe77] Apelin-17(65-77) | 19 |
| ⇔LRP[hArg][Cha]SHKG[Oic][Nle]P[4-Cl-F] PEG-[Leu65;hArg68;Cha69;Oic74;Nle75;4-Cl-Phe77]Apelin-17(65-77) | 21 |
| ⇔LRP[hArg][NMeLeu]SHKG[Oic][Nle]P[4-Cl-F] PEG-[Leu65;hArg68;NMeLeu69;Oic74;Nle75;4-Cl-Phe77]Apelin-17(65-77) | 22 |

Additional examples of APJ agonists are provided below.

**TABLE 14. ADDITIONAL APJ AGONIST EXAMPLES: (Sequence, Chemical names and Structures are listed.)**

| |
|---|
| SEQ ID NO: 23 |
| Sequence: [PE]RP[hArg][Cha][Atz(PEG10)]HKG[Oic][Nle]P[4-Cl-F] |
| Chemical Name: [PE65;hArg68;Cha69;3-(1-(2-aminoethoxy(PEG9)ethyl)-1H-1,2,3-triazol-4-yl)Ala70; Oic74;Nle75 ;4-Cl-F77]Apelin-13(65-77) |
| 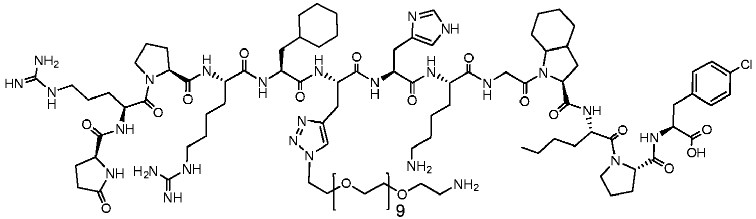 |
| SEQ ID NO: 24 |
| Sequence: Thiopropionyl(20K-mPEG)-KFRRQRP[hArg][Cha]SHKG[Oic][Nle]P[4-Cl-F] |
| Chemical Name: 3-((2-(3-(mPEG(20kDa))propylamino)-2-oxoethyl)thio)propanamide-[hArg68;Cha69;Oic74;Nle75;4-Cl-Phe77]Apelin-17(61-77) |
| 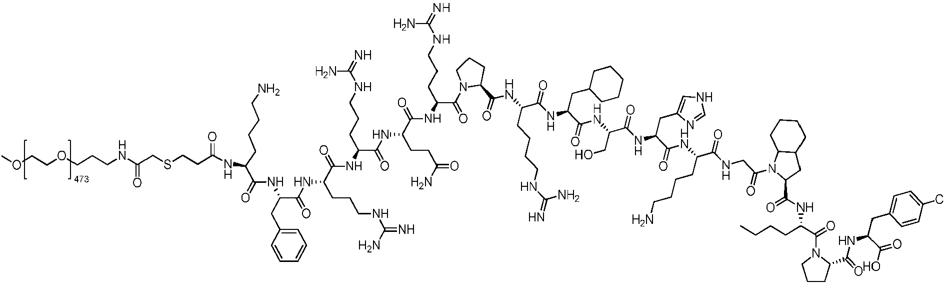 |
| SEQ ID NO: 25 |
| Sequence: Atz(20K-mPEG)-KFRRQRP[hArg][Cha]SHKG[Oic][Nle]P[4-Cl-F] |
| Chemical Name: 3-(1-(2-(mPEG(20kDa))ethyl)-1H-1,2,3-triazol-4-yl)alanine-[hArg68;Cha69;Oic74;Nle75;4-Cl-F77]Apelin-17(61-77) |
| 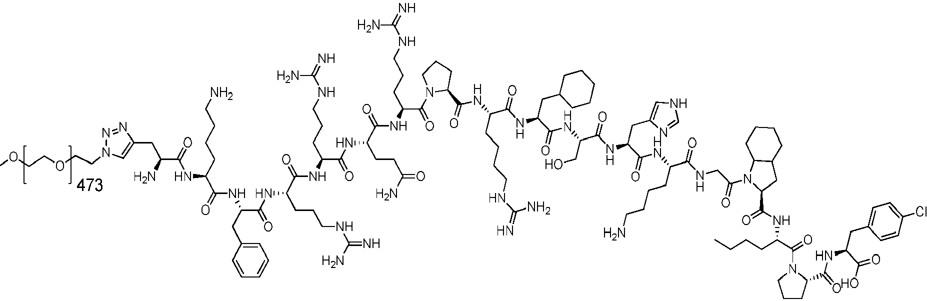 |
| SEQ ID NO: 26 |
| Sequence: Thiopropionyl(20K-mPEG)-LRP[hArg][Cha]SHKG[Oic][Nle]P[4-Cl-F] |
| Chemical Name: 3-((2-(3-(mPEG(20kDa))propylamino)-2-oxoethyl)thio)propanamide-[Leu65;hArg68;Cha69;Oic74;Nle75;4-Cl-F77]Apelin-13(65-77) |
| 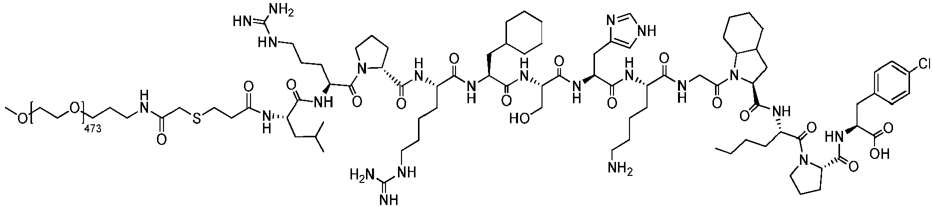 |
| SEQ ID NO: 27 |
| Sequence: Atz(20K-mPEG)-LRP[hArg][Cha]SHKG[Oic][Nle]P[4-Cl-F] |
| Chemical Name: 3-(1-(2-(mPEG(20kDa))ethyl)-1H-1,2,3-triazol-4-yl)alanine-[hArg68;Cha69;Oic74;Nle75;4-Cl-F77]Apelin-17(61-77) |
| 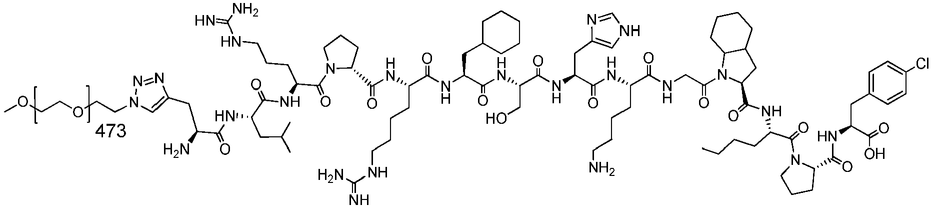 |
| SEQ ID NO: 28 |
| Sequence: NPeg11-KFRRQRP[hArg][Cha]SHKG[Oic][Nle]P[4-Cl-F] |
| Chemical Name: 3-((2-aminoethoxy)PEG1 1)-propanamide-[hArg68;Cha69;Oic74;Nle75;4-Cl-F77]Apelin-17(61-77) |
| 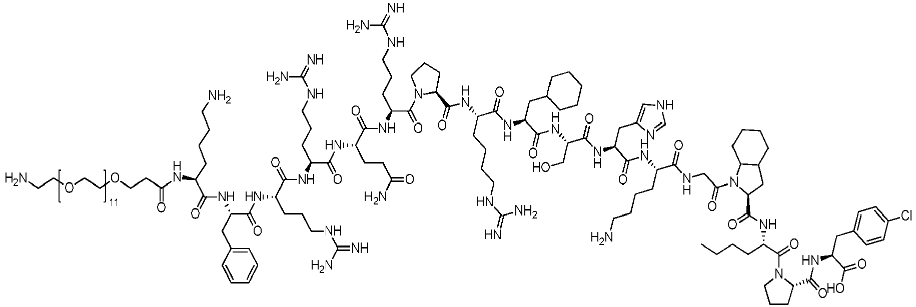 |

In addition to agonists listed in Tables 12 and 13, and Figures 3 and 4 various embodiments can comprise conjugates in which a polymer, peptide, or protein is linked to a different peptide sequence; for example, wherein a Fc is linked to an APJ agonist sequence. Such compositions of matter may be prepared where the APJ agonist is attached to a vehicle through the polypeptide's N-terminus, C-terminus, backbone, or side-chain via chemical modification.

Any of the polypeptides may be attached in tandem (i.e., sequentially), with or without linkers. Any peptide described herein, either as a monomer or as multimers, may be covalently tethered, with or without linkers, to a vehicle. Any of these peptides may be derivatized.

### EXAMPLE 2

Evaluation of *in vitro* plasma stability and identification of major *in vitro* metabolites of apelin peptides across multiple species, including mouse, rat, dog and human were performed.

Apelin peptides were spiked into freshly prepared mouse, rat, dog and human EDTA plasma to a final concentration of 5µM. The plasma samples were then incubated at 37°C over a period of 4hr. Up to 8 time points were sampled to facilitate estimation of *in vitro* half life in plasma matrices. In a typical experiment, aliquot of 100µL of plasma sample was taken from the incubation vial, followed by addition of 300µL of MeOH containing internal standard. The quenched sample was centrifuged at 5500g for 15min after 15min vortex in room temperature. The supernatant was then analyzed by high resolution LC-MS. Accurate mass full scan mass data was used for estimation of parent peptide level over the time course of 4hr, and the data was subsequently used for estimation of *in vitro* half life in plasma. Using the same data set, major metabolites were also identified based on accurate mass full scan MS and MS/MS data. (metabolite data not shown). *In vitro* plasma stability results are shown below in Table 15.

| TABLE 15. | | | in vitro plasma stability % remaining at 1h |
|---|---|---|---|
| | SEQ ID NO | SEQUENCE | RAT |
| Apelin-13 | 2 | QRPRLSHKGPMPF | 0.1 (at 15 min) |
| [PE65] Apelin-13 | 128 | [PE]RPRLSHKGPMPF | 0.7 (at 15 min) |
| | 29 | [PE]RP[hArg][NMeLeu]F[Pra](NPeg9)KGP[Nle]f | 50 |
| | 30 | Acetyl-[Pra](NPeg9)LRP[hArg][NMeLeu][Pra](NPeg9)HKGP[Nle]P[4-Cl-F] | 61 |
| | 31 | [PE] RP[hArg][Cha]SHKGP[Nle][1-Nal] | 63 |
| | 32 | [PE]RP[hArg][Cha]SHKGP[Nle][4-Cl-F] | 64.6 |
| | 33 | [PE]RP[hArg][Cha]F[Pra](NPeg9)KG[Oic][Nle]f | 66 |
| | 34 | [PE]RP[hArg][Cha][Pra](NPeg9)HKG[Oic][Nle]P[4-Cl-F] | 72 |
| | 35 | [PE]RP[hArg][NMeLeu][Pra](NPeg9)HKG[Oic][Nle][4-Cl-F] | 82 |
| | 36 | [PE]RP[hArg][NMeLeu][Pra](NPeg9)HKG[Oic][Nle]P[4-Cl-F] | 88 |
| | 37 | Acetyl-[Pra](NPeg9) LRP[hArg][Cha]SH KG P[N le][D-1Nal] | 96 |

It should be noted in Table 15 and that all of the listed polypeptides have much greater *in vitro* stability than either the native (0.1% at 15 minutes) or the pyroglutamated Apelin 13 (0.7% at 15 minutes). The additionally tested peptides had between 50 and 96% at 1 hour. Additional experiments provided comparative *in vitro* plasma stability in four different species of plasma, i.e. mouse, rat, dog and human (Figure 4). These results demonstrated that the APJ agonists should be able to be used in preclinical models of different species.

### EXAMPLE 3

Assays were performed to determine potency and metabolic stability of the APJ agonists.

Stable CHO cell lines expressing APJ were used for cAMP assays. The cells were incubated with stimulation buffer containing HANK's buffer, forskolin and 0.5mM IBMX in the absence or presence of various concentrations' of peptides at 37°C for 45 min. The cAMP level was determined using a cyclic AMP kit (DiscoverX) according to the manufacturer's instructions.

To assay for GTP, membranes were prepared from stable cell lines and used to determine the efficacy/potency of the modified peptides. The optimal experimental conditions for the concentrations of GDP, MgCl2 and NaCl in the assay buffer were initially determined. The membranes were incubated with modified peptides in the assay buffer. The reaction was initiated by addition of [³⁵S] GTPγS in the absence or presence of peptides and incubated at room temperature for 90 min. Non specific binding was determined in the presence of excess cold GTPγS and was always less than 0.2% of total binding. Bound [³⁵S] GTPγS was separated from free radiolabel by filtration. The filter bound radioactivity was determined by liquid scintillation counting.

Figure 5 shows agonist activity of 20 kDa PEGylated peptides. The (SEQ ID NO: 122), (SEQ ID NO: 123), (SEQ ID NO: 124), (SEQ ID NO: 126) and (SEQ ID NO: 127) are potent and act as a full agonist relative to 2540421 (pyr apelin-13) (SEQ ID NO: 128). Additionally, the stimulation of cAMP can be seen using either human or rat APJ stable cell lines.

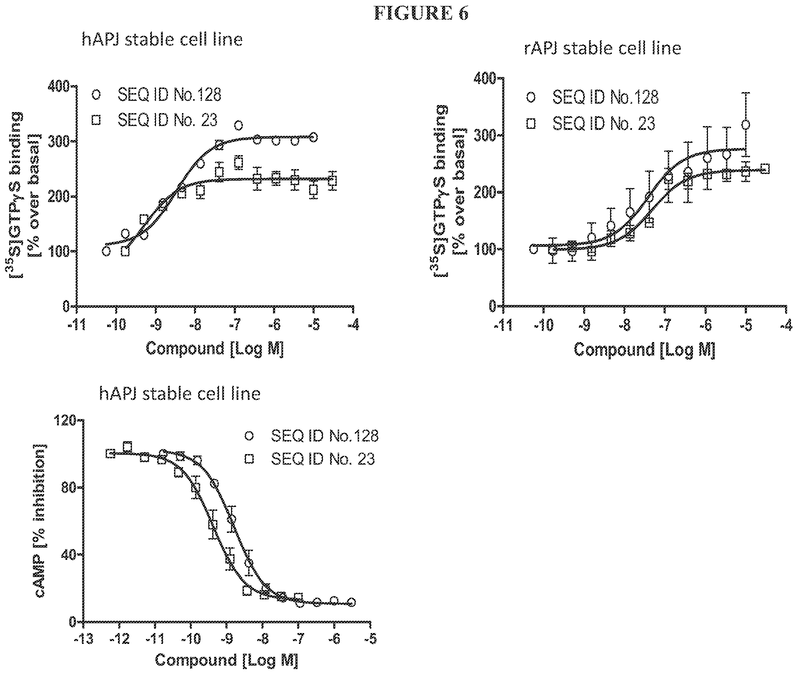

Figure 6 shows a comparison of a 525 dalton PEGylated peptide (SEQ ID NO: 23) and pyr apelin 13 (SEQ ID NO: 128). Relative to the pyr apelin-13 the 525 dalton peptide shows similar *in vitro* potency and efficacy, indicating that although the peptide is highly modified it retains APJ activity similar to the endogenous APJ ligand. Modification of the native apelin peptides by introducing non-canonical amino acids or introduction of PEG had no affect on functional activity in the cAMP and GTPγS assays.

### EXAMPLE 4

To assess the hemodynamic cardiac function of the peptides a Langendorff isolated heart system was used. The isolated heart from rat provides broad spectrum of measurements that can include biochemical, metabolic, morphological and physiological indices. The limitations/advantages of the method include absence of neurohormonal influences, neural regulation and high coronary flows. Such limitations, however, are offset by the ability to study direct cardiac effects without systemic circulation and a host of peripheral interactions.

The reproducibility and accuracy of measurements and relatively low costs over *in vivo* studies for profiling drugs outweigh any limitations of this method. Therefore, this method provides excellent dose-response relationship and sheds critical insight into the pharmacological and physiological properties of the molecules of interest.

The animals were anesthetized upon administration of 50-100mg/kg dose of sodium pentobarbital for rat via the intraperitoneal route. The heart was excised and gently cradled between fingers to avoid injury followed by lifting slightly before incising the aorta, vena cava and pulmonary vessels. Immediately after excision of the heart with aorta, the heart was mounted onto the cannula in the Langendorff apparatus via the aorta. The heart was perfused with modified oxygenated Krebs-Henseleit buffer pH7.5 and equilibrated with 95% O₂/5% CO₂ at 37°C. The perfusion was performed at a constant flow of 10ml/min and paced at 300bpm. The pressure was measured through a pressure sensing balloon catheter inserted in the LV cavity. The intrinsic inotropy (force of muscle contraction) and lusitropy (cardiac relaxation) effects of the heart were assessed by dp/dtₘₐₓ and dp/dtₘᵢₙ.

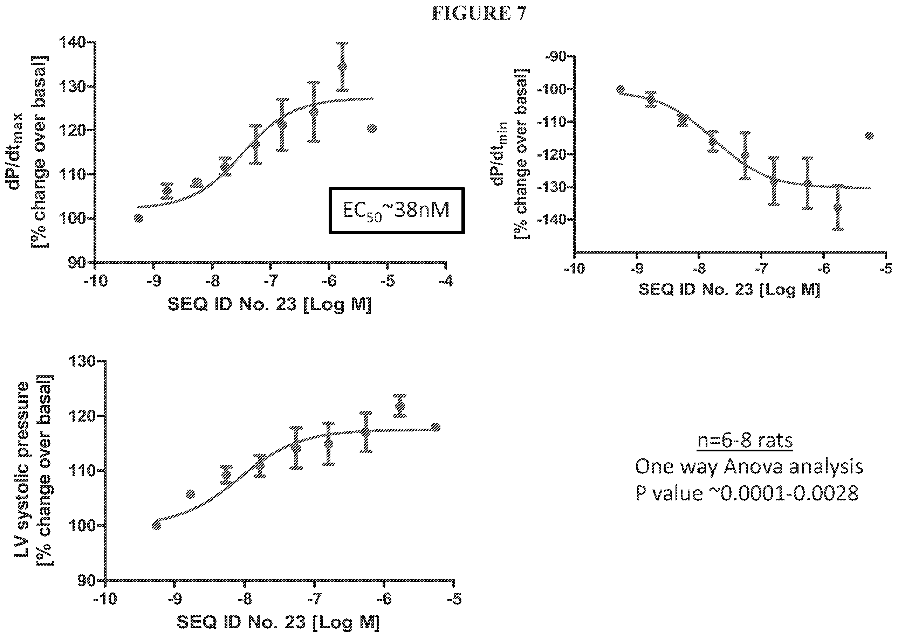

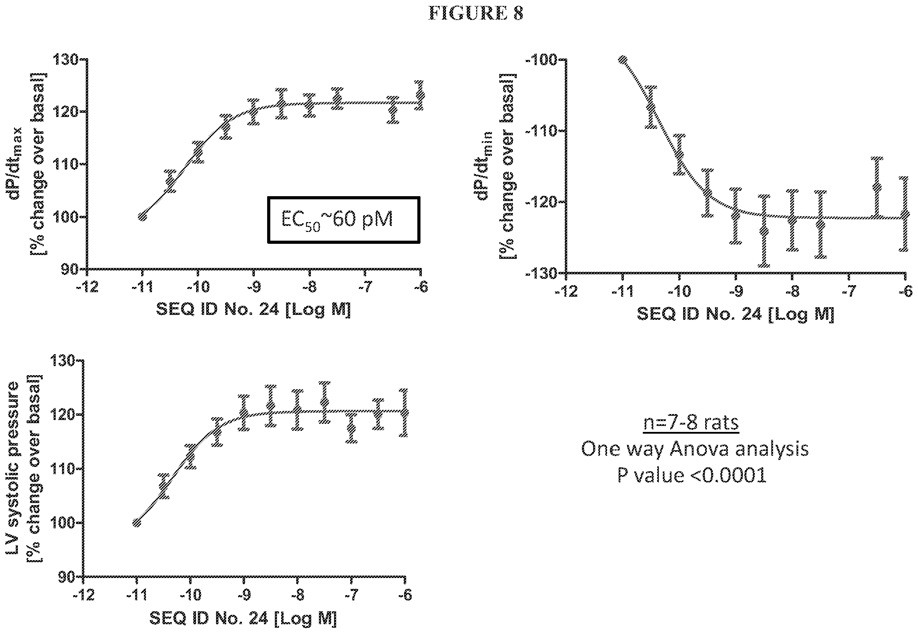

Using the isolated Langendorff system, Figures 7 and 8 show a dose dependent increase in dp/dtₘₐₓ and a decrease in dp/dtₘᵢₙ, along with increases in systolic pressure for two different peptides (SEQ ID NO: 23) and (SEQ ID NO: 24) in control rats. These figures illustrate improvement in both systolic and diastolic function of normal hearts with peptides of SEQ ID NOs: 23 and 24.

### EXAMPLE 5

The *In vivo* cardiac effects of modified peptide SEQ ID NO: 23 was assessed in control and heart failure rats. The cardiac function of healthy rats and the rats having MI (myocardial infarction)-induced heart failure was measured via the Millar PV loop system and femoral arterial pressure catheter were examined.

Male Lewis rats at 2-3 months of age were used for the studies. MI was induced by ligation of the left anterior descending coronary artery (LAD). Echocardiography was performed at 1 week post-MI for animal enrollment. If ejection fraction (EF) was more than 40% and no infarct was identified from echocardiography images, these animals were excluded from the study. The Millar PV loop catheter was inserted into the right common carotid artery and then advanced to the left ventricle (LV) for cardiovascular hemodynamic assessment. The arterial pressure catheter was inserted into a femoral artery for peripheral blood pressure monitoring.

Experiments were performed either on six-seven weeks post-MI rat, or healthy rats. The rats in each experiment were divided into 3 groups: peptide SEQ ID NO: 23 treatment, vehicle, and dobutamine as a positive control. Test articles were given intravenously via jugular vein, and 3 sets of peptide doses were used in the experiment. The peptide having SEQ ID NO: 23 was continuously dosed at 5.5, 27.5 and 55 mg/kg for 10min at each infusion period. At the end of the experiment, the infarct size was verified by gross morphology if MI rats were used in the study.

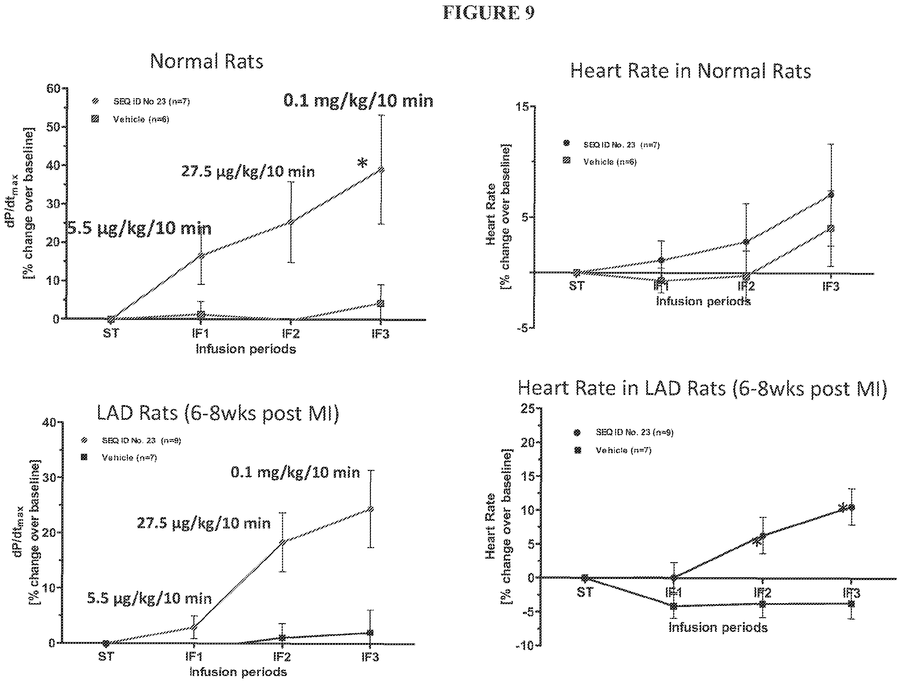

Animals in MI groups had very similar infarct size (38-39% of LV) among the groups (results not shown). Figure 9 shows that the mean dp/dt max increased 32% and 24.5% in healthy and MI rats, respectively. Heart rate (HR) increased up to 10.6% in MI rats at intravenous infusion of 88 µg/kg accumulative dose of peptide 2704121. No other cardiac hemodynamic changes were observed at the dose ranges from 5.5 to 88 µg/kg. No significant changes of peripheral vascular resistant measurement such as mean arterial pressure were seen at the dose ranges from 5.5 to 88 µg/kg.

### EXAMPLE 6

Evaluation of *in vivo* pharmacokinetic profile of apelin peptide in rat was undertaken. Apelin peptides pharmacokinetic profiles were evaluated in Sprague-Dawley rats through intravenous bolus (IV) and subcutaneous (SC) administrations. In a typical IV experiment, an Apelin peptide PBS buffer solution was administered to rats via jugular vein to a final dose of 0.5mg/kg. Blood samples collected over 24hr period was transferred into collection tubes containing potassium EDTA and stored on ice until processed. Plasma was obtained from blood by centrifugation. After transferred into a 96-well container, plasma samples were stored in a freezer maintained at approximately -80°C. Quantitative analysis of Apelin peptide in rat plasma samples were achieved using multiple reaction monitor (MRM) mode on a LC-MS/MS system. Peptides with increased stability relative to pyr-apelin-a3 have enhanced pharmacokinetic profiles, i.e. increased half-life. Peptides conjugated with PEG have a prolonged circulating half-life relative to unconjugated peptides.

### EXAMPLE 7

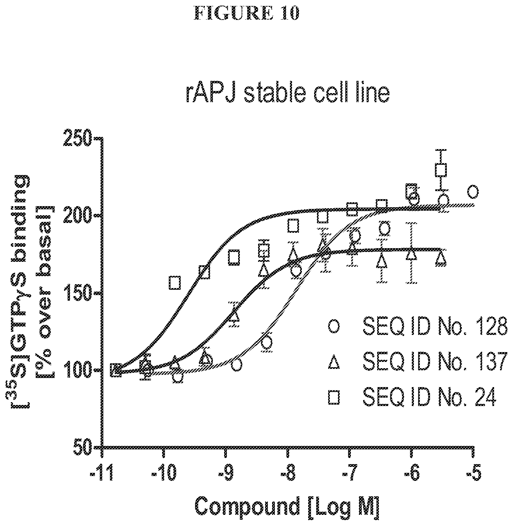

A series of PEGylated peptides were prepared and screened *in vitro* to explore the effects of PEG size and peptide valency on potency and efficacy in the GTPγS assay. In the assay 20kDA and 40 kDA PEG were compared (Figure 10). The potency of the 40 Kda (SEQ ID NO: 474). PEGylated peptide was reduced 5-fold relative 20 kDA (SEQ ID NO: 24) (20 kDa EC₅₀ =0.252 nM vs 40 kDA EC₅₀₌1.37 nM). The efficacy of the 40 Kda PEGylated peptide was also reduced relative 20 kDA (Eₘₐₓ 40 kDa =84% of 20 kDa.

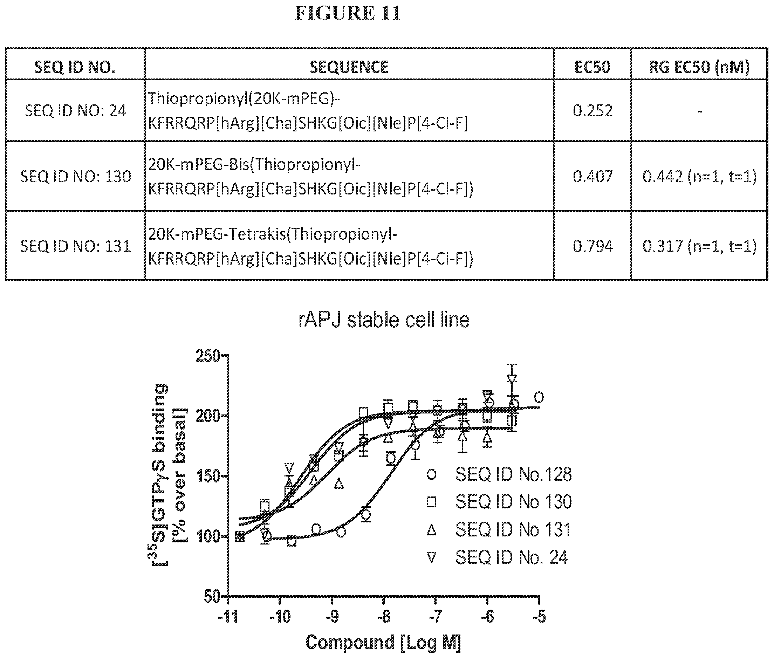

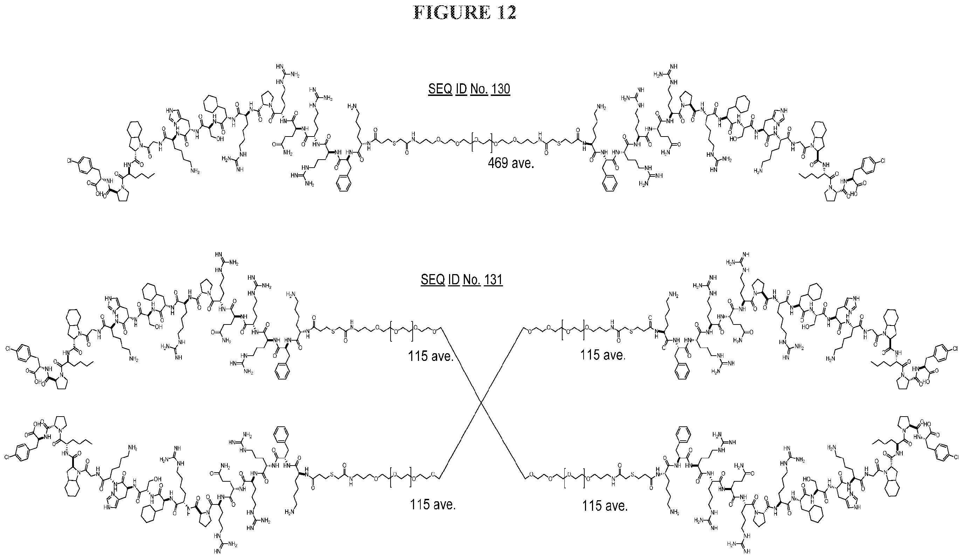

Additionally mono- and multivalent bis, and tetrakis-peptide conjugated PEGS were prepared to explore peptide valency. Figure 11 shows the effects of peptide valency on *in vitro* activity using 20 kDa PEGylated peptides (structures shown in Figure 12) activity in a biological assay. Increased peptide valency had no significant effect on *in vitro* potency when the peptides were conjugated to 20 kDa PEG, although the effects on *in vivo* pharmacodynamic response have not been evaluated. The figure shows mono- bis (SEQ ID NO: 130) and tetrakis- peptides. (SEQ ID NO: 131) 20kDa PEG-mono (SEQ ID NO: 129), bis, and tetrakis (Atz-KFRRQRP [hArg][Cha]SHKG[Oic][Nle]P[4-Cl-F]) (SEQ ID NO: 1562) molecules are approximately equipotent in the *in vitro* rat GTPγS assay, albeit significantly more potent than endogenous pyr apelin 13 (SEQ ID NO: 128).

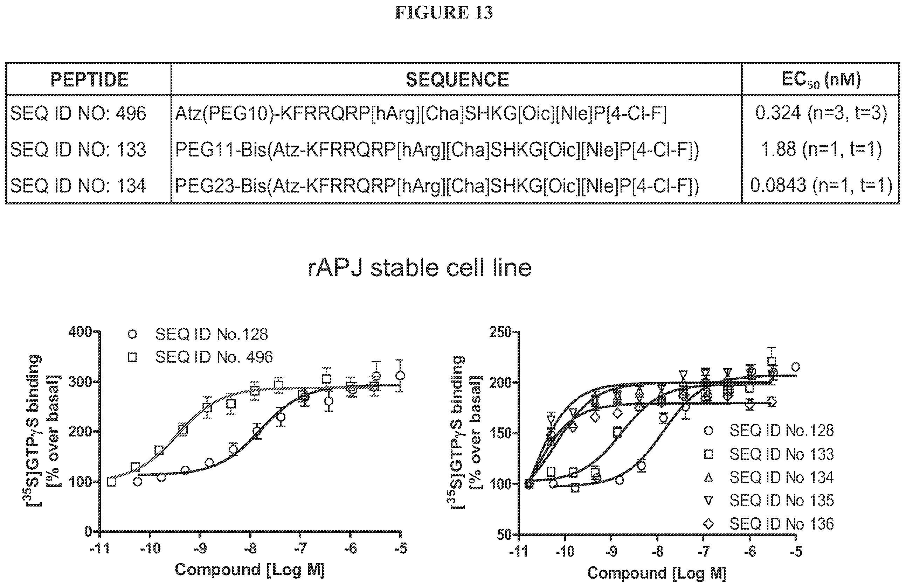

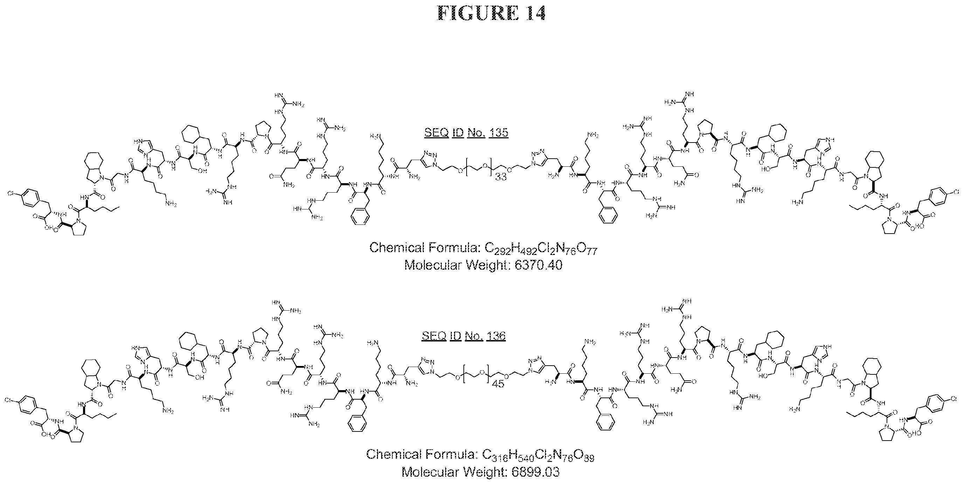

Several additional PEGylated bis- peptides were investigated as shown in Figure 13. Three of the 4 bis-peptides had increased potency relative to the mono-peptide when conjugated to low molecular weight monodispersed PEG derivatives. The shortest PEG conjugate (∼0.6 kDa PEG (SEQ ID NO: 133) had reduced potency, whereas the 1 (SEQ ID NO: 134), 1.7 (SEQ ID NO: 135) and 2.2 kDa PEG (SEQ ID NO: 136) conjugates (Figure 14) had increased potency relative to monopeptide (SEQ ID NO: 494). The most bis-peptide conjugate is 16 pM.

The following conclusions can be reached from this example. 1) Increased PEG MW from 20 kDa to 40 kDa resulted in a conjugate with reduced potency and efficacy in the rat GTPγS assay. 2) Increased multivalency of 20 kDa PEG did not result in increased potency, suggesting that the distance between peptides when conjugated to 20 kDa PEG is not optimal. 3) The majority of bis-peptides conjugated to low MW monodispersed PEG had increased potency relative to mono-conjugated PEG.

Furthermore, it suggested that a) the distance between peptides when conjugated to either 1.7 or 2.2 kDa PEG appears optimal, b) bis-peptide PEG conjugates can offer > 10-fold increased potency relative to mono-peptide conjugates and c) bis-peptide PEG conjugates may have beneficial PK properties.

### EXAMPLE 8

Two discrete APJ agonists (SEQ ID NOs: 1413 and 1414) were used for site selective conjugation to an engineered Fc region of human IgG by reacting the bromoacetamide portion of the peptides with the free cysteine residue to achieve a coavalent thioether linkage. The sequences of the two peptides are shown in Table 16. The Fc protein sequence is shown below.
E53C (E384C) Sequence (SEQ ID NO: 1415) 
1   MDKTHTC1PPC2PAPELLGGPSVFLFPPKPKD   30
31  TLMISRTPEVTC3VVVDVSHEDPCVKFNWYV   60
61  DGVEVHNAKTKPREEQYNSTYRVVSVLTVL   90
91  HQDWLNGKEYKC3KVSNKALPAPIEKTISKA   120
121 KGQPREPQVYTLPPSRDELTKNQVSLTC4LV   150
151 KGFYPSDIAVEWESNGQPENNYKTTPPVLD   180
181 SDGSFFLYSKLTVDKSRWQQGNVFSC4SVMH   210
211 EALHNHYTQKSLSLSPGK
 The underlined C indicates site of engineered cysteine and the point of covalent attachment of the APJ agonist. In the homodimer C7-C7 and C10-C10 are intermolecular disulphides, whereas C42-C102 and C148-C206 are intramolecular disulphides. Additional information related to this may be found in US Pub. No. 2012/0009205

**TABLE 16. Amino acid sequences of APJ agonists for site-selective protein conjugation.**

| AMINO ACID SEQUENCE | DESIGNATION | SEQ ID NO. |
|---|---|---|
| {Bromoacetyl}[NPeg11]LRP[hArg][Cha]S HKG[Oic][Nle]P[4-Cl-F]{FreeAcid} | Bromoacetyl-NPegl 1-[Leu65;hArg68;Cha69;Oic74;Nle7 5;4-Cl-F77]Apelin-13(65-77) | 1413 |
| {Bromoacetyl} [NPeg1 1]KFRR QRP[hArg ][Cha]SHKG[Oic][Nle]P[4-Cl-F]{FreeAcid} | Bromoacetyl-NPegl 1-[hArg68;Cha69;Oic74;Nle75;4-Cl-F77]Apelin-17(61-77) | 1414 |
| {Bromoacetyl}[NPeg11]F[hArg][hArg]Q[ hArg]P[hArg] [Cha]SHKG[Oic][Nle]P[4-Cl-F] {FreeAcid} | Bromoacetyl-NPegl 1-[hArg63,64,66,68;Cha69;Oic74;Nl e75;4-Cl-F77]Apelin-17(61-77) | 1559 |
| {Bromoacetyl}[NPeg11]OF[hArg][hArg]Q [hArg]P[hArg][Cha]SHKG[Oic][Nle]P[4-Cl-F]{FreeAcid} | Bromoacetyl-NPeg11-[Orn61;hArg63,64,66,68;Cha69;O ic74,Nle75;4-ci-F77]Apelin-17(61-77) | 1426 |
| {Bromoacetyl}[NPeg11]QRP[hArg)[Cha] SHKG[Oic][Nle]P[4-Cl-F]{FreeAcid} | Bromoacetyl-NPegl 1-[hArg68;Cha69;Oic74;Nle75;4-Cl-F77]Apelin-13(65-77) | 1427 |
| {Bromoacetyl }GGLRP[hArg][Cha]SHKG[ Oic][Nle]P[4-Cl-F]{FreeAcid} | Bromoacetyl-GG-[Leu65;hArg68;Cha69;Oic74;Nle7 5;4-Cl-F77]Apelin-13(65-77) | 1428 |
| {Bromoacetyl }GGF[hArg][hArg]Q[hArg] P[hArg] [Cha]SHKG[Oic][Nle]P[4-Cl-F]{FreeAcid} | Bromoacetyl-GG-[hArg63,64,66,68;Cha69;Oic74;Nl e75;4-Cl-F77]Apelin-17(61-77) | 1429 |
| {Bromoacetyl}GGOF[hArg][hArg]Q[hArg ]P[hArg][Cha]SHKG[Oic][Nle]P[4-Cl-F]{FreeAcid} | Bromoacetyl-GG-[Orn61;hArg63,64,66,68;Cha69;O ic74;Nle75;4-Cl-F77]Apelin-17(61-77) | 1430 |
| {Bromoacetyl}GGQRP[hArg][Cha]SHKG [Oic][Nle]P[4-Cl-F]{FreeAcid} | Bromoacetyl-GG-[hArg68;Cha69;Oic74;Nle75;4-Cl-F77]Apelin-13(65-77) | 1431 |
| {Bromoacetyl}[Aeea]LRP[hArg][Cha]SH KG[Oic][Nle]P[4-Cl-F]{FreeAcid} | Bromoacetyl-Aeea-[Leu65;hArg68;Cha69;Oic74;Nle7 5;4-Cl-F77]Apelin-13(65-77) | 1432 |
| {Bromoacetyl}[Aeea]F[hArg][hArg]Q[hAr g]P[hArg][Cha]SHKG[Oic][Nle]P[4-Cl-F]{FreeAcid} | Bromoacetyl-Aeea-[hArg63,64,66,68;Cha69;Oic74;Nl e75;4-Cl-F77]Apelin-17(61-77) | 1433 |
| {Bromoacetyl}[Aeea]OF[hArg][hArg]Q[h Arg]P[hArg][Cha]SHKG[Oic][Nle]P[4-Cl-F]{FreeAcid} | Bromoacetyl-Aeea-[Orn61;hArg63,64,66,68;Cha69-0 ic74;Nle75;4-Cl-F77]Apelin-17(61-77) | 1434 |
| {Bromoacetyl}[Aeea]QRP[hArg][Cha]SH KG[Oic][Nle]P[4-Cl-F]{FreeAcid} | Bromoacetyl-Aeea-[hArg68;Cha69;Oic74;Nle75;4-Cl-F77]Apelin-13(65-77) | 1435 |
| {Bromoacetyl }GSGSLRP[hArg][Cha]SH KG[Oic][Nle]P[4-Cl-F]{FreeAcid} | Bromoacetyl-GSGS-[Leu65;hArg68;Cha69;Oic74;Nle7 5;4-Cl-F77]Apelin-13(65-77) | 1436 |
| {Bromoacetyl }GSGSF[hArg][hArg]Q[hAr g]P[hArg][Cha]SHKG[Oic][Nle]P[4-Cl-F]{FreeAcid} | Bromoacetyl-GSGS-[hArg63,64,66,68;Cha69;Oic74;Nl e75;4-Cl-F77]Apelin-17(61-77) | 1437 |
| {Bromoacetyl}GSGSOF[hArg][hArg]Q[h Arg]P[hArg][Cha]SHKG[Oic][Nle]P[4-Cl-F]{FreeAcid} | Bromoacetyl-GSGS-[Orn61;hArg63,64,66,68;Cha69;O ic74;Nle75;4-Cl-F77]Apelin-17(61-77) | 1438 |
| {Bromoacetyl}GSGSQRP[hArg][Cha]SH KG[Oic][Nle]P[4-C1-F]{FreeAcid} | Bromoacetyl-GSGS-[hArg68;Cha69;Oic74;Nle75;4-Cl-F77]Apelin-13(65-77) | 1439 |
| {Bromoacetyl}LRP[hArg][Cha]SHKG[Oic ][Nle]P[4-Cl-F] {FreeAcid} | Bromoacetyl-[Leu65;hArg68;Cha69;Oic74;Nle7 5;4-Cl-F77]Apelin-13(65-77) | 1450 |
| {Bromoacetyl}F[hArg][hArg]Q[hArg]P[h Arg] [Cha]SHKG[Oic][Nle]P[4-Cl-F]{FreeAcid} | Bromoacetyl-[hArg63,64,66,68;Cha69;Oic74;Nl e75;4-Cl-F77]Apelin-17(61-77) | 1451 |
| {Bromoacetyl}OF[hArg][hArg]Q[hArg]P[ hArg] [Cha]SHKG[Oic][Nle]P[4-Cl-F]{FreeAcid} | Bromoacetyl-[Orn61;hArg63,64,66,68;Cha69;O ic74;Nle75;4-Cl-F77]Apelin-17(61-77) | 1452 |
| {Bromoacetyl}QRP[hArg][Cha]SHKG[Oi c][Nle]P[4-Cl-F]{FreeAcid} | Bromoacetyl-[hArg68;Cha69;Oic74;Nle75;4-Cl-F77]Apelin-13(65-77) | 1453 |

The Fc protein forms a homodimer upon folding and thus contains two engineered reactive sites per folded protein. Each Fc-homodimer conjugate will ideally contain two copies of APJ agonist. The conjugates were named FcE52C-ap13 and FcE52C-ap17 (13 and 17 are the number of residues in the peptide, 1x for 1 peptide, 2x for two peptides covalently bound to Fc through a thioether linkage).

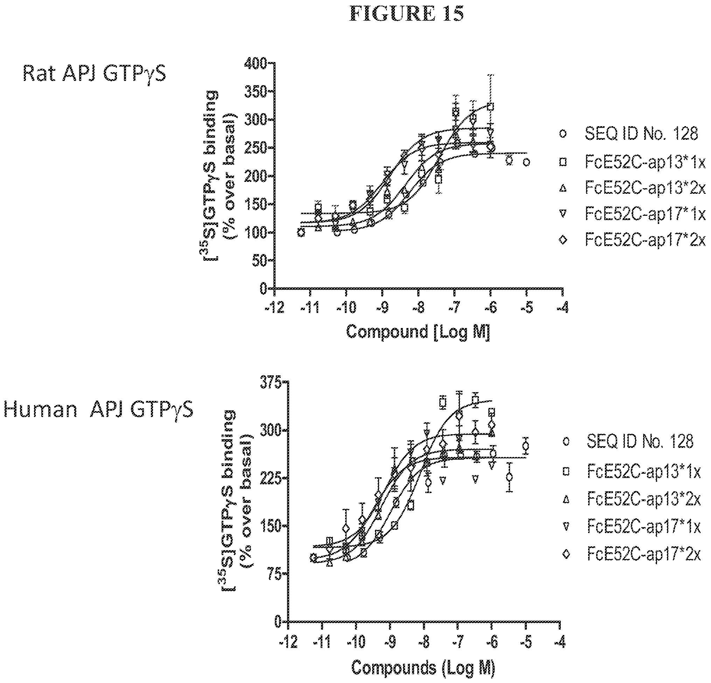

Results of this experiment are shown in Figure 15 with comparison of the activity of the Fc conjugates to SEQ ID NO: 128. Two peptide APJ agonists of different lengths were successfully conjugated to a cysteine engineered Fc protein in a site-selective manner. Fc-conjugates containing one peptide and two peptides per Fc protein were separated by chromatography. Peptide-protein conjugates maintain potency and efficacy at human and rat APJ receptors. The conjugates were compared to wild type pyr-apelin-13 (SEQ ID NO: 128) at both human and rat APJ and were found to have higher or equivalent potency and efficacy.

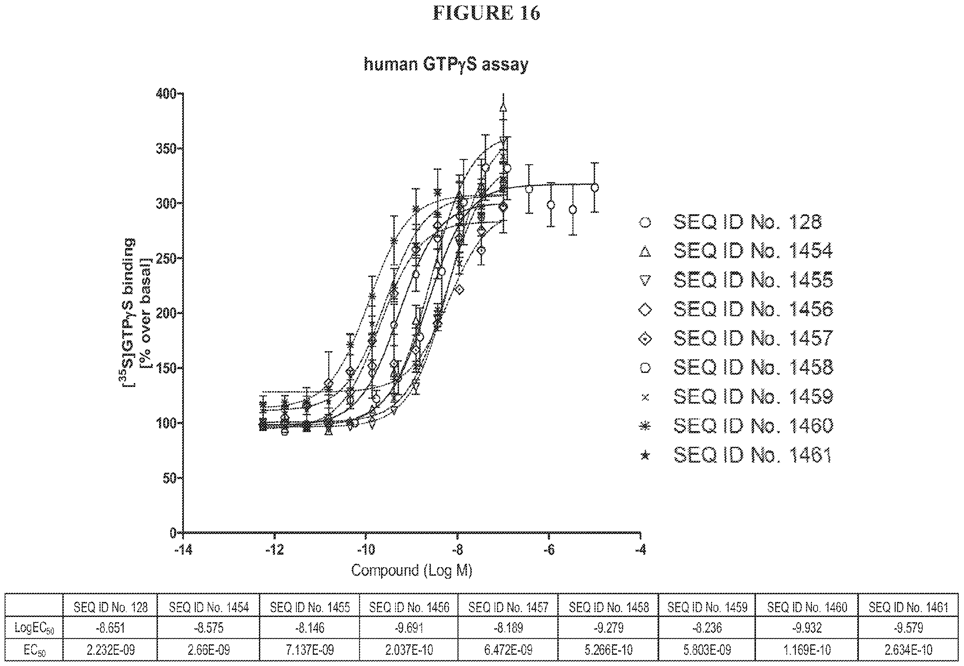

Results of another experiment using several protein conjugates of Table 17 are shown in Figure 16. Results of this experiment are shown in Figure 16 with comparison of the activity of the Fc conjugates to SEQ ID NO: 128. Peptide APJ agonists of varying lengths of spacers between peptide and conjugation linker were successfully conjugated to two different cysteine engineered Fc proteins in a site-selective manner. Fc-conjugates containing one peptide and two peptides per Fc protein were separated by chromatography. Peptide-protein conjugates were tested against human APJ receptor and maintain potency and efficacy. The conjugates were compared to wild type pyr-apelin-13 (SEQ ID NO: 128) and were found to have higher or equivalent potency and efficacy.

**TABLE 17. PROTEIN CONJUGATES**

| DESIGNATION | SEQ ID NO. |
|---|---|
| Fc (E384C){acetamide}-QRP[hArg][Cha]SHKG[Oic][Nle]P[4-Cl-F]) | 1454 |
| Fc (T487C)-{acetamide}-QRP[hArg][Cha]SHKG[Oic][Nle]P[4-Cl-F] | 1455 |
| Fc (E384C)-{acetamide}-GG-QRP[hArg][Cha]SHKG[Oic][Nle]P[4-Cl-F] | 1456 |
| Fc (T487C)-{acetamide}-GG-QRP[hArg][Cha]SHKG[Oic][Nle]P[4-Cl-F] | 1457 |
| Fc (E384C)-{acetamide}-AEEA-QRP[hArg][Cha]SHKG[Oic][Nle]P[4-Cl-F] | 1458 |
| Fc (T487C)-{ oacetamide}-AEEA-QRP[hArg][Cha]SHKG[Oic][Nle]P[4-Cl-F] | 1459 |
| Fc (E384C)-{acetamide}-PEG₁₁-QRP[hArg][Cha]SHKG[Oic][Nle]P[4-Cl-F] | 1460 |
| Fc (T487C)-{acetamide}-PEG₁₁-QRP[hArg][Cha]SHKG[Oic][Nle]P[4-Cl-F] | 1461 |

The following is the sequence for huFc(T487C) (SEQ ID NO: 1462):

DKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAP
IEKTISKAKGQPREPQVYTLPPSREEMCKNQVSLTCLVKGFYPSDIAVEWESNGQPE
NNYKTTPPVLDSDGSFFLYSKLTVDKSRWQQGNVFSCSVMHEALHNHYTQKSLSL
SPGK

### EXAMPLE 9

Additional peptides that can be used as apelin agonists can be found in Table 18.

**TABLE 18.**

| AMINO ACID SEQUENCE | DESIGNATION | SEQ ID NO. |
|---|---|---|
| { Acetyl}[NPeg11]F[hArg][hArg]Q[hArg] P[hArg] [Cha]SHKG[Oic][Nle]P[4-Cl-F]{FreeAcid} | Acetyl-[NPeg1161;hArg63,64,66,68;Cha6 9;Oic74;Nle7 5; 4-Cl-F77]Apelin-17(61-77) | 1463 |
| { Acetyl}[NPeg11]F[hArg][hArg]Q[hArg] P[hArg] [Cha]SHKG[Oic][Nle][1-Nal][4-Cl-F] {FreeAcid} | Acetyl-[NPeg1161;hArg63,64,66,68;Cha6 9;Oic74;Nle75; 1-Nal76;4-Cl-F77]Apelin-17(61-77) | 1464 |
| { Acetyl}[NPeg11]F[hArg][hArg]L[hArg] P[hArg] [Cha]SHKG[Oic][Nle]P[4-Cl-F]{FreeAcid} | Acetyl-[NPeg1161;hArg63,64,66,68;Leu6 5;Cha69;Oic74;Nle75;4-Cl-F77]Apelin-17(61-77) | 1465 |
| { Acetyl}[NPeg11]F[hArg][hArg][Nle][hA rg]P[hArg] [Cha]SHKG[Oic][Nle]P[4-Cl-F]{FreeAcid} | Acetyl-[NPeg1161;hArg63,64,66,68;Nle6 5,75;Cha69;Oic74;4-Cl-F77]Apelin-17(61-77) | 1466 |
| {Acetyl}[NPeg11]F[hArg][hArg]Q[hArg] P[hArg][Cha][NMeSer]HKG[Oic][Nle]P[ 4-Cl-F]{FreeAcid} | Acetyl-[NPeg1161;hArg63,64,66,68;Cha6 9;NMeSer70;Oic74;Nle75;4-Cl-F77]Apelin-17(61-77) | 1467 |
| { Acetyl}[NPeg11]F[hArg][hArg]Q[hArg] P[hArg][Cha]S[2-Nal]KG[Oic][Nle]P[4-Cl-F] {FreeAcid} | Acetyl-[NPeg1161;hArg63,64,66,68;Cha6 9;2-Nal71;Oic74;Nle75;4-Cl-F77]Apelin-17(61-77) | 1468 |
| {Acetyl}[NPeg11]F[hArg][hArg]Q[hArg] P[hArg][Cha]THKG[Oic][Nle]P[4-Cl-F]{FreeAcid} | Acetyl-[NPeg1161;hArg63,64,66,68;Cha6 9;Thr70;Oic74;Nle75;4-Cl-F77]Apelin-17(61-77) | 1469 |
| {Acetyl}[NPeg11]F[hArg][hArg]L[hArg] P[hArg][Cha]S[2-Nal]KG[Oic][Nle][1-Nal][4-Cl-F]{FreeAcid} | Acetyl-[NPeg1161;hArg63,64,66,68;Leu6 5;Cha69;2-Nal71;Oic74;Nle75;1-Nal76;4-Cl-F77]Apelin-17(61-77) | 1470 |
| { Acetyl}[NPeg11]F[hArg][hArg]L[hArg] P[hArg] [Cha]T[2-Nal]KG[Oic][Nle][1-Nal] [4-Cl-F] {FreeAcid} | Acetyl-[NPeg1161;hArg63,64,66,68;Leu6 5;Cha69;Thr70;2-Nal71;Oic74;Nle75;1-Nal76;4-Cl-F77]Apelin-17(61-77) | 1471 |
| { Acetyl}[NPeg11]F[hArg][hArg]L[hArg] P[hArg] [Cha]SAKG[Oic][Nle][1-Nal][4-Cl-F] {FreeAcid} | Acetyl-[NPeg1161;hArg63,64,66,68;Leu6 5;Cha69;Ala71;Oic74;Nle75;1-Nal76;4-Cl-F77]Apelin-17(61-77) | 1472 |
| { Acetyl}[NPeg11]F[hArg][hArg]L[hArg] P[hArg] [Cha]SAKG[Oic][Nle]P[4-Cl-F]{FreeAcid} | Acetyl-[NPeg1161;hArg63,64,66,68;Leu6 5;Cha69;Ala71;Oic74;Nle75;4-Cl-F77]Apelin-17(61-77) | 1473 |
| { Acetyl}[NPeg11]F[hArg][hArg]Q[hArg] P[hArg] [Cha]SHKG[Oic][Nle]P[4-Cl-F]{FreeAcid} | Acetyl-[NPeg1161;hArg63,64,66,68;Cha6 9;Oic74;Nle75;4-Cl-F77]Apelin-17(61-77) | 1474 |
| {Acetyl}[NPeg11]F[hArg][hArg]Q[hArg] P[hArg][Cha][Abu]HKG[Oic][Nle]P[4-Cl-F]{FreeAcid} | Acetyl-[NPeg1161;hArg63,64,66,68;Cha6 9;Abu70;Oic74;Nle75;4-Cl-F77]Apelin-17(61-77) | 1475 |
| { Acetyl}[NPeg11]F[hArg][hArg]Q[hArg] P[hArg] [Cha][Nle]HKG[Oic][Nle]P[4-Cl-F]{FreeAcid} | Acetyl-[NPeg1161;hArg63,64,66,68;Cha6 9;Nle70,75;Oic74;4-Cl-F77]Apelin-17(61-77) | 1476 |
| {Acetyl}[NPeg11]F[hArg][hArg]Q[hArg] P[hArg][Cha]VHKG[Oic][Nle]P[4,-Cl-F]{FreeAcid} | Acetyl-[NPeg1161;hArg63,64,66,68;Cha6 9;Val70;Oic74;Nle75;4-Cl-F77]Apelin-17(61-77) | 1477 |
| {Acetyl}[NPeg11]F[hArg][hArg]Q[hArg] P[hArg][Cha]YHKG[Oic][Nle]P[4-Cl-F]{FreeAcid} | Acetyl-[NPeg1161;hArg63,64,66,68;Cha6 9;Tyr70;Oic74;Nle75;4-Cl-F77]Apelin-17(61-77) | 1478 |
| {Acetyl}[NPeg11]F[hArg][hArg]Q[hArg] P[hArg][Cha]LFKG[Oic][Nle]P[4-Cl-F]{FreeAcid} | Acetyl-[NPeg1161;hArg63,64,66,68;Cha6 9;Leu70;Oic74;Nle75;4-Cl-F77]Apelin-17(61-77) | 1479 |
| {Acetyl}[NPeg11]F[hArg][hArg]Q[hArg] P[hArg][Cha]IHKG[Oic][Nle]P[4-Cl-F]{FreeAcid} | Acetyl-[NPeg1161;hArg63,64,66,68;Cha6 9;Ile70;Oic74;Nle75;4-Cl-F77]Apelin-17(61-77) | 1480 |
| { Acetyl}[NPeg11]F[hArg][hArg]Q[hArg] P[hArg] [Cha][1-Nal]HKG[Oic][Nle]P[4-Cl-F] {FreeAcid} | Acetyl-[NPeg1161;hArg63,64,66,68;Cha6 9;1-Nal70;Oic74;Nle75;4-Cl-F77]Apelin-17(61-77) | 1481 |
| { Acetyl}[NPeg11]F[hArg][hArg]Q[hArg] P[hArg] [Cha][2-Nal]HKG[Oic][Nle]P[4-Cl-F] {FreeAcid} | Acetyl-[NPeg1161;hArg63,64,66,68;Cha6 9;2-Nal70;Oic74;Nle75;4-Cl-F77]Apelin-17(61-77) | 1482 |
| { Acetyl}[NPeg11]F[hArg][hArg]Q[hArg] P[hArg] [Cha][Bip]HKG[Oic][Nle]P[4-Cl-F]{FreeAcid} | Acetyl-[NPeg1161;hArg63,64,66,68;Cha6 9;Bip70;Oic74;Nle75;4-Cl-F77]Apelin-17(61-77) | 1483 |
| {Acetyl}[NPeg11]F[hArg][hArg]Q[hArg] P[hArg][Cha]FHKG[Oic][Nle]P[4-Cl-F]{FreeAcid} | Acetyl-[NPeg1161;hArg63,64,66,68;Cha6 9;Phe70;Oic74;Nle75;4-Cl-F77]Apelin-17(61-77) | 1484 |
| { Acetyl}[NPeg11]F[hArg][hArg]Q[hArg] P[hArg] [Cha]SHKG[Oic][Nle]P[4-Cl-F]{FreeAcid} | Acetyl-[NPeg1161;hArg63,64,66,68;Cha6 9;Oic74;Nle7 5; 4-Cl-F77]Apelin-17(61-77) | 1485 |
| { Acetyl}[NPeg11]F[hArg][hArg]Q[hArg] P[hArg] [Cha]SAKG[Oic][Nle]P[4-Cl-F]{FreeAcid} | Acetyl-[NPeg1161;hArg63,64,66,68;Cha6 9;Ala71;Oic74;Nle75;4-Cl-F77]Apelin-17(61-77) | 1486 |
| { Acetyl}[NPeg11]F[hArg][hArg]Q[hArg] P[hArg][Cha]S[Abu]KG[Oic][Nle]P[4-Cl-F]{FreeAcid} | Acetyl-[NPeg1161;hArg63,64,66,68;Cha6 9;Abu71;Oic74;Nle75;4-Cl-F77]Apelin-17(61-77) | 1487 |
| { Acetyl}[NPeg11]F[hArg][hArg]Q[hArg] P[hArg] [Cha]S[Nle]KG[Oic][Nle]P[4-Cl-F]{FreeAcid} | Acetyl-[NPeg1161;hArg63,64,66,68;Cha6 9;Nle71,75;Oic74;4-Cl-F77]Apelin-17(61-77) | 1488 |
| {Acetyl}[NPeg11]F[hArg][hArg]Q[hArg] P[hArg][Cha]SVKG[Oic][Nle]P[4-Cl-F]{FreeAcid} | Acetyl-[NPeg1161;hArg63,64,66,68;Cha6 9;Val71 ;Oic74;Nle75;4-Cl-F77]Apelin-17(61-77) | 1489 |
| { Acetyl}[NPeg11]F[hArg][hArg]Q[hArg] P[hArg] [Cha]SYKG[Oic][Nle]P[4-Cl-F]{FreeAcid} | Acetyl-[NPeg1161;hArg63,64,66,68;Cha6 9;Tyr71;Oic74;Nle75;4-Cl-F77]Apelin-17(61-77) | 1490 |
| { Acetyl}[NPeg11]F[hArg][hArg]Q[hArg] P[hArg] [Cha]S[Cha]KG[Oic][Nle]P[4-Cl-F]{FreeAcid} | Acetyl-[NPeg1161;hArg63,64,66,68;Cha6 9,71;Oic74;Nle75;4-Cl-F77]Apelin-17(61-77) | 1491 |
| { Acetyl}[NPeg11]F[hArg][hArg]Q[hArg] P[hArg] [Cha]SLKG[Oic][Nle]P[4-Cl-F]{FreeAcid} | Acetyl-[NPeg1161;hArg63,64,66,68;Cha6 9;Leu71;Oic74;Nle75 ;4-Cl-F77]Apelin-17(61-77) | 1492 |
| { Acetyl}[NPeg11]F[hArg][hArg]Q[hArg] P[hArg] [Cha]SIKG[Oic][Nle]P[4-Cl-F]{FreeAcid} | Acetyl-[NPeg1161;hArg63,64,66,68;Cha6 9;Ile71;Oic74;Nle75;4-Cl-F77]Apelin-17(61-77) | 1493 |
| {Acetyl}[NPeg11]F[hArg][hArg]Q[hArg] P[hArg][Cha]SFKG[Oic][Nle]P[4-Cl-F]{FreeAcid} | Acetyl-[NPeg1161;hArg63,64,66,68;Cha6 9;Phe71;Oic74;Nle75;4-Cl-F77]Apelin-17(61-77) | 1494 |
| { Acetyl}[NPeg11]F[hArg][hArg]Q[hArg] P[hArg][Cha]S[2Pal]KG[Oic][Nle]P[4-Cl-F]{FreeAcid} | Acetyl-[NPegl161;hArg63,64,66,68;Cha6 9;2Pal7l;Oic74;Nle75;4-Cl-F77]Apelin-17(61-77) | 1495 |
| {Acetyl}[NPeg11]F[hArg][hArg]Q[hArg] P[hArg][Cha]S[1-Nal]KG[Oic][Nle]P[4-Cl-F]{FreeAcid} | Acetyl-[NPeg1161;hArg63,64,66,68;Cha6 9;1-Nal71;Oic74;Nle75;4-Cl-F77]Apelin-17(61-77) | 1496 |
| {Acetyl}[NPeg11]F[hArg][hArg]Q[hArg] P[hArg][Cha]S[3Pal]KG[Oic][Nle]P[4-Cl-F]{FreeAcid} | Acetyl-[NPeg1161;hArg63,64,66,68;Cha6 9;3Pal71;Oic74;Nle75 ;4-Cl-F77]Apelin-17(61-77) | 1497 |
| { Acetyl}[NPeg11]F[hArg][hArg]Q[hArg] P[hArg] [Cha]S[Bip]KG[Oic][Nle]P[4-Cl-F]{FreeAcid} | Acetyl-[NPeg1161;hArg63,64,66,68;Cha6 9;Bip71;Oic74;Nle75;4-Cl-F77]Apelin-17(61-77) | 1498 |
| {Acetyl}[NPeg11][Orn]F[hArg][hArg]Q[ hArg]P[hArg] [Cha]SHKG[Oic][Nle]P[4-Cl-F] {FreeAcid} | Acetyl-NPeg11-[Orn61;hArg63,64,66,68;Cha69;Oi c74;Nle75;4-Cl-F77]Apelin-17(61-77) | 1499 |
| {Acetyl}[NPeg11][Orn]F[hArg][hArg]Q[ hArg]P[hArg][Cha]SHKG[Oic][Nle][1-Nal][4-Cl-F]{FreeAcid} | Acetyl-NPeg11-[Orn61;hArg63,64,66,68;Cha69;Oi c74;Nle75;1-Nal76;4-Cl-F77]Apelin-17(61-77) | 1505 |
| {Acetyl}[NPeg11][Orn]F[hArg][hArg]L[h Arg]P[hArg][Cha]SHKG[Oic][Nle]P[4-Cl-F]{FreeAcid} | Acetyl-NPeg11-[Orn61;hArg63,64,66,68;Leu65; Ch a69; Oic74;Nle7 5; 4-Cl-F77]Apelin-17(61-77) | 1500 |
| {Acetyl}[NPeg11][Orn]F[hArg][hArg][Nl e][hArg]P[hArg][Cha]SHKG[Oic][Nle]P[ 4-C1-F]{FreeAcid} | Acetyl-NPeg11-[Orn61;hArg63,64,66,68;Nle65, 75; Cha69;Oic74;4-Cl-F77]Apelin-17(61-77) | 1501 |
| {Acetyl}[NPeg11][Orn]F[hArg][hArg]Q[ hArg]P[hArg] [Cha][NMeSer]HKG[Oic][ Nle]P[4-Cl-F]{FreeAcid} | Acetyl-NPeg11-[Orn61;hArg63,64,66,68; Cha69;N MeSer70;Oic74;Nle75;4-Cl-F77]Apelin-17(61-77) | 1502 |
| {Acetyl}[NPeg11][Orn]F[hArg][hArg]Q[ hArg]P [hArg][Cha]S[2-Nal]KG[Oic][Nle]P[4-Cl-F]{FreeAcid} | Acetyl-NPeg11-[Orn61;hArg63,64,66,68; Cha69;2-Nal71;Oic74;Nle75;4-Cl-F77]Apelin-17(61-77) | 1503 |
| {Acetyl}[NPeg11][Orn]F[hArg][hArg]Q[ hArg]P[hArg][Cha]THKG[Oic][Nle]P[4-Cl-F] {FreeAcid} | Acetyl-NPeg11-[Orn61;hArg63,64,66,68;Cha69;Th r70;Oic74;Nle75;4-Cl-F77]Apelin-17(61-77) | 1504 |
| {Acetyl}[NPeg11][Orn]F[hArg][hArg]L[h Arg]P[hArg][Cha]S[2-Nal]KG[Oic][Nle][1-Nal][4-Cl-F]{FreeAcid} | Acetyl-NPeg11-[Orn61;hArg63,64,66,68;Leu65; Ch a69;2-Nal71;Oic74;Nle75;1-Nal76;4-Cl-F77]Apelin-17(61-77) | 1506 |
| {Acetyl}[NPeg11][Orn]F[hArg][hArg]L[h Arg]P[hArg][Cha]T[2-Nal]KG[Oic][Nle][1-Nal][4-Cl-F]{FreeAcid} | Acetyl-NPeg11-[Orn61;hArg63,64,66,68;Leu65;Ch a69;Thr70;2-Nal71;Oic74;Nle75;1-Nal76;4-Cl-F77]Apelin-17(61-77) | 1507 |
| {Acetyl}[NPeg11][Orn]F[hArg][hArg]L[h Arg]P[hArg][Cha]SAKG[Oic][Nle][1-Nal][4-Cl-F]{FreeAcid} | Acetyl-NPeg11-[Orn61;hArg63,64,66,68;Leu65;Ch a69;Ala71 ;Oic74;Nle75; 1-NaI76;4-C1-F77]Apelin-17(61-77) | 1508 |
| {Acetyl}[NPeg11][Orn]F[hArg][hArg]L[h Arg]P[hArg][Cha]SAKG[Oic][Nle]P[4-Cl-F]{FreeAcid} | Acetyl-NPeg11-[Orn61;hArg63,64,66,68;Leu65; Ch a69;Ala71;Oic74;Nle75 ;4-Cl-F77]Apelin-17(61-77) | 1509 |
| {Acetyl}[NPeg11][Orn]F[hArg][hArg]Q[ hArg]P[hArg] [Cha]SHKG[Oic][Nle]P[4-Cl-F] {FreeAcid} | Acetyl-NPeg11-[Orn61;hArg63,64,66,68;Cha69;Oi c74;Nle75;4-Cl-F77]Apelin-17(61-77) | 1510 |
| {Acetyl}[NPeg11][Orn]F[hArg][hArg]Q[ hArg]P[hArg] [Cha][Abu]HKG[Oic][Nle] P[4-Cl-F]{FreeAcid} | Acetyl-NPeg11-[Orn61;hArg63,64,66,68; Cha69;A bu70;Oic74;Nle75;4-Cl-F77]Apelin-17(61-77) | 1511 |
| {Acetyl}[NPeg11][Orn]F[hArg][hArg]Q[ hArg]P[hArg] [Cha] [Nle]HKG[Oic] [Nle]P [4-Cl-F] {FreeAcid} | Acetyl-NPeg11-[Orn61;hArg63,64,66,68; Cha69;Nl e70,75; Oic74;4-Cl-F77]Apelin-17(61-77) | 1512 |
| {Acetyl}[NPeg11][Om]F[hArg][hArg]Q[ hArg]P[hArg] [Cha] VHKG[Oic] [Nle]P[4-Cl-F] {FreeAcid} | Acetyl-NPeg11-[Orn61;hArg63,64,66,68;Cha69;Va 170;Oic74;Nle75;4-Cl-F77]Apelin-17(61-77) | 1513 |
| {Acetyl}[NPeg11][Orn]F[hArg][hArg]Q[ hArg]P[hArg] [Cha]YHKG[Oic][Nle]P[4-Cl-F] {FreeAcid} | Acetyl-NPeg11-[Orn61;hArg63,64,66,68;Cha69;Ty r70;Oic74;Nle75;4-Cl-F77]Apelin-17(61-77) | 1514 |
| {Acetyl}[NPeg11][Orn]F[hArg][hArg]Q[ hArg]P[hArg] [Cha]LHKG[Oic][Nle]P[4-Cl-F] {FreeAcid} | Acetyl-NPeg11-[Orn61;hArg63,64,66,68;Cha69;Le u70;Oic74;Nle75;4-Cl-F77]Apelin-17(61-77) | 1515 |
| {Acetyl}[NPeg11][Orn]F[hArg][hArg]Q[ hArg]P[hArg] [Cha]IHKG[Oic][Nle]P[4-Cl-F] {FreeAcid} | Acetyl-NPeg11-[Orn61;hArg63,64,66,68; Cha69;Ile 70;Oic74;Nle75;4-Cl-F77]Apelin-17(61-77) | 1516 |
| {Acetyl}[NPeg11][Orn]F[hArg][hArg]Q[ hArg]P[hArg] [Cha][1-Nal]HKG[Oic][Nle]P[4-Cl-F]{FreeAcid} | Acetyl-NPeg11-[Orn61;hArg63,64,66,68;Cha69;1-Nal70;Oic74;Nle75;4-Cl-F77]Apelin-17(61-77) | 1517 |
| {Acetyl}[NPeg11][Orn]F[hArg][hArg]Q[ hArg]P[hArg] [Cha][2-Nal]HKG[Oic][Nle]P[4-Cl-F]{FreeAcid} | Acetyl-NPeg11-[Orn61;hArg63,64,66,68; Cha69;2-Nal70;Oic74;Nle75;4-Cl-F77]Apelin-17(61-77) | 1518 |
| {Acetyl}[NPeg11][Orn]F[hArg][hArg]Q[ hArg]P[hArg] [Cha][Bip]HKG[Oic][Nle]P [4-Cl-F] {FreeAcid} | Acetyl-NPeg11-[Orn61;hArg63,64,66,68; Cha69;Bi p70;Oic74;Nle75;4-Cl-F77]Apelin-17(61-77) | 1519 |
| {Acetyl}[NPeg11][Orn]F[hArg][hArg]Q[ hArg]P[hArg] [Cha]FHKG[Oic][Nle]P[4-Cl-F] {FreeAcid} | Acetyl-NPeg11-[Orn61;hArg63,64,66,68; Cha69;Ph e70;Oic74;Nle75;4-Cl-F77]Apelin-17(61-77) | 1520 |
| {Acetyl}[NPeg11][Orn]F[hArg][hArg]Q[ hArg]P[hArg] [Cha]SHKG[Oic][Nle]P[4-Cl-F] {FreeAcid} | Acetyl-NPeg11-[Orn61;hArg63,64,66,68;Cha69;Oi c74;Nle75;4-Cl-F77]Apelin-17(61-77) | 1521 |
| {Acetyl}[NPeg11][Orn]F[hArg][hArg]Q[ hArg]P[hArg] [Cha]SAKG[Oic][Nle]P[4-Cl-F] {FreeAcid} | Acetyl-NPeg11-[Orn61;hArg63,64,66,68; Cha69;Al a71;Oic74;Nle75 ;4-Cl-F77]Apelin-17(61-77) | 1522 |
| {Acetyl}[NPeg11][Orn]F[hArg][hArg]Q[ hArg]P[hArg][Cha]S[Abu]KG[Oic][Nle]P [4-Cl-F]{FreeAcid} | Acetyl-NPeg11-[Orn61;hArg63,64,66,68; Cha69;A bu71;Oic74;Nle75;4-Cl-F77]Apelin-17(61-77) | 1523 |
| {Acetyl}[NPeg11][Orn]F[hArg][hArg]Q[ hArg]P[hArg][Cha]S[Nle]KG[Oic][Nle]P[ 4-Cl-F]{FreeAcid} | Acetyl-NPeg11-[Orn61;hArg63,64,66,68; Cha69;Nl e71,75 ;Oic74;4-Cl-F77]Apelin-17(61-77) | 1524 |
| {Acetyl}[NPeg11][Orn]F[hArg][hArg]Q[ hArg]P[hArg] [Cha]SVKG[Oic][Nle]P[4-Cl-F] {FreeAcid} | Acetyl-NPeg11-[Orn61;hArg63,64,66,68;Cha69;Va 171;Oic74;Nle75;4-Cl-F77]Apelin-17(61-77) | 1525 |
| {Acetyl}[NPeg11][Orn]F[hArg][hArg]Q[ hArg]P[hArg] [Cha]SYKG[Oic][Nle]P[4-Cl-F] {FreeAcid} | Acetyl-NPeg11-[Orn61;hArg63,64,66,68;Cha69;Ty r71;Oic74;Nle75;4-Cl-F77]Apelin-17(61-77) | 1526 |
| {Acetyl}[NPeg11][Orn]F[hArg][hArg]Q[ hArg]P[hArg] [Cha]S[Cha]KG[Oic][Nle]P [4-Cl-F] {FreeAcid} | Acetyl-NPeg11-[Orn61;hArg63,64,66,68;Cha69,71 ;Oic74;Nle75;4-Cl-F77]Apelin-17(61-77) | 1527 |
| {Acetyl}[NPeg11][Orn]F[hArg][hArg]Q[ hArg]P[hArg] [Cha]SLKG[Oic][Nle]P[4-Cl-F] {FreeAcid} | Acetyl-NPeg11-[Orn61;hArg63,64,66,68;Cha69;Le u71;Oic74;Nle75;4-Cl-F77]Apelin-17(61-77) | 1528 |
| {Acetyl}[NPeg11][Orn]F[hArg][hArg]Q[ hArg]P[hArg] [Cha]SIKG[Oic][Nle]P[4-Cl-F] {FreeAcid} | Acetyl-NPeg11-[Orn61;hArg63,64,66,68; Cha69;Ile 71;Oic74;Nle75;4-Cl-F77]Apelin-17(61-77) | 1529 |
| {Acetyl}[NPeg11][Orn]F[hArg][hArg]Q[ hArg]P[hArg] [Cha]SFKG[Oic][Nle]P[4-Cl-F] {FreeAcid} | Acetyl-NPeg11-[Orn61;hArg63,64,66,68; Cha69;Ph e71;Oic74;Nle75 ;4-Cl-F77]Apelin-17(61-77) | 1530 |
| {Acetyl}[NPeg11][Orn]F[hArg][hArg]Q[ hArg]P[hArg][Cha]S[2Pal]KG[Oic][Nle] [4-Cl-F]{FreeAcid} | Acetyl-NPeg11-[Orn61;hArg63,64,66,68; Cha69;2P al71;Oic74;Nle75;4-Cl-F77]Apelin-17(61-77) | 1531 |
| {Acetyl}[NPeg11][Orn]F[hArg][hArg]Q[ hArg]P[hArg][Cha]S[1-Nal]KG[Oic][Nle]P[4-Cl-F]{FreeAcid} | Acetyl-NPeg11-[Orn61;hArg63,64,66,68;Cha69;1-Nal71;Oic74;Nle75;4-Cl-F77]Apelin-17(61-77) | 1532 |
| {Acetyl}[NPeg11][Orn]F[hArg][hArg]Q[ hArg]P[hArg][Cha]S[3Pal]KG[Oic][Nle]P [4-Cl-F]{FreeAcid} | Acetyl-NPeg11-[Orn61;hArg63,64,66,68; Cha69;3P al71;Oic74;Nle75;4-Cl-F77]Apelin-17(61-77) | 1533 |
| {Acetyl}[NPeg11][Orn]F[hArg][hArg]Q[ hArg]P[hArg][Cha]S[Bip]KG[Oic][Nle]P[ 4-Cl-F]{FreeAcid} | Acetyl-NPeg11-[Orn61;hArg63,64,66,68; Cha69;Bi p71;Oic74;Nle75;4-Cl-F77]Apelin-17(61-77) | 1534 |
| { Acetyl}[NPeg11]F[hArg][hArg]L[hArg] P[hArg] [Cha]SAKG[Oic][Nle][1-Nal][4-Cl-F] {FreeAcid} | Acetyl-[NPeg1161;hArg63,64,66,68;Leu6 5;Cha69;Ala71;Oic74;Nle75;1-Nal76;4-Cl-F77]Apelin-17(61-77) | 1535 |
| {Acetyl}[NPeg11]F[hArg][hArg]L[hArg] P[hArg][Cha]S[Cha]KG[Oic][Nle]1-Nal][4-Cl-F]{FreeAcid} | Acetyl-[NPeg1161;hArg63,64,66,68;Leu6 5;Cha69,71;Oic74;Nle75; 1-Nal76;4-Cl-F77]Apelin-17(61-77) | 1536 |
| {Acetyl}[NPeg11]F[hArg][hArg]L[hArg] P[hArg][Cha][NMeSer]AKG[Oic][Nle][1-Nal][4-Cl-F]{FreeAcid} | Acetyl-[NPeg1161;hArg63,64,66,68;Leu6 5;Cha69;NMeSer70;Ala71;Oic74; Nle75; 1-Nal76;4-Cl-F77]Apelin-17(61-77) | 1537 |
| { Acetyl}[NPeg11]F[hArg][hArg]L[hArg] P[hArg][Cha][NMeSer][Cha]KG[Oic][Nle ][1-Nal][4-Cl-F]{FreeAcid} | Acetyl-[NPeg1161;hArg63,64,66,68;Leu6 5;Cha69,71;NMeSer70;Oic74;Nle7 5;1-Nal76;4-Cl-F77]Apelin-17(61-77) | 1538 |
| {Acetyl}[NPeg11][Orn]F[hArg][hArg]L[h Arg]P[hArg][Cha]SAKG[Oic][Nle][1-Nal][4-C1-F]{FreeAcid} | Acetyl-NPeg11-[Orn61;hArg63,64,66,68;Leu65; Ch a69;Ala71;Oic74;Nle75;1-Nal76;4-Cl-F77]Apelin-17(61-77) | 1539 |
| {Acetyl}[NPeg11][Orn]F[hArg][hArg]L[h Arg]P[hArg][Cha]S[Cha]KG[Oic][Nle][1-Nal][4-C1-F]{FreeAcid} | Acetyl-NPeg11-[Orn61;hArg63,64,66,68;Leu65; Ch a69,71;Oic74;Nle75;1-Nal76;4-Cl-F77]Apelin-17(61-77) | 1540 |
| {Acetyl}[NPeg11]F[hArg][hArg]L[hArg] P[hArg][Cha]S[2-Nal]KG[Oic][Nle][1-Nal][4-F-F]{FreeAcid} | Acetyl-[NPeg1161;hArg63,64,66,68;Leu6 5;Cha69;2-Nal71;Oic74;Nle75;1-Nal76;4-F-F77]Apelin-17(61-77) | 1541 |
| {Acetyl}[NPeg11][Orn]F[hArg][hArg]L[h Arg]P[hArg][Cha]S[2-Nal]KG[Oic] [Nle][1-Nal][4-F-F]{FreeAcid} | Acetyl-NPeg11-[Orn61;hArg63,64,66,68;Leu65; Ch a69;2-Nal71;Oic74;Nle75;1-Nal76;4-F-F77]Apelin-17(61-77) | 1542 |
| { Acetyl}[NPeg11]F[hArg][hArg]Q[hArg] P[hArg] [Cha]SHKG[Oic][Nle]P[D-Bip]{FreeAcid} | Acetyl-[NPeg1161;hArg63,64,66,68;Cha6 9;Oic74;Nle75;D-Bip77]Apelin-17(61-77) | 1543 |
| { Acetyl}[NPeg11]F[hArg][hArg]L[hArg] P[hArg][Cha]S[2-Nal]KG[Oic][Nle]P[D-Bip]{FreeAcid} | Acetyl-[NPeg1161;hArg63,64,66,68;Leu6 5;Cha69;2-Nal71;Oic74;Nle75;D-Bip77]Apelin-17(61-77) | 1544 |
| { Acetyl}[NPeg11]F[hArg][hArg]L[hArg] P[hArg] [Cha]S[2-Nal]KG[Oic][Nle][1-Nal][D-Bip]{FreeAcid} | Acetyl-[NPeg1161;hArg63,64,66,68;Leu6 5;Cha69;2-Nal71;Oic74;Nle75;1-Nal76;D-Bip77]Apelin-17(61-77) | 1545 |
| {Acetyl}[NPeg11]F[hArg][hArg]L[hArg] P[hArg][Cha]T[2-Nal]KG[Oic][Nle][1-Nal][D-Bip]{FreeAcid} | Acetyl-[NPeg1161;hArg63,64,66,68;Leu6 5;Cha69;Thr70;2-Nal71;Oic74;Nle75;1-Nal76;D-Bip77]Apelin-17(61-77) | 1546 |
| { Acetyl}[NPeg11]F [hArg] [hArg]L[hArg] P[hArg] [Cha]SAKG[Oic][Nle][1-Nal] [D-Bip]{FreeAcid} | Acetyl-[NPeg1161;hArg63,64,66,68;Leu6 5;Cha69;Ala71;Oic74;Nle75;1-Nal76;D-Bip77]Apelin-17(61-77) | 1547 |
| {Acetyl}[NPeg11][Orn]F[hArg][hArg]Q[ hArg]P[hArg] [Cha] SHKG[Oic] [Nle]P[D-Bip] {FreeAcid} | Acetyl-NPeg11-[Orn61;hArg63,64,66,68;Cha69;Oi c74;Nle75;D-Bip77]Apelin-17(61-77) | 1548 |
| {Acetyl}[NPeg11][Orn]F[hArg][hArg]L[h Arg]P[hArg][Cha]S[2-Nal]KG[Oic] [Nle]P[D-Bip] {FreeAcid} | Acetyl-NPeg11-[Orn61;hArg63,64,66,68;Leu65;Ch a69;2-Nal71;Oic74;Nle75;D-Bip77]Apelin-17(61-77) | 1549 |
| {Acetyl}[NPeg11][Orn]F[hArg][hArg]L[h Arg]P[hArg][Cha]S[2-Nal]KG[Oic] [Nle][1-Nal] [D-Bip] {FreeAcid} | Acetyl-NPeg11-[Orn61;hArg63,64,66,68;Leu65;Ch a69;2-Nal71;Oic74;Nle75;1-Nal76;D-Bip77]Apelin-17(61-77) | 1550 |
| {Acetyl}[NPeg11][Orn]F[hArg][hArg]L[h Arg]P[hArg][Cha]T[2-Nal]KG[Oic] [Nle][1-Nal] [D-Bip] {FreeAcid} | Acetyl-NPeg11-[Orn61;hArg63,64,66,68;Leu65;Ch a69;Thr70;2-Nal71;Oic74;Nle75;1-Nal76;D-Bip77]Apelin-17(61-77) | 1551 |
| {Acetyl}[NPeg11][Orn]F[hArg][hArg]L[h Arg]P[hArg][Cha]SAKG[Oic][Nle][1-Nal][D-Bip]{FreeAcid} | Acetyl-NPeg11-[Orn61;hArg63,64,66,68;Leu65;Ch a69;Ala71;Oic74;Nle75;1-Nal76;D-Bip77]Apelin-17(61-77) | 1552 |
| { Acetyl}[NPeg 11]F [hArg] [hArg]L[hArg] P[hArg] [Cha] SAKG[Oic] [Nle]P[D-Bip] {FreeAcid} | Acetyl-[NPeg1161;hArg63,64,66,68;Leu6 5;Cha69;Ala71;Oic74;Nle75;D-Bip77]Apelin-17(61-77) | 1553 |
| { Acetyl}[NPeg 11]F [hArg] [hArg]L[hArg] P[hArg] [Cha] S [Cha]KG[Oic] [Nle]P[D-Bip] {FreeAcid} | Acetyl-[NPeg1161;hArg63,64,66,68;Leu6 5;Cha69,71;Oic74;Nle75;D-Bip77]Apelin-17(61-77) | 1554 |
| { Acetyl}[NPeg 11]F [hArg] [hArg]L[hArg] P[hArg] [Cha][NMeSer]AKG[Oic][Nle]P[ D-Bip] {FreeAcid} | Acetyl-[NPeg1161;hArg63,64,66,68;Leu6 5;Cha69;NMeSer70;Ala71;Oic74; Nle75;D-Bip77]Apelin-17(61-77) | 1555 |
| {Acetyl}[NPeg11]F[hArg][hArg]L[hArg] P[hArg][Cha][NMeSer][Cha]KG[Oic][Nle ]P[D-Bip]{FreeAcid} | Acetyl-[NPeg1161;hArg63,64,66,68;Leu6 5;Cha69,71;NMeSer70;Oic74;Nle7 5;D-Bip771Apelin-17(61-77) | 1556 |
| {Acetyl}[NPeg11][Orn]F[hArg][hArg]L[h Arg]P[hArg] [Cha] SAKG[Oic] [Nle]P[D-Bip] {FreeAcid} | Acetyl-NPeg11-[Orn61;hArg63,64,66,68;Leu65;Ch a69;Ala71;Oic74;Nle75;D-Bip77]Apelin-17(61-77) | 1557 |
| {Acetyl}[NPeg11][Orn]F[hArg][hArg]L[h Arg]P[hArg][Cha]S[Cha]KG[Oic][Nle]P[ D-Bip] {FreeAcid} | Acetyl-NPeg11-[Orn61;hArg63,64,66,68;Leu65;Ch a69,71;Oic74;Nle75;D-Bip77]Apelin-17(61-77) | 1558 |

Throughout this specification various publications, patents and patent applications have been referenced. The reference to such documents, however, should not be construed as an acknowledgment that such documents are prior art to the application. Further, merely because a document may be referenced, this does not necessarily indicate that the applicants are in complete agreement with the document's contents.

## Claims

1. An APJ peptide agonist having increased *in vitro* stability relative to wild type Apelin-13 (SEQ ID NO: 2), wherein the agonist is modified with a half-life prolonging group at the N-terminus or at a side chain of the peptide agonist, wherein the peptide agonist comprises the amino acid sequence selected from any one of:
SEQ ID NOs 29, 30, 31, 32, 33, 34, 35, 36, 37, 43 and 48,
wherein the peptide agonist binds to and activates the APJ receptor,
wherein the half-life prolonging group is PEG.

2. The APJ peptide agonist of claim 1, wherein the increased stability is an increased *in vitro* half-life.

3. The APJ peptide agonist of claim 1 or claim 2, wherein the PEG group is thiopropionyl(20K-mPEG) or Atz(20K-mPEG).

4. The APJ agonist of any one of claims 1 to 3 for use in a method of improving cardiac contractility in a patient in need thereof.

5. The APJ agonist of any one of claims 1 to 3 for use in a method of improving systolic or diastolic function in a patient in need thereof.

6. The APJ agonist of any one of claims 1 to 3 for use in a method of treating heart failure in a patient in need thereof.

## Patentansprüche

1. APJ-Peptidagonist mit erhöhter In-vitro-Stabilität relativ zu Wildtyp-Apelin-13 (SEQ ID NO: 2), wobei der Agonist mit einer die Halbwertszeit verlängernden Gruppe am N-Terminus oder an einer Seitenkette des Peptidagonisten modifiziert ist, wobei der Peptidagonist die Aminosäuresequenz ausgewählt aus einer der folgenden umfasst:
SEQ ID NOs 29, 30, 31, 32, 33, 34, 35, 36, 37, 43 und 48,
wobei der Peptidagonist an den APJ-Rezeptor bindet und diesen aktiviert,
wobei es sich bei der die Halbwertszeit verlängernden Gruppe um PEG handelt.

2. APJ-Peptidagonist nach Anspruch 1, wobei die erhöhte Stabilität eine erhöhte In-vitro-Halbwertszeit bedeutet.

3. APJ-Peptidagonist nach Anspruch 1 oder Anspruch 2, wobei es sich bei der PEG-Gruppe um Thio-propionyl(20K-mPEG) oder Atz(20K-mPEG) handelt.

4. APJ-Agonist nach einem der Ansprüche 1 bis 3 zur Verwendung bei einem Verfahren zur Verbesserung der kardialen Kontraktilität bei einem diese benötigenden Patienten.

5. APJ-Agonist nach einem der Ansprüche 1 bis 3 zur Verwendung bei einem Verfahren zur Verbesserung der systolischen oder diastolischen Funktion bei einem diese benötigenden Patienten.

6. APJ-Agonist nach einem der Ansprüche 1 bis 3 zur Verwendung bei einem Verfahren zur Behandlung von Herzinsuffizienz bei einem diese benötigenden Patienten.

## Revendications

1. Agoniste peptidique de l'APJ présentant une stabilité *in vitro* accrue par rapport à l'apeline-13 de type sauvage (SEQ ID NO: 2), dans lequel l'agoniste est modifié par un groupe prolongeant la demi-vie au niveau de l'extrémité N-terminale ou d'une chaîne latérale de l'agoniste peptidique, dans lequel l'agoniste peptidique comprend la séquence d'acides aminés choisie parmi l'une quelconque des :
SEQ ID NO: 29, 30, 31, 32, 33, 34, 35, 36, 37, 43 et 48,
dans lequel l'agoniste peptidique se lie au récepteur APJ et l'active,
dans lequel le groupe prolongeant la demi-vie est le PEG.

2. Agoniste peptidique de l'APJ selon la revendication 1, dans lequel la stabilité accrue est une demi-vie *in vitro* accrue.

3. Agoniste peptidique de l'APJ selon la revendication 1 ou la revendication 2, dans lequel le groupe PEG est le thiopropionyl(20K-mPEG) ou l'Atz(20K-mPEG).

4. Agoniste de l'APJ selon l'une quelconque des revendications 1 à 3 pour une utilisation dans un procédé d'amélioration de la contractilité cardiaque chez un patient qui en a besoin.

5. Agoniste de l'APJ selon l'une quelconque des revendications 1 à 3 pour une utilisation dans un procédé d'amélioration de la fonction systolique ou diastolique chez un patient qui en a besoin.

6. Agoniste de l'APJ selon l'une quelconque des revendications 1 à 3 pour une utilisation dans un procédé de traitement d'une insuffisance cardiaque chez un patient qui en a besoin.
